# EUROPEAN PATENT SPECIFICATION

(11) **EP 3 030 658 B1**
(45) Date of publication and mention of the grant of the patent: **28.07.2021**
(21) Application number: 14834532.5
(22) Date of filing: 11.08.2014
(51) Int. Cl.: C12N 15/113, A61K 48/00

(54) **COMPOUNDS AND METHODS FOR MODULATION OF DYSTROPHIA MYOTONICA-PROTEIN KINASE (DMPK) EXPRESSION**
VERBINDUNGEN UND VERFAHREN ZUR MODULATION DER EXPRESSION VON DYSTROPHIA-MYOTONICA-PROTEINKINASE (DMPK)
COMPOSÉS ET PROCÉDÉS POUR LA MODULATION DE L'EXPRESSION DE LA PROTÉINE KINASE DE L'ATROPHIE MYOTONIQUE (DMPK)

(30) Priority: 09.08.2013 US 201361864439 P; 10.10.2013 US 201361889337 P
(43) Date of publication of application: 15.06.2016
(73) Proprietor: Ionis Pharmaceuticals, Inc., Carlsbad, CA 92010 (US)
(72) Inventor: PANDEY, Sanjay, K., Carlsbad, CA 92010 (US); MACLEOD, Robert, A., Carlsbad, CA 92010 (US); SWAYZE, Eric, E., Carlsbad, CA 92010 (US); BENNETT, C., Frank, Carlsbad, CA 92010 (US)
(74) Representative: Carpmaels & Ransford LLP
(86) International application number: PCT/US2014/050481
(87) International publication number: WO 2015/021457

(56) References cited:
- WO-A2-2012/012443
- US-A- 5 552 282
- BALLANTYNE K.N. ET AL.: "Locked nucleic acids in PCR primers increase sensitivity and performance", GENOMICS, ACADEMIC PRESS, SAN DIEGO, US, vol. 91, no. 3, 14 February 2008 (2008-02-14), pages 301-305, XP022482717, ISSN: 0888-7543, DOI: 10.1016/J.YGENO.2007.10.016
- NORONHA DE C.M.C. ET AL.: "Amplimers with 3'-terminal phosphorothioate linkages resist degradation by Vent polymerase and reduce Taq polymerase mispriming", PCR METHODS & APPLICATIONS, COLD SPRING HARBOR LABORATORY PRESS, US, vol. 2, no. 2, 1 November 1992 (1992-11-01), pages 131-136, XP000600686, ISSN: 1054-9803
- LEBEDEV Y. ET AL.: "Oligonucleotides containing 2-aminoadenine and 5-methylcytosine are more effective as primers for PCR amplification than their nonmodified counterparts", GENETIC ANALYSIS: BIOMOLECULAR ENGINEERING, vol. 13, no. 1, May 1996 (1996-05), pages 15-21, XP002766875,
- None

## Description

### Field

Disclosed herein are methods, compounds, and compositions for reducing expression of DMPK mRNA and protein in an animal. Also, disclosed herein are methods, compounds, and compositions comprising a DMPK inhibitor for preferentially reducing CUGexp DMPK RNA, reducing myotonia, or reducing spliceopathy in an animal. Such methods, compounds, and compositions are useful, for example, to treat, prevent, or ameliorate type 1 myotonic dystrophy (DM1) in an animal.

### Background

Myotonic dystrophy type 1 (DM1) is the most common form of muscular dystrophy in adults with an estimated frequency of 1 in 7,500 (Harper PS., Myotonic Dystrophy. London: W.B. Saunders Company; 2001). DM1 is an autosomal dominant disorder caused by expansion of a non-coding CTG repeat in DMPK1. DMPK1 is a gene encoding a cytosolic serine/threonine kinase (Brook JD, et al., Cell., 1992, 68(4):799-808). The physiologic functions and substrates of this kinase have not been fully determined. The expanded CTG repeat is located in the 3' untranslated region (UTR) of DMPK1. This mutation leads to RNA dominance, a process in which expression of RNA containing an expanded CUG repeat (CUGexp) induces cell dysfunction (Osborne RJ and Thornton CA., Human Molecular Genetics., 2006, 15(2): R162-R169).

The DMPK gene normally has 5-37 CTG repeats in the 3' untranslated region. In myotonic dystrophy type I, this number is significantly expanded and is, for example, in the range of 50 to greater than 3,500 (Harper, Myotonic Dystrophy (Saunders, London, ed.3, 2001); Annu. Rev. Neurosci. 29: 259, 2006; EMBO J. 19: 4439, 2000; Curr Opin Neurol. 20: 572, 2007).

The CUGexp tract interacts with RNA binding proteins including muscleblind-like (MBNL) protein, a splicing factor, and causes the mutant transcript to be retained in nuclear foci. The toxicity of this RNA stems from sequestration of RNA binding proteins and activation of signaling pathways. Studies in animal models have shown that phenotypes of DM1 can be reversed if toxicity of CUGexp RNA is reduced (Wheeler TM, et al., Science., 2009, 325(5938):336-339; Mulders SA, et al., Proc Natl Acad Sci USA., 2009, 106(33):13915-13920).

In DM1, skeletal muscle is the most severely affected tissue, but the disease also has important effects on cardiac and smooth muscle, ocular lens, and brain. The cranial, distal limb, and diaphragm muscles are preferentially affected. Manual dexterity is compromised early, which causes several decades of severe disability. The median age at death is 55 years, usually from respiratory failure (de Die-Smulders CE, et al., Brain., 1998, 121(Pt 8):1557-1563).

Antisense technology is emerging as an effective means for modulating expression of certain gene products and may therefore prove to be uniquely useful in a number of therapeutic, diagnostic, and research applications for the modulation of DMPK1. Intramuscular injection of fully modified oligonucleotides targeting with the CAG-repeat were shown in mice to block formation of CUGexp-MBNLl complexes, disperse nuclear foci of CUGexp transcripts, enhance the nucleocytoplasmic transport and translation of CUGexp transcripts, release MBNL proteins to the nucleoplasm, normalize alternative splicing of MBNL-dependent exons, and eliminate myotonia in CUGexp-expressing transgenic mice (Wheeler TM, et al., Science., 2009, 325(5938):336-339; WO2008/036406).

Presently there is no treatment that can modify the course of DM1. The burden of disease, therefore, is significant. It is, therefore, an object herein to provide compounds, compositions, and methods for treating DM1

### Summary

The invention provides a compound comprising a modified oligonucleotide consisting of 10-30 linked nucleosides and having a nucleobase sequence comprising a portion of at least 8 contiguous nucleobases 100% complementary to an equal length portion of nucleobases 1343-1368 of SEQ ID NO: 1 and/or nucleobases 8603-8619 of SEQ ID NO: 2, wherein the nucleobase sequence of the modified oligonucleotide is at least 90% complementary to SEQ ID NO: 1 and/or SEQ ID NO: 2 as measured over the entirety of the modified oligonucleotide and wherein the compound is capable of inhibiting DMPK expression.

The invention also provides a compound comprising a modified oligonucleotide consisting of 10-30 linked nucleosides wherein the modified oligonucleotide has a nucleobase sequence comprising a portion of at least 8 contiguous nucleobases of any of SEQ ID NOs: 30, 25, 26, 123, 124, 125, 126, 127, 128, 378, 379, 380, 381, 382, 383 and 384, wherein the nucleobase sequence of the modified oligonucleotide is at least 90% complementary to SEQ ID NO: 1 and/or SEQ ID NO: 2 as measured over the entirety of the modified oligonucleotide and wherein the compound is capable of inhibiting DMPK expression.

The invention also provides a modified oligonucleotide having a nucleobase sequence as set forth in SEQ ID NO: 30, wherein the modified oligonucleotide consists of 16 linked nucleosides and comprises:
a. a gap segment consisting of eight linked deoxynucleosides;
b. a 5' wing segment consisting of four linked nucleosides and having a E-E-K-K 5'-wing motif;
c. a 3' wing segment consisting of four linked nucleosides and having a K-K-E-E 3'-wing motif;
d. wherein the gap segment is positioned between the 5' wing segment and the 3' wing segment;
e. wherein each E represents 2'-O-methoxyethyl sugar and each K represents a cEt sugar;
f. wherein each internucleoside linkage is a phosphorothioate internucleoside linkage; and
g. wherein each cytosine residue is a 5-methyl cytosine.

The invention also provides a composition comprising the compound of the invention or a salt thereof and a pharmaceutically acceptable carrier or diluent.

The invention also provides a compound of the invention or a composition of the invention for use in a method of treating type 1 myotonic dystrophy in an animal.

Disclosed herein are methods, compounds, and compositions for inhibiting expression of DMPK and treating, preventing, delaying or ameliorating a DMPK related disease and or a symptom thereof. In certain instances, the compounds and compositions disclosed herein inhibit mutant DMPK or CUGexp DMPK.

Also disclosed herein isa method of reducing DMPK expression in an animal comprising administering to the animal a compound comprising a modified oligonucleotide as further described herein targeted to DMPK.

Also disclosed herein is a method of preferentially reducing CUGexp DMPK relative to wild-type DMPK, reducing myotonia, or reducing spliceopathy in an animal comprising administering to the animal a compound comprising a modified oligonucleotide, as further described herein, targeted to CUGexp DMPK. In certain instances, CUGexp DMPK transcripts are believed to be particularly sensitive to antisense knockdown via nuclear ribonucleases (such as RNase H), because of their longer residence time in the nucleus, and this sensitivity is thought to permit effective antisense inhibition of CUGexp DMPK transcripts in relevant tissues such as muscle despite the biodistribution barriers to tissue uptake of antisense oligonucleotides. Antisense mechanisms that do not elicit cleavage via nuclear ribonucleases, such as the CAG-repeat ASOs described in, for example, Wheeler TM, et al., Science., 2009, 325(5938):336-339 and WO2008/036406, do not provide the same therapeutic advantage.

Also disclosed herein is a method of treating an animal having type 1 myotonic dystrophy. In certain instances, the method includes administering to the animal a therapeutically effective amount of a compound comprising a modified oligonucleotide as further described herein targeted to DMPK. In certain instances, the method includes identifying an animal with type 1 myotonic dystrophy.

Also disclosed herein is a method of treating, preventing, delaying, or ameliorating symptoms and outcomes associated with development of DM1 including muscle stiffness, myotonia, disabling distal weakness, weakness in face and jaw muscles, difficulty in swallowing, drooping of the eyelids (ptosis), weakness of neck muscles, weakness in arm and leg muscles, persistent muscle pain, hypersomnia, muscle wasting, dysphagia, respiratory insufficiency, irregular heartbeat, heart muscle damage, apathy, insulin resistance, and cataracts. Also disclosed herein is a method of treating, preventing, delaying, or ameliorating symptoms and outcomes associated with development of DM1 in children, including, developmental delays, learning problems, language and speech issues, and personality development issues.

Also disclosed herein is a method of administering an antisense oligonucleotide to counteract RNA dominance by directing the cleavage of pathogenic transcripts.

In certain embodiments, the DMPK has a sequence as set forth in GenBank Accession No. NM_001081560.1 (incorporated herein as SEQ ID NO: 1). In certain embodiments, the DMPK has the sequence as set forth in GenBank Accession No. NT_011109.15 truncated from nucleotides 18540696 to 18555106 (incorporated herein as SEQ ID NO: 2). In certain embodiments, the DMPK has the sequence as set forth in GenBank Accession No. NT_039413.7 truncated from nucleotides 16666001 to 16681000 (incorporated herein as SEQ ID NO: 3). In certain embodiments, the DMPK has the sequence as set forth in GenBank Accession No. NM_032418.1 (incorporated herein as SEQ ID NO: 4). In certain embodiments, the DMPK has the sequence as set forth in GenBank Accession No. AI007148.1 (incorporated herein as SEQ ID NO: 5). In certain embodiments, the DMPK has the sequence as set forth in GenBank Accession No. AI304033.1 (incorporated herein as SEQ ID NO: 6). In certain embodiments, the DMPK has the sequence as set forth in GenBank Accession No. BC024150.1 (incorporated herein as SEQ ID NO: 7). In certain embodiments, the DMPK has the sequence as set forth in GenBank Accession No. BC056615.1 (incorporated herein as SEQ ID NO: 8). In certain embodiments, the DMPK has the sequence as set forth in GenBank Accession No. BC075715.1 (incorporated herein as SEQ ID NO: 9). In certain embodiments, the DMPK has the sequence as set forth in GenBank Accession No. BU519245.1 (incorporated herein as SEQ ID NO: 10). In certain embodiments, the DMPK has the sequence as set forth in GenBank Accession No. CB247909.1 (incorporated herein as SEQ ID NO: 11). In certain embodiments, the DMPK has the sequence as set forth in GenBank Accession No. CX208906.1 (incorporated herein as SEQ ID NO: 12). In certain embodiments, the DMPK has the sequence as set forth in GenBank Accession No. CX732022.1 (incorporated herein as SEQ ID NO: 13). In certain embodiments, the DMPK has the sequence as set forth in GenBank Accession No. S60315.1 (incorporated herein as SEQ ID NO: 14). In certain embodiments, the DMPK has the sequence as set forth in GenBank Accession No. S60316.1 (incorporated herein as SEQ ID NO: 15). In certain embodiments, the DMPK has the sequence as set forth in GenBank Accession No. NM_001081562.1 (incorporated herein as SEQ ID NO: 16). In certain embodiments, the DMPK has the sequence as set forth in GenBank Accession No. NM_001100.3 (incorporated herein as SEQ ID NO: 17).

### Detailed Description

It is to be understood that both the foregoing general description and the following detailed description are exemplary and explanatory only and are not restrictive of the invention, as claimed. Herein, the use of the singular includes the plural unless specifically stated otherwise. Herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques can be used for chemical synthesis, and chemical analysis.

Unless otherwise indicated, the following terms have the following meanings:
"2'-O-methoxyethyl" (also 2'-MOE and 2'-O(CH₂)₂-OCH₃) refers to an O-methoxy-ethyl modification of the 2' position of a furanosyl ring. A 2'-O-methoxyethyl modified sugar is a modified sugar.
"2'-O-methoxyethyl nucleotide" means a nucleotide comprising a 2'-O-methoxyethyl modified sugar moiety.
"5-methylcytosine" means a cytosine modified with a methyl group attached to position 5. A 5-methylcytosine is a modified nucleobase.
"About" means within ± 7% of a value. For example, if it is stated, "the compound affected at least about 70% inhibition of DMPK", it is implied that the DMPK levels are inhibited within a range of 63% and 77%.
"Active pharmaceutical agent" means the substance or substances in a pharmaceutical composition that provide a therapeutic benefit when administered to an animal. For example, in certain embodiments an antisense oligonucleotide targeted to DMPK is an active pharmaceutical agent.
"Active target region" or "target region" means a region to which one or more active antisense compounds is targeted. "Active antisense compounds" means antisense compounds that reduce target nucleic acid levels or protein levels.
"Administered concomitantly" refers to the co-administration of two agents in any manner in which the pharmacological effects of both are manifest in the patient at the same time. Concomitant administration does not require that both agents be administered in a single pharmaceutical composition, in the same dosage form, or by the same route of administration. The effects of both agents need not manifest themselves at the same time. The effects need only be overlapping for a period of time and need not be coextensive.
"Administering" means providing an agent to an animal, and includes, but is not limited to, administering by a medical professional and self-administering.
"Agent" means an active substance that can provide a therapeutic benefit when administered to an animal. "First Agent" means a therapeutic compound of the invention. For example, a first agent can be an antisense oligonucleotide targeting DMPK. "Second agent" means a second therapeutic compound of the invention (e.g. a second antisense oligonucleotide targeting DMPK) and/or a non-DMPK therapeutic compound.
"Amelioration" refers to a lessening of at least one indicator, sign, or symptom of an associated disease, disorder, or condition. The severity of indicators can be determined by subjective or objective measures, which are known to those skilled in the art.
"Animal" refers to a human or non-human animal, including, but not limited to, mice, rats, rabbits, dogs, cats, pigs, and non-human primates, including, but not limited to, monkeys and chimpanzees.
"Antisense activity" means any detectable or measurable activity attributable to the hybridization of an antisense compound to its target nucleic acid. In certain embodiments, antisense activity is a decrease in the amount or expression of a target nucleic acid or protein encoded by such target nucleic acid.
"Antisense compound" means an oligomeric compound that is capable of undergoing hybridization to a target nucleic acid through hydrogen bonding. Examples of antisense compounds include single-stranded and double-stranded compounds, such as, antisense oligonucleotides, siRNAs, shRNAs, snoRNAs, miRNAs, and satellite repeats.
"Antisense inhibition" means reduction of target nucleic acid levels or target protein levels in the presence of an antisense compound complementary to a target nucleic acid compared to target nucleic acid levels or target protein levels in the absence of the antisense compound.
"Antisense oligonucleotide" means a single-stranded oligonucleotide having a nucleobase sequence that permits hybridization to a corresponding region or segment of a target nucleic acid.
"Bicyclic sugar" means a furanosyl ring modified by the bridging of two non-geminal carbon ring atoms. A bicyclic sugar is a modified sugar.
"Bicyclic nucleic acid" or "BNA" refers to a nucleoside or nucleotide wherein the furanose portion of the nucleoside or nucleotide includes a bridge connecting two carbon atoms on the furanose ring, thereby forming a bicyclic ring system.
"Cap structure" or "terminal cap moiety" means chemical modifications, which have been incorporated at either terminus of an antisense compound.
"Chemically distinct region" refers to a region of an antisense compound that is in some way chemically different than another region of the same antisense compound. For example, a region having 2'-O-methoxyethyl nucleotides is chemically distinct from a region having nucleotides without 2'-O-methoxyethyl modifications.
"Chimeric antisense compound" means an antisense compound that has at least two chemically distinct regions.
"Co-administration" means administration of two or more agents to an individual. The two or more agents can be in a single pharmaceutical composition, or can be in separate pharmaceutical compositions. Each of the two or more agents can be administered through the same or different routes of administration. Co-administration encompasses parallel or sequential administration.
"Complementarity" means the capacity for pairing between nucleobases of a first nucleic acid and a second nucleic acid.
"Contiguous nucleobases" means nucleobases immediately adjacent to each other.
"CUGexp DMPK" means mutant DMPK RNA containing an expanded CUG repeat (CUGexp). The wild-type DMPK gene has 5-37 CTG repeats in the 3' untranslated region. In a "CUGexp DMPK" (such as in a myotonic dystrophy type I patient) this number is significantly expanded and is, for example, in the range of 50 to greater than 3,500 (Harper, Myotonic Dystrophy (Saunders, London, ed.3, 2001); Annu. Rev. Neurosci. 29: 259, 2006; EMBO J. 19: 4439, 2000; Curr Opin Neurol. 20: 572, 2007).
"Diluent" means an ingredient in a composition that lacks pharmacological activity, but is pharmaceutically necessary or desirable. For example, the diluent in an injected composition can be a liquid, e.g. saline solution.
"DMPK" means any nucleic acid or protein of distrophia myotonica protein kinase. DMPK can be a mutant DMPK including CUGexp DMPK nucleic acid.
"DMPK expression" means the level of mRNA transcribed from the gene encoding DMPK or the level of protein translated from the mRNA. DMPK expression can be determined by art known methods such as a Northern or Western blot.
"DMPK nucleic acid" means any nucleic acid encoding DMPK. For example, in certain embodiments, a DMPK nucleic acid includes a DNA sequence encoding DMPK, an RNA sequence transcribed from DNA encoding DMPK (including genomic DNA comprising introns and exons), and an mRNA or pre-mRNA sequence encoding DMPK. "DMPK mRNA" means an mRNA encoding a DMPK protein.
"Dose" means a specified quantity of a pharmaceutical agent provided in a single administration, or in a specified time period. In certain instances, a dose can be administered in one, two, or more boluses, tablets, or injections. For example, in certain instances where subcutaneous administration is desired, the desired dose requires a volume not easily accommodated by a single injection, therefore, two or more injections can be used to achieve the desired dose. In certain instances, the pharmaceutical agent is administered by infusion over an extended period of time or continuously. Doses can be stated as the amount of pharmaceutical agent per hour, day, week, or month.
"Effective amount" or "therapeutically effective amount" means the amount of active pharmaceutical agent sufficient to effectuate a desired physiological outcome in an individual in need of the agent. The effective amount can vary among individuals depending on the health and physical condition of the individual to be treated, the taxonomic group of the individuals to be treated, the formulation of the composition, assessment of the individual's medical condition, and other relevant factors.
"Fully complementary" or "100% complementary" means each nucleobase of a nucleobase sequence of a first nucleic acid has a complementary nucleobase in a second nucleobase sequence of a second nucleic acid. In certain embodiments, a first nucleic acid is an antisense compound and a target nucleic acid is a second nucleic acid.
"Gapmer" means a chimeric antisense compound in which an internal region having a plurality of nucleosides that support RNase H cleavage is positioned between external regions having one or more nucleosides, wherein the nucleosides comprising the internal region are chemically distinct from the nucleoside or nucleosides comprising the external regions. The internal region can be referred to as a "gap segment" and the external regions can be referred to as "wing segments."
"Gap-widened" means a chimeric antisense compound having a gap segment of 12 or more contiguous 2'-deoxyribonucleosides positioned between and immediately adjacent to 5' and 3' wing segments having from one to six nucleosides.
"Hybridization" means the annealing of complementary nucleic acid molecules. In certain embodiments, complementary nucleic acid molecules include an antisense compound and a target nucleic acid.
"Identifying an animal with type 1 myotonic dystrophy" means identifying an animal having been diagnosed with a type 1 myotonic dystrophy, disorder or condition or identifying an animal predisposed to develop a type 1 myotonic dystrophy, disorder or condition. For example, individuals with a familial history can be predisposed to type 1 myotonic dystrophy, disorder or condition. Such identification can be accomplished by any method including evaluating an individual's medical history and standard clinical tests or assessments.
"Immediately adjacent" means there are no intervening elements between the immediately adjacent elements.
"Individual" means a human or non-human animal selected for treatment or therapy.
"Internucleoside linkage" refers to the chemical bond between nucleosides.
"Linked nucleosides" means adjacent nucleosides which are bonded or linked together by an internucleoside linkage.
"Mismatch" or "non-complementary nucleobase" refers to the case when a nucleobase of a first nucleic acid is not capable of pairing with the corresponding nucleobase of a second or target nucleic acid.
"Modified internucleoside linkage" refers to a substitution or any change from a naturally occurring internucleoside bond (i.e. a phosphodiester internucleoside bond).
"Modified nucleobase" refers to any nucleobase other than adenine, cytosine, guanine, thymidine, or uracil. An "unmodified nucleobase" means the purine bases adenine (A) and guanine (G), and the pyrimidine bases thymine (T), cytosine (C), and uracil (U).
"Modified nucleotide" means a nucleotide having, independently, a modified sugar moiety, modified internucleoside linkage, or modified nucleobase. A "modified nucleoside" means a nucleoside having, independently, a modified sugar moiety or modified nucleobase.
"Modified oligonucleotide" means an oligonucleotide comprising at least one modified nucleoside and/or modified internucleoside linkage.
"Modified sugar" refers to a substitution or change from a natural sugar moiety. Modified sugars include substituted sugar moieities and surrogate sugar moieties..
"Motif' means the pattern of chemically distinct regions in an antisense compound.
"Myotonia" means an abnormally slow relaxation of a muscle after voluntary contraction or electrical stimulation.
"Nuclear ribonuclease" means a ribonuclease found in the nucleus. Nuclear ribonucleases include, but are not limited to, RNase H including RNase HI and RNase H2, the double stranded RNase drosha and other double stranded RNases.
"Naturally occurring internucleoside linkage" means a 3' to 5' phosphodiester linkage.
"Natural sugar moiety" means a sugar found in DNA (2'-H) or RNA (2'-OH).
"Nucleic acid" refers to molecules composed of monomeric nucleotides. A nucleic acid includes ribonucleic acids (RNA), deoxyribonucleic acids (DNA), single-stranded nucleic acids, double-stranded nucleic acids, small interfering ribonucleic acids (siRNA), and microRNAs (miRNA). A nucleic acid can also comprise a combination of these elements in a single molecule.
"Nucleobase" means a heterocyclic moiety capable of pairing with a base of another nucleic acid.
"Nucleobase sequence" means the order of contiguous nucleobases independent of any sugar, linkage, or nucleobase modification.
"Nucleoside" means a nucleobase linked to a sugar. In certain embodiments, a nucleoside is linked to a phosphate group.
"Nucleoside mimetic" includes those structures used to replace the sugar or the sugar and the base and not necessarily the linkage at one or more positions of an oligomeric compound such as for example nucleoside mimetics having morpholino, cyclohexenyl, cyclohexyl, tetrahydropyranyl, bicyclo or tricyclo sugar mimetics e.g. non furanose sugar units.
"Nucleotide" means a nucleoside having a phosphate group covalently linked to the sugar portion of the nucleoside.
"Nucleotide mimetic" includes those structures used to replace the nucleoside and the linkage at one or more positions of an oligomeric compound such as for example peptide nucleic acids or morpholinos (morpholinos linked by -N(H)-C(=O)-O- or other non-phosphodiester linkage).
"Oligomeric compound" or "oligomer" means a polymer of linked monomeric subunits which is capable of hybridizing to at least a region of a nucleic acid molecule.
"Oligonucleotide" means a polymer of linked nucleosides, wherein each nucleoside and each internucleoside linkage may be modified or unmodified, independent one from another.
"Parenteral administration" means administration through injection or infusion. Parenteral administration includes subcutaneous administration, intravenous administration, intramuscular administration, intraarterial administration, intraperitoneal administration, or intracranial administration, e.g. intrathecal or intracerebroventricular administration. Administration can be continuous, or chronic, or short or intermittent.
"Peptide" means a molecule formed by linking at least two amino acids by amide bonds. Peptide refers to polypeptides and proteins.
"Pharmaceutical composition" means a mixture of substances suitable for administering to an individual. For example, a pharmaceutical composition can comprise one or more active agents and a sterile aqueous solution.
"Pharmaceutically acceptable salts" means physiologically and pharmaceutically acceptable salts of antisense compounds, i.e., salts that retain the desired biological activity of the parent oligonucleotide and do not impart undesired toxicological effects thereto.
"Phosphorothioate linkage" means a linkage between nucleosides where the phosphodiester bond is modified by replacing one of the non-bridging oxygen atoms with a sulfur atom. A phosphorothioate linkage is a modified internucleoside linkage.
"Portion" means a defined number of contiguous (i.e. linked) nucleobases of a nucleic acid. In certain embodiments, a portion is a defined number of contiguous nucleobases of a target nucleic acid. In certain embodiments, a portion is a defined number of contiguous nucleobases of an antisense compound.
"Preferentially reducing CUG exp DMPK RNA" refers to a preferential reduction of RNA transcripts from a CUGexp DMPK allele relative to RNA transcripts from a normal DMPK allele.
"Prevent" refers to delaying or forestalling the onset or development of a disease, disorder, or condition for a period of time from minutes to indefinitely. Prevent also means reducing risk of developing a disease, disorder, or condition.
"Prodrug" means a therapeutic agent that is prepared in an inactive form that is converted to an active form within the body or cells thereof by the action of endogenous enzymes or other chemicals or conditions.
"Side effects" means physiological responses attributable to a treatment other than the desired effects. In certain embodiments, side effects include injection site reactions, liver function test abnormalities, renal function abnormalities, liver toxicity, renal toxicity, central nervous system abnormalities, myopathies, and malaise. For example, increased aminotransferase levels in serum can indicate liver toxicity or liver function abnormality. For example, increased bilirubin can indicate liver toxicity or liver function abnormality.
"Single-stranded oligonucleotide" means an oligonucleotide which is not hybridized to a complementary strand.
"Specifically hybridizable" refers to an antisense compound having a sufficient degree of complementarity between an antisense oligonucleotide and a target nucleic acid to induce a desired effect, while exhibiting minimal or no effects on non-target nucleic acids under conditions in which specific binding is desired, i.e. under physiological conditions in the case *of in vivo* assays and therapeutic treatments.
"Spliceopathy" means a change in the alternative splicing of one or more RNAs that leads to the expression of altered splice products in a particular tissue.
"Subcutaneous administration" means administration just below the skin.
"Substituted sugar moiety" means a furanosyl other than a natural sugar of RNA or DNA.
"Sugar" or "Sugar moiety" means a natural sugar moiety or a modified sugar.
"Sugar surrogate" overlaps with the slightly broader term "nucleoside mimetic" but is intended to indicate replacement of the sugar unit (furanose ring) only A sugar surrogate is capable of replacing the naturally occurring sugar moiety of a nucleoside, such that the resulting nucleoside sub-units are capable of linking together and/or linking to other nucleosides to form an oligomeric compound which is capable of hybridizing to a complementary oligomeric compound. Such structures include rings comprising a different number of atoms than furanosyl (e.g., 4, 6, or 7-membered rings); replacement of the oxygen of a furanosyl with a non-oxygen atom (e.g., carbon, sulfur, or nitrogen); or both a change in the number of atoms and a replacement of the oxygen. Such structures may also comprise substitutions corresponding to those described for substituted sugar moieties (e.g., 6-membered carbocyclic bicyclic sugar surrogates optionally comprising additional substituents). Sugar surrogates also include more complex sugar replacements (e.g., the non-ring systems of peptide nucleic acid). Sugar surrogates include without limitation morpholinos, cyclohexenyls and cyclohexitols.
"Targeting" or "targeted" means the process of design and selection of an antisense compound that will specifically hybridize to a target nucleic acid and induce a desired effect.
"Target nucleic acid," "target RNA," and "target RNA transcript" all refer to a nucleic acid capable of being targeted by antisense compounds. In certain embodiments, a target nucleic acid comprises a region of a DMPK nucleic acid.
"Target segment" means the sequence of nucleotides of a target nucleic acid to which an antisense compound is targeted. "5' target site" refers to the 5'-most nucleotide of a target segment. "3' target site" refers to the 3'-most nucleotide of a target segment.
"Therapeutically effective amount" means an amount of an agent that provides a therapeutic benefit to an individual.
"Treat" refers to administering a pharmaceutical composition to effect an alteration or improvement of a disease, disorder, or condition.
"Type 1 myotonic dystrophy" or "DM1" means an autosomal dominant disorder caused by expansion of a non-coding CTG repeat in DMPK. This mutation leads to RNA dominance, a process in which expression of RNA containing an expanded CUG repeat (CUGexp) induced cell dysfunction. The CUGexp tract interacts with RNA binding proteins and causes the mutant transcript to be retained in nuclear foci. The toxicity of this RNA stems from sequestration of RNA binding proteins and activation of signaling pathways.
"Unmodified nucleotide" means a nucleotide composed of naturally occurring nucleobases, sugar moieties, and internucleoside linkages. In certain embodiments, an unmodified nucleotide is an RNA nucleotide (i.e. β-D-ribonucleosides) or a DNA nucleotide (i.e. β-D-deoxyribonucleoside).

### Certain Embodiments

Disclosed herein are methods, compounds, and compositions for inhibiting DMPK expression.

Also disclosed herein is a method of reducing DMPK expression in an animal comprising administering to the animal a compound comprising a modified oligonucleotide targeting DMPK.

Also disclosed herein is a method of preferentially reducing CUGexp DMPK RNA, reducing myotonia or reducing spliceopathy in an animal comprising administering to the animal a compound comprising a modified oligonucleotide targeted to DMPK, wherein the modified oligonucleotide preferentially reduces CUGexp DMPK RNA, reduces myotonia or reduces spliceopathy in the animal.

Also disclosed herein is a method of administering an antisense oligonucleotide to counteract RNA dominance by directing the cleavage of pathogenic transcripts.

Also disclosed herein is a method of reducing spliceopathy of *Serca1.* In certain embodiments, methods provided herein result in exon 22 inclusion. In certain instances, the corrective splicing occurs in the tibialis anterior, gastrocnemius, and quadriceps muscles.

Also disclosed herein is a method of reducing spliceopathy of *m-Titin.* In certain instances, methods provided herein result in exon 5 inclusion. In certain instances, the corrective splicing occurs in the tibialis anterior, gastrocnemius, and quadriceps muscles.

Also disclosed herein is a method of reducing spliceopathy of *Clcn1.* In certain instances, methods provided herein result in exon 7a inclusion. In certain instances, the corrective splicing occurs in the tibialis anterior, gastrocnemius, and quadriceps muscles.

Also disclosed herein is a method of reducing spliceopathy of *Zasp.* In certain instances, methods provided herein result in exon 11 inclusion. In certain instances, the corrective splicing occurs in the tibialis anterior, gastrocnemius, and quadriceps muscles.

Also disclosed herein is a method for treating an animal with type 1 myotonic dystrophy comprising: a) identifying said animal with type 1 myotonic dystrophy, and b) administering to said animal a therapeutically effective amount of a compound comprising a modified oligonucleotide targeted to DMPK. In certain instances, the therapeutically effective amount of the compound administered to the animal preferentially reduces CUGexp DMPK RNA, reduces myotonia or reduces spliceopathy in the animal.

Also disclosed herein is a method of achieving a preferential reduction of CUGexp DMPK RNA, including administering to the subject suspected of having type 1 myotonic dystrophy or having a CUGexp DMPK RNA a modified antisense oligonucleotide complementary to a non-repeat region of said CUGexp DMPK RNA. The modified antisense oligonucleotide, when bound to said CUGexp DMPK RNA, achieves a preferential reduction of the CUGexp DMPK RNA.

Also disclosed herein is a method of achieving a preferential reduction of CUGexp DMPK RNA, including selecting a subject having type 1 myotonic dystrophy or having a CUGexp DMPK RNA and administering to said subject a modified antisense oligonucleotide complementary to a non-repeat region of said CUGexp DMPK RNA. The modified antisense oligonucleotide, when bound to the CUGexp DMPK RNA, activates a ribonuclease or nuclear ribonuclease, thereby achieving a preferential reduction of the CUGexp DMPK RNA in the nucleus.

Also disclosed herein is a method of achieving a preferential reduction of CUGexp DMPK RNA, including selecting a subject having type 1 myotonic dystrophy or having a mutant or CUGexp DMPK RNA and systemically administering to said subject a modified antisense oligonucleotide complementary to a non-repeat region of said CUGexp DMPK RNA. The modified antisense oligonucleotide, when bound to the mutant or CUGexp DMPK RNA, achieves a preferential reduction of the mutant or CUGexp DMPK RNA.

Also disclosed herein is a method of reducing myotonia in a subject in need thereof. The method includes administering to the subject a modified antisense oligonucleotide complementary to a non-repeat region of a DMPK RNA, wherein the modified antisense oligonucleotide, when bound to the DMPK RNA, activates a ribonuclease or nuclear ribonuclease, thereby reducing myotonia. In certain instances, the subject has or is suspected of having type 1 myotonic dystrophy or having a mutant DMPK RNA or CUGexp DMPK RNA. In certain instances, the DMPK RNA is nuclear retained.

Also disclosed herein is a method of reducing spliceopathy in a subject in need thereof. The method includes administering to the subject a modified antisense oligonucleotide complementary to a non-repeat region of a DMPK RNA, wherein the modified antisense oligonucleotide, when bound to the DMPK RNA, activates a ribonuclease or nuclear ribonuclease, thereby reducing spliceopathy. In certain instances, the subject has or is suspected of having type 1 myotonic dystrophy or having a nuclear retained CUGexp DMPK RNA. In certain embodiments, the DMPK RNA is nuclear retained. In certain instances, the spliceopathy is MBNL dependent spliceopathy.

In certain embodiments, the modified antisense oligonucleotide of the methods is chimeric. In certain embodiments, the modified antisense oligonucleotide of the methods is a gapmer.

In certain instances, the administering is subcutaneous. In certain instances, the administering is intravenous.

In certain embodiments, the modified antisense oligonucleotide of the methods targets a non-coding sequence within the non-repeat region of a DMPK RNA. In certain instances, the oligonucleotide targets a coding region, an intron, a 5'UTR, or a 3'UTR of the mutant DMPK RNA.

In certain embodiments of the methods provided herein, the nuclear ribonuclease is RNase HI.

In certain embodiments of the methods, the DMPK RNA is reduced in muscle tissue. In certain embodiments, the mutant DMPK RNA CUGexp DMPK RNA is preferentially reduced.

In certain embodiments, the DMPK has the sequence as set forth in GenBank Accession No. NM_001081560.1 (incorporated herein as SEQ ID NO: 1). In certain embodiments, the DMPK has the sequence as set forth in GenBank Accession No. NT_011109.15 truncated from nucleotides 18540696 to 18555106 (incorporated herein as SEQ ID NO: 2). In certain embodiments, the DMPK has the sequence as set forth in GenBank Accession No. NT_039413.7 truncated from nucleotides 16666001 to 16681000 (incorporated herein as SEQ ID NO: 3). In certain embodiments, the DMPK has the sequence as set forth in GenBank Accession No. NM_032418.1 (incorporated herein as SEQ ID NO: 4). In certain embodiments, the DMPK has the sequence as set forth in GenBank Accession No. AI007148.1 (incorporated herein as SEQ ID NO: 5). In certain embodiments, the DMPK has the sequence as set forth in GenBank Accession No. AI304033.1 (incorporated herein as SEQ ID NO: 6). In certain embodiments, the DMPK has the sequence as set forth in GenBank Accession No. BC024150.1 (incorporated herein as SEQ ID NO: 7). In certain embodiments, the DMPK has the sequence as set forth in GenBank Accession No. BC056615.1 (incorporated herein as SEQ ID NO: 8). In certain embodiments, the DMPK has the sequence as set forth in GenBank Accession No. BC075715.1 (incorporated herein as SEQ ID NO: 9). In certain embodiments, the DMPK has the sequence as set forth in GenBank Accession No. BU519245.1 (incorporated herein as SEQ ID NO: 10). In certain embodiments, the DMPK has the sequence as set forth in GenBank Accession No. CB247909.1 (incorporated herein as SEQ ID NO: 11). In certain embodiments, the DMPK has the sequence as set forth in GenBank Accession No. CX208906.1 (incorporated herein as SEQ ID NO: 12). In certain embodiments, the DMPK has the sequence as set forth in GenBank Accession No. CX732022.1 (incorporated herein as SEQ ID NO: 13). In certain embodiments, the DMPK has the sequence as set forth in GenBank Accession No. S60315.1 (incorporated herein as SEQ ID NO: 14). In certain embodiments, the DMPK has the sequence as set forth in GenBank Accession No. S60316.1 (incorporated herein as SEQ ID NO: 15). In certain embodiments, the DMPK has the sequence as set forth in GenBank Accession No. NM_001081562.1 (incorporated herein as SEQ ID NO: 16). In certain embodiments, the DMPK has the sequence as set forth in GenBank Accession No. NM_001100.3 (incorporated herein as SEQ ID NO: 17).

In certain instances, the modified oligonucleotide has a nucleobase sequence comprising at least 8 contiguous nucleobases of a nucleobase sequence recited in any one of SEQ ID NOs: 23, 24, 25, 26, 27, 28, 29, 30, 31, 32, or 33- 874. In certain instances, the modified oligonucleotide has a nucleobase sequence comprising at least 9, at least 10, or at least 11, contiguous nucleobases of a nucleobase sequence recited in any one of SEQ ID NOs: 23, 24, 25, 26, 27, 28, 29, 30, 31, 32, or 33-874.

In certain instances, the modified oligonucleotide has a nucleobase sequence comprising at least 12 contiguous nucleobases of a nucleobase sequence recited in any one of SEQ ID NOs: 23, 24, 25, 26, 27, 28, 29, 30, 31, 32, or 33- 874. In certain instances, the modified oligonucleotide has a nucleobase sequence comprising at least 13, or at least 14, contiguous nucleobases of a nucleobase sequence recited in any one of SEQ ID NOs: 23, 24, 25, 26, 27, 28, 29, 30, 31, 32, or 33- 874.

In certain instances, the modified oligonucleotide has a nucleobase sequence comprising at least 15 contiguous nucleobases of a nucleobase sequence recited in any one of SEQ ID NOs: 23, 24, 25, 26, 27, 28, 29, 30, 31, 32, or 33- 874. In certain instances, the modified oligonucleotide has a nucleobase sequence comprising at least 16 contiguous nucleobases of a nucleobase sequence recited in any one of SEQ ID NOs: 23, 24, 25, 26, 27, 28, 29, 30, 31, 32, or 33- 874.

In certain instances, the modified oligonucleotide has a nucleobase sequence comprising at least 17 contiguous nucleobases of a nucleobase sequence recited in any one of SEQ ID NOs: 24, 25, 27, or 28.

In certain instances, the modified oligonucleotide has a nucleobase sequence comprising at least 18 contiguous nucleobases of a nucleobase sequence recited in any one of SEQ ID NOs: 24 or 25. In certain instances, the modified oligonucleotide has a nucleobase sequence comprising at least 19 contiguous nucleobases of a nucleobase sequence recited in any one of SEQ ID NOs: 24 or 25.

In certain instances, the modified oligonucleotides provided herein are targeted to any one of the following regions of SEQ ID NO: 1: 1343-1368, 1346-1363, 1346-1361 and 1347-1363. In certain embodiments, the modified oligonucleotides provided herein are targeted to any one of the following regions of SEQ ID NO: 1: 1343-1358 and 1344-1359.

In certain embodiments, the modified oligonucleotides provided herein have a nucleobase sequence comprising a complementary region comprising at least 8 contiguous nucleobases complementary to a target region. In certain instances, the modified oligonucleotides provided herein have a nucleobase sequence comprising a complementary region comprising at least 8 contiguous nucleobases complementary to a target region, wherein the target region is targeted to nucleobases 1343-1368, 1346-1363, 1346-1361 or 1347-1363 of SEQ ID NO: 1. In certain embodiments, the modified oligonucleotides provided herein have a nucleobase sequence comprising a complementary region comprising at least 8 contiguous nucleobases complementary to a target region, wherein the target region is targeted to nucleobases 1343-1358 or 1344-1359 of SEQ ID NO: 1.

In certain embodiments, the modified oligonucleotides provided herein have a nucleobase sequence comprising a complementary region comprising at least 10 contiguous nucleobases complementary to a target region. In certain embodiments, the modified oligonucleotides provided herein have a nucleobase sequence comprising a complementary region comprising at least 10 contiguous nucleobases complementary to a target region, wherein the target region is targeted to nucleobases 1343-1368, 1346-1363, 1346-1361 or 1347-1363 of SEQ ID NO: 1. In certain embodiments, the modified oligonucleotides provided herein have a nucleobase sequence comprising a complementary region comprising at least 10 contiguous nucleobases complementary to a target region, wherein the target region is targeted to nucleobases 1343-1358 or 1344-1359 of SEQ ID NO: 1.

In certain embodiments, the modified oligonucleotides provided herein have a nucleobase sequence comprising a complementary region comprising at least 12 contiguous nucleobases complementary to a target region. In certain embodiments, the modified oligonucleotides provided herein have a nucleobase sequence comprising a complementary region comprising at least 12 contiguous nucleobases complementary to a target region, wherein the target region is targeted to nucleobases 1343-1368, 1346-1363, 1346-1361 or 1347-1363 of SEQ ID NO: 1. In certain embodiments, the modified oligonucleotides provided herein have a nucleobase sequence comprising a complementary region comprising at least 12 contiguous nucleobases complementary to a target region, wherein the target region is targeted to nucleobases 1343-1358 or 1344-1359 of SEQ ID NO: 1.

In certain embodiments, the modified oligonucleotides provided herein have a nucleobase sequence comprising a complementary region comprising at least 14 contiguous nucleobases complementary to a target region. In certain embodiments, the modified oligonucleotides provided herein have a nucleobase sequence comprising a complementary region comprising at least 14 contiguous nucleobases complementary to a target region, wherein the target region is targeted to nucleobases 1343-1368, 1346-1363, 1346-1361 or 1347-1363 of SEQ ID NO: 1. In certain embodiments, the modified oligonucleotides provided herein have a nucleobase sequence comprising a complementary region comprising at least 14 contiguous nucleobases complementary to a target region, wherein the target region is targeted to nucleobases 1343-1358 or 1344-1359 of SEQ ID NO: 1.

In certain embodiments, the modified oligonucleotides provided herein have a nucleobase sequence comprising a complementary region comprising at least 16 contiguous nucleobases complementary to a target region. In certain embodiments, the modified oligonucleotides provided herein have a nucleobase sequence comprising a complementary region comprising at least 16 contiguous nucleobases complementary to a target region, wherein the target region is targeted to nucleobases 1343-1368, 1346-1363, 1346-1361 or 1347-1363 of SEQ ID NO: 1. In certain instances, the modified oligonucleotides provided herein have a nucleobase sequence comprising a complementary region comprising at least 16 contiguous nucleobases complementary to a target region, wherein the target region is targeted to nucleobases 1343-1358 or 1344-1359 of SEQ ID NO: 1.

In certain embodiments, the modified oligonucleotides provided herein are targeted to any one of the following regions of SEQ ID NO: 2: 8603-8618 and 8604-8619.

In certain embodiments, the modified oligonucleotides provided herein have a nucleobase sequence comprising a complementary region comprising at least 8 contiguous nucleobases complementary to a target region. In certain embodiments, the modified oligonucleotides provided herein have a nucleobase sequence comprising a complementary region comprising at least 8 contiguous nucleobases complementary to a target region, wherein the target region is targeted to nucleobases 8603-8618 or 8604-8619 of SEQ ID NO: 2.

In certain embodiments, the modified oligonucleotides provided herein have a nucleobase sequence comprising a complementary region comprising at least 10 contiguous nucleobases complementary to a target region. In certain embodiments, the modified oligonucleotides provided herein have a nucleobase sequence comprising a complementary region comprising at least 10 contiguous nucleobases complementary to a target region, wherein the target region is targeted to nucleobases 8603-8618 or 8604-8619 of SEQ ID NO: 2.

In certain embodiments, the modified oligonucleotides provided herein have a nucleobase sequence comprising a complementary region comprising at least 12 contiguous nucleobases complementary to a target region. In certain embodiments, the modified oligonucleotides provided herein have a nucleobase sequence comprising a complementary region comprising at least 12 contiguous nucleobases complementary to a target region, wherein the target region is targeted to nucleobases 8603-8618 or 8604-8619 of SEQ ID NO: 2.

In certain embodiments, the modified oligonucleotides provided herein have a nucleobase sequence comprising a complementary region comprising at least 14 contiguous nucleobases complementary to a target region. In certain embodiments, the modified oligonucleotides provided herein have a nucleobase sequence comprising a complementary region comprising at least 14 contiguous nucleobases complementary to a target region, wherein the target region is targeted to nucleobases 8603-8618 or 8604-8619 of SEQ ID NO: 2.

In certain embodiments, the modified oligonucleotides provided herein have a nucleobase sequence comprising a complementary region comprising at least 16 contiguous nucleobases complementary to a target region. In certain embodiments, the modified oligonucleotides provided herein have a nucleobase sequence comprising a complementary region comprising at least 16 contiguous nucleobases complementary to a target region, wherein the target region is targeted to nucleobases 8603-8618 or 8604-8619 of SEQ ID NO: 2.

In certain embodiments, the animal is a human.

In certain instances, the compounds or compositions disclosed herein are designated as a first agent and the methods disclosed herein further comprise administering a second agent. In certain instances, the first agent and the second agent are co-administered. In certain instances the first agent and the second agent are co-administered sequentially or concomitantly.

In certain instances, administration comprises parenteral administration.

In certain embodiments, the compound is a single-stranded modified oligonucleotide. In certain instances, the nucleobase sequence of the modified oligonucleotide is at least 95% complementary to any one of SEQ ID NOs: 1-19 as measured over the entirety of said modified oligonucleotide. In certain instances, the nucleobase sequence of the modified oligonucleotide is 100% complementary to any one of SEQ ID NOs: 1-19 as measured over the entirety of said modified oligonucleotide. In certain embodiments, the compound is a single-stranded modified oligonucleotide. In certain embodiments, the nucleobase sequence of the modified oligonucleotide is at least 95% complementary to any one of SEQ ID NO: 1 as measured over the entirety of said modified oligonucleotide. In certain embodiments, the nucleobase sequence of the modified oligonucleotide is 100% complementary to any one of SEQ ID NO: 1 as measured over the entirety of said modified oligonucleotide.

In certain embodiments, the nucleobase sequence of the modified oligonucleotide is at least 90% complementary to any one of SEQ ID NO: 1 as measured over the entirety of said modified oligonucleotide. In certain instances, the nucleobase sequence of the modified oligonucleotide is 85% complementary to any one of SEQ ID NOs: 1 as measured over the entirety of said modified oligonucleotide.

In certain embodiments, the nucleobase sequence of the modified oligonucleotide is at least 90% complementary to any one of SEQ ID NO: 2 as measured over the entirety of said modified oligonucleotide. In certain instances, the nucleobase sequence of the modified oligonucleotide is 85% complementary to any one of SEQ ID NO: 2 as measured over the entirety of said modified oligonucleotide.

In certain embodiments, at least one internucleoside linkage of said modified oligonucleotide is a modified internucleoside linkage. In certain embodiments, each internucleoside linkage is a phosphorothioate internucleoside linkage.

In certain embodiments, at least one nucleoside of said modified oligonucleotide comprises a modified sugar. In certain embodiments, at least one modified sugar is a bicyclic sugar. In certain embodiments, at least one modified sugar comprises a 2'-O-methoxyethyl or a 4'- (CH₂)ₙ-O-2' bridge, wherein n is 1 or 2.

In certain embodiments, at least one nucleoside of said modified oligonucleotide comprises a modified nucleobase. In certain embodiments, the modified nucleobase is a 5-methylcytosine.

In certain embodiments, the modified oligonucleotide comprises: a) a gap segment consisting of linked deoxynucleosides; b) a 5' wing segment consisting of linked nucleosides; and c) a 3' wing segment consisting of linked nucleosides. The gap segment is positioned between the 5' wing segment and the 3' wing segment and each nucleoside of each wing segment comprises a modified sugar.

In certain embodiments, the modified oligonucleotide comprises: a) a gap segment consisting of ten linked deoxynucleosides; b) a 5' wing segment consisting of five linked nucleosides; and c) a 3' wing segment consisting of five linked nucleosides. The gap segment is positioned between the 5' wing segment and the 3' wing segment, each nucleoside of each wing segment comprises a 2'-O-methoxyethyl sugar, each internucleoside linkage of said modified oligonucleotide is a phosphorothioate linkage, and each cytosine in said modified oligonucleotide is a 5'-methylcytosine.

In certain embodiments, the modified oligonucleotide consists of 20 linked nucleosides. In certain embodiments, the modified oligonucleotide consists of 19 linked nucleosides. In certain embodiments, the modified oligonucleotide consists of 18 linked nucleosides. In certain embodiments, the modified oligonucleotide consists of 17 linked nucleosides. In certain embodiments, the modified oligonucleotide consists of 16 linked nucleosides.

Also disclosed herein is a method of preferentially reducing CUGexp DMPK RNA, reducing myotonia or reducing spliceopathy in an animal comprising administering to the animal a compound comprising a modified oligonucleotide having a gap segment consisting of ten linked deoxynucleosides, a 5' wing segment consisting of five linked nucleosides and a 3' wing segment consisting of five linked nucleosides. The gap segment is positioned between the 5' wing segment and the 3' wing segment, each nucleoside of each wing segment comprises a 2'-O-methoxyethyl sugar, each internucleoside linkage of said modified oligonucleotide is a phosphorothioate linkage, each cytosine in said modified oligonucleotide is a 5'-methylcytosine.

In certain embodiments, the modified oligonucleotide comprises: a) a gap segment consisting of eight linked deoxynucleosides; b) a 5' wing segment consisting of four linked nucleosides and having a E-E-K-K 5'-wing motif; c) a 3' wing segment consisting of four linked nucleosides and having a K-K-E-E 3'-wing motif; and d) wherein the gap segment is positioned between the 5' wing segment and the 3' wing segment, and wherein each E represents 2'-O-methoxyethyl sugar and each K represents a cEt sugar.

In certain embodiments, the modified oligonucleotide comprises: a) a gap segment consisting of seven linked deoxynucleosides; b) a 5' wing segment consisting of five linked nucleosides and having an E-E-E-K-K 5'-wing motif; c) a 3' wing segment consisting of five linked nucleosides and having a K-K-E-E-E 3'-wing motif; and d) wherein the gap segment is positioned between the 5' wing segment and the 3' wing segment, and wherein each E represents 2'-O-methoxyethyl sugar and each K represents a cEt sugar.

In certain embodiments, the modified oligonucleotide comprises: a) a gap segment consisting of ten linked deoxynucleosides; b) a 5' wing segment consisting of five linked nucleosides; c) a 3' wing segment consisting of five linked nucleosides; and d) wherein the gap segment is positioned between the 5' wing segment and the 3' wing segment, and wherein each nucleoside of each wing segment comprises a 2'-O-methoxyethyl sugar.

In certain embodiments, the modified oligonucleotide comprises: a) a gap segment consisting of ten linked deoxynucleosides; b) a 5' wing segment consisting of three linked nucleosides; c) a 3' wing segment consisting of three linked nucleosides; and d) wherein the gap segment is positioned between the 5' wing segment and the 3' wing segment, and wherein each nucleoside of each wing segment comprises a cEt sugar.

Also disclosed herein is a method of preferentially reducing CUGexp DMPK RNA, reducing myotonia or reducing spliceopathy in an animal comprising administering to the animal a compound comprising a modified oligonucleotide having: a) a gap segment consisting of eight linked deoxynucleosides; b) a 5' wing segment consisting of four linked nucleosides and having a E-E-K-K 5'-wing motif; c) a 3' wing segment consisting of four linked nucleosides and having a K-K-E-E 3'-wing motif; and d) wherein the gap segment is positioned between the 5' wing segment and the 3' wing segment, and wherein each E represents 2'-O-methoxyethyl sugar and each K represents a cEt sugar.

Also disclosed herein is a method of preferentially reducing CUGexp DMPK RNA, reducing myotonia or reducing spliceopathy in an animal comprising administering to the animal a compound comprising a modified oligonucleotide having: a) a gap segment consisting of seven linked deoxynucleosides; b) a 5' wing segment consisting of five linked nucleosides and having an E-E-E-K-K 5'-wing motif; c) a 3' wing segment consisting of five linked nucleosides and having a K-K-E-E-E 3'-wing motif; and d) wherein the gap segment is positioned between the 5' wing segment and the 3' wing segment, and wherein each E represents 2'-O-methoxyethyl sugar and each K represents a cEt sugar.

Also disclosed herein is a method of preferentially reducing CUGexp DMPK RNA, reducing myotonia or reducing spliceopathy in an animal comprising administering to the animal a compound comprising a modified oligonucleotide having: a) a gap segment consisting of ten linked deoxynucleosides; b) a 5' wing segment consisting of five linked nucleosides; c) a 3' wing segment consisting of five linked nucleosides; and d) wherein the gap segment is positioned between the 5' wing segment and the 3' wing segment, and wherein each nucleoside of each wing segment comprises a 2'-O-methoxyethyl sugar.

Also disclosed herein is a method of preferentially reducing CUGexp DMPK RNA, reducing myotonia or reducing spliceopathy in an animal comprising administering to the animal a compound comprising a modified oligonucleotide having: a) a gap segment consisting of ten linked deoxynucleosides; b) a 5' wing segment consisting of three linked nucleosides; c) a 3' wing segment consisting of three linked nucleosides; and d) wherein the gap segment is positioned between the 5' wing segment and the 3' wing segment, and wherein each nucleoside of each wing segment comprises a cEt sugar.

Also disclosed herein is the use of any compound as described herein in the manufacture of a medicament for use in any of the therapeutic methods described herein. For example, disclosed herein is the use of a compound as described herein in the manufacture of a medicament for treating, ameliorating, or preventing type 1 myotonic dystrophy. Also disclosed herein is the use of a compound as described herein in the manufacture of a medicament for inhibiting expression of DMPK and treating, preventing, delaying or ameliorating a DMPK related disease and or a symptom thereof. Also disclosed herein is the use of a compound as described herein in the manufacture of a medicament for reducing DMPK expression in an animal. Also disclosed herein is the use of a compound as described herein in the manufacture of a medicament for preferentially reducing CUGexp DMPK, reducing myotonia, or reducing spliceopathy in an animal. Also disclosed herein is the use of a compound as described herein in the manufacture of a medicament for treating an animal with type 1 myotonic dystrophy. Also disclosed herein is the use of a compound as described herein in the manufacture of a medicament for treating, preventing, delaying, or ameliorating symptoms and outcomes associated with development of DM1 including muscle stiffness, myotonia, disabling distal weakness, weakness in face and jaw muscles, difficulty in swallowing, drooping of the eyelids (ptosis), weakness of neck muscles, weakness in arm and leg muscles, persistent muscle pain, hypersomnia, muscle wasting, dysphagia, respiratory insufficiency, irregular heartbeat, heart muscle damage, apathy, insulin resistance, and cataracts. Also disclosed herein is e the use of a compound as described herein in the manufacture of a medicament for counteracting RNA dominance by directing the cleavage of pathogenic transcripts.

Also disclosed herein is a kit for treating, preventing, or ameliorating type 1 myotonic dystrophy as described herein wherein the kit comprises: a) a compound as described herein; and optionally b) an additional agent or therapy as described herein. The kit can further include instructions or a label for using the kit to treat, prevent, or ameliorate type 1 myotonic dystrophy.

Also disclosed herein is any compound or composition as described herein, for use in any of the therapeutic methods described herein. For example, disclosed herein is a compound or composition as described herein for inhibiting expression of DMPK and treating, preventing, delaying or ameliorating a DMPK related disease and or a symptom thereof. Certain embodiments provide a compound or composition as described in the claims for use in reducing DMPK expression in an animal. Certain embodiments provide a compound or composition as described in the claims for use in preferentially reducing CUGexp DMPK, reducing myotonia, or reducing spliceopathy in an animal. Certain embodiments provide a compound or composition as described in the claims for use in treating an animal with type 1 myotonic dystrophy. Certain embodiments provide a compound or composition as described in the claims for use in treating, preventing, delaying, or ameliorating symptoms and outcomes associated with development of DM1 including muscle stiffness, myotonia, disabling distal weakness, weakness in face and jaw muscles, difficulty in swallowing, drooping of the eyelids (ptosis), weakness of neck muscles, weakness in arm and leg muscles, persistent muscle pain, hypersomnia, muscle wasting, dysphagia, respiratory insufficiency, irregular heartbeat, heart muscle damage, apathy, insulin resistance, and cataracts. Certain embodiments provide a compound or composition as described in the claims for use in counteracting RNA dominance by directing the cleavage of pathogenic transcripts. Also disclosed herein arecompounds comprising a modified oligonucleotide consisting of 12 to 30 linked nucleosides having a nucleobase sequence comprising at least 12 contiguous nucleobases of any of the nucleobase sequences of SEQ ID NOs: 23, 24, 25, 26, 27, 28, 29, 30, 31, 32, or 33- 874.

Other compounds which can be used in the methods described herein are also disclosed.

Also disclosed herein arecompounds comprising a modified oligonucleotide consisting of 10 to 80, 12 to 50, 12 to 30, 15 to 30, 18 to 24, 19 to 22, or 20 linked nucleosides having a nucleobase sequence comprising at least 8, at least 9, at least 10, at least 11, at least 12, at least 13, at least 14, at least 15, at least 16, at least 17, at least 18, or at least 19, contiguous nucleobases of any of the nucleobase sequences of SEQ ID NOs: 23, 24, 25, 26, 27, 28, 29, 30, 31, 32, or 33- 874.

Also disclosed herein arecompounds comprising a modified oligonucleotide consisting of 10 to 80, 12 to 50, 12 to 30, 15 to 30, 18 to 24, 19 to 22, or 20, linked nucleosides having a nucleobase sequence comprising at least 8, at least 9, at least 10, at least 11, at least 12, at least 13, at least 14, at least 15, at least 16, at least 17, at least 18, at least 19, contiguous nucleobases of any of the nucleobase sequences of SEQ ID NOs: 23, 24, 25, 26, 27, 28, 29, 30, 31, 32, or 33- 874.

Also disclosed herein arecompounds comprising a modified oligonucleotide consisting of 10 to 80, 12 to 50, 12 to 30, 15 to 30, or 15 to 17, linked nucleosides having a nucleobase sequence comprising a portion of at least 8, at least 9, at least 10, at least 11, at least 12, at least 13, at least 14, at least 15, at least 16, at least 17, at least 18, or at least 19, or more, contiguous nucleobases complementary to an equal length portion of nucleobases 1343-1368, 1346-1363, 1346-1361, or 1347-1363, of SEQ ID NO: 1.

In certain embodiments, the modified oligonucleotide is a single-stranded oligonucleotide.

In certain instances, the nucleobase sequence of the modified oligonucleotide is at least 70%, at least 75%, at least 80%, at least 85%, at least 90%, at least 95%, or 100%, complementary to any of SEQ ID NOs: 23, 24, 25, 26, 27, 28, 29, 30, 31, 32, or 33- 874.

In certain embodiments, at least one internucleoside linkage is a modified internucleoside linkage.

In certain embodiments, each internucleoside linkage is a phosphorothioate internucleoside linkage.

In certain embodiments, at least one nucleoside comprises a modified sugar.

In certain embodiments, at least one modified sugar is a bicyclic sugar.

In certain embodiments, at least one modified sugar is a cEt.

In certain embodiments, at least one modified sugar comprises a 2'-O-methoxyethyl.

In certain embodiments, at least one nucleoside comprises a modified nucleobase.

In certain embodiments, the modified nucleobase is a 5-methylcytosine. In certain embodiments, each cytosine residue comprises a 5-methylcytosine.

In certain embodiments, the modified oligonucleotide consists of 16 linked nucleosides.

In certain embodiments, the modified oligonucleotide consists of 17 linked nucleosides.

In certain embodiments, the modified oligonucleotide consists of 20 linked nucleosides.

### Antisense Compounds

Oligomeric compounds include, but are not limited to, oligonucleotides, oligonucleosides, oligonucleotide analogs, oligonucleotide mimetics, antisense compounds, antisense oligonucleotides, and siRNAs. An oligomeric compound can be "antisense" to a target nucleic acid, meaning that is capable of undergoing hybridization to a target nucleic acid through hydrogen bonding.

In certain embodiments, an antisense compound has a nucleobase sequence that, when written in the 5' to 3' direction, comprises the reverse complement of the target segment of a target nucleic acid to which it is targeted. In certain such embodiments, an antisense oligonucleotide has a nucleobase sequence that, when written in the 5' to 3' direction, comprises the reverse complement of the target segment of a target nucleic acid to which it is targeted.

In certain embodiments, an antisense compound targeted to DMPK as described herein is 10 to 30 nucleotides in length. In other words, the antisense compounds are in some embodiments from 10 to 30 linked nucleobases. In other instances, the antisense compound comprises a modified oligonucleotide consisting of 8 to 80, 10 to 80, 12 to 30, 12 to 50, 15 to 30, 15 to 18, 15 to 17, 16 to 16, 18 to 24, 19 to 22, or 20 linked nucleobases. In certain such instances, the antisense compound comprises a modified oligonucleotide consisting of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 linked nucleobases in length, or a range defined by any two of the above values. In certain instances, antisense compounds of any of these lengths contain at least 8, at least 9, at least 10, at least 11, at least 12, at least 13, at least 14, at least 15, at least 16, at least 17, at least 18, or at least 19, contiguous nucleobases of the nucleobase sequence of any of the exemplary antisense compounds described herein (e.g., at least 8 contiguous nucleobases of a nucleobase sequence recited in any one of SEQ ID NOs: 23, 24, 25, 26, 27, 28, 29, 30, 31, 32, or 33- 874..

In certain embodiments, the antisense compound comprises a shortened or truncated modified oligonucleotide. The shortened or truncated modified oligonucleotide can have a single nucleoside deleted from the 5' end (5' truncation), or alternatively from the 3' end (3' truncation). A shortened or truncated oligonucleotide can have two nucleosides deleted from the 5' end, or alternatively can have two subunits deleted from the 3' end. Alternatively, the deleted nucleosides can be dispersed throughout the modified oligonucleotide, for example, in an antisense compound having one nucleoside deleted from the 5' end and one nucleoside deleted from the 3' end.

When a single additional nucleoside is present in a lengthened oligonucleotide, the additional nucleoside can be located at the 5' or 3' end of the oligonucleotide. When two or more additional nucleosides are present, the added nucleosides can be adjacent to each other, for example, in an oligonucleotide having two nucleosides added to the 5' end (5' addition), or alternatively to the 3' end (3' addition), of the oligonucleotide. Alternatively, the added nucleoside can be dispersed throughout the antisense compound, for example, in an oligonucleotide having one nucleoside added to the 5' end and one subunit added to the 3' end.

It is possible to increase or decrease the length of an antisense compound, such as an antisense oligonucleotide, and/or introduce mismatch bases without eliminating activity. For example, in Woolf et al. (Proc. Natl. Acad. Sci. USA 89:7305-7309, 1992), a series of antisense oligonucleotides 13-25 nucleobases in length were tested for their ability to induce cleavage of a target RNA in an oocyte injection model. Antisense oligonucleotides 25 nucleobases in length with 8 or 11 mismatch bases near the ends of the antisense oligonucleotides were able to direct specific cleavage of the target mRNA, albeit to a lesser extent than the antisense oligonucleotides that contained no mismatches. Similarly, target specific cleavage was achieved using 13 nucleobase antisense oligonucleotides, including those with 1 or 3 mismatches.

Gautschi et al (J. Natl. Cancer Inst. 93:463-471, March 2001) demonstrated the ability of an oligonucleotide having 100% complementarity to the bcl-2 mRNA and having 3 mismatches to the bcl-xL mRNA to reduce the expression of both bcl-2 and bcl-xL *in vitro* and *in vivo.* Furthermore, this oligonucleotide demonstrated potent anti-tumor activity *in vivo.*

Maher and Dolnick (Nuc. Acid. Res. 16:3341-3358, 1988) tested a series of tandem 14 nucleobase antisense oligonucleotides, and a 28 and 42 nucleobase antisense oligonucleotides comprised of the sequence of two or three of the tandem antisense oligonucleotides, respectively, for their ability to arrest translation of human DHFR in a rabbit reticulocyte assay. Each of the three 14 nucleobase antisense oligonucleotides alone was able to inhibit translation, albeit at a more modest level than the 28 or 42 nucleobase antisense oligonucleotides.

### Target Nucleic Acids, Target Regions and Nucleotide Sequences

Nucleotide sequences that encode DMPK include, without limitation, the following sequences as set forth in GenBank Accession No. NM_001081560.1 (incorporated herein as SEQ ID NO: 1), GenBank Accession No. NT_011109.15 truncated from nucleotides 18540696 to 18555106 (incorporated herein as SEQ ID NO: 2), GenBank Accession No. NT_039413.7 truncated from nucleotides 16666001 to 16681000 (incorporated herein as SEQ ID NO: 3), GenBank Accession No. NM_032418.1 (incorporated herein as SEQ ID NO: 4), GenBank Accession No. AI007148.1 (incorporated herein as SEQ ID NO: 5), GenBank Accession No. AI304033.1 (incorporated herein as SEQ ID NO: 6), GenBank Accession No. BC024150.1 (incorporated herein as SEQ ID NO: 7), GenBank Accession No. BC056615.1 (incorporated herein as SEQ ID NO: 8), GenBank Accession No. BC075715.1 (incorporated herein as SEQ ID NO: 9), GenBank Accession No. BU519245.1 (incorporated herein as SEQ ID NO: 10), GenBank Accession No. CB247909.1 (incorporated herein as SEQ ID NO: 11), GenBank Accession No. CX208906.1 (incorporated herein as SEQ ID NO: 12), GenBank Accession No. CX732022.1 (incorporated herein as SEQ ID NO: 13), GenBank Accession No. S60315.1 (incorporated herein as SEQ ID NO: 14), GenBank Accession No. S60316.1 (incorporated herein as SEQ ID NO: 15), GenBank Accession No. NM_001081562.1 (incorporated herein as SEQ ID NO: 16), and GenBank Accession No. NM_001100.3 (incorporated herein as SEQ ID NO: 17). It is understood that the sequence set forth in each SEQ ID NO in the Examples contained herein is independent of any modification to a sugar moiety, an internucleoside linkage, or a nucleobase. As such, antisense compounds defined by a SEQ ID NO can comprise, independently, one or more modifications to a sugar moiety, an internucleoside linkage, or a nucleobase. Antisense compounds described by Isis Number (Isis No) indicate a combination of nucleobase sequence and motif.

In certain instances, a target region is a structurally defined region of the target nucleic acid. For example, a target region can encompass a 3' UTR, a 5' UTR, an exon, an intron, an exon/intron junction, a coding region, a translation initiation region, translation termination region, or other defined nucleic acid region. The structurally defined regions for DMPK can be obtained by accession number from sequence databases such as NCBI. In certain embodiments, a target region can encompass the sequence from a 5' target site of one target segment within the target region to a 3' target site of another target segment within the target region.

Targeting includes determination of at least one target segment to which an antisense compound hybridizes, such that a desired effect occurs. In certain embodiments, the desired effect is a reduction in mRNA target nucleic acid levels. In certain embodiments, the desired effect is reduction of levels of protein encoded by the target nucleic acid or a phenotypic change associated with the target nucleic acid.

A target region can contain one or more target segments. Multiple target segments within a target region can be overlapping. Alternatively, they can be non-overlapping. In certain instances, target segments within a target region are separated by no more than about 300 nucleotides. In certain instances, target segments within a target region are separated by a number of nucleotides that is, is about, is no more than, is no more than about, 250, 200, 150, 100, 90, 80, 70, 60, 50, 40, 30, 20, or 10 nucleotides on the target nucleic acid, or is a range defined by any two of the preceding values. In certain instances, target segments within a target region are separated by no more than, or no more than about, 5 nucleotides on the target nucleic acid. In certain embodiments, target segments are contiguous. Contemplated are target regions defined by a range having a starting nucleic acid that is any of the 5' target sites or 3' target sites listed herein.

Suitable target segments can be found within a 5' UTR, a coding region, a 3' UTR, an intron, an exon, or an exon/intron junction. Target segments containing a start codon or a stop codon are also suitable target segments. A suitable target segment can specifically exclude a certain structurally defined region such as the start codon or stop codon.

The determination of suitable target segments can include a comparison of the sequence of a target nucleic acid to other sequences throughout the genome. For example, the BLAST algorithm can be used to identify regions of similarity amongst different nucleic acids. This comparison can prevent the selection of antisense compound sequences that can hybridize in a non-specific manner to sequences other than a selected target nucleic acid (i.e., non-target or off-target sequences).

There can be variation in activity (e.g., as defined by percent reduction of target nucleic acid levels) of the antisense compounds within an active target region. In certain embodiments, reductions in DMPK mRNA levels are indicative of inhibition of DMPK protein expression. Reductions in levels of a DMPK protein are also indicative of inhibition of target mRNA expression. Further, phenotypic changes, such as a reducing myotonia or reducing spliceopathy, can be indicative of inhibition of DMPK mRNA and/or protein expression.

### Hybridization

In some embodiments, hybridization occurs between an antisense compound disclosed herein and a DMPK nucleic acid. The most common mechanism of hybridization involves hydrogen bonding (e.g., Watson-Crick, Hoogsteen or reversed Hoogsteen hydrogen bonding) between complementary nucleobases of the nucleic acid molecules.

Hybridization can occur under varying conditions. Stringent conditions are sequence-dependent and are determined by the nature and composition of the nucleic acid molecules to be hybridized.

Methods of determining whether a sequence is specifically hybridizable to a target nucleic acid are well known in the art (Sambrooke and Russell, Molecular Cloning: A Laboratory Manual, 3rd Ed., 2001). In certain embodiments, the antisense compounds provided herein are specifically hybridizable with a DMPK nucleic acid.

### Complementarity

An antisense compound and a target nucleic acid are complementary to each other when a sufficient number of nucleobases of the antisense compound can hydrogen bond with the corresponding nucleobases of the target nucleic acid, such that a desired effect will occur (e.g., antisense inhibition of a target nucleic acid, such as a DMPK nucleic acid).

An antisense compound can hybridize over one or more segments of a DMPK nucleic acid such that intervening or adjacent segments are not involved in the hybridization event (e.g., a loop structure, mismatch or hairpin structure).

In certain instances, the antisense compounds disclosed herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to a DMPK nucleic acid, a target region, target segment, or specified portion thereof. In certain instances, the antisense compounds are at least 70%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% complementary to a DMPK nucleic acid, a target region, target segment, or specified portion thereof, and contain at least 8, at least 9, at least 10, at least 11, at least 12, at least 13, at least 14, at least 15, at least 16, at least 17, at least 18, or at least 19, contiguous nucleobases of the nucleobase sequence of any of the exemplary antisense compounds described herein (e.g., at least 8 contiguous nucleobases of a nucleobase sequence recited in any one of SEQ ID NOs: 23, 24, 25, 26, 27, 28, 29, 30, 31, 32, or 33- 874). Percent complementarity of an antisense compound with a target nucleic acid can be determined using routine methods, and is measured over the entirety of the antisense compound.

For example, an antisense compound in which 18 of 20 nucleobases of the antisense compound are complementary to a target region, and would therefore specifically hybridize, would represent 90 percent complementarity. In this example, the remaining noncomplementary nucleobases can be clustered or interspersed with complementary nucleobases and need not be contiguous to each other or to complementary nucleobases. As such, an antisense compound which is 18 nucleobases in length having 4 (four) noncomplementary nucleobases which are flanked by two regions of complete complementarity with the target nucleic acid would have 77.8% overall complementarity with the target nucleic acid. Percent complementarity of an antisense compound with a region of a target nucleic acid can be determined routinely using BLAST programs (basic local alignment search tools) and PowerBLAST programs known in the art (Altschul et al., J. Mol. Biol., 1990, 215, 403 410; Zhang and Madden, Genome Res., 1997, 7, 649 656). Percent homology, sequence identity or complementarity, can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482 489).

In certain embodiments, the antisense compounds provided herein, or specified portions thereof, are fully complementary (i.e. 100% complementary) to a target nucleic acid, or specified portion thereof. For example, antisense compound can be fully complementary to a DMPK nucleic acid, or a target region, or a target segment or target sequence thereof. As used herein, "fully complementary" means each nucleobase of an antisense compound is capable of precise base pairing with the corresponding nucleobases of a target nucleic acid. For example, a 20 nucleobase antisense compound is fully complementary to a target sequence that is 400 nucleobases long, so long as there is a corresponding 20 nucleobase portion of the target nucleic acid that is fully complementary to the antisense compound. Fully complementary can also be used in reference to a specified portion of the first and /or the second nucleic acid. For example, a 20 nucleobase portion of a 30 nucleobase antisense compound can be "fully complementary" to a target sequence that is 400 nucleobases long. The 20 nucleobase portion of the 30 nucleobase oligonucleotide is fully complementary to the target sequence if the target sequence has a corresponding 20 nucleobase portion wherein each nucleobase is complementary to the 20 nucleobase portion of the antisense compound. At the same time, the entire 30 nucleobase antisense compound can be fully complementary to the target sequence, depending on whether the remaining 10 nucleobases of the antisense compound are also complementary to the target sequence.

The location of a non-complementary nucleobase can be at the 5' end or 3' end of the antisense compound. Alternatively, the non-complementary nucleobase or nucleobases can be at an internal position of the antisense compound. When two or more non-complementary nucleobases are present, they can be either contiguous (i.e. linked) or non-contiguous. In one embodiment, a non-complementary nucleobase is located in the wing segment of a gapmer antisense oligonucleotide.

In certain embodiments, antisense compounds that are, or are up to 10, 12, 13, 14, 15, 16, 17, 18, 19, or 20 nucleobases in length comprise no more than 4, no more than 3, no more than 2, or no more than 1 non-complementary nucleobase(s) relative to a target nucleic acid, such as a DMPK nucleic acid, or specified portion thereof.

In certain embodiments, antisense compounds that are, or are up to 10, 12, 13, 14, 15, 16, 17, 18, 19, 20, 21, 22, 23, 24, 25, 26, 27, 28, 29, or 30 nucleobases in length comprise no more than 6, no more than 5, no more than 4, no more than 3, no more than 2, or no more than 1 non-complementary nucleobase(s) relative to a target nucleic acid, such as a DMPK nucleic acid, or specified portion thereof.

The antisense compounds provided herein also include those which are complementary to a portion of a target nucleic acid. As used herein, "portion" refers to a defined number of contiguous (i.e. linked) nucleobases within a region or segment of a target nucleic acid. A "portion" can also refer to a defined number of contiguous nucleobases of an antisense compound. In certain embodiments, the antisense compounds, are complementary to at least an 8 nucleobase portion of a target segment. In certain embodiments, the antisense compounds are complementary to at least a 10 nucleobase portion of a target segment. In certain embodiments, the antisense compounds are complementary to at least a 15 nucleobase portion of a target segment. Also contemplated are antisense compounds that are complementary to at least an 8, at least a 9, at least a 10, at least an 11, at least a 12, at least a 13, at least a 14, at least a 15, at least a 16, at least a 17, at least an 18, at least a 19, at least a 20, or more nucleobase portion of a target segment, or a range defined by any two of these values.

### Identity

The antisense compounds provided herein can also have a defined percent identity to a particular nucleotide sequence, SEQ ID NO, or compound represented by a specific Isis number, or portion thereof. As used herein, an antisense compound is identical to the sequence disclosed herein if it has the same nucleobase pairing ability. For example, a RNA which contains uracil in place of thymidine in a disclosed DNA sequence would be considered identical to the DNA sequence since both uracil and thymidine pair with adenine. Shortened and lengthened versions of the antisense compounds described herein as well as compounds having non-identical bases relative to the antisense compounds provided herein also are contemplated. The non-identical bases can be adjacent to each other or dispersed throughout the antisense compound. Percent identity of an antisense compound is calculated according to the number of bases that have identical base pairing relative to the sequence to which it is being compared.

In certain instances, the antisense compounds, or portions thereof, are at least 70%, at least 75%, at least 80%, at least 85%, at least 90%, at least 95%, at least 96%, at least 97%, at least 98%, at least 99% or 100% identical to one or more of the exemplary antisense compounds or SEQ ID NOs, or a portion thereof, disclosed herein.

### Modifications

A nucleoside is a base-sugar combination. The nucleobase (also known as base) portion of the nucleoside is normally a heterocyclic base moiety. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. Oligonucleotides are formed through the covalent linkage of adjacent nucleosides to one another, to form a linear polymeric oligonucleotide. Within the oligonucleotide structure, the phosphate groups are commonly referred to as forming the internucleoside linkages of the oligonucleotide.

Modifications to antisense compounds encompass substitutions or changes to internucleoside linkages, sugar moieties, or nucleobases. Modified antisense compounds are often preferred over native forms because of desirable properties such as, for example, enhanced cellular uptake, enhanced affinity for nucleic acid target, increased stability in the presence of nucleases, or increased inhibitory activity.

Chemically modified nucleosides can also be employed to increase the binding affinity of a shortened or truncated antisense oligonucleotide for its target nucleic acid. Consequently, comparable results can often be obtained with shorter antisense compounds that have such chemically modified nucleosides.

### Modified Internucleoside Linkages

The naturally occurring internucleoside linkage of RNA and DNA is a 3' to 5' phosphodiester linkage. Antisense compounds having one or more modified, i.e. non-naturally occurring, internucleoside linkages are often selected over antisense compounds having naturally occurring internucleoside linkages because of desirable properties such as, for example, enhanced cellular uptake, enhanced affinity for target nucleic acids, and increased stability in the presence of nucleases.

Oligonucleotides having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates, phosphoramidate, and phosphorothioates. Methods of preparation of phosphorous-containing and non-phosphorous-containing linkages are well known.

In certain embodiments, antisense compounds targeted to a DMPK nucleic acid comprise one or more modified internucleoside linkages. In certain embodiments, the modified internucleoside linkages are phosphorothioate linkages. In certain embodiments, each internucleoside linkage of an antisense compound is a phosphorothioate internucleoside linkage.

### Modified Sugar Moieties

Antisense compounds of the invention can optionally contain one or more nucleosides wherein the sugar group has been modified. Such sugar modified nucleosides may impart enhanced nuclease stability, increased binding affinity, or some other beneficial biological property to the antisense compounds. In certain embodiments, nucleosides comprise chemically modified ribofuranose ring moieties. Examples of chemically modified ribofuranose rings include without limitation, addition of substitutent groups (including 5' and 2' substituent groups, bridging of non-geminal ring atoms to form bicyclic nucleic acids (BNA), replacement of the ribosyl ring oxygen atom with S, N(R), or C(R₁)(R₂) (R, R₁ and R₂ are each independently H, C₁-C₁₂ alkyl or a protecting group) and combinations thereof. Examples of chemically modified sugars include 2'-F-5'-methyl substituted nucleoside (see PCT International Application WO 2008/101157 Published on 8/21/08 for other disclosed 5',2'-bis substituted nucleosides) or replacement of the ribosyl ring oxygen atom with S with further substitution at the 2'-position (see published U.S. Patent Application US2005-0130923, published on June 16, 2005) or alternatively 5'-substitution of a BNA (see PCT International Application WO 2007/134181 Published on 11/22/07 wherein LNA is substituted with for example a 5'-methyl or a 5'-vinyl group).

Examples of nucleosides having modified sugar moieties include without limitation nucleosides comprising 5'-vinyl, 5'-methyl (*R* or *S*), 4'-S, 2'-F, 2'-OCH₃, 2'-OCH₂CH₃, 2'-OCH₂CH₂F and 2'-O(CH₂)₂OCH₃ substituent groups. The substituent at the 2' position can also be selected from allyl, amino, azido, thio, O-allyl, O-C₁-C₁₀ alkyl, OCF₃, OCH₂F, O(CH₂)₂SCH₃, O(CH₂)₂-O-N(Rₘ)(Rₙ), O-CH₂-C(=O)-N(Rₘ)(Rₙ), and O-CH₂-C(=O)-N(R)-(CH₂)₂-N(Rₘ)(Rₙ), where each Ri, Rₘ and Rₙ is, independently, H or substituted or unsubstituted C₁-C₁₀ alkyl.

Examples of bicyclic nucleic acids (BNAs) include without limitation nucleosides comprising a bridge between the 4' and the 2' ribosyl ring atoms. In certain embodiments, antisense compounds provided herein include one or more BNA nucleosides wherein the bridge comprises one of the formulas: 4'-(CH₂)-O-2' (LNA); 4'-(CH₂)-S-2'; 4'-(CH₂)₂-O-2' (ENA); 4'-CH(CH₃)-O-2' and 4'-CH(CH₂OCH₃)-O-2' (and analogs thereof see U.S. Patent 7,399,845, issued on July 15, 2008); 4'-C(CH₃)(CH₃)-O-2' (and analogs thereof see PCT/US2008/068922 published as WO/2009/006478, published January 8, 2009); 4'-CH₂-N(OCH₃)-2' (and analogs thereof see PCT/US2008/064591 published as WO/2008/150729, published December 11, 2008); 4'-CH₂-ON(CH₃)-2' (see published U.S. Patent Application US2004-0171570, published September 2, 2004 ); 4'-CH₂-N(R)-O-2', wherein R is H, C₁-C₁₂ alkyl, or a protecting group (see U.S. Patent 7,427,672, issued on September 23, 2008); 4'-CH₂-C(H)(CH₃)-2' (see Chattopadhyaya et al., J. Org. Chem., 2009, 74, 118-134); and 4'-CH₂-C(=CH₂)-2' (and analogs thereof see PCT/US2008/066154 published as WO 2008/154401, published on December 8, 2008).

Further bicyclic nucleosides have been reported in published literature (see for example: Srivastava et al., J. Am. Chem. Soc., 2007, 129(26) 8362-8379; Frieden et al., Nucleic Acids Research, 2003, 21, 6365-6372; Elayadi et al., Curr. Opinion Invens. Drugs, 2001, 2, 558-561; Braasch et al., Chem. Biol., 2001, 8, 1-7; Orum et al., Curr. Opinion Mol. Ther., 2001, 3, 239-243; Wahlestedt et al., Proc. Natl. Acad. Sci. U. S. A., 2000, 97, 5633-5638; Singh et al., Chem. Commun., 1998, 4, 455-456; Koshkin et al., Tetrahedron, 1998, 54, 3607-3630; Kumar et al., Bioorg. Med. Chem. Lett., 1998, 8, 2219-2222; Singh et al., J. Org. Chem., 1998, 63, 10035-10039; U.S. Patents Nos.: 9,012,421; 8,501,805; 8,546,556; 8,530,640; 7,399,845; 7,053,207; 7,034,133; 6,794,499; 6,770,748; 6,670,461; 6,525,191; 6,268,490 ; U.S. Patent Publication Nos.: US2008-0039618; US2007-0287831; US2004-0171570; U.S. Patent Applications, Serial Nos.: 12/129,154; International applications WO 2007/134181; WO 2005/021570; WO 2004/106356; WO 94/14226; and PCT International Applications Nos.: PCT/US2008/068922; PCT/US2008/066154; and PCT/US2008/064591). Each of the foregoing bicyclic nucleosides can be prepared having one or more stereochemical sugar configurations including for example α-L-ribofuranose and β-D-ribofuranose (see PCT international application PCT/DK98/00393, published on March 25, 1999 as WO 99/14226).

In certain embodiments, bicyclic nucleosides comprise a bridge between the 4' and the 2' carbon atoms of the pentofuranosyl sugar moiety including without limitation, bridges comprising 1 or from 1 to 4 linked groups independently selected from -[C(Rₐ)(R_{b})]ₙ-, -C(Rₐ)=C(R_{b})-, -C(Rₐ)=N-, -C(=NRₐ)-, -C(=O)-, -C(=S)-, -O-, -Si(Rₐ)₂-, -S(=O)ₓ-, and -N(Rₐ)-; wherein: x is 0, 1, or 2; n is 1, 2, 3, or 4; each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJi, NJ₁J₂, SJi, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In certain embodiments, the bridge of a bicyclic sugar moiety is, -[C(Rₐ)(R_{b})]ₙ-, -[C(Rₐ)(R_{b})]ₙ-O-, -C(RₐR_{b})-N(R)-O- or -C(RₐR_{b})-O-N(R)-. In certain embodiments, the bridge is 4'-CH₂-2', 4'-(CH₂)₂-2', 4'-(CH₂)₃-2', 4'-CH₂-O-2', 4'-(CH₂)₂-O-2', 4'-CH₂-O-N(R)-2' and 4'-CH₂-N(R)-O-2'- wherein each R is, independently, H, a protecting group or C₁-C₁₂ alkyl.

In certain embodiments, bicyclic nucleosides are further defined by isomeric configuration. For example, a nucleoside comprising a 4'-(CH₂)-O-2' bridge, may be in the α-L configuration or in the β-D configuration. Previously, α-L-methyleneoxy (4'-CH₂-O-2') BNA's have been incorporated into antisense oligonucleotides that showed antisense activity (Frieden et al., Nucleic Acids Research, 2003, 21, 6365-6372).

In certain embodiments, bicyclic nucleosides include those having a 4' to 2' bridge wherein such bridges include without limitation, α-L-4'-(CH₂)-O-2', β-D-4'-CH₂-O-2', 4'-(CH₂)₂-O-2', 4'-CH₂-O-N(R)-2', 4'-CH₂-N(R)-O-2', 4'-CH(CH₃)-O-2', 4'-CH₂-S-2', 4'-CH₂-N(R)-2', 4'-CH₂-CH(CH₃)-2', and 4'-(CH₂)₃-2', wherein R is H, a protecting group or C₁-C₁₂ alkyl.

In certain embodiments, bicyclic nucleosides have the formula: 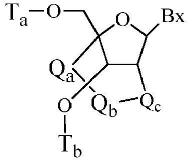 wherein:
Bx is a heterocyclic base moiety;
-Qa-Qb-Qc- is -CH₂-N(R_{c})-CH₂-, -C(=O)-N(R_{c})-CH₂-, -CH₂-O-N(R_{c})-, -CH₂-N(R_{c})-O- or - N(R_{c})-O-CH₂;
R_{c} is C₁-C₁₂ alkyl or an amino protecting group; and
Tₐ and T_{b} are each, independently H, a hydroxyl protecting group, a conjugate group, a reactive phosphorus group, a phosphorus moiety or a covalent attachment to a support medium.

In certain embodiments, bicyclic nucleosides have the formula: 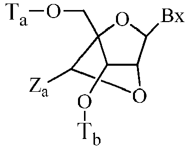 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Zₐ is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, substituted amide, thiol or substituted thiol.

In one embodiment, each of the substituted groups, is, independently, mono or poly substituted with substituent groups independently selected from halogen, oxo, hydroxyl, OJ_{c}, NJ_{c}J_{d}, SJ_{c}, N3, OC(=X)Jc, and NJeC(=X)NJcJd, wherein each J_{c}, J_{d} and Jₑ is, independently, H, C₁-C₆ alkyl, or substituted C₁-C₆ alkyl and X is O or NJ_{c}.

In certain embodiments, bicyclic nucleosides have the formula: 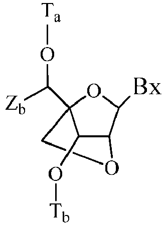 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Z_{b} is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl or substituted acyl (C(=O)-).

In certain embodiments, bicyclic nucleosides have the formula: 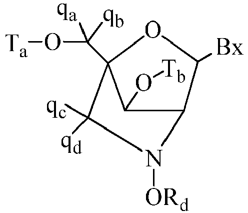 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
R_{d} is C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl;
each qₐ, q_{b}, q_{c} and q_{d} is, independently, H, halogen, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl, C₁-C₆ alkoxyl, substituted C₁-C₆ alkoxyl, acyl, substituted acyl, C₁-C₆ aminoalkyl or substituted C₁-C₆ aminoalkyl;

In certain embodiments, bicyclic nucleosides have the formula: 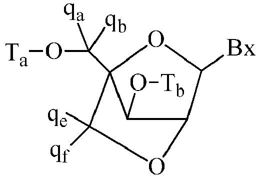 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
qₐ, q_{b}, qₑ and q_{f} are each, independently, hydrogen, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJj, SJj, SOJⱼ, SO₂Jⱼ, NJjJk, N3, CN, C(=O)OJⱼ, C(=O)NJⱼJₖ, C(=O)Jⱼ, O-C(=O)NJⱼJₖ, N(H)C(=NH)NJjJk, N(H)C(=O)NJⱼJₖ or N(H)C(=S)NJjJk;
or qₑ and q_{f} together are =C(q_{g})(qₕ);
q_{g} and qₕ are each, independently, H, halogen, C₁-C₁₂ alkyl or substituted C₁-C₁₂ alkyl.

The synthesis and preparation of adenine, cytosine, guanine, 5-methyl-cytosine, thymine and uracil bicyclic nucleosides having a 4'-CH₂-O-2' bridge, along with their oligomerization, and nucleic acid recognition properties have been described (Koshkin et al., Tetrahedron, 1998, 54, 3607-3630). The synthesis of bicyclic nucleosides has also been described in WO 98/39352 and WO 99/14226.

Analogs of various bicyclic nucleosides that have 4' to 2' bridging groups such as 4'-CH₂-O-2' and 4'-CH₂-S-2', have also been prepared (Kumar et al., Bioorg. Med. Chem. Lett., 1998, 8, 2219-2222). Preparation of oligodeoxyribonucleotide duplexes comprising bicyclic nucleosides for use as substrates for nucleic acid polymerases has also been described (Wengel et al., WO 99/14226). Furthermore, synthesis of 2'-amino-BNA, a novel conformationally restricted high-affinity oligonucleotide analog has been described in the art (Singh et al., J. Org. Chem., 1998, 63, 10035-10039). In addition, 2'-amino- and 2'-methylamino-BNA's have been prepared and the thermal stability of their duplexes with complementary RNA and DNA strands has been previously reported.

In certain embodiments, bicyclic nucleosides have the formula: 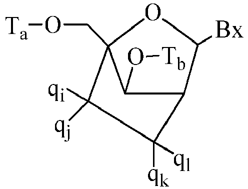 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
each qᵢ, qⱼ, qₖ and qₗ is, independently, H, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxyl, substituted C₁-C₁₂ alkoxyl, OJj, SJj, SOJⱼ, SO₂Jⱼ, NJjJk, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ; and
qi and qⱼ or qi and qₖ together are =C(q_{g})(qₕ), wherein q_{g} and qₕ are each, independently, H, halogen, C₁-C₁₂ alkyl or substituted C₁-C₁₂ alkyl.

One carbocyclic bicyclic nucleoside having a 4'-(CH₂)₃-2' bridge and the alkenyl analog bridge 4'-CH=CH-CH₂-2' have been described (Frier et al., Nucleic Acids Research, 1997, 25(22), 4429-4443 and Albaek et al., J. Org. Chem., 2006, 71, 7731-7740). The synthesis and preparation of carbocyclic bicyclic nucleosides along with their oligomerization and biochemical studies have also been described (Srivastava et al., J. Am. Chem. Soc. 2007, 129(26), 8362-8379).

In certain embodiments, bicyclic nucleosides include, but are not limited to, (A) α-L-methyleneoxy (4'-CH₂-O-2') BNA, (B) β-D-methyleneoxy (4'-CH₂-O-2') BNA, (C) ethyleneoxy (4'-(CH₂)₂-O-2') BNA, (D) aminooxy (4'-CH₂-O-N(R)-2') BNA, (E) oxyamino (4'-CH₂-N(R)-O-2') BNA, (F) methyl(methyleneoxy) (4'-CH(CH₃)-O-2') BNA (also referred to as constrained ethyl or cEt), (G) methylene-thio (4'-CH₂-S-2') BNA, (H) methylene-amino (4'-CH₂-N(R)-2') BNA, (I) methyl carbocyclic (4'-CH₂-CH(CH₃)-2') BNA, (J) propylene carbocyclic (4'-(CH₂)₃-2') BNA, and (K) vinyl BNA as depicted below. 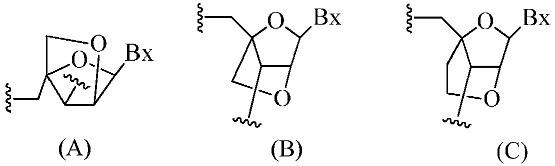 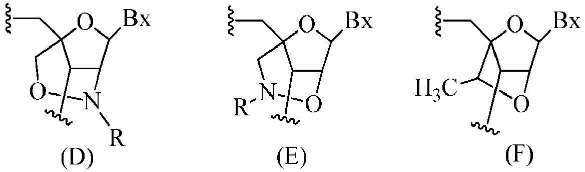 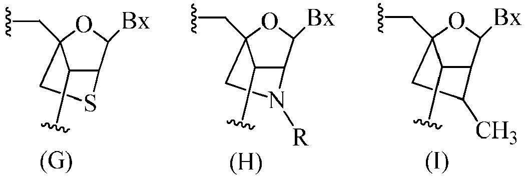 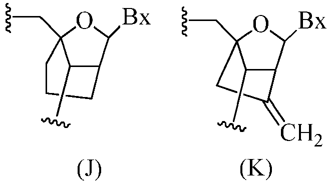 wherein Bx is the base moiety and R is, independently, H, a protecting group, C₁-C₆ alkyl or C₁-C₆ alkoxy.

In certain embodiments, nucleosides are modified by replacement of the ribosyl ring with a sugar surrogate. Such modification includes without limitation, replacement of the ribosyl ring with a surrogate ring system (sometimes referred to as DNA analogs) such as a morpholino ring, a cyclohexenyl ring, a cyclohexyl ring or a tetrahydropyranyl ring such as one having one of the formula:

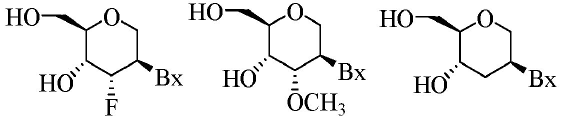

In certain embodiments, sugar surrogates are selected having the formula: 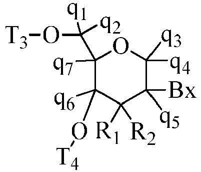 wherein:
Bx is a heterocyclic base moiety;
T₃ and T₄ are each, independently, an internucleoside linking group linking the tetrahydropyran nucleoside analog to the oligomeric compound or one of T₃ and T₄ is an internucleoside linking group linking the tetrahydropyran nucleoside analog to an oligomeric compound or oligonucleotide and the other of T₃ and T₄ is H, a hydroxyl protecting group, a linked conjugate group or a 5' or 3'-terminal group;
   q₁, q₂, q₃, q₄, qs, q₆ and q₇ are each independently, H, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl; and
one of R₁ and R₂ is hydrogen and the other is selected from halogen, substituted or unsubstituted alkoxy, NJ₁J₂, SJ₁, N₃, OC(=X)J₁, OC(=X)NJ₁J₂, NJ₃C(=X)NJ₁J₂ and CN, wherein X is O, S or NJ₁ and each Ji, J₂ and J₃ is, independently, H or C₁-C₆ alkyl.

In certain embodiments, q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, THP nucleosides are provided wherein one of R₁ and R₂ is F. In certain embodiments, R₁ is fluoro and R₂ is H; R₁ is methoxy and R₂ is H, and R₁ is methoxyethoxy and R₂ is H.

Such sugar surrogates include, but are not limited to, what is referred to in the art as hexitol nucleic acid (HNA), altritol nucleic acid (ANA), and mannitol nucleic acid (MNA) *(see* Leumann, C. J., Bioorg. & Med. Chem., 2002, 10, 841-854).

In certain embodiments, sugar surrogates comprise rings having more than 5 atoms and more than one heteroatom. For example nucleosides comprising morpholino sugar moieties and their use in oligomeric compounds has been reported (see for example: Braasch et al., Biochemistry, 2002, 41, 4503-4510; and U.S. Patents 5,698,685; 5,166,315; 5,185,444; and 5,034,506).

As used here, the term "morpholino" means a sugar surrogate having the following structure:

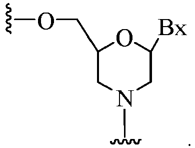

In certain embodiments, morpholinos may be modified, for example by adding or altering various substituent groups from the above morpholino structure. Such sugar surrogates are refered to herein as "modifed morpholinos."

In certain embodiments, antisense compounds comprise one or more modified cyclohexenyl nucleosides, which is a nucleoside having a six-membered cyclohexenyl in place of the pentofuranosyl residue in naturally occurring nucleosides. Modified cyclohexenyl nucleosides include, but are not limited to those described in the art (see for example commonly owned, published PCT Application WO 2010/036696, published on April 10, 2010, Robeyns et al., J. Am. Chem. Soc., 2008, 130(6), 1979-1984; Horvath et al., Tetrahedron Letters, 2007, 48, 3621-3623; Nauwelaerts et al., J. Am. Chem. Soc., 2007, 129(30), 9340-9348; Gu et al.,, Nucleosides, Nucleotides & Nucleic Acids, 2005, 24(5-7), 993-998; Nauwelaerts et al., Nucleic Acids Research, 2005, 33(8), 2452-2463; Robeyns et al., Acta Crystallographica, Section F: Structural Biology and Crystallization Communications, 2005, F61(6), 585-586; Gu et al., Tetrahedron, 2004, 60(9), 2111-2123; Gu et al., Oligonucleotides, 2003, 13(6), 479-489; Wang et al., J. Org. Chem., 2003, 68, 4499-4505; Verbeure et al., Nucleic Acids Research, 2001, 29(24), 4941-4947; Wang et al., J. Org. Chem., 2001, 66, 8478-82; Wang et al., Nucleosides, Nucleotides & Nucleic Acids, 2001, 20(4-7), 785-788; Wang et al., J. Am. Chem., 2000, 122, 8595-8602; Published PCT application, WO 06/047842; and Published PCT Application WO 01/049687). Certain modified cyclohexenyl nucleosides have the formula: 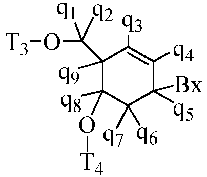 wherein:
Bx is a heterocyclic base moiety;
T₃ and T₄ are each, independently, an internucleoside linking group linking the cyclohexenyl nucleoside analog to an antisense compound or one of T₃ and T₄ is an internucleoside linking group linking the tetrahydropyran nucleoside analog to an antisense compound and the other of T₃ and T₄ is H, a hydroxyl protecting group, a linked conjugate group, or a 5'-or 3'-terminal group; and q₁, q₂, q₃, q₄, q₅, q₆, q₇, q₈ and q₉ are each, independently, H, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl or other sugar substituent group.

Many other bicyclic and tricyclic sugar surrogate ring systems are also known in the art that can be used to modify nucleosides for incorporation into antisense compounds (see for example review article: Leumann, Christian J., Bioorg. & Med. Chem., 2002, 10, 841-854). Such ring systems can undergo various additional substitutions to enhance activity.

Methods for the preparations of modified sugars are well known to those skilled in the art. Some representative U.S. patents that teach the preparation of such modified sugars include without limitation, U.S.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,670,633; 5,700,920; 5,792,847 and 6,600,032 and International Application PCT/US2005/019219, filed June 2, 2005 and published as WO 2005/121371 on December 22, 2005.

In nucleotides having modified sugar moieties, the nucleobase moieties (natural, modified or a combination thereof) are maintained for hybridization with an appropriate nucleic acid target.

In certain embodiments, antisense compounds targeted to a DMPK nucleic acid comprise one or more nucleotides having modified sugar moieties. In certain embodiments, the modified sugar moiety is 2'-MOE. In certain embodiments, the 2'-MOE modified nucleotides are arranged in a gapmer motif.

### Modified Nucleobases

Nucleobase (or base) modifications or substitutions are structurally distinguishable from, yet functionally interchangeable with, naturally occurring or synthetic unmodified nucleobases. Both natural and modified nucleobases are capable of participating in hydrogen bonding. Such nucleobase modifications can impart nuclease stability, binding affinity or some other beneficial biological property to antisense compounds. Modified nucleobases include synthetic and natural nucleobases such as, for example, 5-methylcytosine (5-me-C). Certain nucleobase substitutions, including 5-methylcytosine substitutions, are particularly useful for increasing the binding affinity of an antisense compound for a target nucleic acid. For example, 5-methylcytosine substitutions have been shown to increase nucleic acid duplex stability by 0.6-1.2°C (Sanghvi, Y.S., Crooke, S.T. and Lebleu, B., eds., Antisense Research and Applications, CRC Press, Boca Raton, 1993, pp. 276-278).

Additional unmodified nucleobases include 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine.

Heterocyclic base moieties can also include those in which the purine or pyrimidine base is replaced with other heterocycles, for example 7-deaza-adenine, 7-deazaguanosine, 2-aminopyridine and 2-pyridone. Nucleobases that are particularly useful for increasing the binding affinity of antisense compounds include 5-substituted pyrimidines, 6-azapyrimidines and N-2, N-6 and O-6 substituted purines, including 2 aminopropyladenine, 5-propynyluracil and 5-propynylcytosine.

In certain embodiments, antisense compounds targeted to a DMPK nucleic acid comprise one or more modified nucleobases. In certain embodiments, gap-widened antisense oligonucleotides targeted to a DMPK nucleic acid comprise one or more modified nucleobases. In certain embodiments, the modified nucleobase is 5-methylcytosine. In certain embodiments, each cytosine is a 5-methylcytosine.

### Certain Antisense Compound Motifs

In certain embodiments, antisense compounds targeted to a DMPK nucleic acid have chemically modified subunits arranged in patterns, or motifs, to confer to the antisense compounds properties such as enhanced the inhibitory activity, increased binding affinity for a target nucleic acid, or resistance to degradation by *in vivo* nucleases.

Chimeric antisense compounds typically contain at least one region modified so as to confer increased resistance to nuclease degradation, increased cellular uptake, increased binding affinity for the target nucleic acid, and/or increased inhibitory activity. A second region of a chimeric antisense compound can optionally serve as a substrate for the cellular endonuclease RNase H, which cleaves the RNA strand of an RNA:DNA duplex.

Antisense compounds having a gapmer motif are considered chimeric antisense compounds. In a gapmer an internal region having a plurality of nucleotides that supports RNaseH cleavage is positioned between external regions having a plurality of nucleotides that are chemically distinct from the nucleosides of the internal region. In the case of an antisense oligonucleotide having a gapmer motif, the gap segment generally serves as the substrate for endonuclease cleavage, while the wing segments comprise modified nucleosides. In certain embodiments, the regions of a gapmer are differentiated by the types of sugar moieties comprising each distinct region. The types of sugar moieties that are used to differentiate the regions of a gapmer can in some embodiments include β-D-ribonucleosides, β-D-deoxyribonucleosides, 2'-modified nucleosides (such 2'-modified nucleosides can include 2'-MOE, and 2'-O-CH₃, among others), and bicyclic sugar modified nucleosides (such bicyclic sugar modified nucleosides can include those having a 4'-(CH₂)ₙ-O-2' bridge, where n=1 or n=2). The wing-gap-wing motif is frequently described as "X-Y-Z", where "X" represents the length of the 5' wing region, "Y" represents the length of the gap region, and "Z" represents the length of the 3' wing region. As used herein, a gapmer described as "X-Y-Z" has a configuration such that the gap segment is positioned immediately adjacent each of the 5' wing segment and the 3' wing segment. Thus, no intervening nucleotides exist between the 5' wing segment and gap segment, or the gap segment and the 3' wing segment. Any of the antisense compounds described herein can have a gapmer motif. In some embodiments, X and Z are the same, in other embodiments they are different. In a preferred embodiment, Y is between 8 and 15 nucleotides. X, Y or Z can be any of 1, 2, 3, 4, 5, 6, 7, 8, 9, 10, 11, 12, 13, 14, 15, 16, 17, 18, 19, 20, 25, 30 or more nucleotides. Thus, gapmers include, but are not limited to, for example 5-10-5, 4-8-4, 4-12-3, 4-12-4, 3-14-3, 2-13-5, 2-16-2, 1-18-1, 3-10-3, 2-10-2, 1-10-1, 2-8-2, 6-8-6, 5-8-5, 5-7-5, 1-8-1, or 2-6-2.

In certain embodiments, the antisense compound as a "wingmer" motif, having a wing-gap or gap-wing configuration, i.e. an X-Y or Y-Z configuration as described above for the gapmer configuration. Thus, wingmer configurations include, but are not limited to, for example 5-10, 8-4, 4-12, 12-4, 3-14, 16-2, 18-1, 10-3, 2-10, 1-10, 8-2, 2-13, or 5-13.

In certain embodiments, antisense compounds targeted to a DMPK nucleic acid possess a 5-10-5 gapmer motif. In certain embodiments, antisense compounds targeted to a DMPK nucleic acid possess a 5-7-5 gapmer motif. In certain embodiments, antisense compounds targeted to a DMPK nucleic acid possess a 3-10-3 gapmer motif. In certain embodiments, antisense compounds targeted to a DMPK nucleic acid possess a 4-8-4 gapmer motif.

In certain embodiments, an antisense compound targeted to a DMPK nucleic acid has a gap-widened motif.

In certain embodiments, antisense compounds of any of these gapmer or wingmer motifs contain at least 8, at least 9, at least 10, at least 11, at least 12, at least 13, at least 14, at least 15, at least 16, at least 17, at least 18, or at least 19, contiguous nucleobases of the nucleobase sequence of any of the exemplary antisense compounds described herein (e.g., at least 8 contiguous nucleobases of a nucleobase sequence recited in any one of SEQ ID NOs: 23, 24, 25, 26, 27, 28, 29, 30, 31, 32, or 33- 874.

In certain embodiments, the present invention provides oligomeric compounds comprising oligonucleotides. In certain embodiments, such oligonucleotides comprise one or more chemical modification. In certain embodiments, chemically modified oligonucleotides comprise one or more modified sugars. In certain embodiments, chemically modified oligonucleotides comprise one or more modified nucleobases. In certain embodiments, chemically modified oligonucleotides comprise one or more modified internucleoside linkages. In certain embodiments, the chemically modifications (sugar modifications, nucleobase modifications, and/or linkage modifications) define a pattern or motif. In certain embodiments, the patterns of chemical modifications of sugar moieties, internucleoside linkages, and nucleobases are each independent of one another. Thus, an oligonucleotide may be described by its sugar modification motif, internucleoside linkage motif and/or nucleobase modification motif (as used herein, nucleobase modification motif describes the chemical modifications to the nucleobases independent of the sequence of nucleobases).

### Certain sugar motifs

In certain embodiments, oligonucleotides comprise one or more type of modified sugar moieties and/or naturally occurring sugar moieties arranged along an oligonucleotide or region thereof in a defined pattern or sugar modification motif. Such motifs may include any of the sugar modifications discussed herein and/or other known sugar modifications.

In certain embodiments, the oligonucleotides comprise or consist of a region having a gapmer sugar modification motif, which comprises two external regions or "wings" and an internal region or "gap." The three regions of a gapmer motif (the 5'-wing, the gap, and the 3'-wing) form a contiguous sequence of nucleosides wherein at least some of the sugar moieties of the nucleosides of each of the wings differ from at least some of the sugar moieties of the nucleosides of the gap. Specifically, at least the sugar moieties of the nucleosides of each wing that are closest to the gap (the 3'-most nucleoside of the 5'-wing and the 5'-most nucleoside of the 3'-wing) differ from the sugar moiety of the neighboring gap nucleosides, thus defining the boundary between the wings and the gap. In certain embodiments, the sugar moieties within the gap are the same as one another. In certain embodiments, the gap includes one or more nucleoside having a sugar moiety that differs from the sugar moiety of one or more other nucleosides of the gap. In certain embodiments, the sugar modification motifs of the two wings are the same as one another (symmetric gapmer). In certain embodiments, the sugar modification motifs of the 5'-wing differs from the sugar modification motif of the 3'-wing (asymmetric gapmer).

### Certain 5'-wings

In certain embodiments, the 5'- wing of a gapmer consists of 1 to 5 linked nucleosides. In certain embodiments, the 5'- wing of a gapmer consists of 2 to 5 linked nucleosides. In certain embodiments, the 5'-wing of a gapmer consists of 3 to 5 linked nucleosides. In certain embodiments, the 5' - wing of a gapmer consists of 4 or 5 linked nucleosides. In certain embodiments, the 5'- wing of a gapmer consists of 1 to 4 linked nucleosides. In certain embodiments, the 5'- wing of a gapmer consists of 1 to 3 linked nucleosides. In certain embodiments, the 5'- wing of a gapmer consists of 1 or 2 linked nucleosides. In certain embodiments, the 5'- wing of a gapmer consists of 2 to 4 linked nucleosides. In certain embodiments, the 5'-wing of a gapmer consists of 2 or 3 linked nucleosides. In certain embodiments, the 5'- wing of a gapmer consists of 3 or 4 linked nucleosides. In certain embodiments, the 5'- wing of a gapmer consists of 1 nucleoside. In certain embodiments, the 5'- wing of a gapmer consists of 2 linked nucleosides. In certain embodiments, the 5'- wing of a gapmer consists of 3linked nucleosides. In certain embodiments, the 5'- wing of a gapmer consists of 4 linked nucleosides. In certain embodiments, the 5'- wing of a gapmer consists of 5 linked nucleosides.

In certain embodiments, the 5'- wing of a gapmer comprises at least one bicyclic nucleoside. In certain embodiments, the 5'- wing of a gapmer comprises at least two bicyclic nucleosides. In certain embodiments, the 5'- wing of a gapmer comprises at least three bicyclic nucleosides. In certain embodiments, the 5'- wing of a gapmer comprises at least four bicyclic nucleosides. In certain embodiments, the 5'- wing of a gapmer comprises at least one constrained ethyl nucleoside. In certain embodiments, the 5'-wing of a gapmer comprises at least one LNA nucleoside. In certain embodiments, each nucleoside of the 5'-wing of a gapmer is a bicyclic nucleoside. In certain embodiments, each nucleoside of the 5'- wing of a gapmer is a constrained ethyl nucleoside. In certain embodiments, each nucleoside of the 5'- wing of a gapmer is a LNA nucleoside.

In certain embodiments, the 5'- wing of a gapmer comprises at least one non-bicyclic modified nucleoside. In certain embodiments, the 5'- wing of a gapmer comprises at least one 2'-substituted nucleoside. In certain embodiments, the 5'- wing of a gapmer comprises at least one 2'-MOE nucleoside. In certain embodiments, the 5'- wing of a gapmer comprises at least one 2'-OMe nucleoside. In certain embodiments, each nucleoside of the 5'- wing of a gapmer is a non-bicyclic modified nucleoside. In certain embodiments, each nucleoside of the 5'- wing of a gapmer is a 2'-substituted nucleoside. In certain embodiments, each nucleoside of the 5'- wing of a gapmer is a 2'-MOE nucleoside. In certain embodiments, each nucleoside of the 5' - wing of a gapmer is a 2'-OMe nucleoside.

In certain embodiments, the 5'-wing of a gapmer comprises at least one bicyclic nucleoside and at least one non-bicyclic modified nucleoside. In certain embodiments, the 5'-wing of a gapmer comprises at least one bicyclic nucleoside and at least one 2'-substituted nucleoside. In certain embodiments, the 5'-wing of a gapmer comprises at least one bicyclic nucleoside and at least one 2'-MOE nucleoside. In certain embodiments, the 5'-wing of a gapmer comprises at least one bicyclic nucleoside and at least one 2'-OMe nucleoside. In certain embodiments, the 5'-wing of a gapmer comprises at least one bicyclic nucleoside and at least one 2'-deoxynucleoside.

In certain embodiments, the 5'-wing of a gapmer comprises at least one constrained ethyl nucleoside and at least one non-bicyclic modified nucleoside. In certain embodiments, the 5'-wing of a gapmer comprises at least one constrained ethyl nucleoside and at least one 2'-substituted nucleoside. In certain embodiments, the 5'-wing of a gapmer comprises at least one constrained ethyl nucleoside and at least one 2'-MOE nucleoside. In certain embodiments, the 5'-wing of a gapmer comprises at least one constrained ethyl nucleoside and at least one 2'-OMe nucleoside. In certain embodiments, the 5'-wing of a gapmer comprises at least one constrained ethyl nucleoside and at least one 2'-deoxynucleoside.

In certain embodiments, the 5'-wing of a gapmer comprises at least one LNA nucleoside and at least one non-bicyclic modified nucleoside. In certain embodiments, the 5'-wing of a gapmer comprises at least one LNA nucleoside and at least one 2'-substituted nucleoside. In certain embodiments, the 5'-wing of a gapmer comprises at least one LNA nucleoside and at least one 2'-MOE nucleoside. In certain embodiments, the 5'-wing of a gapmer comprises at least one LNA nucleoside and at least one 2'-OMe nucleoside. In certain embodiments, the 5'-wing of a gapmer comprises at least one LNA nucleoside and at least one 2'-deoxynucleoside.

In certain embodiments, the 5'-wing of a gapmer comprises three constrained ethyl nucleosides. In certain embodiments, the 5'-wing of a gapmer comprises two bicyclic nucleosides and two non bicyclic modified nucleosides. In certain embodiments, the 5'-wing of a gapmer comprises two constrained ethyl nucleosides and two 2'-MOE nucleosides. In certain embodiments, the 5'-wing of a gapmer comprises two bicyclic nucleosides and two non bicyclic modified nucleosides. In certain embodiments, the 5'-wing of a gapmer comprises two constrained ethyl nucleosides and two 2'-MOE nucleosides. In certain embodiments, the 5'-wing of a gapmer comprises two constrained ethyl nucleosides and three 2'-MOE nucleosides.

In certain embodiments, the 5'-wing of a gapmer comprises three LNA nucleosides. In certain embodiments, the 5'-wing of a gapmer comprises two LNAnucleosides and two non bicyclic modified nucleosides. In certain embodiments, the 5'-wing of a gapmer comprises two LNA nucleosides and two 2'-MOE nucleosides. In certain embodiments, the 5'-wing of a gapmer comprises two LNA and two non bicyclic modified nucleosides. In certain embodiments, the 5'-wing of a gapmer comprises two LNA nucleosides and two 2'-MOE nucleosides. In certain embodiments, the 5'-wing of a gapmer comprises two LNA nucleosides and three 2'-MOE nucleosides.

In certain embodiments, the 5'-wing of a gapmer comprises three constrained ethyl nucleosides. In certain embodiments, the 5'-wing of a gapmer comprises two bicyclic nucleosides and two non bicyclic modified nucleosides. In certain embodiments, the 5'-wing of a gapmer comprises two constrained ethyl nucleosides and two 2'-OMe nucleosides. In certain embodiments, the 5'-wing of a gapmer comprises two bicyclic nucleosides and two non bicyclic modified nucleosides. In certain embodiments, the 5'-wing of a gapmer comprises two constrained ethyl nucleosides and two 2'-OMe nucleosides. In certain embodiments, the 5'-wing of a gapmer comprises two constrained ethyl nucleosides and three 2'-OMe nucleosides.

In certain embodiments, the 5'-wing of a gapmer comprises three LNA nucleosides. In certain embodiments, the 5'-wing of a gapmer comprises two LNAnucleosides and two non bicyclic modified nucleosides. In certain embodiments, the 5'-wing of a gapmer comprises two LNA nucleosides and two 2'-OMe nucleosides. In certain embodiments, the 5'-wing of a gapmer comprises two LNA and two non bicyclic modified nucleosides. In certain embodiments, the 5'-wing of a gapmer comprises two LNA nucleosides and two 2'-OMe nucleosides. In certain embodiments, the 5'-wing of a gapmer comprises two LNA nucleosides and three 2'-OMe nucleosides.

In certain embodiments, the 5'-wing of a gapmer has an AABB motif, wherein each A is selected from among a 2'-MOE nucleoside and a 2'OMe nucleoside. In certain embodiments, the 5'-wing of a gapmer has an AABB motif, wherein each B is selected from among a cEt, LNA, α-L-LNA, ENA and 2'-thio LNA nucleoside. In certain embodiments, the 5'-wing of a gapmer has an AABB motif, wherein each A represents a 2'-MOE nucleoside and each B represents a constrained ethyl nucleoside.

In certain embodiments, the 5'-wing of a gapmer has an AAABB motif, wherein each A is selected from among a 2'-MOE nucleoside and a 2'OMe nucleoside. In certain embodiments, the 5'-wing of a gapmer has an AABB motif, wherein each B is selected from among a cEt, LNA, α-L-LNA, ENA and 2'-thio LNA nucleoside. In certain embodiments, the 5'-wing of a gapmer has an AABB motif, wherein each A represents a 2'-MOE nucleoside and each B represents a constrained ethyl nucleoside.

### Certain 3'-wings

In certain embodiments, the 3'- wing of a gapmer consists of 1 to 5 linked nucleosides. In certain embodiments, the 3'- wing of a gapmer consists of 2 to 5 linked nucleosides. In certain embodiments, the 3'-wing of a gapmer consists of 3 to 5 linked nucleosides. In certain embodiments, the 3'- wing of a gapmer consists of 4 or 5 linked nucleosides. In certain embodiments, the 3'- wing of a gapmer consists of 1 to 4 linked nucleosides. In certain embodiments, the 3'- wing of a gapmer consists of 1 to 3 linked nucleosides. In certain embodiments, the 3'- wing of a gapmer consists of 1 or 2 linked nucleosides. In certain embodiments, the 3'- wing of a gapmer consists of 2 to 4 linked nucleosides. In certain embodiments, the 3'-wing of a gapmer consists of 2 or 3 linked nucleosides. In certain embodiments, the 3'- wing of a gapmer consists of 3 or 4 linked nucleosides. In certain embodiments, the 3'- wing of a gapmer consists of 1 nucleoside. In certain embodiments, the 3'- wing of a gapmer consists of 2 linked nucleosides. In certain embodiments, the 3'- wing of a gapmer consists of 3linked nucleosides. In certain embodiments, the 3'- wing of a gapmer consists of 4 linked nucleosides. In certain embodiments, the 3'- wing of a gapmer consists of 5 linked nucleosides.

In certain embodiments, the 3'- wing of a gapmer comprises at least one bicyclic nucleoside. In certain embodiments, the 3'- wing of a gapmer comprises at least one constrained ethyl nucleoside. In certain embodiments, the 3'- wing of a gapmer comprises at least one LNA nucleoside. In certain embodiments, each nucleoside of the 3'- wing of a gapmer is a bicyclic nucleoside. In certain embodiments, each nucleoside of the 3'- wing of a gapmer is a constrained ethyl nucleoside. In certain embodiments, each nucleoside of the 3'- wing of a gapmer is a LNA nucleoside.

In certain embodiments, the 3'- wing of a gapmer comprises at least one non-bicyclic modified nucleoside. In certain embodiments, the 3'- wing of a gapmer comprises at least two non-bicyclic modified nucleosides. In certain embodiments, the 3'- wing of a gapmer comprises at least three non-bicyclic modified nucleosides. In certain embodiments, the 3'- wing of a gapmer comprises at least four non-bicyclic modified nucleosides. In certain embodiments, the 3'- wing of a gapmer comprises at least one 2'-substituted nucleoside. In certain embodiments, the 3'- wing of a gapmer comprises at least one 2'-MOE nucleoside. In certain embodiments, the 3'- wing of a gapmer comprises at least one 2'-OMe nucleoside. In certain embodiments, each nucleoside of the 3'- wing of a gapmer is a non-bicyclic modified nucleoside. In certain embodiments, each nucleoside of the 3'- wing of a gapmer is a 2'-substituted nucleoside. In certain embodiments, each nucleoside of the 3'- wing of a gapmer is a 2'-MOE nucleoside. In certain embodiments, each nucleoside of the 3'- wing of a gapmer is a 2'-OMe nucleoside.

In certain embodiments, the 3'-wing of a gapmer comprises at least one bicyclic nucleoside and at least one non-bicyclic modified nucleoside. In certain embodiments, the 3'-wing of a gapmer comprises at least one bicyclic nucleoside and at least one 2'-substituted nucleoside. In certain embodiments, the 3'-wing of a gapmer comprises at least one bicyclic nucleoside and at least one 2'-MOE nucleoside. In certain embodiments, the 3'-wing of a gapmer comprises at least one bicyclic nucleoside and at least one 2'-OMe nucleoside. In certain embodiments, the 3'-wing of a gapmer comprises at least one bicyclic nucleoside and at least one 2'-deoxynucleoside.

In certain embodiments, the 3'-wing of a gapmer comprises at least one constrained ethyl nucleoside and at least one non-bicyclic modified nucleoside. In certain embodiments, the 3'-wing of a gapmer comprises at least one constrained ethyl nucleoside and at least one 2'-substituted nucleoside. In certain embodiments, the 3'-wing of a gapmer comprises at least one constrained ethyl nucleoside and at least one 2'-MOE nucleoside. In certain embodiments, the 3'-wing of a gapmer comprises at least one constrained ethyl nucleoside and at least one 2'-OMe nucleoside. In certain embodiments, the 3'-wing of a gapmer comprises at least one constrained ethyl nucleoside and at least one 2'-deoxynucleoside.

In certain embodiments, the 3'-wing of a gapmer comprises at least one LNA nucleoside and at least one non-bicyclic modified nucleoside. In certain embodiments, the 3'-wing of a gapmer comprises at least one LNA nucleoside and at least one 2'-substituted nucleoside. In certain embodiments, the 3'-wing of a gapmer comprises at least one LNA nucleoside and at least one 2'-MOE nucleoside. In certain embodiments, the 3'-wing of a gapmer comprises at least one LNA nucleoside and at least one 2'-OMe nucleoside. In certain embodiments, the 3'-wing of a gapmer comprises at least one LNA nucleoside and at least one 2'-deoxynucleoside.

In certain embodiments, the 3'-wing of a gapmer comprises three constrained ethyl nucleosides. In certain embodiments, the 3'-wing of a gapmer comprises two bicyclic nucleosides and two non bicyclic modified nucleosides. In certain embodiments, the 3'-wing of a gapmer comprises two constrained ethyl nucleosides and two 2'-MOE nucleosides. In certain embodiments, the 3'-wing of a gapmer comprises two bicyclic nucleosides and two non bicyclic modified nucleosides. In certain embodiments, the 3'-wing of a gapmer comprises two constrained ethyl nucleosides and two 2'-MOE nucleosides. In certain embodiments, the 5'-wing of a gapmer comprises two constrained ethyl nucleosides and three 2'-MOE nucleosides.

In certain embodiments, the 3'-wing of a gapmer comprises three LNA nucleosides. In certain embodiments, the 3'-wing of a gapmer comprises two LNAnucleosides and two non bicyclic modified nucleosides. In certain embodiments, the 3'-wing of a gapmer comprises two LNA nucleosides and two 2'-MOE nucleosides. In certain embodiments, the 3'-wing of a gapmer comprises two LNA and two non bicyclic modified nucleosides. In certain embodiments, the 3'-wing of a gapmer comprises two LNA nucleosides and two 2'-MOE nucleosides. In certain embodiments, the 3'-wing of a gapmer comprises two LNA nucleosides and three 2'-MOE nucleosides.

In certain embodiments, the 3'-wing of a gapmer comprises three constrained ethyl nucleosides. In certain embodiments, the 3'-wing of a gapmer comprises two bicyclic nucleosides and two non bicyclic modified nucleosides. In certain embodiments, the 3'-wing of a gapmer comprises two constrained ethyl nucleosides and two 2'-OMe nucleosides. In certain embodiments, the 3'-wing of a gapmer comprises two bicyclic nucleosides and two non bicyclic modified nucleosides. In certain embodiments, the 3'-wing of a gapmer comprises two constrained ethyl nucleosides and two 2'-OMe nucleosides. In certain embodiments, the 3'-wing of a gapmer comprises two constrained ethyl nucleosides and three 2'-OMe nucleosides.

In certain embodiments, the 3'-wing of a gapmer comprises three LNA nucleosides. In certain embodiments, the 3'-wing of a gapmer comprises two LNAnucleosides and two non bicyclic modified nucleosides. In certain embodiments, the 3'-wing of a gapmer comprises two LNA nucleosides and two 2'-OMe nucleosides. In certain embodiments, the 3'-wing of a gapmer comprises two LNA and two non bicyclic modified nucleosides. In certain embodiments, the 3'-wing of a gapmer comprises two LNA nucleosides and two 2'-OMe nucleosides. In certain embodiments, the 3'-wing of a gapmer comprises two LNA nucleosides and three 2'-OMe nucleosides.

In certain embodiments, the 3'-wing of a gapmer has a BBAA motif, wherein each A is selected from among a 2'-MOE nucleoside and a 2'OMe nucleoside. In certain embodiments, the 3'-wing of a gapmer has an BBAA motif, wherein each B is selected from among a cEt, LNA, α-L-LNA, ENA and 2'-thio LNA nucleoside. In certain embodiments, the 3'-wing of a gapmer has a BBAA motif, wherein each A represents a 2'-MOE nucleoside and each B represents a constrained ethyl nucleoside.

In certain embodiments, the 3'-wing of a gapmer has a BBAAA motif, wherein each A is selected from among a 2'-MOE nucleoside and a 2'OMe nucleoside. In certain embodiments, the 3'-wing of a gapmer has a BBAA motif, wherein each B is selected from among a cEt, LNA, α-L-LNA, ENA and 2'-thio LNA nucleoside. In certain embodiments, the 3'-wing of a gapmer has a BBAA motif, wherein each A represents a 2'-MOE nucleoside and each B represents a constrained ethyl nucleoside.

### Compositions and Methods for Formulating Pharmaceutical Compositions

Antisense oligonucleotides can be admixed with pharmaceutically acceptable active or inert substance for the preparation of pharmaceutical compositions or formulations. Compositions and methods for the formulation of pharmaceutical compositions are dependent upon a number of criteria, including, but not limited to, route of administration, extent of disease, or dose to be administered.

Antisense compound targeted to a DMPK nucleic acid can be utilized in pharmaceutical compositions by combining the antisense compound with a suitable pharmaceutically acceptable diluent or carrier. A pharmaceutically acceptable diluent includes phosphate-buffered saline (PBS). PBS is a diluent suitable for use in compositions to be delivered parenterally. Accordingly, in one embodiment, employed in the methods described herein is a pharmaceutical composition comprising an antisense compound targeted to a DMPK nucleic acid and a pharmaceutically acceptable diluent. In certain embodiments, the pharmaceutically acceptable diluent is PBS. In certain embodiments, the antisense compound is an antisense oligonucleotide.

Pharmaceutical compositions comprising antisense compounds encompass any pharmaceutically acceptable salts, esters, or salts of such esters, or any other oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

A prodrug can include the incorporation of additional nucleosides at one or both ends of an antisense compound which are cleaved by endogenous nucleases within the body, to form the active antisense compound.

### Conjugated Antisense Compounds

Antisense compounds can be covalently linked to one or more moieties or conjugates which enhance the activity, cellular distribution or cellular uptake of the resulting antisense oligonucleotides. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

Antisense compounds can also be modified to have one or more stabilizing groups that are generally attached to one or both termini of antisense compounds to enhance properties such as, for example, nuclease stability. Included in stabilizing groups are cap structures. These terminal modifications protect the antisense compound having terminal nucleic acid from exonuclease degradation, and can help in delivery and/or localization within a cell. The cap can be present at the 5'-terminus (5'-cap), or at the 3'-terminus (3'-cap), or can be present on both termini. Cap structures are well known in the art and include, for example, inverted deoxy abasic caps. Further 3' and 5'-stabilizing groups that can be used to cap one or both ends of an antisense compound to impart nuclease stability include those disclosed in WO 03/004602 published on January 16, 2003.

### Cell culture and antisense compounds treatment

The effects of antisense compounds on the level, activity or expression of DMPK nucleic acids can be tested in vitro in a variety of cell types. Cell types used for such analyses are available from commercial vendors (e.g. American Type Culture Collection, Manassus, VA; Zen-Bio, Inc., Research Triangle Park, NC; Clonetics Corporation, Walkersville, MD) and cells are cultured according to the vendor's instructions using commercially available reagents (e.g. Invitrogen Life Technologies, Carlsbad, CA). Illustrative cell types include, but are not limited to, HepG2 cells, Hep3B cells, primary hepatocytes, A549 cells, GM04281 fibroblasts and LLC-MK2 cells.

### In vitro testing of antisense oligonucleotides

Described herein are methods for treatment of cells with antisense oligonucleotides, which can be modified appropriately for treatment with other antisense compounds.

In general, cells are treated with antisense oligonucleotides when the cells reach approximately 60-80% confluence in culture.

One reagent commonly used to introduce antisense oligonucleotides into cultured cells includes the cationic lipid transfection reagent LIPOFECTIN® (Invitrogen, Carlsbad, CA). Antisense oligonucleotides are mixed with LIPOFECTIN® in OPTI-MEM® 1 (Invitrogen, Carlsbad, CA) to achieve the desired final concentration of antisense oligonucleotide and a LIPOFECTIN® concentration that typically ranges 2 to 12 ug/mL per 100 nM antisense oligonucleotide.

Another reagent used to introduce antisense oligonucleotides into cultured cells includes LIPOFECTAMINE 2000® (Invitrogen, Carlsbad, CA). Antisense oligonucleotide is mixed with LIPOFECTAMINE 2000® in OPTI-MEM® 1 reduced serum medium (Invitrogen, Carlsbad, CA) to achieve the desired concentration of antisense oligonucleotide and a LIPOFECTAMINE® concentration that typically ranges 2 to 12 ug/mL per 100 nM antisense oligonucleotide.

Another reagent used to introduce antisense oligonucleotides into cultured cells includes Cytofectin® (Invitrogen, Carlsbad, CA). Antisense oligonucleotide is mixed with Cytofectin® in OPTI-MEM® 1 reduced serum medium (Invitrogen, Carlsbad, CA) to achieve the desired concentration of antisense oligonucleotide and a Cytofectin® concentration that typically ranges 2 to 12 ug/mL per 100 nM antisense oligonucleotide.

Another technique used to introduce antisense oligonucleotides into cultured cells includes electroporation.

Cells are treated with antisense oligonucleotides by routine methods. Cells are typically harvested 16-24 hours after antisense oligonucleotide treatment, at which time RNA or protein levels of target nucleic acids are measured by methods known in the art and described herein. In general, when treatments are performed in multiple replicates, the data are presented as the average of the replicate treatments.

The concentration of antisense oligonucleotide used varies from cell line to cell line. Methods to determine the optimal antisense oligonucleotide concentration for a particular cell line are well known in the art. Antisense oligonucleotides are typically used at concentrations ranging from 1 nM to 300 nM when transfected with LIPOFΓCTAMINF2000®, Lipofectin or Cytofectin. Antisense oligonucleotides are used at higher concentrations ranging from 625 to 20,000 nM when transfected using electroporation.

### RNA Isolation

RNA analysis can be performed on total cellular RNA or poly(A)+ mRNA. Methods of RNA isolation are well known in the art. RNA is prepared using methods well known in the art, for example, using the TRIZOL® Reagent (Invitrogen, Carlsbad, CA) according to the manufacturer's recommended protocols.

### Analysis of inhibition of target levels or expression

Inhibition of levels or expression of a DMPK nucleic acid can be assayed in a variety of ways known in the art. For example, target nucleic acid levels can be quantitated by, e.g., Northern blot analysis, competitive polymerase chain reaction (PCR), or quantitaive real-time PCR. RNA analysis can be performed on total cellular RNA or poly(A)+ mRNA. Methods of RNA isolation are well known in the art. Northern blot analysis is also routine in the art. Quantitative real-time PCR can be conveniently accomplished using the commercially available ABI PRISM® 7600, 7700, or 7900 Sequence Detection System, available from PE-Applied Biosystems, Foster City, CA and used according to manufacturer's instructions.

### Quantitative Real-Time PCR Analysis of Target RNA Levels

Quantitation of target RNA levels can be accomplished by quantitative real-time PCR using the ABI PRISM® 7600, 7700, or 7900 Sequence Detection System (PE-Applied Biosystems, Foster City, CA) according to manufacturer's instructions. Methods of quantitative real-time PCR are well known in the art.

Prior to real-time PCR, the isolated RNA is subjected to a reverse transcriptase (RT) reaction, which produces complementary DNA (cDNA) that is then used as the substrate for the real-time PCR amplification. The RT and real-time PCR reactions are performed sequentially in the same sample well. RT and real-time PCR reagents are obtained from Invitrogen (Carlsbad, CA). RT, real-time-PCR reactions are carried out by methods well known to those skilled in the art.

Gene (or RNA) target quantities obtained by real time PCR are normalized using either the expression level of a gene whose expression is constant, such as cyclophilin A, or by quantifying total RNA using RIBOGREEN® (Invitrogen, Inc. Carlsbad, CA). Cyclophilin A expression is quantified by real time PCR, by being run simultaneously with the target, multiplexing, or separately. Total RNA is quantified using RIBOGREEN® RNA quantification reagent (Invitrogen, Inc. Eugene, OR). Methods of RNA quantification by RIBOGREEN® are taught in Jones, L.J., et al, (Analytical Biochemistry, 1998, 265, 368-374). A CYTOFLUOR® 4000 instrument (PE Applied Biosystems) is used to measure RIBOGREEN® fluorescence.

Probes and primers are designed to hybridize to a DMPK nucleic acid. Methods for designing real-time PCR probes and primers are well known in the art, and can include the use of software such as PRIMER FXPRFSS® Software (Applied Biosystems, Foster City, CA).

### Analysis of Protein Levels

Antisense inhibition of DMPK nucleic acids can be assessed by measuring DMPK protein levels. Protein levels of DMPK can be evaluated or quantitated in a variety of ways well known in the art, such as immunoprecipitation, Western blot analysis (immunoblotting), enzyme-linked immunosorbent assay (ELISA), quantitative protein assays, protein activity assays (for example, caspase activity assays), immunohistochemistry, immunocytochemistry or fluorescence-activated cell sorting (FACS). Antibodies directed to a target can be identified and obtained from a variety of sources, such as the MSRS catalog of antibodies (Aerie Corporation, Birmingham, MI), or can be prepared via conventional monoclonal or polyclonal antibody generation methods well known in the art.

### In vivo testing of antisense compounds

Antisense compounds, for example, antisense oligonucleotides, are tested in animals to assess their ability to inhibit expression of DMPK and produce phenotypic changes. Testing can be performed in normal animals, or in experimental disease models, for example, the HSA^{LR} mouse model of myotonic dystrophy (DM1).

The HSA^{LR} mouse model is an established model for DM1 (Mankodi, A. et al. Science. 289: 1769, 2000). The mice carry a human skeletal actin (*hACTA1*) transgene with 220 CTG repeats inserted in the 3' UTR of the gene. The *hACTA1*-CUG^{exp} transcript accumulates in nuclear foci in skeletal muscles and results in myotonia similar to that in human DM1 (Mankodi, A. et al. Mol. Cell 10: 35, 2002; Lin, X. et al. Hum. Mol. Genet. 15: 2087, 2006). Hence, it is expected that amelioration of DM1 symptoms in the HSA^{LR} mouse by antisense inhibition of the *hACTA1* transgene would predict amelioration of similar symptoms in human patients by antisense inhibition of the DMPK transcript.

Expression of CUG^{exp} RNA in mice causes extensive remodeling of the muscle transcriptome, much of which is reproduced by ablation of MBNL1. Hence, it is expected that normalization of the transcriptome in HSA^{LR} mice would predict normalization of the human transcriptome in DM1 patients by antisense inhibition of the DMPK transcript.

For administration to animals, antisense oligonucleotides are formulated in a pharmaceutically acceptable diluent, such as phosphate-buffered saline. Administration includes parenteral routes of administration. Following a period of treatment with antisense oligonucleotides, RNA is isolated from tissue and changes in DMPK nucleic acid expression are measured. Changes in DMPK protein levels are also measured.

### Splicing

Myotonic dystrophy (DM1) is caused by CTG repeat expansions in the 3' untranslated region of the DMPK gene (Brook, J.D. et al. Cell. 68: 799, 1992). This mutation leads to RNA dominance, a process in which expression of RNA containing an expanded CUG repeat (CUGexp) induces cell dysfunction (Osborne RJ and Thornton CA., Human Molecular Genetics., 2006, 15(2): R162-R169). Such CUGexp are retained in the nuclear foci of skeletal muscles (Davis, B.M. et al. Proc. Natl. Acad. Sci. U.S.A. 94:7388, 1997). The accumulation of CUGexp in the nuclear foci leads to the sequestration of poly(CUG)-binding proteins, such as, Muscleblind-like 1 (MBLN1) (Miller, J.W. et al. EMBO J. 19: 4439, 2000). MBLN1 is a splicing factor and regulates the splicing of genes such as Serca1, CIC-1, Titin, and Zasp. Therefore, sequestration of MBLN1 by CUGexp triggers misregulated alternative splicing of the exons of genes that MBLN1 normally controls (Lin, X. et al. Hum. Mol. Genet. 15: 2087, 2006). Correction of alternative splicing in an animal displaying such disregulation, such as, for example, in a DM1 patient and the HSALR mouse model, is a useful indicator for the efficacy of a treatment, including treatment with an antisense oligonucleotide.

### Certain Antisense Mechanisms

Myotonic dystrophy (DM1) is caused by CTG repeat expansions in the 3' untranslated region of the DMPK gene. In certain embodiments, expansions in the 3' untranslated region of the DMPK gene results in the transcription of RNA containing an expanded CUG repeat, and RNA containing an expanded CUG repeat (CUGexp) is retained in the nuclear foci of skeletal muscles. In certain instances, the cellular machinery responsible for exporting mRNA from the nucleus into the cytoplasm does not export RNA containing an expanded CUG repeat from the nucleus or does so less efficiently. In certain embodiments, cells do not export DMPK CUGexp mRNA from the nucleus or such export is reduced. Accordingly, in certain embodiments, DMPK CUGexp mRNA accumulates in the nucleus. In certain embodiments, more copies of DMPK CUGexp mRNA are present in the nucleus of a cell than are copies of wild-type DMPK mRNA, which is exported normally. In such embodiments, antisense compounds that reduce target in the nucleus will preferentially reduce mutant DMPK CUGexp mRNA relative to wild type DMPK mRNA, due to their relative abundences in the nucleus, even if the antisense compound does not otherwise distinguish between mutant and wild type. Since RNase H dependent antisense compounds are active in the nucleus, such compounds are particularly well suited for such use.

In certain instances, wild-type DMPK pre-mRNA and mutant CUGexp DMPK pre-mRNA are expected to be processed into mRNA at similar rate. Accordingly, approximately the same amount of wild-type DMPK pre-mRNA and mutant CUGexp DMPK pre-mRNA are expected to be present in the nucleus of a cell. However, after proceesing, wild type DMPK mRNA is exported from the nucleus relatively quickly, and mutant CUGexp DMPK mRNA is exported slowly or not at all. In certan such embodiments, mutant CUGexp DMPK mRNA accumulates in the nucleus in greater amounts than wild-type DMPK mRNA. In certain such embodiments, an antisense oligonucleotide targeted to the mRNA, will preferentially reduce the expression of the mutant CUGexp DMPK mRNA compared to the wild-type DMPK mRNA because more copies of the mutant CUGexp DMPK mRNA are present in the nucleus of the cell. In certain instances, antisense compounds targeted to pre-mRNA and not mRNA (e.g., targeting an intron) are not expected to preferentially reduce mutant DMPK relative to wild type, because the nuclear abundance of the two pre-mRNAs is likely to be similar. In certain embodiments, antisense compounds described herein are not targeted to introns of DMPK pre-mRNA. In certain embodiments, antisense compounds described herein are targeted to exons or exon-exon junctions present in DMPK mRNA. In certain embodiments, use of an antisense oligonucleotide to target the mRNA is therefore preferred because an antisense oligonucleotide having one or more features described herein (i) has activity in the nucleus of a cell and (2) will preferentially reduce mutant CUGexp DMPK mRNA compared to wild-type DMPK mRNA.

### Certain Biomarkers

DM1 severity in mouse models is determined, at least in part, by the level of CUG^{exp} transcript accumulation in the nucleus or nuclear foci. A useful physiological marker for DM1 severity is the development of high-frequency runs of involuntary action potentials (myotonia).

### Certain Indications

Disclosed herein are methods of treating an individual comprising administering one or more pharmaceutical compositions as described herein. In certain instances, the individual has type 1 myotonic dystrophy (DM1).

Accordingly, dislcosedherein are methods for ameliorating a symptom associated with type 1 myotonic dystrophy in a subject in need thereof. Also disclosed herein is a method for reducing the rate of onset of a symptom associated with type 1 myotonic dystrophy. Also disclosed herein is a method for reducing the severity of a symptom associated with type 1 myotonic dystrophy. In certain instances, symptoms associated with DM1 include muscle stiffness, myotonia, disabling distal weakness, weakness in face and jaw muscles, difficulty in swallowing, drooping of the eyelids (ptosis), weakness of neck muscles, weakness in arm and leg muscles, persistent muscle pain, hypersomnia, muscle wasting, dysphagia, respiratory insufficiency, irregular heartbeat, heart muscle damage, apathy, insulin resistance, and cataracts. In children, the symptoms may also be developmental delays, learning problems, language and speech issues, and personality development issues.

In certain instances, the methods comprise administering to an individual in need thereof a therapeutically effective amount of a compound targeted to a DMPK nucleic acid.

In certain intances, administration of an antisense compound targeted to a DMPK nucleic acid results in reduction of DMPK expression by at least about 15%, by at least about 20%, by at least about 25%, by at least about 30%, by at least about 35%, by at least about 40%, by at least about 45%, by at least about 50%, by at least about 55%, by at least about 60%, by least about 65%, by least about 70%, by least about 75%, by least about 80%, by at least about 85%, by at least about 90%, by at least about 95% or by at least about 99%, or a range defined by any two of these values.

In certain instances, pharmaceutical compositions comprising an antisense compound targeted to DMPK are used for the preparation of a medicament for treating a patient suffering or susceptible to type 1 myotonic dystrophy.

In certain instances, the methods described herein include administering a compound comprising a modified oligonucleotide having a contiguous nucleobases portion as described herein of a sequence recited in SEQ ID NOs: 23, 24, 25, 26, 27, 28, 29, 30, 31, 32, or 33- 874.

### Administration

In certain instances, the compounds and compositions as described herein are administered parenterally.

In certain instances, parenteral administration is by infusion. Infusion can be chronic or continuous or short or intermittent. In certain instances, infused pharmaceutical agents are delivered with a pump. In certain instances, parenteral administration is by injection (e.g., bolus injection). The injection can be delivered with a syringe.

Parenteral administration includes subcutaneous administration, intravenous administration, intramuscular administration, intraarterial administration, intraperitoneal administration, or intracranial administration, e.g., intrathecal or intracerebroventricular administration. Administration can be continuous, or chronic, or short, or intermittent.

In certain instances, the administering is subcutaneous, intravenous, intracerebral, intracerebroventricular, intrathecal or another administration that results in a systemic effect of the oligonucleotide (systemic administration is characterized by a systemic effect, i.e., an effect in more than one tissue) or delivery to the CNS or to the CSF.

The duration of action as measured by inhibition of alpha 1 actin and reduction of myotonia in the HSA^{LR} mouse model of DM1 is prolonged in muscle tissue including quadriceps, gastrocnemius, and the tibialis anterior (see Examples, below). Subcutaneous injections of antisense oligonucleotide for 4 weeks results in inhibition of alpha 1 actin by at least 70% in quadriceps, gastrocnemius, and the tibialis anterior in HSA^{LR} mice for at least 11 weeks (77 days) after termination of dosing. Subcutaneous injections of antisense oligonucleotide for 4 weeks results in elimination of myotonia in quadriceps, gastrocnemius, and the tibialis anterior in HSA^{LR} mice for at least 11 weeks (77 days) after termination of dosing.

In certain instances, delivery of a compound of composition, as described herein, results in at least 70% down-regulation of a target mRNA and/or target protein for at least 77 days. In certain instances, delivery of a compound or composition, as described herein, results in 50%, 55%, 60%, 65%, 70%, 75%, 80%, 85%, 90%, 95%, or 100% down-regulation of a target mRNA and/or target protein for at least 30 days, at least 35 days, at least 40 days, at least 45 days, at least 50 days, at least 55 days, at least 60 days, at least 65 days, at least 70 days, at least 75 days, at least 76 days, at least 77 days, at least 78 days, at least 79 days, at least 80 days, at least 85 days, at least 90 days, at least 95 days, at least 100 days, at least 105 days, at least 110 days, at least 115 days, at least 120 days, at least 1 year.

In certain instances, an antisense oligonucleotide is delivered by injection or infusion once every 77 days. In certain instances, an antisense oligonucleotide is delivered by injection or infusion once every month, every two months, every three months, every 6 months, twice a year or once a year.

### Certain Combination Therapies

In certain instances, a first agent comprising a modified oligonucleotide disclosed herein is co-administered with one or more secondary agents. In certain instances, such second agents are designed to treat the same type 1 myotonic dystrophy as the first agent described herein. In certain instances, such second agents are designed to treat a different disease, disorder, or condition as the first agent described herein. In certain instances, such second agents are designed to treat an undesired side effect of one or more pharmaceutical compositions as described herein. In certain instances, second agents are co-administered with the first agent to treat an undesired effect of the first agent. In certain instances, second agents are co-administered with the first agent to produce a combinational effect. In certain instances, second agents are co-administered with the first agent to produce a synergistic effect.

In certain instances, a first agent and one or more second agents are administered at the same time. In certain instances, the first agent and one or more second agents are administered at different times. In certain instances, the first agent and one or more second agents are prepared together in a single pharmaceutical formulation. In certain instances, the first agent and one or more second agents are prepared separately.

### Certain Comparator Compounds

In certain instances, the compounds disclosed herein benefit from one or more improved *in vitro* and/or *in vivo* properties relative to an appropriate comparator compound.

In certain embodiments, ISIS 445569, a 5-10-5 MOE gapmer, having a sequence of (from 5' to 3') CGGAGCGGTTGTGAACTGGC (incorporated herein as SEQ ID NO: 24), wherein each internucleoside linkage is a phosphorothioate linkage, each cytosine is a 5-methylcytosine, and each of nucleosides 1-5 and 16-20 comprise a 2'-O-methoxyethyl moiety, which was previously described in WO 2012/012443, is a comparator compound.

ISIS 445569 is an appropriate representative comparator compound because ISIS 445569 demonstrates statistically significant reduction of human DMPK in vitro as measured using a plurality of primer probe sets *(see* e.g. Example 1 and Example 2 of WO 2012/012443). Additionally, ISIS 445569 demonstrates statistically significant dose-dependent inhibition of human DMPK in vitro in both human skeletal muscle cells and DM1 fibroblasts *(see* e.g. Example 4 and Example 5 of WO 2012/012443 and Example 28 of WO 2012/012467). ISIS 445569 also reduces human DMPK transcript expression in transgenic mice (Examples 23 and 24 of WO 2012/012443 and Examples 29 and 30 of WO 2012/012467). ISIS 445569 was a preferred human DMPK antisense compound in WO 2012/012443 and WO 2012/012467.

### Certain Compounds

In certain embodiments, the compounds disclosed herein benefit from improved activity and/or improved tolerability relative to appropriate comparator compounds, such as ISIS 445569. For example, in certain embodiments, ISIS 598769, ISIS 598768, and/or ISIS 486178 have more activity and/or tolerability than appropriate comparator compounds, such as ISIS 445569.

In certain embodiments, the compounds disclosed herein are more potent than appropriate comparator compounds, such as ISIS 445569. For example, as provided in Example 10 (described herein), ISIS 598769 achieved an IC₅₀ of 1.9 µM, ISIS 598768 achieved an IC₅₀ of 1.2 µM, and ISIS 486178 achieved an IC₅₀ of 0.7 µM in a 6 point dose response curve (61.7 nM, 185.2 nM, 555.6 nM, 1666.7 nM, 5000.0 nM, and 15000.0 nM) in cultured in HepG2 cells when transfected using electroporation, whereas ISIS 445569 achieved an IC₅₀ of 2.3 µM. Thus, ISIS 598769, ISIS 598768, and ISIS 486178 are more potent than the comparator compound, ISIS 445569.

In certain embodiments, the compounds disclosed herein have greater activity than appropriate comparator compounds, such as ISIS 445569, at achieving dose-dependent inhibition of DMPK across multiple different muscle tissues. In another example, as provided in Example 16 (described herein), ISIS 598768 and ISIS 598769 achieved greater dose-dependent inhibition than the comparator compound ISIS 445569 across several different muscle tissues when administered subcutaneously to DMSXL transgenic mice twice a week for 4 weeks with 25 mg/kg/week, 50 mg/kg/wk, or 100 mg/kg/wk. In some muscle tissues, for example, in the tibialis anterior, both ISIS 598768 and ISIS 598769 achieved greater inhibition of DMPK at 25, 50 and 100 mg/kg/wk than ISIS 445569 achieved at 200 mg/kg/wk. In the quadriceps and gastrocnemius, both ISIS 598768 and ISIS 598769 achieved equal or greater inhibition of DMPK at 50 mg/kg/wk than ISIS 445569 achieved at 100 or 200 mg/kg/wk. Thus, ISIS 598768 and ISIS 598769 have greater activity than ISIS 445569 at achieving dose-dependent inhibition of DMPK across multiple different muscle tissues.

In certain embodiments, the compounds disclosed herein are more tolerable than appropriate comparator compounds, such as ISIS 445569, when administered to CD-1 mice. In another example, as provided in Example 17 (described herein), ISIS 598769, ISIS 598768, and ISIS 486178 exhibited more favorable tolerability markers than ISIS 445569 when administered to CD-1 mice. ISIS 598769, ISIS 598768, and ISIS 486178 were administered subcutaneously twice a week for 6 weeks at 50 mg/kg/wk. ISIS 445569 was administered subcutaneously twice a week for 6 weeks at 100mg/kg/wk. After treatment, ALT, AST, and BUN levels were lower in ISIS 486178 and ISIS 598768 treated mice than in ISIS 445569 treated mice. After treatment, ALT and AST levels were lower in ISIS 598769 treated mice than in ISIS 445569 treated mice. Therefore, ISIS 598769, ISIS 598768, and ISIS 486178 are more tolerable than the comparator compound, ISIS 445569 in CD-1 mice.

In certain embodiments, the compounds disclosed herein are more tolerable than appropriate comparator compounds, such as ISIS 445569, when administered to Sprague-Dawley rats. In another example, as provided in Example 18 (described herein), ISIS 598769, ISIS 598768, and ISIS 486178 exhibited more favorable tolerability markers than ISIS 445569 when administered to Sprague-Dawley rats. ISIS 598769, ISIS 598768, and ISIS 486178 were administered subcutaneously twice a week for 6 weeks at 50 mg/kg/wk. ISIS 445569 was administered subcutaneously twice a week for 6 weeks at 100mg/kg/wk. After treatment, ALT and AST levels were lower in ISIS 486178, ISIS 598769, and ISIS 598768 treated mice than in ISIS 445569 treated mice. Therefore ISIS 598769, ISIS 598768, and ISIS 486178 are more tolerable than the comparator compound, ISIS 445569 in Sprague-Dawley rats.

In certain embodiments, the compounds disclosed herein exhibit more favorable tolerability markers in cynomolgous monkeys than appropriate comparator compounds, such as ISIS 445569. In another example, as provided in Example 19 (described herein), ISIS 598769, ISIS 598768, and ISIS 486178 exhibited more favorable tolerability markers in cynomolgous monkeys including Alanine aminotransferase (ALT), aspartate aminotransferase (AST), lactate dehydrogenase (LDH), and creatine kinase (CK) assessment. In certain embodiments, ALT and AST levels are used as indicators of hepatotoxicity. For example, in certain embodiments, elevated ALT and AST levels indicate trauma to liver cells. In certain embodiments, elevated CK levels are associated with damage to cells in muscle tissue. In certain embodiments, elevated LDH levels are associated with cellular tissue damage.

In certain embodiments, the compounds disclosed herein are more tolerable than appropriate comparator compounds, such as ISIS 445569, when administered to cynomolgous monkeys. As provided in Example 19, groups of cynomolgous monkeys were treated with 40 mg/kg/wk of ISIS 598769, ISIS 598768, ISIS 486178, and ISIS 445569. Treatment with ISIS 445569 resulted in elevated ALT and AST levels at 93 days into treatment. Treatment with ISIS 598768, and ISIS 486178 resulted in lower ALT and AST levels at 58 and 93 days into treatment compared to ISIS 445569. Treatment with ISIS 598769, resulted in lower AST levels at 58 and 93 days into treatment and lower ALT levels at 93 days of treatment compared to ISIS 445569. Furthermore, the ALT and AST levels of monkeys receiving doses of ISIS 598769, ISIS 598768, and ISIS 486178 were consistent with the ALT and AST levels of monkeys given saline. Treatment with ISIS 445569 resulted in elevated LDH levels compared to the LDH levels measured in animals given ISIS 598769, ISIS 598768, and ISIS 486178 at 93 days into treatment. Additionally, treatment with ISIS 445569 resulted in elevated CK levels compared to the CK levels measured in animals given ISIS 598769, ISIS 598768, and ISIS 486178 at 93 days into treatment. Therefore, ISIS 598769, ISIS 598768, and ISIS 486178 are more tolerable than the comparator compound, ISIS 445569.

As the data discussed above demonstrate, ISIS 598769, ISIS 598768, and ISIS 486178 possess a wider range of well-tolerated doses at which ISIS 598769, ISIS 598768, and ISIS 486178 are active compared to the comparator compound, ISIS 445569. Additionally, the totality of the data presented in the examples herein and discussed above demonstrate that each of ISIS 598769, ISIS 598768, and ISIS 486178 possess a number of safety and activity advantages over the comparator compound, ISIS 445569. In other words, each of ISIS 598769, ISIS 598768, and ISIS 486178 are likely to be safer and more active drugs in humans than ISIS 445569.

In certain embodiments, ISIS 445569 is likely to be a safer and more active drug in humans for reducing CUGexp DMPK mRNA and\or treating conditions or symptoms associated with having myotonic dystrophy type 1 than the other compounds disclosed in WO 2012/012443 and/or WO 2012/012467.

In certain embodiments, ISIS 512497 has a better safety profile in primates and CD-1 mice than ISIS 445569. In certain embodiments, ISIS 512497 achieves greater knockdown of human DMPK nucleic acid in multiple muscle tissues when administered at the same dose and at lower doses than ISIS 445569.

In certain embodiments, ISIS 598768 achieves greater knockdown of human DMPK nucleic acid in one or more muscle tissues when administered at the same dose as ISIS 445569. In certain embodiments, ISIS 598768 has a better safety profile in primates than ISIS 445569.

In certain embodiments, ISIS 598769 achieves greater knockdown of human DMPK nucleic acid in one or more muscle tissues when administered at the same dose as ISIS 445569. In certain embodiments, ISIS 598769 has a better safety profile in primates than ISIS 445569.

### Nonlimiting disclosure

While certain compounds, compositions and methods described herein have been described with specificity in accordance with certain embodiments, the following examples serve only to illustrate the compounds described herein and are not intended to limit the same. Although the sequence listing accompanying this filing identifies each sequence as either "RNA" or "DNA" as required, in reality, those sequences may be modified with any combination of chemical modifications. One of skill in the art will readily appreciate that such designation as "RNA" or "DNA" to describe modified oligonucleotides is, in certain instances, arbitrary. For example, an oligonucleotide comprising a nucleoside comprising a 2'-OH sugar moiety and a thymine base could be described as a DNA having a modified sugar (2'-OH for the natural 2'-H of DNA) or as an RNA having a modified base (thymine (methylated uracil) for natural uracil of RNA).

Accordingly, nucleic acid sequences provided herein, including, but not limited to those in the sequence listing, are intended to encompass nucleic acids containing any combination of natural or modified RNA and/or DNA, including, but not limited to such nucleic acids having modified nucleobases. By way of further example and without limitation, an oligomeric compound having the nucleobase sequence "ATCGATCG" encompasses any oligomeric compounds having such nucleobase sequence, whether modified or unmodified, including, but not limited to, such compounds comprising RNA bases, such as those having sequence "AUCGAUCG" and those having some DNA bases and some RNA bases such as "AUCGATCG" and oligomeric compounds having other modified or naturally occurring bases, such as "AT^{me}CGAUCG," wherein ^{me}C indicates a cytosine base comprising a methyl group at the 5-position.

### EXAMPLES

### Non-limiting disclosure

While certain compounds, compositions and methods described herein have been described with specificity in accordance with certain embodiments, the following examples serve only to illustrate the compounds described herein and are not intended to limit the same.

### Example 1: Design of antisense oligonucleotides targeting human dystrophia myotonica protein kinase (hDMPK)

A series of antisense oligonucleotides (ASOs) were designed to target hDMPK. The newly designed ASOs were prepared using standard oligonucleotide synthesis well known in the art and are described in Tables 1 and 2, below. Subscripts "s" indicate phosphorothioate internucleoside linkages; subscripts "k" indicate 6'-*(S)*-CH₃ bicyclic nucleosides (cEt); subscripts "e" indicate 2'-O-methoxyethyl (MOE) modified nucleosides; and subscripts "d" indicate β-D-2'-deoxyribonucleosides. "^{m}C" indicates 5-methylcytosine nucleosides.

The antisense oligonucleotides are targeted to either SEQ ID NO: 1 (GENBANK Accession No. NM_001081560.1) and/or SEQ ID NO: 2 (the complement of GENBANK Accession No. NT_011109.15 truncated from nucleotides 18540696 to 18555106 ). "Start site" indicates the 5'-most nucleoside to which the gapmer is targeted in the human gene sequence. "Stop site" indicates the 3'-most nucleoside to which the gapmer is targeted human gene sequence.

**Table 1**

| Design of antisense oligonucleotides targeting hDMPK and targeted to SEQ ID NO 2 | | | | | | |
|---|---|---|---|---|---|---|
| ISIS No. | Composition (5' to 3') | Motif | Length | Start Site | Stop Site | SEQ ID No. |
| 486178 | | kkk-10-kkk | 16 | 13836 | 13851 | 23 |
| 445569 | | e5-d10-e5 | 20 | 13226 | 13245 | 24 |
| 512497 | | e5-d10-e5 | 20 | 8608 | 8627 | 25 |
| | | | | | | |
| 598768 | | eekk-d8-kkee | 16 | 8603 | 8618 | 26 |
| 594300 | | eeekk-d7-kkeee | 17 | 13229 | 13245 | 27 |
| 594292 | | eeekk-d7-kkeee | 17 | 13835 | 13851 | 28 |
| 569473 | | kkk-d10-kkk | 16 | 5082 | 5097 | 29 |
| 598769 | | eekk-d8-kkee | 16 | 8604 | 8619 | 30 |
| 570808 | | kkk-d10-kkk | 16 | 10201 | 10216 | 31 |
| 598777 | | eekk-d8-kkee | 16 | 10202 | 10217 | 32 |

**Table 2**

| Design of antisense oligonucleotides targeting hDMPK and targeted to SEQ ID NO 1 | | | | | |
|---|---|---|---|---|---|
| ISIS No. | Composition (5' to 3') | Motif | Length | Start Site | Stop Site |
| 486178 | | kkk-10-kkk | 16 | 2773 | 2788 |
| 445569 | | e5-d10-e5 | 20 | 2163 | 2182 |
| 512497 | | e5-d10-e5 | 20 | 1348 | 1367 |
| 598768 | | eekk-d8-kkee | 16 | 1343 | 1358 |
| 594300 | | eeekk-d7-kkeee | 17 | 2166 | 2182 |
| 594292 | | eeekk-d7-kkeee | 17 | 2772 | 2788 |
| 569473 | | kkk-d10-kkk | 16 | 730 | 745 |
| | | | | | |
| 598769 | | eekk-d8-kkee | 16 | 1344 | 1359 |

### Example 2: Antisense inhibition of human DMPK in human skeletal muscle cells (hSKMc)

Antisense oligonucleotides targeted to a human DMPK nucleic acid were tested for their effect on DMPK RNA transcript *in vitro.* Cultured hSKMc cells at a density of 20,000 cells per well were transfected using electroporation with 10,000 nM antisense oligonucleotide. After approximately 24 hours, RNA was isolated from the cells and DMPK transcript levels were measured by quantitative real-time PCR. DMPK RNA transcript levels were adjusted according to total RNA content, as measured by RIBOGREEN®. Results are presented as percent expression of DMPK, relative to untreated control cells.

The antisense oligonucleotides in Tables 3, 4, 5, and 6 are 5-10-5 gapmers, where the gap segment comprises ten 2'-deoxynucleosides and each wing segment comprises five 2'-MOE nucleosides. The internucleoside linkages throughout each gapmer are phosphorothioate (P=S) linkages. All cytsoine residues throughout each gapmer are 5-methylcytosines. 'Target start site' indicates the 5'-most nucleoside to which the antisense oligonucleotide is targeted in the human genomic gene sequence. 'Target stop site' indicates the 3'-most nucleoside to which the antisense oligonucleotide is targeted in the human genomic sequence. All the antisense oligonucleotides listed in Table 3, 4, or 5 target SEQ ID NO: 1 (GENBANK Accession No. NM_001081560.1). All the antisense oligonucleotides listed in Table 6 target SEQ ID NO: 2 (the complement of GENBANK Accession No. NT_011109.15 truncated from nucleotides 18540696 to 18555106).

Several of the antisense oligonucleotides in Tables 2, 3, 4, and 5 demonstrated significant inhibition of DMPK mRNA levels under the conditions specified above.

**Table 3**

| Inhibition of human DMPK RNA transcript in hSKMc by 5-10-5 gapmers targeting SEQ ID NO: 1 | | | | | |
|---|---|---|---|---|---|
| ISIS No. | Sequence | % Target Expression | Start Site on Seq ID: 1 | Stop Site on Seq ID: 1 | SEQ ID NO. |
| UTC | N/A | 100.0 | N/A | N/A | 33 |
| 444401 | TTGCACTTTGCGAACCAACG | 7.3 | 2490 | 2509 | 34 |
| 512326 | CGACACCTCGCCCCTCTTCA | 13.4 | 528 | 547 | 35 |
| 512327 | ACGACACCTCGCCCCTCTTC | 40.8 | 529 | 548 | 36 |
| 512328 | CACGACACCTCGCCCCTCTT | 27.8 | 530 | 549 | 37 |
| 512329 | GCACGACACCTCGCCCCTCT | 16.5 | 531 | 550 | 38 |
| 512330 | AGCACGACACCTCGCCCCTC | 17.9 | 532 | 551 | 39 |
| 512331 | AAGCACGACACCTCGCCCCT | 18.8 | 533 | 552 | 40 |
| 512332 | GAAGCACGACACCTCGCCCC | 23.3 | 534 | 553 | 41 |
| 512333 | GGAAGCACGACACCTCGCCC | 28.1 | 535 | 554 | 42 |
| 512334 | CGGAAGCACGACACCTCGCC | 16.3 | 536 | 555 | 43 |
| 512335 | ACGGAAGCACGACACCTCGC | 28.7 | 537 | 556 | 44 |
| 512336 | CACGGAAGCACGACACCTCG | 15.9 | 538 | 557 | 45 |
| 512337 | TCACGGAAGCACGACACCTC | 18.8 | 539 | 558 | 46 |
| 512338 | CTCACGGAAGCACGACACCT | 16.4 | 540 | 559 | 47 |
| 512339 | CCTCACGGAAGCACGACACC | 20.2 | 541 | 560 | 48 |
| 512340 | TCCTCACGGAAGCACGACAC | 19.3 | 542 | 561 | 49 |
| 512341 | CTCCTCACGGAAGCACGACA | 15.2 | 543 | 562 | 50 |
| 512342 | TCTCCTCACGGAAGCACGAC | 16.2 | 544 | 563 | 51 |
| 512343 | CTCTCCTCACGGAAGCACGA | 16.4 | 545 | 564 | 52 |
| 512344 | CCTCTCCTCACGGAAGCACG | 15.7 | 546 | 565 | 53 |
| 512345 | CCCTCTCCTCACGGAAGCAC | 14.7 | 547 | 566 | 54 |
| 512346 | TCCCTCTCCTCACGGAAGCA | 20.6 | 548 | 567 | 55 |
| 512347 | GTCCCTCTCCTCACGGAAGC | 32.6 | 549 | 568 | 56 |
| 512348 | CGTCCCTCTCCTCACGGAAG | 31.5 | 550 | 569 | 57 |
| 512349 | GGTCCCCATTCACCAACACG | 41.6 | 568 | 587 | 58 |
| 512350 | CGGTCCCCATTCACCAACAC | 31.6 | 569 | 588 | 59 |
| 512351 | CCGGTCCCCATTCACCAACA | 38.1 | 570 | 589 | 60 |
| 512352 | GCCGGTCCCCATTCACCAAC | 55.5 | 571 | 590 | 61 |
| 512353 | CGCCGGTCCCCATTCACCAA | 42.9 | 572 | 591 | 62 |
| 512354 | CCGCCGGTCCCCATTCACCA | 35.7 | 573 | 592 | 63 |
| 512355 | ACCGCCGGTCCCCATTCACC | 51.4 | 574 | 593 | 64 |
| 512356 | CACCGCCGGTCCCCATTCAC | 34.4 | 575 | 594 | 65 |
| 512357 | CCACCGCCGGTCCCCATTCA | 40.4 | 576 | 595 | 66 |
| 512358 | TCCACCGCCGGTCCCCATTC | 35.5 | 577 | 596 | 67 |
| 512359 | ATCCACCGCCGGTCCCCATT | 41.7 | 578 | 597 | 68 |
| 512360 | GATCCACCGCCGGTCCCCAT | 51.0 | 579 | 598 | 69 |
| 512361 | TGATCCACCGCCGGTCCCCA | 35.9 | 580 | 599 | 70 |
| 512362 | GTGATCCACCGCCGGTCCCC | 53.2 | 581 | 600 | 71 |
| 512363 | CGTGATCCACCGCCGGTCCC | 28.2 | 582 | 601 | 72 |
| 512364 | TTCTCATCCTGGAAGGCGAA | 34.6 | 611 | 630 | 73 |
| 512365 | GTTCTCATCCTGGAAGGCGA | 57.1 | 612 | 631 | 74 |
| 512366 | AGTTCTCATCCTGGAAGGCG | 72.1 | 613 | 632 | 75 |
| 512367 | GTAGTTCTCATCCTGGAAGG | 47.1 | 615 | 634 | 76 |
| 512368 | GGTAGTTCTCATCCTGGAAG | 56.0 | 616 | 635 | 77 |
| 512369 | AGGTAGTTCTCATCCTGGAA | 48.3 | 617 | 636 | 78 |
| 512370 | CAGGTAGTTCTCATCCTGGA | 20.2 | 618 | 637 | 79 |
| 512371 | TACAGGTAGTTCTCATCCTG | 44.0 | 620 | 639 | 80 |
| 512372 | GTACAGGTAGTTCTCATCCT | 64.1 | 621 | 640 | 81 |
| 512373 | GGTACAGGTAGTTCTCATCC | 54.2 | 622 | 641 | 82 |
| 512374 | AGGTACAGGTAGTTCTCATC | 65.6 | 623 | 642 | 83 |
| 512375 | CCAGGTACAGGTAGTTCTCA | 45.7 | 625 | 644 | 84 |
| 512376 | ACCAGGTACAGGTAGTTCTC | 60.4 | 626 | 645 | 85 |
| 512377 | GACCAGGTACAGGTAGTTCT | 62.2 | 627 | 646 | 86 |
| 512378 | TGACCAGGTACAGGTAGTTC | 64.9 | 628 | 647 | 87 |
| 512379 | CATGACCAGGTACAGGTAGT | 39.2 | 630 | 649 | 88 |
| 512380 | CCATGACCAGGTACAGGTAG | 27.7 | 631 | 650 | 89 |
| 512381 | TCCATGACCAGGTACAGGTA | 21.6 | 632 | 651 | 90 |
| 512382 | CTCCATGACCAGGTACAGGT | 25.7 | 633 | 652 | 91 |
| 512383 | ACTCCATGACCAGGTACAGG | 28.6 | 634 | 653 | 92 |
| 512384 | TACTCCATGACCAGGTACAG | 23.7 | 635 | 654 | 93 |
| 512385 | ATACTCCATGACCAGGTACA | 20.8 | 636 | 655 | 94 |
| 512386 | AATACTCCATGACCAGGTAC | 22.0 | 637 | 656 | 95 |
| 512387 | TAATACTCCATGACCAGGTA | 14.7 | 638 | 657 | 96 |
| 512388 | CGTAATACTCCATGACCAGG | 10.4 | 640 | 659 | 97 |
| 512389 | AGCAGTGTCAGCAGGTCCCC | 15.0 | 665 | 684 | 98 |
| 512390 | CAGCAGTGTCAGCAGGTCCC | 13.0 | 666 | 685 | 99 |
| 512391 | TCAGCAGTGTCAGCAGGTCC | 22.3 | 667 | 686 | 100 |
| 512392 | CTCAGCAGTGTCAGCAGGTC | 16.4 | 668 | 687 | 101 |
| 512393 | GCTCAGCAGTGTCAGCAGGT | 22.2 | 669 | 688 | 102 |
| 512394 | TGCTCAGCAGTGTCAGCAGG | 26.2 | 670 | 689 | 103 |
| 512395 | TTGCTCAGCAGTGTCAGCAG | 27.4 | 671 | 690 | 104 |
| 512396 | CTTGCTCAGCAGTGTCAGCA | 15.7 | 672 | 691 | 105 |
| 512397 | ACTTGCTCAGCAGTGTCAGC | 43.5 | 673 | 692 | 106 |
| 512398 | AACTTGCTCAGCAGTGTCAG | 26.9 | 674 | 693 | 107 |
| 512399 | AAACTTGCTCAGCAGTGTCA | 20.0 | 675 | 694 | 108 |
| 512400 | CAAACTTGCTCAGCAGTGTC | 23.1 | 676 | 695 | 109 |
| 512401 | CCAAACTTGCTCAGCAGTGT | 20.5 | 677 | 696 | 110 |
| 512402 | CCCAAACTTGCTCAGCAGTG | 13.5 | 678 | 697 | 33 |

**Table 4**

| Inhibition of human DMPK RNA transcript in hSKMc by 5-10-5 gapmers targeting SEQ ID NO: 1 | | | | | |
|---|---|---|---|---|---|
| ISIS No. | Sequence | % Target Expression | Start Site on Seq ID: 1 | Stop Site on Seq ID: 1 | SEQ ID NO. |
| UTC | N/A | 100 | N/A | N/A | |
| 444401 | TTGCACTTTGCGAACCAACG | 13.4 | 2490 | 2509 | 33 |
| 512480 | GTGAGCCCGTCCTCCACCAA | 29.8 | 1310 | 1329 | 111 |
| 512481 | AGTGAGCCCGTCCTCCACCA | 15.6 | 1311 | 1330 | 112 |
| 512482 | CAGTGAGCCCGTCCTCCACC | 10.7 | 1312 | 1331 | 113 |
| 512483 | GCAGTGAGCCCGTCCTCCAC | 33.3 | 1313 | 1332 | 114 |
| 512484 | GGCAGTGAGCCCGTCCTCCA | 9.6 | 1314 | 1333 | 115 |
| 512485 | TGGCAGTGAGCCCGTCCTCC | 8.8 | 1315 | 1334 | 116 |
| 512486 | CATGGCAGTGAGCCCGTCCT | 10.5 | 1317 | 1336 | 117 |
| 512487 | CCATGGCAGTGAGCCCGTCC | 10.1 | 1318 | 1337 | 118 |
| 512488 | TCCATGGCAGTGAGCCCGTC | 13.7 | 1319 | 1338 | 119 |
| 512489 | CTCCATGGCAGTGAGCCCGT | 16.9 | 1320 | 1339 | 120 |
| 512490 | TCTCCATGGCAGTGAGCCCG | 29.1 | 1321 | 1340 | 121 |
| 512491 | GTCTCCATGGCAGTGAGCCC | 41.3 | 1322 | 1341 | 122 |
| 512492 | CCTTCCCGAATGTCCGACAG | 8.8 | 1343 | 1362 | 123 |
| 512493 | ACCTTCCCGAATGTCCGACA | 12.1 | 1344 | 1363 | 124 |
| 512494 | CACCTTCCCGAATGTCCGAC | 6 | 1345 | 1364 | 125 |
| 512495 | GCACCTTCCCGAATGTCCGA | 8.5 | 1346 | 1365 | 126 |
| 512496 | CGCACCTTCCCGAATGTCCG | 5.6 | 1347 | 1366 | 127 |
| 512497 | GCGCACCTTCCCGAATGTCC | 7.7 | 1348 | 1367 | 25 |
| 512498 | GGCGCACCTTCCCGAATGTC | 15 | 1349 | 1368 | 128 |
| 512499 | ACAAAAGGCAGGTGGACCCC | 22.8 | 1373 | 1392 | 129 |
| 512500 | CACAAAAGGCAGGTGGACCC | 22 | 1374 | 1393 | 130 |
| 512501 | CCACAAAAGGCAGGTGGACC | 16.4 | 1375 | 1394 | 131 |
| 512502 | CCCACAAAAGGCAGGTGGAC | 15.8 | 1376 | 1395 | 132 |
| 512503 | GCCCACAAAAGGCAGGTGGA | 25.1 | 1377 | 1396 | 133 |
| 512504 | AG CCCACAAAAG GCAGGTGG | 24.7 | 1378 | 1397 | 134 |
| 512505 | TAGCCCACAAAAGGCAGGTG | 20.7 | 1379 | 1398 | 135 |
| 512506 | GTAGCCCACAAAAGGCAGGT | 20.7 | 1380 | 1399 | 136 |
| 512507 | AGTAGCCCACAAAAGGCAGG | 27.8 | 1381 | 1400 | 137 |
| 512508 | GAGTAGCCCACAAAAGGCAG | 43.9 | 1382 | 1401 | 138 |
| 512509 | GGAGTAGCCCACAAAAGGCA | 29.9 | 1383 | 1402 | 139 |
| 512510 | AGGAGTAGCCCACAAAAGGC | 31.9 | 1384 | 1403 | 140 |
| 512511 | TAGGAGTAGCCCACAAAAGG | 59.9 | 1385 | 1404 | 141 |
| 512512 | GTAGGAGTAGCCCACAAAAG | 40.1 | 1386 | 1405 | 142 |
| 512513 | AGTAGGAGTAGCCCACAAAA | 48.1 | 1387 | 1406 | 143 |
| 512514 | GAGTAGGAGTAGCCCACAAA | 53.3 | 1388 | 1407 | 144 |
| 512515 | GGAGTAGGAGTAGCCCACAA | 24.7 | 1389 | 1408 | 145 |
| 512516 | AGGAGTAGGAGTAGCCCACA | 22.1 | 1390 | 1409 | 146 |
| 512517 | CAGGAGTAGGAGTAGCCCAC | 15.4 | 1391 | 1410 | 147 |
| 512518 | GCAGGAGTAGGAGTAGCCCA | 32.8 | 1392 | 1411 | 148 |
| 512519 | TGCAGGAGTAGGAGTAGCCC | 37.6 | 1393 | 1412 | 149 |
| 512520 | ATGCAGGAGTAGGAGTAGCC | 47.4 | 1394 | 1413 | 150 |
| 512521 | CATGCAGGAGTAGGAGTAGC | 67.2 | 1395 | 1414 | 151 |
| 512522 | CCATGCAGGAGTAGGAGTAG | 58.8 | 1396 | 1415 | 152 |
| 512523 | GCCATGCAGGAGTAGGAGTA | 42.4 | 1397 | 1416 | 153 |
| 512524 | GGCCATGCAGGAGTAGGAGT | 34.1 | 1398 | 1417 | 154 |
| 512525 | GGGCCATGCAGGAGTAGGAG | 44.5 | 1399 | 1418 | 155 |
| 512526 | AGGGCCATGCAGGAGTAGGA | 42 | 1400 | 1419 | 156 |
| 512527 | GAGGGCCATGCAGGAGTAGG | 46.3 | 1401 | 1420 | 157 |
| 512528 | CTGAGGGCCATGCAGGAGTA | 25.3 | 1403 | 1422 | 158 |
| 512529 | CCTGAGGGCCATGCAGGAGT | 28.1 | 1404 | 1423 | 159 |
| 512530 | CCCTGAGGGCCATGCAGGAG | 22.8 | 1405 | 1424 | 160 |
| 512531 | TCCCTGAGGGCCATGCAGGA | 25.7 | 1406 | 1425 | 161 |
| 512532 | GTCCCTGAGGGCCATGCAGG | 17 | 1407 | 1426 | 162 |
| 512533 | TGTCCCTGAGGGCCATGCAG | 18.9 | 1408 | 1427 | 163 |
| 512534 | CTGTCCCTGAGGGCCATGCA | 27.3 | 1409 | 1428 | 164 |
| 512535 | ACTGTCCCTGAGGGCCATGC | 16.5 | 1410 | 1429 | 165 |
| 512536 | CACTGTCCCTGAGGGCCATG | 26 | 1411 | 1430 | 166 |
| 512537 | TCACTGTCCCTGAGGGCCAT | 22.7 | 1412 | 1431 | 167 |
| 512538 | CTCACTGTCCCTGAGGGCCA | 20.2 | 1413 | 1432 | 168 |
| 512539 | CCTCACTGTCCCTGAGGGCC | 19.3 | 1414 | 1433 | 169 |
| 512540 | ACCTCACTGTCCCTGAGGGC | 31 | 1415 | 1434 | 170 |
| 512541 | GACCTCACTGTCCCTGAGGG | 51.4 | 1416 | 1435 | 171 |
| 512542 | GGACCTCACTGTCCCTGAGG | 28 | 1417 | 1436 | 172 |
| 512543 | GGGACCTCACTGTCCCTGAG | 42.6 | 1418 | 1437 | 173 |
| 512544 | CCTCCAGTTCCATGGGTGTG | 16.7 | 1444 | 1463 | 174 |
| 512545 | GCCTCCAGTTCCATGGGTGT | 21.9 | 1445 | 1464 | 175 |
| 512546 | GGCCTCCAGTTCCATGGGTG | 19 | 1446 | 1465 | 176 |
| 512547 | CGGCCTCCAGTTCCATGGGT | 14.9 | 1447 | 1466 | 177 |
| 512548 | TCGGCCTCCAGTTCCATGGG | 23 | 1448 | 1467 | 178 |
| 512549 | CTCGGCCTCCAGTTCCATGG | 15.7 | 1449 | 1468 | 179 |
| 512550 | GCTCGGCCTCCAGTTCCATG | 16.2 | 1450 | 1469 | 180 |
| 512551 | TGCTCGGCCTCCAGTTCCAT | 17.7 | 1451 | 1470 | 181 |
| 512552 | CTGCTCGGCCTCCAGTTCCA | 18.4 | 1452 | 1471 | 182 |
| 512553 | GCTGCTCGGCCTCCAGTTCC | 22 | 1453 | 1472 | 183 |
| 512554 | AGCTGCTCGGCCTCCAGTTC | 32.4 | 1454 | 1473 | 184 |
| 512555 | CAGCTGCTCGGCCTCCAGTT | 15.7 | 1455 | 1474 | 185 |
| 512556 | GCAGCTGCTCGGCCTCCAGT | 16.3 | 1456 | 1475 | 186 |

**Table 5**

| Inhibition of human DMPK RNA transcript in hSKMc by 5-10-5 gapmers targeting SEQ ID NO: 1 | | | | | |
|---|---|---|---|---|---|
| ISIS No. | Sequence | % Target Expression | Start Site on Seq ID: 1 | Stop Site on Seq ID: 1 | SEQ ID NO. |
| UTC | N/A | 100.0 | N/A | N/A | |
| 444401 | TTGCACTTTGCGAACCAACG | 7.0 | 2490 | 2509 | 33 |
| 512557 | AGCAGCTGCTCGGCCTCCAG | 25.2 | 1457 | 1476 | 187 |
| 512558 | AAGCAGCTGCTCGGCCTCCA | 16.1 | 1458 | 1477 | 188 |
| 512559 | CAAGCAGCTGCTCGGCCTCC | 21.9 | 1459 | 1478 | 189 |
| 512560 | TCAAGCAGCTGCTCGGCCTC | 24.8 | 1460 | 1479 | 190 |
| 512561 | CTCAAGCAGCTGCTCGGCCT | 19.8 | 1461 | 1480 | 191 |
| 512562 | GCTCAAGCAGCTGCTCGGCC | 11.6 | 1462 | 1481 | 192 |
| 512563 | GGCTCAAGCAGCTGCTCGGC | 19.8 | 1463 | 1482 | 193 |
| 512564 | TGGCTCAAGCAGCTGCTCGG | 31.9 | 1464 | 1483 | 194 |
| 512565 | GTGGCTCAAGCAGCTGCTCG | 27.5 | 1465 | 1484 | 195 |
| 512566 | TGTGGCTCAAGCAGCTGCTC | 35.4 | 1466 | 1485 | 196 |
| 512567 | GTGTGGCTCAAGCAGCTGCT | 24.8 | 1467 | 1486 | 197 |
| 512568 | CCACTTCAGCTGTTTCATCC | 43.1 | 1525 | 1544 | 198 |
| 512569 | TGCCACTTCAGCTGTTTCAT | 35.0 | 1527 | 1546 | 199 |
| 512570 | CTGCCACTTCAGCTGTTTCA | 27.8 | 1528 | 1547 | 200 |
| 512571 | ACTGCCACTTCAGCTGTTTC | 78.9 | 1529 | 1548 | 201 |
| 512572 | AACTGCCACTTCAGCTGTTT | 36.4 | 1530 | 1549 | 202 |
| 512573 | GAACTGCCACTTCAGCTGTT | 30.3 | 1531 | 1550 | 203 |
| 512574 | GGAACTGCCACTTCAGCTGT | 66.7 | 1532 | 1551 | 204 |
| 512575 | TGGAACTGCCACTTCAGCTG | 22.6 | 1533 | 1552 | 205 |
| 512576 | CTGGAACTGCCACTTCAGCT | 22.9 | 1534 | 1553 | 206 |
| 512577 | GCTGGAACTGCCACTTCAGC | 59.5 | 1535 | 1554 | 207 |
| 512578 | CGCTGGAACTGCCACTTCAG | 24.9 | 1536 | 1555 | 208 |
| 512579 | CCGCTGGAACTGCCACTTCA | 42.5 | 1537 | 1556 | 209 |
| 512580 | GCCGCTGGAACTGCCACTTC | 20.0 | 1538 | 1557 | 210 |
| 512581 | AGCCGCTGGAACTGCCACTT | 19.4 | 1539 | 1558 | 211 |
| 512582 | CTCAGCCTCTGCCGCAGGGA | 22.1 | 1560 | 1579 | 212 |
| 512583 | CCTCAGCCTCTGCCGCAGGG | 33.7 | 1561 | 1580 | 213 |
| 512584 | GGCCTCAGCCTCTGCCGCAG | 24.6 | 1563 | 1582 | 214 |
| 512585 | CGGCCTCAGCCTCTGCCGCA | 55.1 | 1564 | 1583 | 215 |
| 512586 | TCGG CCTCAG CCTCTG CCG C | 60.8 | 1565 | 1584 | 216 |
| 512587 | CTCG G CCTCAG CCTCTG CCG | 31.8 | 1566 | 1585 | 217 |
| 512588 | CCTCG G CCTCAG CCTCTG CC | 16.4 | 1567 | 1586 | 218 |
| 512589 | ACCTCGGCCTCAGCCTCTGC | 31.1 | 1568 | 1587 | 219 |
| 512590 | CACCTCGGCCTCAGCCTCTG | 39.7 | 1569 | 1588 | 220 |
| 512591 | TCACCTCGGCCTCAGCCTCT | 24.8 | 1570 | 1589 | 221 |
| 512592 | GTCACCTCGGCCTCAGCCTC | 28.7 | 1571 | 1590 | 222 |
| 512593 | CGTCACCTCGGCCTCAGCCT | 20.3 | 1572 | 1591 | 223 |
| 512594 | AGCACCTCCTCCTCCAGGGC | 18.4 | 1610 | 1629 | 224 |
| 512595 | GAGCACCTCCTCCTCCAGGG | 19.9 | 1611 | 1630 | 225 |
| 512596 | TGAGCACCTCCTCCTCCAGG | 15.6 | 1612 | 1631 | 226 |
| 512597 | GTGAGCACCTCCTCCTCCAG | 22.3 | 1613 | 1632 | 227 |
| 512598 | GGTGAGCACCTCCTCCTCCA | 19.4 | 1614 | 1633 | 228 |
| 512599 | GGGTGAGCACCTCCTCCTCC | 17.3 | 1615 | 1634 | 229 |
| 512600 | CGGGTGAGCACCTCCTCCTC | 12.2 | 1616 | 1635 | 230 |
| 512601 | CCGGGTGAGCACCTCCTCCT | 15.9 | 1617 | 1636 | 231 |
| 512602 | GCCGGGTGAGCACCTCCTCC | 15.7 | 1618 | 1637 | 232 |
| 512603 | TGCCGGGTGAGCACCTCCTC | 15.1 | 1619 | 1638 | 233 |
| 512604 | CTGCCGGGTGAGCACCTCCT | 24.5 | 1620 | 1639 | 234 |
| 512605 | TCTGCCGGGTGAGCACCTCC | 33.8 | 1621 | 1640 | 235 |
| 512606 | GCTCTGCCGGGTGAGCACCT | 26.1 | 1623 | 1642 | 236 |
| 512607 | GGCTCTGCCGGGTGAGCACC | 50.4 | 1624 | 1643 | 237 |
| 512608 | AGGCTCTGCCGGGTGAGCAC | 42.9 | 1625 | 1644 | 238 |
| 512609 | CAGGCTCTGCCGGGTGAGCA | 39.2 | 1626 | 1645 | 239 |
| 512610 | TCAGGCTCTGCCGGGTGAGC | 20.2 | 1627 | 1646 | 240 |
| 512611 | GCTCAGGCTCTGCCGGGTGA | 22.5 | 1629 | 1648 | 241 |
| 512612 | CGGCTCAGGCTCTGCCGGGT | 27.0 | 1631 | 1650 | 242 |
| 512613 | CCGGCTCAGGCTCTGCCGGG | 68.8 | 1632 | 1651 | 243 |
| 512614 | CCCGGCTCAGGCTCTGCCGG | 58.8 | 1633 | 1652 | 244 |
| 512615 | TCCCGGCTCAGGCTCTGCCG | 24.8 | 1634 | 1653 | 245 |
| 512616 | CTCCCGGCTCAGGCTCTGCC | 10.4 | 1635 | 1654 | 246 |
| 512617 | TCTCCCGGCTCAGGCTCTGC | 12.8 | 1636 | 1655 | 247 |
| 512618 | ATCTCCCGGCTCAGGCTCTG | 13.3 | 1637 | 1656 | 248 |
| 512619 | CATCTCCCGGCTCAGGCTCT | 7.7 | 1638 | 1657 | 249 |
| 512620 | CCATCTCCCGGCTCAGGCTC | 2.8 | 1639 | 1658 | 250 |
| 512621 | TCCATCTCCCGGCTCAGGCT | 2.6 | 1640 | 1659 | 251 |
| 512622 | CTCCATCTCCCGGCTCAGGC | 1.5 | 1641 | 1660 | 252 |
| 512623 | CCTCCATCTCCCGGCTCAGG | 1.4 | 1642 | 1661 | 253 |
| 512624 | GCCTCCATCTCCCGGCTCAG | 2.0 | 1643 | 1662 | 254 |
| 512625 | GGCCTCCATCTCCCGGCTCA | 8.3 | 1644 | 1663 | 255 |
| 512626 | TGGCCTCCATCTCCCGGCTC | 9.4 | 1645 | 1664 | 256 |
| 512627 | ATGGCCTCCATCTCCCGGCT | 6.3 | 1646 | 1665 | 257 |
| 512628 | GATGGCCTCCATCTCCCGGC | 2.7 | 1647 | 1666 | 258 |
| 512629 | GGATGGCCTCCATCTCCCGG | 1.3 | 1648 | 1667 | 259 |
| 512630 | CGGATGGCCTCCATCTCCCG | 1.5 | 1649 | 1668 | 260 |
| 512631 | GCGGATGGCCTCCATCTCCC | 2.4 | 1650 | 1669 | 261 |
| 512632 | TGCGGATGGCCTCCATCTCC | 2.2 | 1651 | 1670 | 262 |
| 512633 | GTTCCGAGCCTCTGCCTCGC | 29.2 | 1701 | 1720 | 263 |

**Table 6**

| Inhibition of human DMPK RNA transcript in hSKMc by 5-10-5 gapmers targeting SEQ ID NO: 2 | | | | | |
|---|---|---|---|---|---|
| ISIS No. | Sequence | % Target Expression | Start Site on Seq ID: 2 | Stop Site on Seq ID: 2 | SEQ ID NO. |
| UTC | N/A | 100.0 | N/A | N/A | |
| 444401 | TTGCACTTTGCGAACCAACG | 7.0 | 13553 | 13572 | 33 |
| 444436 | GTCGGAGGACGAGGTCAATA | 9.7 | 13748 | 13767 | 264 |
| 512634 | AGGGCCTCAGCCTGGCCGAA | 31.7 | 13452 | 13471 | 265 |
| 512635 | CAGGGCCTCAGCCTGGCCGA | 39.5 | 13453 | 13472 | 266 |
| 512636 | GTCAGGGCCTCAGCCTGGCC | 20.5 | 13455 | 13474 | 267 |
| 512637 | CGTCAGGGCCTCAGCCTGGC | 23.3 | 13456 | 13475 | 268 |
| 512638 | AGCAAATTTCCCGAGTAAGC | 14.7 | 13628 | 13647 | 269 |
| 512639 | AAGCAAATTTCCCGAGTAAG | 21.2 | 13629 | 13648 | 270 |
| 512640 | AAAAGCAAATTTCCCGAGTA | 23.0 | 13631 | 13650 | 271 |
| 512641 | CAAAAGCAAATTTCCCGAGT | 19.7 | 13632 | 13651 | 272 |
| 512642 | GCAAAAGCAAATTTCCCGAG | 26.6 | 13633 | 13652 | 273 |
| 512643 | GGCAAAAGCAAATTTCCCGA | 12.8 | 13634 | 13653 | 274 |
| 512644 | TGGCAAAAGCAAATTTCCCG | 12.2 | 13635 | 13654 | 275 |
| 512645 | TTTGGCAAAAGCAAATTTCC | 24.2 | 13637 | 13656 | 276 |
| 512646 | GTTTGGCAAAAGCAAATTTC | 25.5 | 13638 | 13657 | 277 |
| 512647 | GGGTTTGGCAAAAGCAAATT | 43.0 | 13640 | 13659 | 278 |
| 512648 | CGGGTTTGGCAAAAGCAAAT | 27.2 | 13641 | 13660 | 279 |
| 512649 | AAGCGGGTTTGGCAAAAGCA | 27.0 | 13644 | 13663 | 280 |
| 512650 | AATATCCAAACCGCCGAAGC | 45.7 | 13728 | 13747 | 281 |
| 512651 | AAATATCCAAACCGCCGAAG | 56.6 | 13729 | 13748 | 282 |
| 512652 | ATAAATATCCAAACCGCCGA | 39.0 | 13731 | 13750 | 283 |
| 512653 | AATAAATATCCAAACCGCCG | 34.7 | 13732 | 13751 | 284 |
| 512654 | TCAATAAATATCCAAACCGC | 34.7 | 13734 | 13753 | 285 |
| 512655 | GTCAATAAATATCCAAACCG | 19.1 | 13735 | 13754 | 286 |
| 512656 | GGTCAATAAATATCCAAACC | 24.3 | 13736 | 13755 | 287 |
| 512657 | AGGTCAATAAATATCCAAAC | 23.5 | 13737 | 13756 | 288 |
| 512658 | GAGGTCAATAAATATCCAAA | 24.2 | 13738 | 13757 | 289 |
| 512659 | ACGAGGTCAATAAATATCCA | 28.3 | 13740 | 13759 | 290 |
| 512660 | GACGAGGTCAATAAATATCC | 17.8 | 13741 | 13760 | 291 |
| 512661 | AGGACGAGGTCAATAAATAT | 45.7 | 13743 | 13762 | 292 |
| 512662 | GAGGACGAGGTCAATAAATA | 27.6 | 13744 | 13763 | 293 |
| 512663 | CGGAGGACGAGGTCAATAAA | 15.8 | 13746 | 13765 | 294 |
| 512664 | TCGGAGGACGAGGTCAATAA | 10.8 | 13747 | 13766 | 295 |
| 512665 | AGTCGGAGGACGAGGTCAAT | 15.4 | 13749 | 13768 | 296 |
| 512666 | GAGTCGGAGGACGAGGTCAA | 18.8 | 13750 | 13769 | 297 |
| 512667 | GCGAGTCGGAGGACGAGGTC | 26.0 | 13752 | 13771 | 298 |
| 512668 | AGCGAGTCGGAGGACGAGGT | 21.7 | 13753 | 13772 | 299 |
| 512669 | CAGCGAGTCGGAGGACGAGG | 13.7 | 13754 | 13773 | 300 |
| 512670 | TCAGCGAGTCGGAGGACGAG | 16.5 | 13755 | 13774 | 301 |
| 512671 | GTCAGCGAGTCGGAGGACGA | 17.4 | 13756 | 13775 | 302 |
| 512672 | CTGTCAGCGAGTCGGAGGAC | 25.2 | 13758 | 13777 | 303 |
| 512673 | CCTGTCAGCGAGTCGGAGGA | 18.4 | 13759 | 13778 | 304 |
| 512674 | AGCCTGTCAGCGAGTCGGAG | 16.8 | 13761 | 13780 | 305 |
| 512675 | GTCTCAGTGCATCCAAAACG | 11.8 | 13807 | 13826 | 306 |
| 512676 | GGTCTCAGTGCATCCAAAAC | 17.7 | 13808 | 13827 | 307 |
| 512677 | GGGTCTCAGTGCATCCAAAA | 11.2 | 13809 | 13828 | 308 |
| 512678 | GGAGGGCCTTTTATTCGCGA | 17.8 | 13884 | 13903 | 309 |
| 512679 | TGGAGGGCCTTTTATTCGCG | 13.2 | 13885 | 13904 | 310 |
| 512680 | ATGGAGGGCCTTTTATTCGC | 19.3 | 13886 | 13905 | 311 |
| 512681 | GATGGAGGGCCTTTTATTCG | 30.5 | 13887 | 13906 | 312 |
| 512682 | AGATGGAGGGCCTTTTATTC | 50.8 | 13888 | 13907 | 313 |
| 512683 | CAGATGGAGGGCCTTTTATT | 46.1 | 13889 | 13908 | 314 |
| 512684 | GCAGATGGAGGGCCTTTTAT | 50.4 | 13890 | 13909 | 315 |
| 512685 | CCCTCAGGCTCTCTGCTTTA | 34.7 | 655 | 674 | 316 |
| 512686 | GCCCTCAGGCTCTCTGCTTT | 47.9 | 656 | 675 | 317 |
| 512687 | AGCCCTCAGGCTCTCTGCTT | 47.4 | 657 | 676 | 318 |
| 512688 | TAGCCCTCAGGCTCTCTGCT | 54.1 | 658 | 677 | 319 |
| 512689 | TTAGCCCTCAGGCTCTCTGC | 48.0 | 659 | 678 | 320 |
| 512690 | TTTAGCCCTCAGGCTCTCTG | 50.7 | 660 | 679 | 321 |
| 512691 | ATTTAGCCCTCAGGCTCTCT | 47.3 | 661 | 680 | 322 |
| 512692 | AATTTAGCCCTCAGGCTCTC | 44.8 | 662 | 681 | 323 |
| 512693 | AAATTTAGCCCTCAGGCTCT | 39.2 | 663 | 682 | 324 |
| 512694 | TAAATTTAGCCCTCAGGCTC | 48.0 | 664 | 683 | 325 |
| 512695 | TTAAATTTAGCCCTCAGGCT | 54.9 | 665 | 684 | 326 |
| 512696 | GTTAAATTTAGCCCTCAGGC | 48.1 | 666 | 685 | 327 |
| 512697 | AGTTAAATTTAGCCCTCAGG | 39.3 | 667 | 686 | 328 |
| 512698 | CAGTTAAATTTAGCCCTCAG | 47.5 | 668 | 687 | 329 |
| 512699 | ACAGTTAAATTTAGCCCTCA | 68.2 | 669 | 688 | 330 |
| 512700 | GACAGTTAAATTTAGCCCTC | 59.2 | 670 | 689 | 331 |
| 512701 | GGACAGTTAAATTTAGCCCT | 63.7 | 671 | 690 | 332 |
| 512702 | CGGACAGTTAAATTTAGCCC | 50.7 | 672 | 691 | 333 |
| 512703 | TCGGACAGTTAAATTTAGCC | 39.6 | 673 | 692 | 334 |
| 512704 | CTCGGACAGTTAAATTTAGC | 36.5 | 674 | 693 | 335 |
| 512705 | ACTCGGACAGTTAAATTTAG | 59.1 | 675 | 694 | 336 |
| 512706 | GACTCGGACAGTTAAATTTA | 50.0 | 676 | 695 | 337 |
| 512707 | CGACTCGGACAGTTAAATTT | 63.0 | 677 | 696 | 338 |
| 512708 | CCGACTCGGACAGTTAAATT | 34.3 | 678 | 697 | 339 |
| 512709 | TCCGACTCGGACAGTTAAAT | 39.5 | 679 | 698 | 340 |

### Example 3: Design of antisense oligonucleotides targeting human dystrophia myotonica protein kinase (hDMPK)

A series of antisense oligonucleotides (ASOs) were designed to target hDMPK. The newly designed ASOs were prepared using standard oligonucleotide synthesis well known in the art and are described in Table 7, below. Subscripts "s" indicate phosphorothioate internucleoside linkages; subscripts "k" indicate 6'-*(S)*-CH₃ bicyclic nucleosides (cEt); subscripts "e" indicate 2'-O-methoxyethyl (MOE) modified nucleosides; and subscripts "d" indicate β-D-2'-deoxyribonucleosides. "^{m}C" indicates 5-methylcytosine nucleosides.

The antisense oligonucleotides targeted to a human DMPK nucleic acid were tested for their effect on DMPK RNA transcript *in vitro.* Cultured HepG2 cells at a density of 20,000 cells per well were transfected using electroporation with 4,500 nM antisense oligonucleotide. After approximately 24 hours, RNA was isolated from the cells and DMPK transcript levels were measured by quantitative real-time PCR. DMPK RNA transcript levels were adjusted according to total RNA content, as measured by RIBOGREEN®. Results are presented as percent expression of DMPK, relative to untreated control cells.

'Target start site' indicates the 5'-most nucleoside to which the antisense oligonucleotide is targeted in the human genomic gene sequence. 'Target stop site' indicates the 3'-most nucleoside to which the antisense oligonucleotide is targeted in the human genomic sequence. All the antisense oligonucleotides listed in Table 7 target SEQ ID NO: 1 (GENBANK Accession No. NM_001081560.1).

Several of the antisense oligonucleotides demonstrated significant inhibition of DMPK mRNA levels under the conditions specified above.

**Table 7**

| Inhibition of human DMPK RNA transcript in HepG2 cells targeting SEQ ID NO: 1 | | | | | |
|---|---|---|---|---|---|
| ISIS No. | Sequence | % Target Expression | Start Site on Seq ID: 1 | Stop Site on Seq ID: 1 | Seq ID No. |
| UTC | N/A | 100 | N/A | N/A | |
| 533424 | Tₑₛ^{m}CₑₛTₑₛ^{m}Cₑₛ^{m}C_{ds}T_{ds}^{m}C_{ds}A_{ds}^{m}C_{ds}G_{ds}G_{ds}A_{ds}A_{ds}Gₖₛ^{m}CₖₛAₖ | 34.4 | 548 | 563 | 341 |
| 533425 | ^{m}CₑₛTₑₛ^{m}CₑₛT_{ds}^{m}C_{ds}^{m}C_{ds}T_{ds}^{m}C_{ds}A_{ds}^{m}C_{ds}G_{ds}G_{ds}A_{ds}AₖₛGₖₛ^{m}Cₖ | 32.1 | 549 | 564 | 342 |
| 533426 | ^{m}Cₑₛ^{m}CₑₛTₑₛ^{m}C_{ds}T_{ds}^{m}C_{ds}^{m}C_{ds}T_{ds}^{m}C_{ds}A_{ds}^{m}C_{ds}G_{ds}G_{ds}AₖₛAₖₛGₖ | 52.1 | 550 | 565 | 343 |
| 533427 | AₑₛAₑₛAₑₛ^{m}C_{ds}T _{ds}T _{ds}G_{ds}^{m}C_{ds}T_{ds}^{m}C_{ds}A_{ds}G_{ds}^{m}C_{ds}A_{kS}GₖₛTₖ | 36.8 | 679 | 694 | 344 |
| 533428 | ^{m}CₑₛAₑₛAₑₛA_{ds}^{m}C_{ds}T_{ds}T_{ds}G_{ds}^{m}C_{ds}T_{ds}^{m}C_{ds}A_{ds}G_{ds}^{m}CₖₛAₖₛGₖ | 59.9 | 680 | 695 | 345 |
| 533429 | ^{m}Cₑₛ^{m}CₑₛAₑₛA_{ds}A_{ds}^{m}C_{ds}T_{ds}T_{ds}G_{ds}^{m}C_{ds}T_{ds}^{m}C_{ds}A_{ds}Gₖₛ^{m}CₖₛAₖ | 39.3 | 681 | 696 | 346 |
| 533430 | ^{m}Cₑₛ^{m}Cₑₛ^{m}CₑₛA_{ds}A_{ds}A_{ds}^{m}C_{ds}T_{ds}T_{ds}G_{ds}^{m}C_{ds}T_{ds}^{m}C_{ds}AₖₛGₖₛ^{m}Cₖ | 37.6 | 682 | 697 | 347 |
| 533431 | ^{m}Cₑₛ^{m}Ceₛ^{m}Cₑₛ^{m}C_{ds}A_{ds}A_{ds}A_{ds}^{m}C_{ds}T_{ds}T_{ds}G_{ds}^{m}C_{ds}T_{ds}^{m}CₖₛAₖₛGₖ | 39.6 | 683 | 698 | 348 |
| 533432 | Tₑₛ^{m}Cₑₛ^{m}Cₑₛ^{m}C_{ds}^{m}C_{ds}A_{ds}A_{ds}A_{ds}^{m}C_{ds}T_{ds}T_{ds}G_{ds}^{m}C_{ds}Tₖₛ^{m}CₖₛAₖ | 52.1 | 684 | 699 | 349 |
| 533433 | GₑₛTₑₛTₑₛT_{ds}G_{ds}A_{ds}T_{ds}G_{ds}T_{ds}^{m}C_{ds}^{m}C_{ds}^{m}C_{ds}T_{ds}GₖₛTₖₛGₖ | 53.9 | 782 | 797 | 350 |
| 533434 | GₑₛGₑₛTₑₛT_{ds}T_{ds}G_{ds}A_{ds}T_{ds}G_{ds}T_{ds}^{m}C_{ds}^{m}C_{ds}^{m}C_{ds}TₖₛGₖₛTₖ | 38.1 | 783 | 798 | 351 |
| 533435 | GₑₛGₑₛGₑₛT_{ds}T_{ds}T_{ds}G_{ds}A_{ds}T_{ds}G_{ds}T_{d}s C_{ds} C_{ds} CₖₛTₖₛGₖ | 43.7 | 784 | 799 | 352 |
| 533436 | Aₑₛ^{m}CₑₛAₑₛG_{ds}^{m}C_{ds}^{m}C_{ds}T_{ds}G_{ds}^{m}C_{ds}A_{ds}G_{ds}G_{ds}A_{ds}Tₖₛ^{m}CₖₛTₖ | 29.5 | 927 | 942 | 353 |
| 533437 | ^{m}CₑₛAₑₛ^{m}CₑₛA_{ds}G_{ds}^{m}C_{ds}^{m}C_{ds}T_{ds}G_{ds}^{m}C_{ds}A_{ds}G_{ds}G_{ds}AₖₛTₖₛ^{m}Cₖ | 48.6 | 928 | 943 | 354 |
| 533438 | ^{m}Cₑₛ^{m}CₑₛAₑₛ^{m}C_{ds}A_{ds}G_{ds}^{m}C_{ds}^{m}C_{ds}T_{ds}G_{ds}^{m}C_{ds}A_{ds}G_{ds}GₖₛAₖₛTₖ | 46.9 | 929 | 944 | 355 |
| 533439 | ^{m}Cₑₛ^{m}Cₑₛ^{m}CₑₛA_{ds}^{m}C_{ds}A_{ds}G_{ds}^{m}C_{ds}^{m}C_{ds}T_{ds}G_{ds}^{m}C_{ds}A_{ds}GₖₛGₖₛAₖ | 43.6 | 930 | 945 | 356 |
| 533440 | Gₑₛ^{m}Cₑₛ^{m}Cₑₛ^{m}C_{ds}A_{ds}^{m}C_{ds}A_{ds}G_{ds}^{m}C_{ds}^{m}C_{ds}T_{ds}G_{ds}^{m}C_{ds}AₖₛGₖₛGₖ | 26.9 | 931 | 946 | 357 |
| 533441 | ^{m}CₑₛGₑₛ^{m}Cₑₛ^{m}C_{ds}^{m}C_{ds}A_{ds}^{m}C_{ds}A_{ds}G_{ds}^{m}C_{ds}^{m}C_{ds}T_{ds}G_{ds}^{m}CₖₛAₖₛGₖ | 31.3 | 932 | 947 | 358 |
| 533442 | ^{m}Cₑₛ^{m}CₑₛGₑₛ^{m}C_{ds}^{m}C_{ds}^{m}C_{ds}A_{ds}^{m}C_{ds}A_{ds}G_{ds}^{m}C_{ds}^{m}C_{ds}T_{ds}Gₖₛ^{m}CₖₛAₖ | 20.5 | 933 | 948 | 359 |
| 533443 | Aₑₛ^{m}Cₑₛ^{m}CₑₛG_{ds}^{m}C_{ds}^{m}C_{ds}^{m}C_{ds}A_{ds}^{m}C_{ds}A_{ds}G_{ds}^{m}C_{ds}^{m}C_{ds}TₖₛGₖₛ^{m}Cₖ | 13.7 | 934 | 949 | 360 |
| 533444 | ^{m}CₑₛAₑₛ^{m}Cₑₛ^{m}C_{ds}G_{ds}^{m}C_{ds}^{m}C_{ds}^{m}C_{ds}A_{ds}^{m}C_{ds}A_{ds}G_{ds}^{m}C_{ds}^{m}CₖₛTₖₛGₖ | 29.4 | 935 | 950 | 361 |
| 533445 | ^{m}Cₑₛ^{m}CₑₛAₑₛ^{m}C_{ds}^{m}C_{ds}G_{ds}^{m}C_{ds}^{m}C_{ds}^{m}C_{ds}A_{ds}^{m}C_{ds}A_{ds}G_{ds}^{m}Cₖₛ^{m}CₖₛTₖ | 32 | 936 | 951 | 362 |
| 533446 | ^{m}Cₑₛ^{m}Cₑₛ^{m}CₑₛA_{ds}^{m}C_{ds}^{m}C_{ds}G_{ds}^{m}C_{ds}^{m}C_{ds}^{m}C_{ds}A_{ds}^{m}C_{ds}A_{ds}Gₖₛ^{m}Cₖₛ^{m}Cₖ | 8.3 | 937 | 952 | 363 |
| 533447 | Gₑₛ^{m}Cₑₛ^{m}Cₑₛ^{m}C_{ds}A_{ds}^{m}C_{ds}mC_{ds}G_{ds}^{m}C_{ds}^{m}C_{ds}^{m}C_{ds}A_{ds}^{m}C_{ds}AₖₛGₖₛ^{m}Cₖ | 18.3 | 938 | 953 | 364 |
| 533448 | ^{m}Cₑₛ^{m}CₑₛAₑₛG_{ds}G_{ds}^{m}C_{ds}^{m}C_{ds}^{m}C_{ds}A_{ds}^{m}C_{ds}^{m}C_{ds}G_{ds}^{m}C_{ds}^{m}Cₖₛ^{m}CₖₛAₖ | 19.4 | 942 | 957 | 365 |
| 533449 | ^{m}Cₑₛ^{m}Cₑₛ^{m}CₑₛA_{ds}G_{ds}G_{ds}^{m}C_{ds}^{m}C_{ds}^{m}C_{ds}A_{ds}^{m}C_{ds}^{m}C_{ds}G_{ds}^{m}Cₖₛ^{m}Cₖₛ^{m}Cₖ | 24.2 | 943 | 958 | 366 |
| 533450 | Tₑₛ^{m}Cₑₛ^{m}Cₑₛ^{m}C_{ds}A_{ds}G_{ds}G_{ds}^{m}C_{ds}^{m}C_{ds}^{m}C_{ds}A_{ds}^{m}C_{ds}^{m}C_{ds}Gₖₛ^{m}Cₖₛ^{m}Cₖ | 39.2 | 944 | 959 | 367 |
| 533451 | TₑₛGₑₛ^{m}Cₑₛ^{m}C_{ds}T_{ds}G_{ds}T_{ds}^{m}C_{ds}^{m}C_{ds}^{m}C_{ds}A_{ds}G_{ds}G_{ds}^{m}Cₖₛ^{m}Cₖₛ^{m}Cₖ | 44.2 | 950 | 965 | 368 |
| 533452 | ^{m}CₑₛTₑₛGₑₛ^{m}C_{ds}^{m}C_{ds}T_{ds}G_{ds}T_{ds}^{m}C_{ds}^{m}C_{ds}^{m}C_{ds}A_{ds}G_{ds}Gₖₛ^{m}Cₖₛ^{m}Cₖ | 55.6 | 951 | 966 | 369 |
| 533453 | Gₑₛ^{m}CₑₛTₑₛG_{ds}^{m}C_{ds}^{m}C_{ds}T_{ds}G_{ds}T_{ds}^{m}C_{ds}^{m}C_{ds}^{m}C_{ds}A_{ds}GₖₛGₖₛ^{m}Cₖ | 71.2 | 952 | 967 | 370 |
| 533454 | GₑₛGₑₛTₑₛG_{ds}G_{ds}^{m}C_{ds}A_{ds}^{m}C_{ds}^{m}C_{ds}T_{ds}T_{ds}^{m}C_{ds}G_{ds}AₖₛAₖₛAₖ | 39.6 | 1276 | 1291 | 371 |
| 533455 | ^{m}CₑₛGₑₛGₑₛT_{ds}G_{ds}G_{ds}^{m}C_{ds}A_{ds}^{m}C_{ds}^{m}C_{ds}T_{ds}T_{ds}^{m}C_{ds}GₖₛAₖₛAₖ | 52.9 | 1277 | 1292 | 372 |
| 533456 | Tₑₛ^{m}CₑₛGₑₛG_{ds}T_{ds}G_{ds}G_{ds}^{m}C_{ds}A_{ds}^{m}C_{ds}^{m}C_{ds}T_{ds}T_{ds}^{m}CₖₛGₖₛAₖ | 27 | 1278 | 1293 | 373 |
| 533457 | AₑₛGₑₛTₑₛG_{ds}A_{ds}G_{ds}^{m}C_{ds}^{m}C_{ds}^{m}C_{ds}G_{ds}T_{ds}^{m}C_{ds}^{m}C_{ds}Tₖₛ^{m}Cₖₛ^{m}Cₖ | 51.5 | 1315 | 1330 | 374 |
| 533458 | ^{m}CₑₛAₑₛGₑₛT_{ds}G_{ds}A_{ds}G_{ds}^{m}C_{ds}^{m}C_{ds}^{m}C_{ds}G_{ds}T_{ds}^{m}C_{ds}^{m}CₖₛTₖₛ^{m}Cₖ | 55.1 | 1316 | 1331 | 375 |
| 533459 | Gₑₛ^{m}CₑₛAₑₛG_{ds}T_{ds}G_{ds}A_{ds}G_{ds}^{m}C_{ds}^{m}C_{ds}^{m}C_{ds}G_{ds}T_{ds}^{m}Cₖₛ^{m}CₖₛTₖ | 33.7 | 1317 | 1332 | 376 |
| 533460 | Tₑₛ^{m}Cₑₛ^{m}Cₑₛ^{m}C_{ds}G_{ds}A_{ds}A_{ds}T_{ds}G_{ds}T_{ds}^{m}C_{ds}^{m}C_{ds}G_{ds}Aₖₛ^{m}CₖₛAₖ | 28.7 | 1344 | 1359 | 377 |
| 533461 | TₑₛTₑₛ^{m}Cₑₛ^{m}C_{ds}^{m}C_{ds}G_{ds}A_{ds}A_{ds}T_{ds}G_{ds}T_{ds}^{m}C_{ds}^{m}C_{ds}GₖₛAₖₛ^{m}Cₖ | 36.2 | 1345 | 1360 | 378 |
| 533462 | ^{m}CₑₛTₑₛTₑₛ^{m}C_{ds}^{m}C_{ds}^{m}C_{ds}G_{ds}A_{ds}A_{ds}T_{ds}G_{ds}T_{ds}^{m}C_{ds}^{m}CₖₛGₖₛAₖ | 23 | 1346 | 1361 | 379 |
| 533463 | ^{m}Cₑₛ^{m}CₑₛTₑₛT_{ds}^{m}C_{ds}^{m}C_{ds}^{m}C_{ds}G_{ds}A_{ds}A_{ds}T_{ds}G_{ds}T_{ds}^{m}Cₖₛ^{m}CₖₛGₖ | 11.5 | 1347 | 1362 | 380 |
| 533464 | Aₑₛ^{m}Cₑₛ^{m}CₑₛT_{ds}T_{ds}^{m}C_{ds}^{m}C_{ds}^{m}C_{ds}G_{ds}A_{ds}A_{ds}T_{ds}G_{ds}Tₖₛ^{m}Cₖₛ^{m}Cₖ | 19.9 | 1348 | 1363 | 381 |
| 533465 | ^{m}CₑₛAₑₛ^{m}Cₑₛ^{m}C_{ds}T_{ds}T_{ds}^{m}C_{ds}^{m}C_{ds}^{m}C_{ds}G_{ds}A_{ds}A_{ds}T_{ds}GₖₛTₖₛ^{m}Cₖ | 30.2 | 1349 | 1364 | 382 |
| 533466 | Gₑₛ^{m}CₑₛAₑₛ^{m}C_{ds}^{m}C_{ds}T_{ds}T_{ds}^{m}C_{ds}^{m}C_{ds}^{m}C_{ds}G_{ds}A_{ds}A_{ds}T_{ds}GₖₛTₖ | 30.2 | 1350 | 1365 | 383 |
| 533467 | ^{m}CₑₛGₑₛ^{m}CₑₛA_{ds}^{m}C_{ds}^{m}C_{ds}T_{ds}T_{ds}^{m}C_{ds}^{m}C_{ds}^{m}C_{ds}G_{ds}A_{ds}AₖₛTₖₛGₖ | 35.5 | 1351 | 1366 | 384 |
| 533468 | AₑₛTₑₛ^{m}Cₑₛ^{m}C_{ds}G_{ds}^{m}C_{ds}T_{ds}^{m}C_{ds}^{m}C_{ds}T_{ds}G_{ds}^{m}C_{ds}A_{ds}Aₖₛ^{m}CₖₛTₖ | 47.4 | 1746 | 1761 | 385 |
| 533469 | ^{m}CₑₛAₑₛTₑₛ^{m}C_{ds}^{m}C_{ds}G_{ds}^{m}C_{ds}T_{ds}^{m}C_{ds}^{m}C_{ds}T_{ds}G_{ds}^{m}C_{ds}AₖₛAₖₛ^{m}Cₖ | 51.2 | 1747 | 1762 | 386 |
| 533470 | ^{m}Cₑₛ^{m}CₑₛAₑₛT_{ds}^{m}C_{ds}^{m}C_{ds}G_{ds}^{m}C_{ds}T_{ds}^{m}C_{ds}^{m}C_{ds}T_{ds}G_{ds}^{m}CₖₛAₖₛAₖ | 35.5 | 1748 | 1763 | 387 |
| 533471 | Gₑₛ^{m}CₑₛTₑₛ^{m}C_{ds}^{m}C_{ds}^{m}C_{ds}T_{ds}^{m}C_{ds}T_{ds}G_{ds}^{m}C_{ds}^{m}C_{ds}T_{ds}Gₖₛ^{m}CₖₛAₖ | 65.6 | 1770 | 1785 | 388 |
| 533472 | AₑₛGₑₛGₑₛT_{ds}G_{ds}G_{ds}A_{ds}T_{ds}^{m}C_{ds}^{m}C_{ds}G_{ds}T_{ds}G_{ds}Gₖₛ^{m}Cₖₛ^{m}Cₖ | 51.8 | 1816 | 1831 | 389 |
| 533473 | GₑₛGₑₛGₑₛA_{ds}A_{ds}G_{ds}G_{ds}T_{ds}G_{ds}G_{ds}A_{ds}T_{ds}^{m}C_{ds}^{m}CₖₛGₖₛTₖ | 44.9 | 1820 | 1835 | 390 |
| 533474 | Aₑₛ^{m}CₑₛAₑₛG_{ds}G_{ds}A_{ds}G_{ds}^{m}C_{ds}A_{ds}G_{ds}G_{ds}G_{ds}A_{ds}AₖₛAₖₛGₖ | 80.8 | 1955 | 1970 | 391 |
| 533475 | ^{m}CₑₛAₑₛGₑₛA_{ds}^{m}C_{ds}T_{ds}G_{ds}^{m}C_{ds}G_{ds}G_{ds}T_{ds}G_{ds}A_{ds}GₖₛTₖₛTₖ | 95.5 | 2034 | 2049 | 392 |
| 533476 | GₑₛGₑₛ^{m}CₑₛT_{ds}^{m}C_{ds}^{m}C_{ds}T_{ds}G_{ds}G_{ds}G_{ds}^{m}C_{ds}G_{ds}G_{ds}^{m}CₖₛGₖₛ^{m}Cₖ | 55.7 | 2050 | 2065 | 393 |
| 533477 | GₑₛGₑₛ^{m}CₑₛG_{ds}G_{ds}^{m}C_{ds}T_{ds}^{m}C_{ds}^{m}C_{ds}T_{ds}G_{ds}G_{ds}G_{ds}^{m}CₖₛGₖₛGₖ | 45.8 | 2053 | 2068 | 394 |
| 533478 | ^{m}CₑₛGₑₛ^{m}CₑₛG_{ds}G_{ds}G_{ds}^{m}C_{ds}G_{ds}G_{ds}^{m}C_{ds}T_{ds}^{m}C_{ds}^{m}C_{ds}TₖₛGₖₛGₖ | 83.7 | 2057 | 2072 | 395 |
| 533479 | GₑₛAₑₛGₑₛ^{m}C_{ds}G_{ds}^{m}C_{ds}G_{ds}G_{ds}G_{ds}^{m}C_{ds}G_{ds}G_{ds}^{m}C_{ds}Tₖₛ^{m}Cₖₛ^{m}Cₖ | 79.8 | 2060 | 2075 | 396 |
| 533480 | GₑₛGₑₛTₑₛT_{ds}^{m}C_{ds}A_{ds}G_{ds}G_{ds}G_{ds}A_{ds}G_{ds}^{m}C_{ds}G_{ds}^{m}CₖₛGₖₛGₖ | 49.4 | 2068 | 2083 | 397 |
| 533481 | AₑₛGₑₛTₑₛT_{ds}^{m}C_{ds}T_{ds}A_{ds}G_{ds}G_{ds}G_{ds}T_{ds}T_{ds}^{m}C_{ds}AₖₛGₖₛGₖ | 37 | 2076 | 2091 | 398 |
| 533482 | ^{m}CₑₛAₑₛGₑₛT_{ds}T_{ds}^{m}C_{ds}T_{ds}A_{ds}G_{ds}G_{ds}G_{ds}T_{ds}T_{ds}^{m}CₖₛAₖₛGₖ | 28.5 | 2077 | 2092 | 399 |
| 533483 | Aₑₛ^{m}CₑₛAₑₛG_{ds}T_{ds}T_{ds}^{m}C_{ds}T_{ds}A_{ds}G_{ds}G_{ds}G_{ds}T_{ds}Tₖₛ^{m}CₖₛAₖ | 42 | 2078 | 2093 | 400 |
| 533484 | GₑₛAₑₛ^{m}CₑₛA_{ds}G_{ds}T_{ds}T_{ds}^{m}C_{ds}T_{ds}A_{ds}G_{ds}G_{ds}G_{ds}TₖₛTₖₛ^{m}Cₖ | 37.4 | 2079 | 2094 | 401 |
| 533485 | AₑₛGₑₛAₑₛ^{m}C_{ds}A_{ds}G_{ds}T_{ds}T_{ds}^{m}C_{ds}T_{ds}A_{ds}G_{ds}G_{ds}GₖₛTₖₛTₖ | 66.5 | 2080 | 2095 | 402 |
| 533486 | AₑₛAₑₛGₑₛA_{ds}^{m}C_{ds}A_{ds}G_{ds}T_{ds}T_{ds}^{m}C_{ds}T_{ds}A_{ds}G_{ds}GₖₛGₖₛtₖ | 62.4 | 2081 | 2096 | 403 |
| 533487 | GₑₛAₑₛAₑₛG_{ds}A_{ds}^{m}C_{ds}A_{ds}G_{ds}T_{ds}T_{ds}^{m}C_{ds}T_{ds}A_{ds}GₖₛGₖₛGₖ | 56.9 | 2082 | 2097 | 404 |
| 533488 | ^{m}CₑₛGₑₛAₑₛA_{ds}G_{ds}A_{ds}^{m}C_{ds}A_{ds}G_{ds}T_{ds}T_{ds}^{m}C_{ds}T_{ds}AₖₛGₖₛGₖ | 36.8 | 2083 | 2098 | 405 |
| 533489 | Tₑₛ^{m}CₑₛGₑₛA_{ds}A_{ds}G_{ds}A_{ds}^{m}C_{ds}A_{ds}G_{ds}T_{ds}T_{ds}^{m}C_{ds}TₖₛAₖₛGₖ | 49.6 | 2084 | 2099 | 406 |
| 533490 | GₑₛTₑₛ^{m}CₑₛG_{ds}A_{ds}A_{ds}G_{ds}A_{ds}^{m}C_{ds}A_{ds}G_{ds}T_{ds}T_{ds}^{m}CₖₛTₖₛAₖ | 40.4 | 2085 | 2100 | 407 |
| 533491 | AₑₛGₑₛTₑₛ^{m}C_{ds}G_{ds}A_{ds}A_{ds}G_{ds}A_{ds}^{m}C_{ds}A_{ds}G_{ds}T_{ds}Tₖₛ^{m}CₖₛTₖ | 37.4 | 2086 | 2101 | 408 |
| 533492 | GₑₛAₑₛGₑₛT_{ds}^{m}C_{ds}G_{ds}A_{ds}A_{ds}G_{ds}A_{ds}^{m}C_{ds}A_{ds}G_{ds}TₖₛTₖₛ^{m}Cₖ | 36.6 | 2087 | 2102 | 409 |
| 533493 | GₑₛGₑₛAₑₛG_{ds}T_{ds}^{m}C_{ds}G_{ds}A_{ds}A_{ds}G_{ds}A_{ds}^{m}C_{ds}A_{ds}GₖₛTₖₛTₖ | 33.2 | 2088 | 2103 | 410 |
| 533494 | ^{m}CₑₛGₑₛGₑₛA_{ds}G_{ds}T_{ds}^{m}C_{ds}G_{ds}A_{ds}A_{ds}G_{ds}A_{ds}^{m}C_{ds}AₖₛGₖₛTₖ | 45.3 | 2089 | 2104 | 411 |
| 533495 | ^{m}Cₑₛ^{m}CₑₛGₑₛG_{ds}A_{ds}G_{ds}T_{ds}^{m}C_{ds}G_{ds}A_{ds}A_{ds}G_{ds}A_{ds}^{m}CₖₛAₖₛGₖ | 45.9 | 2090 | 2105 | 412 |
| 533496 | ^{m}Cₑₛ^{m}Cₑₛ^{m}CₑₛG_{ds}G_{ds}A_{ds}G_{ds}T_{ds}^{m}C_{ds}G_{ds}A_{ds}A_{ds}G_{ds}Aₖₛ^{m}CₖₛAₖ | 51.3 | 2091 | 2106 | 413 |
| 533497 | ^{m}Cₑₛ^{m}Cₑₛ^{m}Cₑₛ^{m}C_{ds}G_{ds}G_{ds}A_{ds}G_{ds}T_{ds}^{m}C_{ds}G_{ds}A_{ds}A_{ds}GₖₛAₖₛ^{m}Cₖ | 49.2 | 2092 | 2107 | 414 |
| 533498 | Gₑₛ^{m}Cₑₛ^{m}Cₑₛ^{m}C_{ds}^{m}C_{ds}G_{ds}G_{ds}A_{ds}G_{ds}T_{ds}^{m}C_{ds}G_{ds}A_{ds}AₖₛGₖₛAₖ | 52.3 | 2093 | 2108 | 415 |
| 533499 | GₑₛGₑₛ^{m}Cₑₛ^{m}C_{ds}^{m}C_{ds}^{m}C_{ds}G_{ds}G_{ds}A_{ds}G_{ds}T_{ds}^{m}C_{ds}G_{ds}AₖₛAₖₛGₖ | 54.9 | 2094 | 2109 | 416 |
| 533500 | GₑₛGₑₛGₑₛ^{m}C_{ds}^{m}C_{ds}^{m}C_{ds}^{m}C_{ds}G_{ds}G_{ds}A_{ds}G_{ds}T_{ds}^{m}C_{ds}GₖₛAₖₛAₖ | 46.7 | 2095 | 2110 | 417 |
| 533809 | Aₑₛ^{m}CₑₛAₑₛA_{ds}T_{ds}A_{ds}A_{ds}A_{ds}T_{ds}A_{ds}^{m}C_{ds}^{m}C_{ds}G_{ds}AₖₛGₖₛGₖ | 51.4 | 2773 | 2788 | 418 |

### Example 4: Design of antisense oligonucleotides targeting human dystrophia myotonica protein kinase (hDMPK)

### Dose Response HepG2

A series of antisense oligonucleotides (ASOs) were designed to target hDMPK. The newly designed ASOs were prepared using standard oligonucleotide synthesis well known in the art and are described in Table 8, below. Subscripts "s" indicate phosphorothioate internucleoside linkages; subscripts "k" indicate 6'-*(S)*-CH₃ bicyclic nucleosides (cEt); subscripts "e" indicate 2'-O-methoxyethyl (MOE) modified nucleosides; and subscripts "d" indicate β-D-2'-deoxyribonucleosides. "^{m}C" indicates 5-methylcytosine nucleosides.

The antisense oligonucleotides are targeted to SEQ ID NO: 1 (GENBANK Accession No. NM_001081560.1). "Start site" indicates the 5'-most nucleoside to which the gapmer is targeted in the human gene sequence. "Stop site" indicates the 3'-most nucleoside to which the gapmer is targeted human gene sequence.

**Table 8**

| Design of antisense oligonucleotides targeting hDMPK | | | | |
|---|---|---|---|---|
| ISIS No. | Composition (5' to 3') | Start Site | Stop Site | SEQ ID NO |
| 533440 | Gₑₛ^{m}Cₑₛ^{m}Cₑₛ^{m}C_{ds}A_{ds}^{m}C_{ds}A_{ds}G_{ds}^{m}C_{ds}^{m}C_{ds}T_{ds}G_{ds}^{m}C_{ds}AₖₛGₖₛGₖ | 931 | 946 | 357 |
| 533442 | ^{m}Cₑₛ^{m}CₑₛGₑₛ^{m}C_{ds}^{m}C_{ds}^{m}C_{ds}A_{ds}^{m}C_{ds}A_{ds}G_{ds}^{m}C_{ds}^{m}C_{ds}T_{ds}Gₖₛ^{m}CₖₛAₖ | 933 | 948 | 359 |
| 533443 | Aₑₛ^{m}Cₑₛ^{m}CₑₛG_{ds}^{m}C_{ds}^{m}C_{ds}^{m}C_{ds}A_{ds}^{m}C_{ds}A_{ds}G_{ds}^{m}C_{ds}^{m}C_{ds}TₖₛGₖₛ^{m}Cₖ | 934 | 949 | 360 |
| 533446 | ^{m}Cₑₛ^{m}Cₑₛ^{m}CₑₛA_{ds}^{m}C_{ds}^{m}C_{ds}G_{ds}^{m}C_{ds}^{m}C_{ds}^{m}C_{ds}A_{ds}^{m}C_{ds}A_{ds}Gₖₛ^{m}Cₖₛ^{m}Cₖ | 937 | 952 | 363 |
| 533447 | Gₑₛ^{m}Cₑₛ^{m}Cₑₛ^{m}C_{ds}A_{ds}^{m}C_{ds}^{m}C_{ds}G_{ds}^{m}C_{ds}^{m}C_{ds}^{m}C_{ds}A_{ds}^{m}C_{ds}AₖₛGₖₛ^{m}Cₖ | 938 | 953 | 364 |
| 533448 | ^{m}Cₑₛ^{m} CₑₛAₑₛG_{ds}G_{ds}^{m}C_{ds}^{m}C_{ds}^{m}C_{ds}A_{ds}^{m}C_{ds}^{m}C_{ds}G_{ds}^{m}C_{ds}^{m}Cₖₛ^{m}CₖₛAₖ | 942 | 957 | 365 |
| 533449 | ^{m}Cₑₛ^{m}Cₑₛ^{m}CₑₛA_{ds}G_{ds}G_{ds}^{m}C_{ds}^{m}C_{ds}C_{ds}A_{ds}^{m}C_{ds}^{m}C_{ds}G_{ds}^{m}Cₖₛ^{m}Cₖₛ^{m}Cₖ | 943 | 958 | 366 |
| 533462 | ^{m}CₑₛTₑₛTₑₛ^{m}C_{ds}^{m}C_{ds}^{m}C_{ds}G_{ds}A_{ds}A_{ds}T_{ds}G_{ds}T_{ds}^{m}C_{ds}^{m}CₖₛGₖₛAₖ | 1346 | 1361 | 379 |
| 533463 | ^{m}Cₑₛ^{m}CₑₛTₑₛT_{ds}^{m}C_{ds}^{m}C_{ds}^{m}C_{ds}G_{ds}A_{ds}A_{ds}T_{ds}G_{ds}T_{ds}^{m}Cₖₛ^{m}CₖₛGₖ | 1347 | 1362 | 380 |
| 533464 | Aₑₛ^{m}Cₑₛ^{m}CₑₛT_{ds}T_{ds}^{m}C_{ds}^{m}C_{ds}^{m}C_{ds}G_{ds}A_{ds}A_{ds}T_{ds}G_{ds}Tₖₛ^{m}Cₖₛ^{m}Cₖ | 1348 | 1363 | 381 |
| 533529 | ^{m}CₑₛGₑₛGₑₛT_{ds}T_{ds}G_{ds}T_{ds}G_{ds}A_{ds}A_{ds}^{m}C_{ds}T_{ds}G_{ds}Gₖₛ^{m}CₖₛAₖ | 2162 | 2177 | 23 |
| 533530 | AₑₛGₑₛ^{m}CₑₛG_{ds}G_{ds}T_{ds}T_{ds}G_{ds}T_{ds}G_{ds}A_{ds}A_{ds}^{m}C_{ds}TₖₛGₖₛGₖ | 2164 | 2179 | 419 |
| 533599 | Gₑₛ^{m}CₑₛAₑₛ^{m}C_{ds}T_{ds}T_{ds}T_{ds}G_{ds}^{m}C_{ds}G_{ds}A_{ds}A_{ds}^{m}C_{ds}^{m}CₖₛAₖₛAₖ | 2492 | 2507 | 420 |
| 533600 | TₑₛGₑₛ^{m}CₑₛA_{ds}^{m}C_{ds}T_{ds}T_{ds}T_{ds}G_{ds}^{m}C_{ds}G_{ds}A_{ds}A_{ds}^{m}Cₖₛ^{m}CₖₛAₖ | 2493 | 2508 | 421 |

### Example 5: Dose Response HepG2

Antisense oligonucleotides targeted to a human DMPK nucleic acid were tested for their effect on human DMPK RNA transcript *in vitro.* Cultured HepG2 cells at a density of 20,000 cells per well were transfected using electroporation with 625 nM, 1250 nM, 2500 nM, 5000 nM, and 10000.0 nM concentrations of each antisense oligonucleotide. After approximately 24 hours, RNA was isolated from the cells and DMPK RNA transcript levels were measured by quantitative real-time PCR using primer probe set RTS3164 (forward sequence AGCCTGAGCCGGGAGATG, designated herein as SEQ ID NO: 20; reverse sequence GCGTAGTTGACTGGCGAAGTT, designated herein as SEQ ID NO: 21; probe sequence AGGCCATCCGCACGGACAACCX, designated herein as SEQ ID NO: 22). Human DMPK RNA transcript levels were adjusted according to total RNA content, as measured by RIBOGREEN®. Results are presented in the table below as percent expression of human DMPK, relative to untreated control (UTC) cells. The tested antisense oligonucleotide sequences demonstrated dose-dependent inhibition of human DMPK mRNA levels under the conditions specified above.

**Table 9**

| Inhibition of human DMPK RNA transcript in HepG2 cells targeting SEQ ID NO: 1 | | | | |
|---|---|---|---|---|
| ISIS No. | Dose (nM) | % Target Expression | Start Site on Seq ID: 1 | Stop Site on Seq ID: 1 |
| UTC | N/A | 100 | N/A | N/A |
| 486178 | 625.0 | 39.4 | 2773 | 2788 |
| 486178 | 1250.0 | 31.2 | 2773 | 2788 |
| 486178 | 2500.0 | 20.6 | 2773 | 2788 |
| 486178 | 5000.0 | 13 | 2773 | 2788 |
| 486178 | 10000.0 | 11.5 | 2773 | 2788 |
| 533440 | 625.0 | 55.4 | 931 | 946 |
| 533440 | 1250.0 | 40.4 | 931 | 946 |
| 533440 | 2500.0 | 25.4 | 931 | 946 |
| 533440 | 5000.0 | 22.6 | 931 | 946 |
| 533440 | 10000.0 | 10.3 | 931 | 946 |
| 533442 | 625.0 | 55.2 | 933 | 948 |
| 533442 | 1250.0 | 33.1 | 933 | 948 |
| 533442 | 2500.0 | 29 | 933 | 948 |
| 533442 | 5000.0 | 16.9 | 933 | 948 |
| 533442 | 10000.0 | 7.2 | 933 | 948 |
| 533443 | 625.0 | 44.8 | 934 | 949 |
| 533443 | 1250.0 | 29.4 | 934 | 949 |
| 533443 | 2500.0 | 19.9 | 934 | 949 |
| 533443 | 5000.0 | 10.8 | 934 | 949 |
| 533443 | 10000.0 | 7 | 934 | 949 |
| 533446 | 625.0 | 50.9 | 937 | 952 |
| 533446 | 1250.0 | 35.5 | 937 | 952 |
| 533446 | 2500.0 | 30.4 | 937 | 952 |
| 533446 | 5000.0 | 14.6 | 937 | 952 |
| 533446 | 10000.0 | 14 | 937 | 952 |
| 533447 | 625.0 | 53.3 | 938 | 953 |
| 533447 | 1250.0 | 31.7 | 938 | 953 |
| 533447 | 2500.0 | 16.8 | 938 | 953 |
| 533447 | 5000.0 | 11.7 | 938 | 953 |
| 533447 | 10000.0 | 4.4 | 938 | 953 |
| 533448 | 625.0 | 58.8 | 942 | 957 |
| 533448 | 1250.0 | 36.9 | 942 | 957 |
| 533448 | 2500.0 | 24.8 | 942 | 957 |
| 533448 | 5000.0 | 11.5 | 942 | 957 |
| 533448 | 10000.0 | 10.1 | 942 | 957 |
| 533449 | 625.0 | 61.1 | 943 | 958 |
| 533449 | 1250.0 | 42.8 | 943 | 958 |
| 533449 | 2500.0 | 30.4 | 943 | 958 |
| 533449 | 5000.0 | 20.2 | 943 | 958 |
| 533449 | 10000.0 | 10.1 | 943 | 958 |
| 533462 | 625.0 | 50.7 | 1346 | 1361 |
| 533462 | 1250.0 | 32.3 | 1346 | 1361 |
| 533462 | 2500.0 | 29.2 | 1346 | 1361 |
| 533462 | 5000.0 | 12.5 | 1346 | 1361 |
| 533462 | 10000.0 | 5.8 | 1346 | 1361 |
| 533463 | 625.0 | 39.1 | 1347 | 1362 |
| 533463 | 1250.0 | 23.7 | 1347 | 1362 |
| 533463 | 2500.0 | 12.6 | 1347 | 1362 |
| 533463 | 5000.0 | 9.3 | 1347 | 1362 |
| 533463 | 10000.0 | 3.2 | 1347 | 1362 |
| 533464 | 625.0 | 48.8 | 1348 | 1363 |
| 533464 | 1250.0 | 36.4 | 1348 | 1363 |
| 533464 | 2500.0 | 24.5 | 1348 | 1363 |
| 533464 | 5000.0 | 11.7 | 1348 | 1363 |
| 533464 | 10000.0 | 5 | 1348 | 1363 |
| 533529 | 625.0 | 35.8 | 2162 | 2177 |
| 533529 | 1250.0 | 26.4 | 2162 | 2177 |
| 533529 | 2500.0 | 18.3 | 2162 | 2177 |
| 533529 | 5000.0 | 14.8 | 2162 | 2177 |
| 533529 | 10000.0 | 14.7 | 2162 | 2177 |
| 533530 | 625.0 | 47.4 | 2164 | 2179 |
| 533530 | 1250.0 | 22.1 | 2164 | 2179 |
| 533530 | 2500.0 | 21.5 | 2164 | 2179 |
| 533530 | 5000.0 | 14.4 | 2164 | 2179 |
| 533530 | 10000.0 | 8 | 2164 | 2179 |
| 533599 | 625.0 | 31.3 | 2492 | 2507 |
| 533599 | 1250.0 | 21.9 | 2492 | 2507 |
| 533599 | 2500.0 | 13.1 | 2492 | 2507 |
| 533599 | 5000.0 | 8.8 | 2492 | 2507 |
| 533599 | 10000.0 | 7.3 | 2492 | 2507 |
| 533600 | 625.0 | 33.8 | 2493 | 2508 |
| 533600 | 1250.0 | 20.9 | 2493 | 2508 |
| 533600 | 2500.0 | 16.5 | 2493 | 2508 |
| 533600 | 5000.0 | 10.4 | 2493 | 2508 |
| 533600 | 10000.0 | 12.1 | 2493 | 2508 |

### Example 6: Design of antisense oligonucleotides targeting human dystrophia myotonica protein kinase (hDMPK)

A series of antisense oligonucleotides (ASOs) were designed to target hDMPK. The newly designed ASOs were prepared using standard oligonucleotide synthesis well known in the art and are described in Table 10, below. Subscripts "s" indicate phosphorothioate internucleoside linkages; subscripts "k" indicate 6'-*(S)-*CH₃ bicyclic nucleosides (cEt); subscripts "e" indicate 2'-O-methoxyethyl (MOE) modified nucleosides; and subscripts "d" indicate β-D-2'-deoxyribonucleosides. "^{m}C" indicates 5-methylcytosine nucleosides.

The antisense oligonucleotides are targeted to SEQ ID NO: 2 (the complement of GENBANK Accession No. NT_011109.15 truncated from nucleotides 18540696 to 18555106). "Start site" indicates the 5'-most nucleoside to which the gapmer is targeted in the human gene sequence. "Stop site" indicates the 3'-most nucleoside to which the gapmer is targeted human gene sequence.

**Table 10**

| Design of antisense oligonucleotides targeting hDMPK | | | | |
|---|---|---|---|---|
| ISIS No. | Sequence | Start Site on Seq ID: 2 | Stop Site on Seq ID: 2 | Seq ID No. |
| UTC | N/A | N/A | N/A | |
| 486178 | Aₖₛ^{m}CₖₛAₖₛA_{ds}T_{ds}A_{ds}A_{ds}A_{ds}T_{ds}A_{ds}^{m}C_{ds}^{m}C_{ds}G_{ds}AₖₛGₖₛGₖ | 13836 | 13851 | 23 |
| 533597 | Aₑₛ^{m}CₑₛTₑₛT_{ds}T_{ds}G_{ds}^{m}C_{ds}G_{ds}A_{ds}A_{ds}^{m}C_{ds}^{m}C_{ds}A_{ds}Aₖₛ^{m}CₖₛGₖ | 13553 | 13568 | 422 |
| 533603 | AₑₛAₑₛAₑₛG _{ds}^{m}C_{ds}T_{ds}T_{ds}T_{ds}G_{ds}^{m}C_{ds}A_{ds}^{m}C_{ds}T_{ds}TₖₛTₖₛGₖ | 13563 | 13578 | 423 |
| 533617 | Tₑₛ^{m}cₑₛ^{m}cₑₛ^{m}C_{ds}G_{ds}A_{ds}G_{ds}T_{ds}A_{ds}A_{ds}G_{ds}^{m}C_{ds}A_{ds}GₖₛGₖₛ^{m}Cₖ | 13624 | 13639 | 424 |
| 533649 | Gₑₛ^{m}CₑₛAₑₛG_{ds}^{m}C_{ds}G_{ds}^{m}C_{ds}A_{ds}A_{ds}G_{ds}T_{ds}G_{ds}A_{ds}GₖₛGₖₛAₖ | 13686 | 13701 | 425 |
| 533694 | GₑₛTₑₛ^{m}CₑₛA_{ds}G_{ds}^{m}C_{ds}G_{ds}A_{ds}G_{ds}T_{ds}^{m}C_{ds}G_{ds}G_{ds}AₖₛGₖₛGₖ | 13760 | 13775 | 426 |
| 533697 | ^{m}Cₑₛ^{m}CₑₛTₑₛG_{ds}T_{ds}^{m}C_{ds}A_{ds}G_{ds}^{m}C_{ds}G_{ds}A_{ds}G_{ds}T_{ds}^{m}CₖₛGₖₛGₖ | 13763 | 13778 | 427 |
| 533698 | Gₑₛ^{m}Cₑₛ^{m}CₑₛT_{ds}G_{ds}T_{ds}^{m}C_{ds}A_{ds}G_{ds}^{m}C_{ds}G_{ds}A_{ds}G_{ds}Tₖₛ^{m}CₖₛGₖ | 13764 | 13779 | 428 |
| 533699 | AₑₛGₑₛ^{m}Cₑₛ^{m}C_{ds}T_{ds}G_{ds}T_{ds}^{m}C_{ds}A_{ds}G_{ds}^{m}C_{ds}G_{ds}A_{ds}GₖₛTₖₛ^{m}Cₖ | 13765 | 13780 | 429 |
| 533711 | GₑₛGₑₛGₑₛT_{ds}^{m}C_{ds}T_{ds}^{m}C_{ds}A_{ds}G_{ds}T_{ds}G_{ds}^{m}C_{ds}A_{ds}Tₖₛ^{m}Cₖₛ^{m}Cₖ | 13813 | 13828 | 430 |
| 533721 | AₑₛGₑₛGₑₛT_{ds}T_{ds}T_{ds}T_{ds}T_{ds}^{m}C_{ds}^{m}C_{ds}A_{ds}G_{ds}A_{ds}GₖₛGₖₛ^{m}Cₖ | 2580 | 2595 | 431 |
| 533722 | AₑₛAₑₛGₑₛG_{ds}T_{ds}T_{ds}T_{ds}T_{ds}T_{ds}^{m}C_{ds}^{m}C_{ds}A_{ds}G_{ds}AₖₛGₖₛGₖ | 2581 | 2596 | 432 |
| 533751 | GₑₛGₑₛTₑₛ^{m}C_{ds}A_{ds}^{m}C_{ds}T_{ds}G_{ds}^{m}C_{ds}T_{ds}G_{ds}G_{ds}G_{ds}Tₖₛ^{m}Cₖₛ^{m}Cₖ | 6446 | 6461 | 433 |
| 533786 | GₑₛTₑₛGₑₛG_{ds}T_{ds}T_{ds}T_{ds}^{m}C_{ds}T_{ds}G_{ds}T_{ds}^{m}C_{ds}T_{ds}Gₖₛ^{m}CₖₛTₖ | 11099 | 11114 | 434 |
| 533787 | ^{m}CₑₛGₑₛTₑₛG_{ds}G_{ds}T_{ds}T_{ds}T_{ds}^{m}C_{ds}T_{ds}G_{ds}T_{ds}^{m}C_{ds}TₖₛGₖₛ^{m}Cₖ | 11100 | 11115 | 435 |

### Example 7: Dose Response for ASOs targeted to a human DMPK RNA transcript in HepG2 cells

Antisense oligonucleotides targeted to a human DMPK nucleic acid were tested for their effect on human DMPK RNA transcript *in vitro.* Cultured HepG2 cells at a density of 20,000 cells per well were transfected using electroporation with 625 nM, 1250 nM, 2500 nM, 5000 nM, and 10000.0 nM concentrations of each antisense oligonucleotide. After approximately 24 hours, RNA was isolated from the cells and DMPK RNA transcript levels were measured by quantitative real-time PCR using primer probe set RTS3164 (forward sequence AGCCTGAGCCGGGAGATG, designated herein as SEQ ID NO: 20; reverse sequence GCGTAGTTGACTGGCGAAGTT, designated herein as SEQ ID NO: 21; probe sequence AGGCCATCCGCACGGACAACCX, designated herein as SEQ ID NO: 22). Human DMPK RNA transcript levels were adjusted according to total RNA content, as measured by RIBOGREEN®. Results are presented as percent expression of human DMPK, relative to untreated control (UTC) cells and are shown in the table below. The tested antisense oligonucleotide sequences demonstrated dose-dependent inhibition of human DMPK mRNA levels under the conditions specified above.

**Table 11**

| Inhibition of human DMPK RNAtranscript in HepG2 cells targeting SEQ ID NO: 1 | | | | |
|---|---|---|---|---|
| ISIS No. | Dose (nM) | % Target Expression | Start Site on Seq ID: 2 | Stop Site on Seq ID: 2 |
| UTC | NA | 100 | N/A | N/A |
| 486178 | 625.000 | 39.4 | 13836 | 13851 |
| 486178 | 1250.000 | 27.3 | 13836 | 13851 |
| 486178 | 2500.000 | 14 | 13836 | 13851 |
| 486178 | 5000.000 | 16.3 | 13836 | 13851 |
| 486178 | 10000.000 | 8.3 | 13836 | 13851 |
| 533597 | 625.000 | 42.4 | 13553 | 13568 |
| 533597 | 1250.000 | 30.3 | 13553 | 13568 |
| 533597 | 2500.000 | 15.3 | 13553 | 13568 |
| 533597 | 5000.000 | 10 | 13553 | 13568 |
| 533597 | 10000.000 | 10.6 | 13553 | 13568 |
| 533603 | 625.000 | 48.2 | 13563 | 13578 |
| 533603 | 1250.000 | 31.1 | 13563 | 13578 |
| 533603 | 2500.000 | 22.4 | 13563 | 13578 |
| 533603 | 5000.000 | 15.6 | 13563 | 13578 |
| 533603 | 10000.000 | 9.9 | 13563 | 13578 |
| 533617 | 625.000 | 38.4 | 13624 | 13639 |
| 533617 | 1250.000 | 26.3 | 13624 | 13639 |
| 533617 | 2500.000 | 21.6 | 13624 | 13639 |
| 533617 | 5000.000 | 15.8 | 13624 | 13639 |
| 533617 | 10000.000 | 14.6 | 13624 | 13639 |
| 533649 | 625.000 | 52.2 | 13686 | 13701 |
| 533649 | 1250.000 | 27.8 | 13686 | 13701 |
| 533649 | 2500.000 | 24.6 | 13686 | 13701 |
| 533649 | 5000.000 | 20.5 | 13686 | 13701 |
| 533649 | 10000.000 | 14.5 | 13686 | 13701 |
| 533694 | 625.000 | 53.3 | 13760 | 13775 |
| 533694 | 1250.000 | 29.4 | 13760 | 13775 |
| 533694 | 2500.000 | 23.6 | 13760 | 13775 |
| 533694 | 5000.000 | 18.7 | 13760 | 13775 |
| 533694 | 10000.000 | 13.5 | 13760 | 13775 |
| 533697 | 625.000 | 30.6 | 13763 | 13778 |
| 533697 | 1250.000 | 14.9 | 13763 | 13778 |
| 533697 | 2500.000 | 13.8 | 13763 | 13778 |
| 533697 | 5000.000 | 9.7 | 13763 | 13778 |
| 533697 | 10000.000 | 7.1 | 13763 | 13778 |
| 533698 | 625.000 | 23.4 | 13764 | 13779 |
| 533698 | 1250.000 | 15.5 | 13764 | 13779 |
| 533698 | 2500.000 | 13.8 | 13764 | 13779 |
| 533698 | 5000.000 | 12.4 | 13764 | 13779 |
| 533698 | 10000.000 | 10.2 | 13764 | 13779 |
| 533699 | 625.000 | 38.2 | 13765 | 13780 |
| 533699 | 1250.000 | 26.9 | 13765 | 13780 |
| 533699 | 2500.000 | 17.6 | 13765 | 13780 |
| 533699 | 5000.000 | 12.9 | 13765 | 13780 |
| 533699 | 10000.000 | 9.3 | 13765 | 13780 |
| 533711 | 625.000 | 35.1 | 13813 | 13828 |
| 533711 | 1250.000 | 34.6 | 13813 | 13828 |
| 533711 | 2500.000 | 22.4 | 13813 | 13828 |
| 533711 | 5000.000 | 22 | 13813 | 13828 |
| 533711 | 10000.000 | 13 | 13813 | 13828 |
| 533721 | 625.000 | 36.3 | 2580 | 2595 |
| 533721 | 1250.000 | 29.8 | 2580 | 2595 |
| 533721 | 2500.000 | 23.2 | 2580 | 2595 |
| 533721 | 5000.000 | 17.8 | 2580 | 2595 |
| 533721 | 10000.000 | 17.2 | 2580 | 2595 |
| 533722 | 625.000 | 48.5 | 2581 | 2596 |
| 533722 | 1250.000 | 28.6 | 2581 | 2596 |
| 533722 | 2500.000 | 21.9 | 2581 | 2596 |
| 533722 | 5000.000 | 28.1 | 2581 | 2596 |
| 533722 | 10000.000 | 13.8 | 2581 | 2596 |
| 533751 | 625.000 | 37.7 | 6446 | 6461 |
| 533751 | 1250.000 | 21.6 | 6446 | 6461 |
| 533751 | 2500.000 | 12.6 | 6446 | 6461 |
| 533751 | 5000.000 | 9.7 | 6446 | 6461 |
| 533751 | 10000.000 | 8.5 | 6446 | 6461 |
| 533786 | 625.000 | 53.6 | 11099 | 11114 |
| 533786 | 1250.000 | 26.6 | 11099 | 11114 |
| 533786 | 2500.000 | 14.7 | 11099 | 11114 |
| 533786 | 5000.000 | 9.6 | 11099 | 11114 |
| 533786 | 10000.000 | 5.5 | 11099 | 11114 |
| 533787 | 625.000 | 43.8 | 11100 | 11115 |
| 533787 | 1250.000 | 27.7 | 11100 | 11115 |
| 533787 | 2500.000 | 16.3 | 11100 | 11115 |
| 533787 | 5000.000 | 7 | 11100 | 11115 |
| 533787 | 10000.000 | 4.5 | 11100 | 11115 |

### Example 8: ASOs designed to target a human DMPK RNA transcript

A series of antisense oligonucleotides (ASOs) were designed to target hDMPK. The newly designed ASOs were prepared using standard oligonucleotide synthesis well known in the art and are described in Table 12, below. Subscripts "s" indicate phosphorothioate internucleoside linkages; subscripts "k" indicate 6'-*(S)*-CH₃ bicyclic nucleosides (cEt); subscripts "e" indicate 2'-O-methoxyethyl (MOE) modified nucleosides; and subscripts "d" indicate β-D-2'-deoxyribonucleosides. "^{m}C" indicates 5-methylcytosine nucleosides.

The antisense oligonucleotides targeted to a human DMPK nucleic acid were tested for their effect on DMPK RNA transcript *in vitro.* Cultured hSKMC cells at a density of 20,000 cells per well were transfected using electroporation with 800 nM antisense oligonucleotide. After approximately 24 hours, RNA was isolated from the cells and DMPK transcript levels were measured by quantitative real-time PCR. DMPK RNA transcript levels were adjusted according to total RNA content, as measured by RIBOGREEN^{®}. Results are presented as percent expression of DMPK, relative to untreated control cells.

'Target start site' indicates the 5'-most nucleoside to which the antisense oligonucleotide is targeted in the human genomic gene sequence. 'Target stop site' indicates the 3'-most nucleoside to which the antisense oligonucleotide is targeted in the human genomic sequence. All the antisense oligonucleotides listed in Table 12 target SEQ ID NO: 1 (GENBANK Accession No. NM_001081560.1).

Several of the antisense oligonucleotides demonstrated significant inhibition of DMPK mRNA levels under the conditions specified above.

**Table 12**

| Inhibition of human DMPK RNA transcript in HepG2 cells using ASOs targeting SEQ ID NO: 1 | | | | | |
|---|---|---|---|---|---|
| ISIS No. | Sequence | % Target Expression | Start Site on Seq ID: 1 | Stop Site on Seq ID: 1 | Seq ID No. |
| UTC | N/A | 100 | N/A | N/A | |
| 444401 | TₑₛTₑₛGₑₛ^{m}CₑₛAₑₛ^{m}C_{ds}T_{ds}T_{ds}T_{ds}G_{ds}^{m}C_{ds}G_{ds}A_{ds}A_{ds}^{m}C_{ds}^{m}CₑₛAₑₛAₑₛ^{m}CₑₛGₑ | 25.2 | 2490 | 2509 | 33 |
| 444436 | GₑₛTₑₛ^{m}CₑₛGₑₛGₑₛA_{ds}G_{ds}G_{ds}A_{ds}^{m}C_{ds}G_{ds}A_{ds}G_{ds}G_{ds}T_{ds}^{m}CₑₛAₑₛAₑₛTₑₛAₑ | 30.8 | 2685 | 2704 | 264 |
| 486072 | AₖₛAₖₛGₖₛA_{ds}^{m}C_{ds}A_{ds}G_{ds}T_{ds}T_{ds}^{m}C_{ds}T_{ds}A_{ds}G_{ds}GₖₛGₖₛTₖ | 36.8 | 2081 | 2096 | 403 |
| 486073 | ^{m}CₖₛGₖₛAₖₛA_{ds}G_{ds}A_{ds}^{m}C_{ds}A_{ds}G_{ds}T_{ds}T_{ds}^{m}C_{ds}T_{ds}AₖₛGₖₛGₖ | 22.4 | 2083 | 2098 | 405 |
| 486075 | GₖₛTₖₛ^{m}CₖₛG_{ds}A_{ds}A_{ds}G_{ds}A_{ds}^{m}C_{ds}A_{ds}G_{ds}T_{ds}T_{ds}^{m}CₖₛTₖₛAₖ | 41.3 | 2085 | 2100 | 407 |
| 486076 | AₖₛGₖₛTₖₛ^{m}C_{ds}G_{ds}A_{ds}A_{ds}G_{ds}A_{ds}^{m}C_{ds}A_{ds}G_{ds}T_{ds}Tₖₛ^{m}CₖₛTₖ | 22.4 | 2086 | 2101 | 408 |
| 486077 | GₖₛAₖₛGₖₛT_{ds}^{m}C_{ds}G_{ds}A_{ds}A_{ds}G_{ds}A_{ds}^{m}C_{ds}A_{ds}G_{ds}TₖₛTₖₛ^{m}Cₖ | 35.2 | 2087 | 2102 | 409 |
| 486078 | ^{m}CₖₛGₖₛGₖₛA_{ds}G_{ds}T_{ds}^{m}C_{ds}G_{ds}A_{ds}A_{ds}G_{ds}A_{ds}^{m}C_{ds}AₖₛGₖₛTₖ | 12.4 | 2089 | 2104 | 411 |
| 486079 | ^{m}Cₖₛ^{m}Cₖₛ^{m}CₖₛG_{ds}G_{ds}A_{ds}G_{ds}T_{ds}^{m}C_{ds}G_{ds}A_{ds}A_{ds}G_{ds}Aₖₛ^{m}CₖₛAₖ | 36.5 | 2091 | 2106 | 413 |
| 486080 | ^{m}Cₖₛ^{m}Cₖₛ^{m}Cₖₛ^{m}C_{ds}G_{ds}G_{ds}A_{ds}G_{ds}T_{ds}^{m}C_{ds}G_{ds}A_{ds}A_{ds}GₖₛAₖₛ^{m}Cₖ | 19.9 | 2092 | 2107 | 414 |
| 486085 | GₖₛAₖₛAₖₛ^{m}C_{ds}T_{ds}G_{ds}G_{ds}^{m}C_{ds}A_{ds}G_{ds}G_{ds}^{m}C_{ds}G_{ds}GₖₛTₖₛGₖ | 30.1 | 2155 | 2170 | 436 |
| 486086 | TₖₛGₖₛTₖₛG_{ds}A_{ds}A_{ds}^{m}C_{ds}T_{ds}G_{ds}G_{ds}^{m}C_{ds}A_{ds}G_{ds}Gₖₛ^{m}CₖₛGₖ | 17.2 | 2158 | 2173 | 437 |
| 486087 | GₖₛGₖₛTₖₛT_{ds}G_{ds}T_{ds}G_{ds}A_{ds}A_{ds}^{m}C_{ds}T_{ds}G_{ds}G_{ds}^{m}CₖₛAₖₛGₖ | 11.5 | 2161 | 2176 | 438 |
| 486088 | GₖₛAₖₛGₖₛ^{m}C_{ds}G_{ds}G_{ds}T_{ds}T_{ds}G_{ds}T_{ds}G_{ds}A_{ds}A_{ds}^{m}CₖₛTₖₛGₖ | 21.7 | 2165 | 2180 | 439 |
| 486094 | Aₖₛ^{m}CₖₛTₖₛG_{ds}G_{ds}A_{ds}G_{ds}^{m}C_{ds}T_{ds}G_{ds}G_{ds}G_{ds}^{m}C_{ds}GₖₛGₖₛAₖ | 30.2 | 2193 | 2208 | 440 |
| 486096 | AₖₛGₖₛGₖₛA_{ds}^{m}C_{ds}T_{ds}G_{ds}G_{ds}A_{ds}G_{ds}^{m}C_{ds}T_{ds}G_{ds}GₖₛGₖₛ^{m}Cₖ | 43.5 | 2196 | 2211 | 441 |
| 486097 | Tₖₛ^{m}CₖₛAₖₛ^{m}C_{ds}A_{ds}G_{ds}G_{ds}A_{ds}^{m}C_{ds}T_{ds}G_{ds}G_{ds}A_{ds}Gₖₛ^{m}CₖₛTₖ | 54.5 | 2200 | 2215 | 442 |
| 486098 | AₖₛTₖₛ^{m}CₖₛA_{ds}^{m}C_{ds}A_{ds}G_{ds}G_{ds}A_{ds}^{m}C_{ds}T_{ds}G_{ds}G_{ds}AₖₛGₖₛ^{m}Cₖ | 77.3 | 2201 | 2216 | 443 |
| 486099 | GₖₛGₖₛAₖₛT_{ds}^{m}C_{ds}A_{ds}^{m}C_{ds}A_{ds}G_{ds}G_{ds}A_{ds}^{m}C_{ds}T_{ds}GₖₛGₖₛAₖ | 24.8 | 2203 | 2218 | 444 |
| 486101 | ^{m}CₖₛAₖₛGₖₛ^{m}C_{ds}^{m}C_{ds}T_{ds}G_{ds}G_{ds}^{m}C_{ds}^{m}C_{ds}G_{ds}A_{ds}A_{ds}AₖₛGₖₛAₖ | 31.6 | 2386 | 2401 | 445 |
| 486102 | ^{m}CₖₛTₖₛ^{m}CₖₛA_{ds}G_{ds}^{m}C_{ds}^{m}C_{ds}T_{ds}G_{ds}G_{ds}^{m}C_{ds}^{m}C_{ds}G_{ds}AₖₛAₖₛAₖ | 35.1 | 2388 | 2403 | 446 |
| 486104 | GₖₛTₖₛ^{m}CₖₛA_{ds}G_{ds}G_{ds}G_{ds}^{m}C_{ds}^{m}C_{ds}T_{ds}^{m}C_{ds}A_{ds}G_{ds}^{m}Cₖₛ^{m}CₖₛTₖ | 26.9 | 2396 | 2411 | 447 |
| 486105 | ^{m}CₖₛGₖₛTₖₛ^{m}C_{ds}A_{ds}G_{ds}G_{ds}G_{ds}^{m}C_{ds}^{m}C_{ds}T_{ds}^{m}C_{ds}A_{ds}Gₖₛ^{m}Cₖₛ^{m}Cₖ | 48.4 | 2397 | 2412 | 448 |
| 486110 | TₖₛTₖₛTₖₛG_{ds}^{m}C_{ds}A_{ds}^{m}C_{ds}T_{ds}T_{ds}T_{ds}G_{ds}^{m}C_{ds}G_{ds}AₖₛAₖₛ^{m}Cₖ | 31.6 | 2495 | 2510 | 449 |
| 486111 | GₖₛAₖₛAₖₛA_{ds}G_{ds}^{m}C_{ds}T_{ds}T_{ds}T_{ds}G_{ds}^{m}C_{ds}A_{ds}^{m}C_{ds}TₖₛTₖₛTₖ | 31.9 | 2501 | 2516 | 450 |
| 486112 | AₖₛAₖₛTₖₛT_{ds}T_{ds}^{m}C_{ds}^{m}C_{ds}^{m}C_{ds}G_{ds}A_{ds}G_{ds}T_{ds}A_{ds}AₖₛGₖₛ^{m}Cₖ | 47.4 | 2565 | 2580 | 451 |
| 486115 | Gₖₛ^{m}CₖₛAₖₛA_{ds}A_{ds}T_{ds}T_{ds}T_{ds}^{m}C_{ds}^{m}C_{ds}^{m}C_{ds}G_{ds}A_{ds}GₖₛTₖₛAₖ | 20.8 | 2568 | 2583 | 452 |
| 486116 | AₖₛGₖₛ^{m}CₖₛA_{ds}A_{ds}A_{ds}T_{dS}T_{dS}T_{dS}^{m}C_{ds}^{m}C_{ds}^{m}C_{dS}G_{ds}AₖₛG_{kS}Tₖ | 23.9 | 2569 | 2584 | 453 |
| 486117 | AₖₛAₖₛGₖₛ^{m}C_{ds}A_{ds}A_{ds}A_{ds}T_{ds}T_{ds}T_{ds}^{m}C_{ds}^{m}C_{ds}^{m}C_{ds}GₖₛAₖₛGₖ | 22 | 2570 | 2585 | 454 |
| 486118 | AₖₛAₖₛAₖₛG_{ds}^{m}C_{ds}A_{ds}A_{ds}A_{ds}T_{ds}T_{ds}T_{ds}^{m}C_{ds}^{m}C_{ds}^{m}CₖₛGₖₛAₖ | 26.7 | 2571 | 2586 | 455 |
| 486119 | AₖₛAₖₛAₖₛA_{ds}G_{ds}^{m}C_{ds}A_{ds}A_{ds}A_{ds}T_{ds}T_{ds}T_{ds}^{m}C_{ds}^{m}Cₖₛ^{m}CₖₛGₖ | 33.5 | 2572 | 2587 | 456 |
| 486120 | Gₖₛ^{m}CₖₛAₖₛA_{ds}A_{ds}A_{ds}G_{ds}^{m}C_{ds}A_{ds}A_{ds}A_{ds}T_{ds}T_{ds}Tₖₛ^{m}Cₖₛ^{m}Cₖ | 51.4 | 2574 | 2589 | 457 |
| 486121 | GₖₛGₖₛ^{m}CₖₛA_{ds}A_{ds}A_{ds}A_{ds}G_{ds}^{m}C_{ds}A_{ds}A_{ds}A_{ds}T_{ds}TₖₛTₖₛ^{m}Cₖ | 60.8 | 2575 | 2590 | 458 |
| 486123 | TₖₛTₖₛGₖₛG_{ds} C_{ds}A_{ds}A_{ds}A_{ds}A_{ds}G_{ds} C_{ds}A_{ds}A_{ds}AₖₛTₖₛTₖ | 39.8 | 2577 | 2592 | 459 |
| 486125 | GₖₛTₖₛTₖₛT_{ds}G_{ds}G_{ds}C_{ds}A_{ds}A_{ds}A_{ds}A_{ds}G_{ds}C_{ds}AₖₛAₖₛAₖ | 32.7 | 2579 | 2594 | 460 |
| 486126 | GₖₛGₖₛTₖₛT_{ds}T_{ds}G_{ds}G_{ds}C_{ds}A_{ds}A_{ds}A_{ds}A_{ds}G_{ds}CₖₛAₖₛAₖ | 19.2 | 2580 | 2595 | 461 |
| 486127 | GₖₛGₖₛGₖₛT_{ds}T_{ds}T_{ds}G_{ds}G_{ds}^{m}C_{ds}A_{ds}A_{ds}A_{ds}A_{ds}Gₖₛ^{m}CₖₛAₖ | 36.1 | 2581 | 2596 | 462 |
| 486128 | Gₖₛ^{m}CₖₛGₖₛG_{ds}G_{ds}T_{ds}T_{ds}T_{ds}G_{ds}G_{ds}^{m}C_{ds}A_{ds}A_{ds}AₖₛAₖₛGₖ | 39.1 | 2583 | 2598 | 463 |
| 486129 | AₖₛGₖₛ^{m}CₖₛG_{ds}G_{ds}G_{ds}T_{ds}T_{ds}T_{ds}G_{ds}G_{ds}^{m}C_{ds}A_{ds}AₖₛAₖₛAₖ | 31.4 | 2584 | 2599 | 464 |
| 486130 | AₖₛAₖₛGₖₛ^{m}C_{ds}G_{ds}G_{ds}G_{ds}T_{ds}T_{ds}T_{ds}G_{ds}G_{ds}^{m}C_{ds}AₖₛAₖₛAₖ | 35.7 | 2585 | 2600 | 465 |
| 486133 | ^{m}CₖₛTₖₛ^{m}Cₖₛ^{m}C_{ds}G_{ds}A_{ds}G_{ds}A_{ds}G_{ds}^{m}C_{ds}A_{ds}G_{ds}^{m}C_{ds}Gₖₛ^{m}CₖₛAₖ | 45.9 | 2631 | 2646 | 466 |
| 486134 | Gₖₛ^{m}CₖₛTₖₛ^{m}C_{ds}^{m}C_{ds}G_{ds}A_{ds}G_{ds}A_{ds}G_{ds}^{m}C_{ds}A_{ds}G_{ds}^{m}CₖₛGₖₛ^{m}Cₖ | 29.5 | 2632 | 2647 | 467 |
| 486135 | GₖₛGₖₛ^{m}CₖₛT_{ds}^{m}C_{ds}^{m}C_{ds}G_{ds}A_{ds}G_{ds}A_{ds}G_{ds}^{m}C_{ds}A_{ds}Gₖₛ^{m}CₖₛGₖ | 51.4 | 2633 | 2648 | 468 |
| 486142 | TₖₛAₖₛAₖₛA_{ds}T_{ds}A_{ds}T_{ds}^{m}C_{ds}^{m}C_{ds}A_{ds}A_{ds}A_{ds}^{m}C_{ds}^{m}CₖₛGₖₛ^{m}Cₖ | 64.4 | 2671 | 2686 | 469 |
| 486147 | GₖₛTₖₛ^{m}CₖₛA_{ds}A_{ds}T_{ds}A_{ds}A_{ds}A_{ds}T_{ds}A_{ds}T_{ds}^{m}C_{ds}^{m}CₖₛAₖₛAₖ | 16.1 | 2676 | 2691 | 470 |
| 486148 | AₖₛGₖₛGₖₛT_{ds}^{m}C_{ds}A_{ds}A_{ds}T_{ds}A_{ds}A_{ds}A_{ds}T_{d}sA_{d}sTₖs^{m}Cₖs^{m}Cₖ | 18.3 | 2678 | 2693 | 471 |
| 486149 | CₖₛGₖₛAₖₛG_{ds}G_{ds}T_{ds}C_{ds}A_{ds}A_{ds}T_{ds}A_{ds}A_{ds}A_{ds}TₖₛAₖₛTₖ | 37.9 | 2680 | 2695 | 472 |
| 486150 | Aₖₛ^{m}CₖₛGₖₛA_{ds}G_{ds}G_{ds}T_{ds}^{m}C_{ds}A_{ds}A_{ds}T_{ds}A_{ds}A_{ds}AₖₛTₖₛAₖ | 45.3 | 2681 | 2696 | 473 |
| 486151 | GₖₛAₖₛCₖₛG_{ds}A_{ds}G_{ds}G_{ds}T_{ds}C_{ds}A_{ds}A_{ds}T_{ds}A_{ds}AₖₛAₖₛTₖ | 52.2 | 2682 | 2697 | 474 |
| 486152 | GₖₛGₖₛAₖₛC_{ds}G_{ds}A_{ds}G_{ds}G_{ds}T_{ds}C_{ds}A_{ds}A_{ds}T_{ds}AₖₛAₖₛAₖ | 19.8 | 2683 | 2698 | 475 |
| 486153 | AₖₛGₖₛGₖₛA_{ds} C_{ds}G_{ds}A_{ds}G_{ds}G_{ds}T_{ds} C_{ds}A_{ds}A_{ds}TₖₛAₖₛAₖ | 19.9 | 2684 | 2699 | 476 |
| 486154 | GₖₛAₖₛGₖₛG_{ds}A_{ds}C_{ds}G_{ds}A_{ds}G_{ds}G_{ds}T_{ds}C_{ds}A_{ds}AₖₛTₖₛAₖ | 19.6 | 2685 | 2700 | 477 |
| 486155 | GₖₛGₖₛAₖₛG_{ds}G_{ds}A_{ds}^{m}C_{ds}G_{ds}A_{ds}G_{ds}G_{ds}T_{ds}^{m}C_{ds}AₖₛAₖₛTₖ | 38.3 | 2686 | 2701 | 478 |
| 486156 | ^{m}CₖₛGₖₛGₖₛA_{ds}G_{ds}G_{ds}A_{ds}^{m}C_{ds}G_{ds}A_{ds}G_{ds}G_{ds}T_{ds}^{m}CₖₛAₖₛAₖ | 14.1 | 2687 | 2702 | 479 |
| 486157 | Tₖₛ^{m}CₖₛGₖₛG_{ds}A_{ds}G_{ds}G_{ds}A_{ds}^{m}C_{ds}G_{ds}A_{ds}G_{ds}G_{ds}Tₖₛ^{m}CₖₛAₖ | 23.2 | 2688 | 2703 | 480 |
| 486158 | GₖₛTₖₛ^{m}CₖₛG_{ds}G_{ds}A_{ds}G_{ds}G_{ds}A_{ds}^{m}C_{ds}G_{ds}A_{ds}G_{ds}GₖₛTₖₛ^{m}Cₖ | 34.5 | 2689 | 2704 | 481 |
| 486159 | AₖₛGₖₛTₖₛ^{m}C_{ds}G_{ds}G_{ds}A_{ds}G_{ds}G_{ds}A_{ds}^{m}C_{ds}G_{ds}A_{ds}GₖₛGₖₛTₖ | 23.7 | 2690 | 2705 | 482 |
| 486160 | GₖₛAₖₛGₖₛT_{ds}^{m}C_{ds}G_{ds}G_{ds}A_{ds}G_{ds}G_{ds}A_{ds}^{m}C_{ds}G_{ds}AₖₛGₖₛGₖ | 14.3 | 2691 | 2706 | 483 |
| 486161 | ^{m}CₖₛGₖₛAₖₛG_{ds}T_{ds}^{m}C_{ds}G_{ds}G_{ds}A_{ds}G_{ds}G_{ds}A_{ds}^{m}C_{ds}GₖₛAₖₛGₖ | 29 | 2692 | 2707 | 484 |
| 486162 | AₖₛGₖₛ^{m}CₖₛG_{ds}A_{ds}G_{ds}T_{ds}^{m}C_{ds}G_{ds}G_{ds}A_{ds}G_{ds}G_{ds}Aₖₛ^{m}CₖₛGₖ | 20.6 | 2694 | 2709 | 485 |
| 486163 | ^{m}CₖₛAₖₛGₖₛ^{m}C_{ds}G_{ds}A_{ds}G_{ds}T_{ds}^{m}C_{ds}G_{ds}G_{ds}A_{ds}G_{ds}GₖₛAₖₛ^{m}Cₖ | 29 | 2695 | 2710 | 486 |
| 486164 | Tₖₛ^{m}CₖₛAₖₛG_{ds}^{m}C_{ds}G_{ds}A_{ds}G_{ds}T_{ds}^{m}C_{ds}G_{ds}G_{ds}A_{ds}GₖₛGₖₛAₖ | 17 | 2696 | 2711 | 487 |
| 486165 | GₖₛTₖₛ^{m}CₖₛA_{ds}G_{ds}^{m}C_{ds}G_{ds}A_{ds}G_{ds}T_{ds}^{m}C_{ds}G_{ds}G_{ds}AₖₛGₖₛGₖ | 14.2 | 2697 | 2712 | 426 |
| 486166 | TₖₛGₖₛTₖₛ^{m}C_{ds}A_{ds}G_{ds}^{m}C_{ds}G_{ds}A_{ds}G_{ds}T_{ds}^{m}C_{ds}G_{ds}GₖₛAₖₛGₖ | 25.1 | 2698 | 2713 | 488 |
| 486167 | ^{m}CₖₛTₖₛGₖₛT_{ds}^{m}C_{ds}A_{ds}G_{ds}^{m}C_{ds}G_{ds}A_{ds}G_{ds}T_{ds}^{m}C_{ds}GₖₛGₖₛAₖ | 15 | 2699 | 2714 | 489 |
| 486168 | ^{m}Cₖₛ^{m}CₖₛTₖₛG_{ds}T_{ds}^{m}C_{ds}A_{ds}G_{ds}^{m}C_{ds}G_{ds}A_{ds}G_{ds}T_{ds}^{m}CₖₛGₖₛGₖ | 12.4 | 2700 | 2715 | 427 |
| 486169 | Gₖₛ^{m}Cₖₛ^{m}CₖₛT_{ds}G_{ds}T_{ds}^{m}C_{ds}A_{ds}G_{ds}^{m}C_{ds}G_{ds}A_{ds}G_{ds}Tₖₛ^{m}CₖₛGₖ | 24.5 | 2701 | 2716 | 428 |
| 486170 | AₖₛGₖₛ^{m}Cₖₛ^{m}C_{ds}T_{ds}G_{ds}T_{ds}^{m}C_{ds}A_{ds}G_{ds}^{m}C_{ds}G_{ds}A_{ds}GₖₛTₖₛ^{m}Cₖ | 16.3 | 2702 | 2717 | 429 |
| 486171 | ^{m}CₖₛAₖₛGₖₛT_{ds}G_{ds}^{m}C_{ds}A_{ds}T_{ds}^{m}C_{ds}^{m}C_{ds}A_{ds}A_{ds}A_{ds}Aₖₛ^{m}CₖₛGₖ | 31.8 | 2744 | 2759 | 490 |
| 486172 | Tₖₛ^{m}CₖₛAₖₛG_{ds}T_{ds}G_{ds}^{m}C_{ds}A_{ds}T_{ds}^{m}C_{ds}^{m}C_{ds}A_{ds}A_{ds}AₖₛAₖₛ^{m}Cₖ | 23.1 | 2745 | 2760 | 491 |
| 486173 | ^{m}CₖₛTₖₛ^{m}CₖₛA_{ds}G_{ds}T_{ds}G_{ds}^{m}C_{ds}A_{ds}T_{ds}^{m}C_{ds}^{m}C_{ds}A_{ds}AₖₛAₖₛAₖ | 23 | 2746 | 2761 | 492 |
| 486174 | Tₖₛ^{m}CₖₛTₖₛ^{m}C_{ds}A_{ds}G_{ds}T_{ds}G_{ds}^{m}C_{ds}A_{ds}T_{ds}^{m}C_{ds}^{m}C_{ds}AₖₛAₖₛAₖ | 50.9 | 2747 | 2762 | 493 |
| 486175 | GₖₛTₖₛ^{m}CₖₛT_{ds}^{m}C_{ds}A_{ds}G_{ds}T_{ds}G_{ds}^{m}C_{ds}A_{ds}T_{ds}^{m}C_{ds}^{m}CₖₛAₖₛAₖ | 17.2 | 2748 | 2763 | 494 |
| 486176 | GₖₛGₖₛGₖₛT_{ds}^{m}C_{ds}T_{ds}^{m}C_{ds}A_{ds}G_{ds}T_{ds}G_{ds}^{m}C_{ds}A_{ds}Tₖₛ^{m}Cₖₛ^{m}Cₖ | 37.6 | 2750 | 2765 | 430 |
| 486177 | ^{m}CₖₛAₖₛAₖₛT_{ds}A_{ds}A_{ds}A_{ds}T_{ds}A_{ds}^{m}C_{ds}^{m}C_{ds}G_{ds}A_{ds}GₖₛGₖₛAₖ | 40 | 2772 | 2787 | 495 |
| 486178 | Aₖₛ^{m}CₖₛAₖₛA_{ds}T_{ds}A_{ds}A_{ds}A_{ds}T_{ds}A_{ds}^{m}C_{ds}^{m}C_{ds}G_{ds}AₖₛGₖₛGₖ | 11.3 | 2773 | 2788 | 23 |
| 486179 | AₖₛGₖₛAₖₛ^{m}C_{ds}A_{ds}A_{ds}T_{ds}A_{ds}A_{ds}A_{ds}T_{ds}A_{ds}^{m}C_{ds}^{m}CₖₛGₖₛAₖ | 13.5 | 2775 | 2790 | 496 |
| 486180 | ^{m}CₖₛAₖₛGₖₛA_{ds}^{m}C_{ds}A_{ds}A_{ds}T_{ds}A_{ds}A_{ds}A_{ds}T_{ds}A_{ds}^{m}Cₖₛ^{m}CₖₛGₖ | 18.6 | 2776 | 2791 | 497 |

### Example 9: ASOs designed to target a human DMPK RNA transcript

A series of antisense oligonucleotides (ASOs) were designed to target hDMPK. The newly designed ASOs were prepared using standard oligonucleotide synthesis well known in the art and are described in Table 13 to 18, below. Subscripts "s" indicate phosphorothioate internucleoside linkages; subscripts "k" indicate 6'-*(S)*-CH₃ bicyclic nucleosides (cEt); subscripts "e" indicate 2'-O-methoxyethyl (MOE) modified nucleosides; and subscripts "d" indicate β-D-2'-deoxyribonucleosides. "^{m}C" indicates 5-methylcytosine nucleosides.

The antisense oligonucleotides targeted to a human DMPK nucleic acid were tested for their effect on DMPK RNA transcript *in vitro.* Cultured HepG2 cells at a density of 20,000 cells per well were transfected using electroporation with 4,500 nM antisense oligonucleotide. After approximately 24 hours, RNA was isolated from the cells and DMPK transcript levels were measured by quantitative real-time PCR. DMPK RNA transcript levels were adjusted according to total RNA content, as measured by RIBOGREEN^{®}. Results are presented as percent expression of DMPK, relative to untreated control cells, with "% Target Expression" representing the percent expression of DMPK relative to untreated control cells

All the antisense oligonucleotides listed in Table 13 target SEQ ID NO: 1 (GENBANK Accession No. NM_001081560.1). All the antisense oligonucleotides listed in Table 14 to 18 target SEQ ID NO: 2 (the complement of GENBANK Accession No. NT_011109.15 truncated from nucleotides 18540696 to 18555106). 'Target start site' indicates the 5'-most nucleoside to which the antisense oligonucleotide is targeted in the human genomic gene sequence. 'Target stop site' indicates the 3'-most nucleoside to which the antisense oligonucleotide is targeted in the human genomic sequence.

**Table13**

| Inhibition of human DMPK RNA transcript in HepG2 cells targeting SEQ ID NO: 1 | | | | | |
|---|---|---|---|---|---|
| ISIS No. | Sequence | % Target Expression | Start Site on Seq ID: 1 | Stop Site on Seq ID: 1 | Seq ID No. |
| UTC | N/A | 100 | N/A | N/A | |
| 445569 | ^{m}CₑₛGₑₛGₑₛAₑₛGₑₛ^{m}C_{ds}G_{ds}G_{ds}T_{ds}T_{ds}G_{ds}T_{ds}G_{ds}A_{ds}A_{ds}^{m}CₑₛTₑₛGₑₛGₑₛ^{m}Cₑ | 36.7 | 2163 | 2182 | 24 |
| 486178 | AₖₛCₖₛAₖₛA_{ds}T_{ds}A_{ds}A_{ds}A_{ds}T_{ds}A_{ds}C_{ds}C_{ds}G_{ds}AₖₛGₖₛGₖ | 21.3 | 2773 | 2788 | 23 |
| 569403 | ^{m}CₖₛAₖₛ^{m}CₖₛG_{ds}G_{ds}A_{ds}G_{ds}^{m}C_{ds}A_{ds}^{m}C_{ds}G_{ds}^{m}CₖₛAₖₛ^{m}Cₖ | 18.8 | 542 | 557 | 498 |
| 569404 | Tₖₛ^{m}CₖₛAₖₛ^{m}C_{ds}G_{ds}G_{ds}A_{ds}A_{ds}G_{ds}^{m}C_{ds}A_{ds}^{m}C_{ds}G_{ds}Aₖₛ^{m}CₖₛAₖ | 25.2 | 543 | 558 | 499 |
| 569405 | ^{m}CₖₛTₖₛ^{m}CₖₛA_{ds}^{m}C_{ds}G_{ds}G_{ds}A_{ds}A_{ds}G_{ds}^{m}C_{ds}A_{ds}^{m}C_{ds}GₖₛAₖₛ^{m}Cₖ | 21.2 | 544 | 559 | 500 |
| 569406 | ^{m}Cₖₛ^{m}CₖₛTₖₛ^{m}C_{ds}T_{ds}^{m}C_{ds}^{m}C_{ds}T_{ds}^{m}C_{ds}A_{ds}^{m}C_{ds}G_{ds}G_{ds}AₖₛAₖₛGₖ | 27.9 | 550 | 565 | 343 |
| 569407 | GₖₛTₖₛ^{m}Cₖₛ^{m}C_{ds}^{m}C_{ds}T_{ds}^{m}C_{ds}T_{ds}^{m}C_{ds}^{m}C_{ds}T_{ds}^{m}C_{ds}A_{ds}^{m}CₖₛGₖₛGₖ | 30.9 | 553 | 568 | 501 |
| 569408 | ^{m}CₖₛGₖₛTₖₛ^{m}C_{ds}^{m}C_{ds}^{m}C_{ds}T_{ds}^{m}C_{ds}T_{ds}^{m}C_{ds}^{m}C_{ds}T_{ds}^{m}C_{ds}Aₖₛ^{m}CₖₛGₖ | 32.8 | 554 | 569 | 502 |
| 569409 | ^{m}Cₖₛ^{m}Cₖₛ^{m}CₖₛA_{ds}T_{ds}T_{ds}^{m}C_{dAds}^{m}C_{ds}^{m}C_{ds}A_{ds}A_{ds}^{m}C_{ds}Aₖₛ^{m}CₖₛGₖ | 33 | 568 | 583 | 503 |
| 569410 | ^{m}Cₖₛ^{m}Cₖₛ^{m}Cₖₛ^{m}C_{ds}A_{ds}T_{ds}T_{ds}^{m}C_{ds}A_{ds}^{m}C_{ds}^{m}C_{ds}A_{ds}A_{ds}^{m}CₖₛAₖₛ^{m}Cₖ | 42.1 | 569 | 584 | 504 |
| 569411 | Tₖₛ^{m}Cₖₛ^{m}Cₖₛ^{m}C_{ds}^{m}C_{ds}A_{ds}T_{ds}T_{ds}^{m}C_{ds}A_{ds}^{m}C_{ds}^{m}C_{ds}AₖₛAₖₛ^{m}CₖₛAₖ | 68.6 | 570 | 585 | 505 |
| 569412 | GₖₛTₖₛ^{m}Cₖₛ^{m}C_{ds}^{m}C_{ds}^{m}C_{ds}A_{ds}T_{ds}T_{ds}^{m}C_{ds}A_{ds}^{m}C_{ds}^{m}C_{ds}AₖₛAₖₛ^{m}Cₖ | 60.7 | 571 | 586 | 506 |
| 569413 | GₖₛGₖₛTₖₛ^{m}C_{ds}^{m}C_{ds}^{m}C_{ds}^{m}C_{ds}A_{ds}T_{ds}T_{ds}^{m}C_{ds}A_{ds}^{m}C_{ds}^{m}CₖₛAₖₛAₖ | 65.1 | 572 | 587 | 507 |
| 569414 | ^{m}CₖₛGₖₛGₖₛT_{ds}^{m}C_{ds}^{m}C_{ds}^{m}C_{ds}^{m}C_{ds}A_{ds}T_{ds}T_{ds}^{m}C_{ds}A_{ds}^{m}Cₖₛ^{m}CₖₛAₖ | 54.4 | 573 | 588 | 508 |
| 569415 | ^{m}Cₖₛ^{m}CₖₛGₖₛG_{ds}T_{ds}^{m}C_{ds}^{m}C_{ds}^{m}C_{ds}^{m}C_{ds}A_{ds}T_{ds}T_{ds}^{m}C_{ds}Aₖₛ^{m}Cₖₛ^{m}Cₖ | 51.3 | 574 | 589 | 509 |
| 569416 | Gₖₛ^{m}Cₖₛ^{m}CₖₛG_{ds}G_{ds}T_{ds}^{m}C_{ds}^{m}C_{ds}^{m}C_{ds}^{m}C_{ds}A_{ds}T_{ds}T_{ds}^{m}CₖₛAₖₛ^{m}Cₖ | 57.9 | 575 | 590 | 510 |
| 569417 | ^{m}CₖₛGₖₛ^{m}Cₖₛ^{m}C_{ds}G_{ds}G_{ds}T_{ds}^{m}C_{ds}^{m}C_{ds}^{m}C_{ds}^{m}C_{ds}A_{ds}T_{ds}Tₖₛ^{m}CₖₛAₖ | 43.2 | 576 | 591 | 511 |
| 569418 | ^{m}Cₖₛ^{m}CₖₛGₖₛ^{m}C_{ds}^{m}C_{ds}G_{ds}G_{ds}T_{ds}^{m}C_{ds}^{m}C_{ds}^{m}C_{ds}^{m}C_{ds}A_{ds}TₖₛTₖₛ^{m}Cₖ | 79.3 | 577 | 592 | 512 |
| 569419 | Aₖₛ^{m}Cₖₛ^{m}CₖₛG_{ds}^{m}C_{ds}^{m}C_{ds}G_{ds}G_{ds}T_{ds}^{m}C_{ds}^{m}C_{ds}^{m}C_{ds}^{m}C_{ds}AₖₛTₖₛTₖ | 36 | 578 | 593 | 513 |
| 569420 | ^{m}CₖₛAₖₛ^{m}Cₖₛ^{m}C_{ds}G_{ds}^{m}C_{ds}^{m}C_{ds}G_{ds}G_{ds}T_{ds}^{m}C_{ds}^{m}C_{ds}^{m}C_{ds}^{m}CₖₛAₖₛTₖ | 36.2 | 579 | 594 | 514 |
| 569421 | ^{m}Cₖₛ^{m}CₖₛAₖₛ^{m}C_{ds}^{m}C_{ds}G_{ds}^{m}C_{ds}^{m}C_{ds}G_{ds}G_{ds}T_{ds}^{m}C_{ds}^{m}C_{ds}^{m}Cₖₛ^{m}CₖₛAₖ | 34.7 | 580 | 595 | 515 |
| 569422 | Tₖₛ^{m}Cₖₛ^{m}CₖₛA_{ds}^{m}C_{ds}^{m}C_{ds}G_{ds}^{m}C_{ds}^{m}C_{ds}G_{ds}G_{ds}T_{ds}^{m}C_{ds}^{m}Cₖₛ^{m}Cₖₛ^{m}Cₖ | 40 | 581 | 596 | 516 |
| 569423 | AₖₛTₖₛ^{m}Cₖₛ^{m}C_{ds}A_{ds}^{m}C_{ds}^{m}C_{ds}G_{ds}^{m}C_{ds}^{m}C_{ds}G_{ds}G_{ds}T_{ds}^{m}Cₖₛ^{m}Cₖₛ^{m}Cₖ | 31.6 | 582 | 597 | 517 |
| 569424 | GₖₛAₖₛTₖₛ^{m}C_{ds}^{m}C_{ds}A_{ds}^{m}C_{ds}^{m}C_{ds}G_{ds}^{m}C_{ds}^{m}C_{ds}G_{ds}G_{ds}Tₖₛ^{m}Cₖₛ^{m}Cₖ | 56 | 583 | 598 | 518 |
| 569425 | TₖₛGₖₛAₖₛT_{ds}^{m}C_{ds}^{m}C_{ds}A_{ds}^{m}C_{ds}^{m}C_{ds}G_{ds}^{m}C_{ds}^{m}C_{ds}G_{ds}GₖₛTₖₛ^{m}Cₖ | 53.9 | 584 | 599 | 519 |
| 569426 | GₖₛTₖₛGₖₛA_{ds}T_{ds}^{m}C_{ds}^{m}C_{ds}A_{ds}^{m}C_{ds}^{m}C_{ds}G_{ds}^{m}C_{ds}^{m}C_{ds}GₖₛGₖₛTₖ | 54.1 | 585 | 600 | 520 |
| 569427 | ^{m}CₖₛGₖₛTₖₛG_{ds}A_{ds}T_{ds}^{m}C_{ds}^{m}C_{ds}A_{ds}^{m}C_{ds}^{m}C_{ds}G_{ds}^{m}C_{ds}^{m}CₖₛGₖₛGₖ | 34.8 | 586 | 601 | 521 |
| 569428 | ^{m}CₖₛAₖₛTₖₛ^{m}C_{ds}^{m}C_{ds}T_{ds}G_{ds}G_{ds}A_{ds}A_{ds}G_{ds}G_{ds}^{m}C_{ds}GₖₛAₖₛAₖ | 71 | 611 | 626 | 522 |
| 569429 | Tₖₛ^{m}CₖₛAₖₛT_{ds}^{m}C_{ds}^{m}C_{ds}T_{ds}G_{ds}G_{ds}A_{ds}A_{ds}G_{ds}G_{ds}^{m}CₖₛGₖₛAₖ | 51.1 | 612 | 627 | 523 |
| 569430 | AₖₛGₖₛTₖₛT_{ds}^{m}C_{ds}T_{ds}^{m}C_{ds}A_{ds}T_{ds}^{m}C_{ds}^{m}C_{ds}T_{ds}G_{ds}GₖₛAₖₛAₖ | 69.2 | 617 | 632 | 524 |
| 569431 | TₖₛAₖₛGₖₛT_{ds}T_{ds}^{m}C_{ds}T_{ds}^{m}C_{ds}A_{ds}T_{ds}^{m}C_{ds}^{m}C_{ds}T_{ds}GₖₛGₖₛAₖ | 48.6 | 618 | 633 | 525 |
| 569432 | GₖₛTₖₛAₖₛG_{ds}T_{ds}T_{ds}^{m}C_{ds}T_{ds}^{m}C_{ds}A_{ds}T_{ds}^{m}C_{ds}^{m}C_{ds}TₖₛGₖₛGₖ | 29.6 | 619 | 634 | 526 |
| 569433 | ^{m}CₖₛAₖₛGₖₛG_{ds}T_{ds}A_{ds}^{m}C_{ds}A_{ds}G_{ds}G_{ds}T_{ds}A_{ds}G_{ds}TₖₛTₖₛ^{m}Cₖ | 36.5 | 628 | 643 | 527 |
| 569434 | ^{m}Cₖₛ^{m}CₖₛAₖₛG_{ds}G_{ds}T_{ds}A_{ds}^{m}C_{ds}A_{ds}G_{ds}G_{ds}T_{ds}A_{ds}GₖₛTₖₛTₖ | 51 | 629 | 644 | 528 |
| 569435 | GₖₛAₖₛ^{m}Cₖₛ^{m}C_{ds}A_{ds}G_{ds}G_{ds}T_{ds}A_{ds}^{m}C_{ds}A_{ds}G_{ds}G_{ds}TₖₛAₖₛGₖ | 49.9 | 631 | 646 | 529 |
| 569436 | ^{m}CₖₛTₖₛ^{m}Cₖₛ^{m}C_{ds}A_{ds}T_{ds}G_{ds}A_{ds}^{m}C_{ds}^{m}C_{ds}A_{ds}G_{ds}G_{ds}TₖₛAₖₛ^{m}Cₖ | 41 | 637 | 652 | 530 |
| 569437 | Aₖₛ^{m}CₖₛTₖₛ^{m}C_{ds}^{m}C_{ds}A_{ds}T_{ds}G_{ds}A_{ds}^{m}C_{ds}^{m}C_{ds}A_{ds}G_{ds}GₖₛTₖₛAₖ | 32.9 | 638 | 653 | 531 |
| 569438 | TₖₛAₖₛ^{m}CₖₛT_{ds}^{m}C_{ds}^{m}C_{ds}A_{ds}T_{ds}G_{ds}A_{ds}^{m}C_{ds}^{m}C_{ds}A_{ds}GₖₛGₖₛTₖ | 25.7 | 639 | 654 | 532 |
| 569439 | AₖₛTₖₛAₖₛ^{m}C_{ds}T_{ds}^{m}C_{ds}^{m}C_{ds}A_{ds}T_{ds}G_{ds}A_{ds}^{m}C_{ds}^{m}C_{ds}AₖₛGₖₛGₖ | 9.4 | 640 | 655 | 533 |
| 569440 | AₖₛAₖₛTₖₛA_{ds}^{m}C_{ds}T_{ds}^{m}C_{ds}^{m}C_{ds}A_{ds}T_{ds}G_{ds}A_{ds}^{m}C_{ds}^{m}CₖₛAₖₛGₖ | 21.2 | 641 | 656 | 534 |
| 569441 | TₖₛAₖₛAₖₛT_{ds}A_{ds}^{m}C_{ds}T_{ds}^{m}C_{ds}^{m}C_{ds}A_{ds}T_{ds}G_{ds}A_{ds}^{m}Cₖₛ^{m}CₖₛAₖ | 30.8 | 642 | 657 | 535 |
| 569442 | GₖₛTₖₛAₖₛA_{ds}T_{ds}A_{ds}^{m}C_{ds}T_{ds}^{m}C_{ds}^{m}C_{ds}A_{ds}T_{ds}G_{ds}Aₖₛ^{m}Cₖₛ^{m}Cₖ | 29.8 | 643 | 658 | 536 |
| 569443 | ^{m}CₖₛGₖₛTₖₛA_{ds}A_{ds}T_{ds}A_{ds}^{m}C_{ds}T_{ds}^{m}C_{ds}^{m}C_{ds}A_{ds}T_{ds}GₖₛAₖₛ^{m}Cₖ | 25.3 | 644 | 659 | 537 |
| 569444 | ^{m}CₖₛTₖₛTₖₛG_{ds}^{m}C_{ds}T_{ds}^{m}C_{ds}A_{ds}G_{ds}^{m}C_{ds}A_{ds}G_{ds}T_{ds}GₖₛTₖₛ^{m}Cₖ | 19.3 | 676 | 691 | 538 |
| 569445 | Aₖₛ^{m}CₖₛTₖₛT_{ds}G_{ds}^{m}C_{ds}T_{ds}^{m}C_{ds}A_{ds}G_{ds}^{m}C_{ds}A_{ds}G_{ds}TₖₛGₖₛTₖ | 35 | 677 | 692 | 539 |
| 569446 | AₖₛAₖₛ^{m}CₖₛT_{ds}T_{ds}G_{ds}^{m}C_{ds}T_{ds}^{m}C_{ds}A_{ds}G_{ds}^{m}C_{ds}A_{ds}GₖₛTₖₛGₖ | 30 | 678 | 693 | 540 |
| 569447 | AₖₛAₖₛAₖₛ^{m}C_{ds}T_{ds}T_{ds}G_{ds}^{m}C_{ds}T_{ds}^{m}C_{ds}A_{ds}G_{ds}^{m}C_{ds}AₖₛGₖₛTₖ | 32.2 | 679 | 694 | 344 |
| 569448 | ^{m}Cₖₛ^{m}CₖₛAₖₛA_{ds}A_{ds}^{m}C_{ds}T_{ds}T_{ds}G_{ds}^{m}C_{ds}T_{ds}^{m}C_{ds}A_{ds}Gₖₛ^{m}CₖₛAₖ | 30.1 | 681 | 696 | 346 |
| 569449 | ^{m}Cₖₛ^{m}Cₖₛ^{m}CₖₛA_{ds}A_{ds}A_{ds}^{m}C_{ds}T_{ds}T_{ds}G_{ds}^{m}C_{ds}T_{ds}^{m}C_{ds}AₖₛGₖₛ^{m}Cₖ | 18.4 | 682 | 697 | 347 |
| 569450 | ^{m}Cₖₛ^{m}Cₖₛ^{m}Cₖₛ^{m}C_{ds}A_{ds}A_{ds}A_{ds}^{m}C_{ds}T_{ds}T_{ds}G_{ds}^{m}C_{ds}T_{ds}^{m}CₖₛAₖₛGₖ | 44.8 | 683 | 698 | 348 |
| 569451 | Gₖₛ^{m}CₖₛTₖₛ^{m}C_{ds}^{m}C_{ds}^{m}C_{ds}^{m}C_{ds}A_{ds}A_{ds}A_{ds}^{m}C_{ds}T_{ds}T_{ds}Gₖₛ^{m}CₖₛTₖ | 47 | 686 | 701 | 541 |
| 569452 | ^{m}CₖₛGₖₛ^{m}CₖₛT_{ds}^{m}C_{ds}^{m}C_{ds}^{m}C_{ds}^{m}C_{ds}A_{ds}A_{ds}A_{ds}^{m}C_{ds}T_{ds}TₖₛGₖₛ^{m}Cₖ | 35.4 | 687 | 702 | 542 |
| 569453 | ^{m}Cₖₛ^{m}CₖₛGₖₛ^{m}C_{ds}T_{ds}^{m}C_{ds}^{m}C_{ds}^{m}C_{ds}^{m}C_{ds}A_{ds}A_{ds}A_{ds}^{m}C_{ds}TₖₛTₖₛGₖ | 46.6 | 688 | 703 | 543 |
| 569454 | Tₖₛ^{m}Cₖₛ^{m}CₖₛG_{ds}^{m}C_{ds}T_{ds}^{m}C_{ds}^{m}C_{ds}^{m}C_{ds}^{m}C_{ds}A_{ds}A_{ds}A_{ds}^{m}CₖₛTₖₛTₖ | 29.4 | 689 | 704 | 544 |
| 569455 | AₖₛTₖₛ^{m}Cₖₛ^{m}C_{ds}G_{ds}^{m}C_{ds}T_{ds}^{m}C_{ds}^{m}C_{ds}^{m}C_{ds}^{m}C_{ds}A_{ds}A_{ds}Aₖₛ^{m}CₖₛTₖ | 36.9 | 690 | 705 | 545 |
| 569456 | AₖₛAₖₛTₖₛ^{m}C_{ds}^{m}C_{ds}G_{ds}^{m}C_{ds}T_{ds}^{m}C_{ds}^{m}C_{ds}^{m}C_{ds}^{m}C_{ds}A_{ds}AₖₛAₖₛ^{m}Cₖ | 32.9 | 691 | 706 | 546 |
| 569457 | GₖₛAₖₛAₖₛT_{ds}^{m}C_{ds}^{m}C_{ds}G_{ds}^{m}C_{ds}T_{ds}^{m}C_{ds}^{m}C_{ds}^{m}C_{ds}^{m}C_{ds}AₖₛAₖₛAₖ | 41.7 | 692 | 707 | 547 |
| 569458 | GₖₛGₖₛAₖₛA_{ds}T_{ds}^{m}C_{ds}^{m}C_{ds}G_{ds}^{m}C_{ds}T_{ds}^{m}C_{ds}^{m}C_{ds}^{m}C_{ds}^{m}CₖₛAₖₛAₖ | 36.4 | 693 | 708 | 548 |
| 569459 | ^{m}CₖₛGₖₛGₖₛA_{ds}A_{ds}T_{ds}^{m}C_{ds}^{m}C_{ds}G_{ds}^{m}C_{ds}T_{ds}^{m}C_{ds}^{m}C_{ds}^{m}Cₖₛ^{m}CₖₛAₖ | 30 | 694 | 709 | 549 |
| 569460 | ^{m}Cₖₛ^{m}CₖₛGₖₛG_{ds}A_{ds}A_{ds}T_{ds}^{m}C_{ds}^{m}C_{ds}G_{ds}^{m}C_{ds}T_{ds}^{m}C_{ds}^{m}Cₖₛ^{m}Cₖₛ^{m}Cₖ | 26.5 | 695 | 710 | 550 |
| 569461 | Gₖₛ^{m}Cₖₛ^{m}CₖₛG_{ds}G_{ds}A_{ds}A_{ds}T_{ds}^{m}C_{ds}^{m}C_{ds}G_{ds}^{m}C_{ds}T_{ds}^{m}Cₖₛ^{m}Cₖₛ^{m}Cₖ | 36.5 | 696 | 711 | 551 |
| 569462 | AₖₛGₖₛAₖₛA_{ds}G_{ds}^{m}C_{ds}G_{ds}^{m}C_{ds}G_{ds}^{m}C_{ds}^{m}C_{ds}A_{ds}T_{ds}^{m}CₖₛTₖₛ^{m}Cₖ | 26 | 713 | 728 | 552 |
| 569463 | TₖₛAₖₛGₖₛA_{ds}A_{ds}G_{ds}^{m}C_{ds}G_{ds}^{m}C_{ds}G_{ds}^{m}C_{ds}^{m}C_{ds}A_{ds}Tₖₛ^{m}CₖₛTₖ | 40.3 | 714 | 729 | 553 |
| 569464 | GₖₛTₖₛAₖₛG_{ds}A_{ds}A_{ds}G_{ds}^{m}C_{ds}G_{ds}^{m}C_{ds}G_{ds}^{m}C_{ds}^{m}C_{ds}AₖₛTₖₛ^{m}Cₖ | 28.9 | 715 | 730 | 554 |
| 569465 | GₖₛGₖₛTₖₛA_{ds}G_{ds}A_{ds}A_{ds}G_{ds}^{m}C_{ds}G_{ds}^{m}C_{ds}G_{ds}^{m}C_{ds}^{m}CₖₛAₖₛTₖ | 35.7 | 716 | 731 | 555 |
| 569466 | AₖₛGₖₛGₖₛT_{ds}A_{ds}G_{ds}A_{ds}A_{ds}G_{ds}^{m}C_{ds}G_{ds}^{m}C_{ds}G_{ds}^{m}Cₖₛ^{m}CₖₛAₖ | 31.1 | 717 | 732 | 556 |
| 569467 | ^{m}CₖₛAₖₛGₖₛG_{ds}T_{ds}A_{ds}G_{ds}A_{ds}A_{ds}G_{ds}^{m}C_{ds}G_{ds}^{m}C_{ds}Gₖₛ^{m}Cₖₛ^{m}Cₖ | 14.8 | 718 | 733 | 557 |
| 569468 | ^{m}Cₖₛ^{m}CₖₛAₖₛG_{ds}G_{ds}T_{ds}A_{ds}G_{ds}A_{ds}A_{ds}G_{ds}^{m}C_{ds}G_{ds}^{m}CₖₛGₖₛ^{m}Cₖ | 32.1 | 719 | 734 | 558 |
| 569469 | Gₖₛ^{m}Cₖₛ^{m}CₖₛA_{ds}G_{ds}G_{ds}T_{ds}A_{ds}G_{ds}A_{ds}A_{ds}G_{ds}^{m}C_{ds}Gₖₛ^{m}CₖₛGₖ | 54.5 | 720 | 735 | 559 |
| 569470 | ^{m}CₖₛGₖₛ^{m}Cₖₛ^{m}C_{ds}A_{ds}G_{ds}G_{ds}T_{ds}A_{ds}G_{ds}A_{ds}A_{ds}G_{ds}^{m}CₖₛGₖₛ^{m}Cₖ | 50.5 | 721 | 736 | 560 |
| 569471 | ^{m}Cₖₛ^{m}CₖₛGₖₛ^{m}C_{ds}^{m}C_{ds}A_{ds}G_{ds}G_{ds}T_{ds}A_{ds}G_{ds}A_{ds}A_{ds}Gₖₛ^{m}CₖₛGₖ | 56.6 | 722 | 737 | 561 |
| 569472 | Tₖₛ^{m}Cₖₛ^{m}CₖₛG_{ds}^{m}C_{ds}^{m}C_{ds}A_{ds}G_{ds}G_{ds}T_{ds}A_{ds}G_{ds}A_{ds}AₖₛGₖₛ^{m}Cₖ | 44.1 | 723 | 738 | 562 |
| 569473 | GₖₛAₖₛ^{m}CₖₛA_{ds}A_{ds}T_{ds}^{m}C_{ds}T_{ds}^{m}C_{ds}^{m}C_{ds}G_{ds}^{m}C_{ds}^{m}C_{ds}AₖₛGₖₛGₖ | 14.2 | 730 | 745 | 29 |
| 569474 | TₖₛGₖₛAₖₛ^{m}C_{ds}A_{ds}A_{ds}T_{ds}^{m}C_{ds}T_{ds}^{m}C_{ds}^{m}C_{ds}G_{ds}^{m}C_{ds}^{m}CₖₛAₖₛGₖ | 25.9 | 731 | 746 | 563 |
| 569475 | AₖₛTₖₛGₖₛA_{ds}^{m}C_{ds}A_{ds}A_{ds}T_{ds}^{m}C_{ds}T_{ds}^{m}C_{ds}^{m}C_{ds}G_{ds}^{m}Cₖₛ^{m}CₖₛAₖ | 28.7 | 732 | 747 | 564 |
| 569476 | ^{m}CₖₛAₖₛTₖₛG_{ds}A_{ds}^{m}C_{ds}A_{ds}A_{ds}T_{ds}^{m}C_{ds}T_{ds}^{m}C_{ds}^{m}C_{ds}Gₖₛ^{m}Cₖₛ^{m}Cₖ | 27.4 | 733 | 748 | 565 |
| 569477 | ^{m}Cₖₛ^{m}CₖₛAₖₛT_{ds}G_{ds}A_{ds}^{m}C_{ds}A_{ds}A_{ds}T_{ds}^{m}C_{ds}T_{ds}^{m}C_{ds}^{m}CₖₛGₖₛ^{m}Cₖ | 52.4 | 734 | 749 | 566 |
| 569478 | Gₖₛ^{m}Cₖₛ^{m}CₖₛA_{ds}T_{ds}G_{ds}A_{ds}^{m}C_{ds}A_{ds}A_{ds}T_{ds}^{m}C_{ds}T_{ds}^{m}Cₖₛ^{m}CₖₛGₖ | 50.5 | 735 | 750 | 567 |
| 569479 | GₖₛGₖₛ^{m}Cₖₛ^{m}C_{ds}A_{ds}T_{ds}G_{ds}A_{ds}^{m}C_{ds}A_{ds}A_{ds}T_{ds}^{m}C_{ds}Tₖₛ^{m}Cₖₛ^{m}Cₖ | 48.4 | 736 | 751 | 568 |

**Table 14**

| Inhibition of human DMPK RNA transcript in HepG2 cells targeting SEQ ID NO: 2 | | | | | |
|---|---|---|---|---|---|
| ISIS No. | Sequence | % Target Expression | Start Site on Seq ID: 2 | Stop Site on Seq ID: 2 | Seq ID No. |
| UTC | N/A | 100 | N/A | N/A | |
| 445569 | ^{m}CₑₛGₑₛGₑₛAₑₛGₑₛ^{m}C_{ds}G_{ds}G_{ds}T_{ds}T_{ds}G_{ds}T_{ds}G_{ds}A_{ds}A_{ds}^{m}CₑₛTₑₛGₑₛGₑₛ^{m}Cₑ | 31.4 | 13226 | 13245 | 24 |
| 486178 | AₖₛCₖₛAₖₛA_{ds}T_{ds}A_{ds}A_{ds}A_{ds}T_{ds}A_{ds}C_{ds}C_{ds}G_{ds}AₖₛGₖₛGₖ | 25.3 | 13836 | 13851 | 23 |
| 570801 | ^{m}Cₖₛ^{m}CₖₛAₖₛA_{ds}^{m}CdₛT_{ds}G_{ds}T_{ds}T_{ds}^{m}CdₛT_{ds}^{m}CdₛT_{ds}TₖₛAₖₛGₖ | 22.7 | 10165 | 10180 | 569 |
| 570802 | AₖₛAₖₛ^{m}Cₖₛ^{m}C_{ds}A_{ds}A_{ds}^{m}C_{ds}T_{ds}G_{ds}T_{ds}T_{ds}^{m}C_{ds}T_{ds}^{m}CₖₛTₖₛTₖ | 22.6 | 10167 | 10182 | 570 |
| 570803 | ^{m}Cₖₛ^{m}CₖₛAₖₛG_{ds}T_{ds}A_{ds}A_{ds}T_{ds}A_{ds}A_{ds}A_{ds}A_{ds}G_{ds}^{m}CₖₛTₖₛGₖ | 37.4 | 10190 | 10205 | 571 |
| 570804 | GₖₛTₖₛ^{m}Cₖₛ^{m}C_{ds}A_{ds}G_{ds}T_{ds}A_{ds}A_{ds}T_{ds}A_{ds}A_{ds}A_{ds}AₖₛGₖₛ^{m}Cₖ | 24.9 | 10192 | 10207 | 572 |
| 570805 | GₖₛTₖₛTₖₛG_{ds}T_{ds}^{m}C_{ds}^{m}C_{ds}A_{ds}G_{ds}T_{ds}A_{ds}A_{ds}T_{ds}AₖₛAₖₛAₖ | 23.8 | 10195 | 10210 | 573 |
| 570806 | AₖₛTₖₛGₖₛT_{ds}T_{ds}G_{ds}T_{ds}^{m}C_{ds}^{m}C_{ds}A_{ds}G_{ds}T_{ds}A_{ds}AₖₛTₖₛAₖ | 21.9 | 10197 | 10212 | 574 |
| 570807 | TₖₛAₖₛAₖₛT_{ds}G_{ds}T_{ds}T_{ds}G_{ds}T_{ds}^{m}C_{ds}^{m}C_{ds}A_{ds}G_{ds}TₖₛAₖₛAₖ | 20 | 10199 | 10214 | 575 |
| 570808 | TₖₛGₖₛTₖₛA_{ds}A_{ds}T_{ds}G_{ds}T_{ds}T_{ds}G_{ds}T_{ds}^{m}C_{ds}^{m}C_{ds}AₖₛGₖₛTₖ | 11.5 | 10201 | 10216 | 31 |
| 570809 | TₖₛTₖₛ^{m}CₖₛA_{ds}A_{ds}T_{ds}^{m}C_{ds}^{m}C_{ds}T_{ds}G_{ds}A_{ds}^{m}C_{ds}^{m}C_{ds}^{m}CₖₛAₖₛ^{m}Cₖ | 34.7 | 10279 | 10294 | 576 |
| 570810 | GₖₛGₖₛTₖₛT_{ds}^{m}C_{ds}A_{ds}A_{ds}T_{ds}^{m}C_{ds}^{m}C_{ds}T_{ds}G_{ds}A_{ds}^{m}Cₖₛ^{m}Cₖₛ^{m}Cₖ | 76.4 | 10281 | 10296 | 577 |
| 570811 | TₖₛGₖₛGₖₛG_{ds}T_{ds}T_{ds}^{m}C_{ds}A_{ds}A_{ds}T_{ds}^{m}C_{ds}^{m}C_{ds}T_{ds}GₖₛAₖₛ^{m}Cₖ | 72.4 | 10283 | 10298 | 578 |
| 570812 | GₖₛAₖₛTₖₛG_{ds}G_{ds}G_{ds}T_{ds}T_{ds}^{m}C_{ds}A_{ds}A_{ds}T_{ds}^{m}C_{ds}^{m}CₖₛTₖₛGₖ | 49 | 10285 | 10300 | 579 |
| 570813 | AₖₛGₖₛGₖₛA_{ds}T_{ds}G_{ds}G_{ds}G_{ds}T_{ds}T_{ds}^{m}C_{ds}A_{ds}A_{ds}Tₖₛ^{m}Cₖₛ^{m}Cₖ | 80.8 | 10287 | 10302 | 580 |
| 570814 | AₖₛGₖₛAₖₛG_{ds}G_{ds}A_{ds}T_{ds}G_{ds}G_{ds}G_{ds}T_{ds}T_{ds}^{m}C_{ds}AₖₛAₖₛTₖ | 43.3 | 10289 | 10304 | 581 |
| 570815 | AₖₛTₖₛAₖₛG_{ds}A_{ds}G_{ds}G_{ds}A_{ds}T_{ds}G_{ds}G_{ds}G_{ds}T_{ds}Tₖₛ^{ffi}CₖₛAₖ | 63.2 | 10291 | 10306 | 582 |
| 570816 | ^{m}Cₖₛ^{m}Cₖₛ^{m}CₖₛT_{ds}^{m}C_{ds}^{m}C_{ds}T_{ds}G_{ds}T_{ds}G_{ds}G_{ds}G_{ds}A_{ds}Aₖₛ^{m}CₖₛAₖ | 38.8 | 10349 | 10364 | 583 |
| 570817 | GₖₛTₖₛ^{m}Cₖₛ^{m}C_{ds}^{m}C_{ds}T_{ds}^{m}C_{ds}^{m}C_{ds}T_{ds}G_{ds}T_{ds}G_{ds}G_{ds}GₖₛAₖₛAₖ | 91 | 10351 | 10366 | 584 |
| 570818 | ^{m}CₖₛAₖₛGₖₛT_{ds}^{m}C_{ds}^{m}C_{ds}^{m}C_{ds}T_{ds}^{m}C_{ds}^{m}C_{ds}T_{ds}G_{ds}T_{ds}GₖₛGₖₛGₖ | 64.8 | 10353 | 10368 | 585 |
| 570819 | AₖₛGₖₛ^{m}CₖₛA_{ds}G_{ds}T_{ds}^{m}C_{ds}^{m}C_{ds}^{m}C_{ds}T_{ds}^{m}C_{ds}^{m}C_{ds}T_{ds}GₖₛTₖₛGₖ | 28.5 | 10355 | 10370 | 586 |
| 570820 | Aₖₛ^{m}CₖₛTₖₛ^{m}C_{ds}A_{ds}G_{ds}^{m}C_{ds}T_{ds}G_{ds}T_{ds}G_{ds}G_{ds}G_{ds}AₖₛAₖₛGₖ | 62.9 | 10417 | 10432 | 587 |
| 570821 | ^{m}Cₖₛ^{m}Cₖₛ^{m}CₖₛA_{ds}^{m}C_{ds}T_{ds}^{m}C_{ds}A_{ds}G_{ds}^{m}C_{ds}T_{ds}G_{ds}T_{ds}GₖₛGₖₛGₖ | 79.9 | 10420 | 10435 | 588 |
| 570822 | Aₖₛ^{m}Cₖₛ^{m}Cₖₛ^{m}C_{ds}^{m}C_{ds}A_{ds}^{m}C_{ds}T_{ds}^{m}C_{ds}A_{ds}G_{ds}^{m}C_{ds}T_{ds}GₖₛTₖₛGₖ | 47.5 | 10422 | 10437 | 589 |
| 570823 | Aₖₛ^{m}CₖₛAₖₛ^{m}C_{ds}^{m}C_{ds}^{m}C_{ds}^{m}C_{ds}A_{ds}^{m}C_{ds}T_{ds}^{m}C_{ds}A_{ds}G_{ds}^{m}CₖₛTₖₛGₖ | 78.1 | 10424 | 10439 | 590 |
| 570824 | Gₖₛ^{m}CₖₛAₖₛ^{m}C_{ds}A_{ds}^{m}C_{ds}^{m}C_{ds}^{m}C_{ds}^{m}C_{ds}A_{ds}^{m}C_{ds}T_{ds}^{m}C_{ds}AₖₛGₖₛ^{m}Cₖ | 82.5 | 10426 | 10441 | 591 |
| 570825 | Tₖₛ^{m}CₖₛAₖₛG_{ds}^{m}C_{ds}A_{ds}^{m}C_{ds}A_{ds}^{m}C_{ds}^{m}C_{ds}^{m}C_{ds}^{m}C_{ds}A_{ds}^{m}CₖₛTₖₛ^{m}Cₖ | 52.6 | 10429 | 10444 | 592 |
| 570826 | GₖₛTₖₛGₖₛG_{ds}T_{ds}^{m}C_{ds}^{m}C_{ds}T_{ds}A_{ds}A_{ds}G_{ds}A_{ds}^{m}C_{ds}TₖₛGₖₛGₖ | 30.9 | 10474 | 10489 | 593 |
| 570827 | GₖₛAₖₛTₖₛG_{ds}T_{ds}G_{ds}G_{ds}T_{ds}^{m}C_{ds}^{m}C_{ds}T_{ds}A_{ds}A_{ds}GₖₛAₖₛ^{m}Cₖ | 25.5 | 10477 | 10492 | 594 |
| 570828 | ^{m}CₖₛAₖₛGₖₛA_{ds}T_{ds}G_{ds}T_{ds}G_{ds}G_{ds}T_{ds}^{m}C_{ds}^{m}C_{ds}T_{ds}AₖₛAₖₛGₖ | 18.6 | 10479 | 10494 | 595 |
| 570829 | ^{m}Cₖₛ^{m}CₖₛTₖₛ^{m}C_{ds}^{m}C_{ds}A_{ds}^{m}C_{ds}A_{ds}G_{ds}A_{ds}T_{ds}G_{ds}T_{ds}GₖₛGₖₛTₖ | 44.5 | 10485 | 10500 | 596 |
| 570830 | ^{m}CₖₛAₖₛ^{m}Cₖₛ^{m}C_{ds}T_{ds}^{m}C_{ds}^{m}C_{ds}A_{ds}^{m}C_{ds}A_{ds}G_{ds}A_{ds}T_{ds}GₖₛTₖₛGₖ | 67.4 | 10487 | 10502 | 597 |
| 570831 | GₖₛGₖₛ^{m}Cₖₛ^{m}C_{ds}A_{ds}^{m}C_{ds}^{m}C_{ds}T_{ds}^{m}C_{ds}^{m}C_{ds}A_{ds}^{m}C_{ds}A_{ds}GₖₛAₖₛTₖ | 56.3 | 10490 | 10505 | 598 |
| 570832 | TₖₛGₖₛ^{m}CₖₛT_{ds}T_{ds}G_{ds}G_{ds}^{m}C_{ds}T_{ds}^{m}C_{ds}T_{ds}G_{ds}G_{ds}^{m}Cₖₛ^{m}CₖₛAₖ | 42.4 | 10501 | 10516 | 599 |
| 570833 | Aₖₛ^{m}CₖₛTₖₛG_{ds}^{m}C_{ds}T_{ds}T_{ds}G_{ds}G_{ds}^{m}C_{ds}T_{ds}^{m}C_{ds}T_{ds}GₖₛGₖₛ^{m}Cₖ | 16 | 10503 | 10518 | 600 |
| 570834 | AₖₛGₖₛAₖₛ^{m}C_{ds}T_{ds}G_{ds}^{m}C_{ds}T_{ds}T_{ds}G_{ds}G_{ds}^{m}C_{ds}T_{ds}^{m}CₖₛTₖₛGₖ | 47.5 | 10505 | 10520 | 601 |
| 570835 | GₖₛGₖₛAₖₛG_{ds}A_{ds}^{m}C_{ds}T_{ds}G_{ds}^{m}C_{ds}T_{ds}T_{ds}G_{ds}G_{ds}^{m}CₖₛTₖₛ^{m}Cₖ | 37.2 | 10507 | 10522 | 602 |
| 570836 | TₖₛGₖₛ^{m}CₖₛA_{ds}G_{ds}A_{ds}^{m}C_{ds}^{m}C_{ds}^{m}C_{ds}^{m}C_{ds}T_{ds}^{m}C_{ds}T_{ds}Tₖₛ^{m}CₖₛTₖ | 63.1 | 10556 | 10571 | 603 |
| 570837 | ^{m}CₖₛTₖₛ^{m}Cₖₛ^{m}C_{ds}T_{ds}^{m}C_{ds}^{m}C_{ds}^{m}C_{ds}T_{ds}T_{ds}G_{ds}A_{ds}^{m}C_{ds}AₖₛTₖₛGₖ | 60.7 | 10579 | 10594 | 604 |
| 570838 | ^{m}Cₖₛ^{m}CₖₛAₖₛG_{ds}A_{ds}^{m}C_{ds}^{m}C_{ds}^{m}C_{ds}^{m}C_{ds}^{m}C_{ds}A_{ds}T_{ds}G_{ds}TₖₛTₖₛ^{m}Cₖ | 42.9 | 10609 | 10624 | 605 |
| 570839 | GₖₛTₖₛ^{m}Cₖₛ^{m}C_{ds}A_{ds}G_{ds}A_{ds}^{m}C_{ds}^{m}C_{ds}^{m}C_{ds}^{m}C_{ds}^{m}C_{ds}A_{ds}TₖₛGₖₛTₖ | 64.3 | 10611 | 10626 | 606 |
| 570840 | GₖₛGₖₛGₖₛT_{ds}^{m}C_{ds}^{m}C_{ds}A_{ds}G_{ds}A_{ds}^{m}C_{ds}^{m}C_{ds}^{m}C_{ds}^{m}C_{ds}^{m}CₖₛAₖₛTₖ | 68.5 | 10613 | 10628 | 607 |
| 570841 | Aₖₛ^{m}Cₖₛ^{m}CₖₛT_{ds}T_{ds}^{m}C_{ds}T_{ds}G_{ds}^{m}C_{ds}A_{ds}G_{ds}G_{ds}G_{ds}Aₖₛ^{m}cₖₛTₖ | 14.9 | 10631 | 10646 | 608 |
| 570842 | TₖₛAₖₛAₖₛA_{ds}^{m}C_{ds}^{m}C_{ds}T_{ds}T_{ds}^{m}C_{ds}T_{ds}G_{ds}^{m}C_{ds}A_{ds}GₖₛGₖₛGₖ | 51.7 | 10634 | 10649 | 609 |
| 570843 | GₖₛAₖₛAₖₛA_{ds}A_{ds}G_{ds}^{m}C_{ds}^{m}C_{ds}^{m}C_{ds}T_{ds}G_{ds}^{m}C_{ds}^{m}C_{ds}^{m}Cₖₛ^{m}CₖₛTₖ | 46.3 | 10684 | 10699 | 610 |
| 570844 | TₖₛAₖₛGₖₛG_{ds}A_{ds}A_{ds}A_{ds}A_{ds}G_{ds}^{m}C_{ds}^{m}C_{ds}^{m}C_{ds}T_{ds}Gₖₛ^{m}Cₖₛ^{m}Cₖ | 52.3 | 10687 | 10702 | 611 |
| 570845 | ^{m}CₖₛTₖₛTₖₛA_{ds}G_{ds}G_{ds}A_{ds}A_{ds}A_{ds}A_{ds}G_{ds}^{m}C_{ds}^{m}C_{ds}^{m}CₖₛTₖₛGₖ | 53.8 | 10689 | 10704 | 612 |
| 570846 | TₖₛGₖₛ^{m}CₖₛT_{ds}T_{ds}A_{ds}G_{ds}G_{ds}A_{ds}A_{ds}A_{ds}A_{ds}G_{ds}^{m}Cₖₛ^{m}Cₖₛ^{m}Cₖ | 47.8 | 10691 | 10706 | 613 |
| 570847 | Tₖₛ^{m}CₖₛTₖₛG_{ds}^{m}C_{ds}T_{ds}T_{ds}A_{ds}G_{ds}G_{ds}A_{ds}A_{ds}A_{ds}AₖₛGₖₛ^{m}Cₖ | 43.9 | 10693 | 10708 | 614 |
| 570848 | ^{m}CₖₛTₖₛ^{m}Cₖₛ^{m}C_{ds}T_{ds}^{m}C_{ds}T_{ds}G_{ds}^{m}C_{ds}T_{ds}T_{ds}A_{ds}G_{ds}GₖₛAₖₛAₖ | 67.9 | 10697 | 10712 | 615 |
| 570849 | ^{m}Cₖₛ^{m}Cₖₛ^{m}CₖₛT_{ds}^{m}C_{ds}^{m}C_{ds}T_{ds}^{m}C_{ds}T_{ds}G_{ds}^{m}C_{ds}T_{ds}T_{ds}AₖₛGₖₛGₖ | 50.8 | 10699 | 10714 | 616 |
| 570850 | ^{m}CₖₛTₖₛGₖₛA_{ds}T_{ds}T_{ds}T_{ds}G_{ds}A_{ds}G_{ds}G_{ds}A_{ds}A_{ds}GₖₛGₖₛGₖ | 41.1 | 10759 | 10774 | 617 |
| 570851 | Tₖₛ^{m}Cₖₛ^{m}CₖₛT_{ds}G_{ds}A_{ds}T_{ds}T_{ds}T_{ds}G_{ds}A_{ds}G_{ds}G_{ds}AₖₛAₖₛGₖ | 87.4 | 10761 | 10776 | 618 |
| 570852 | ^{m}Cₖₛ^{m}CₖₛTₖₛ^{m}C_{ds}^{m}C_{ds}T_{ds}G_{dS}A_{ds}T_{ds}T_{ds}T_{ds}G_{ds}A_{ds}GₖₛGₖₛAₖ | 75.8 | 10763 | 10778 | 619 |
| 570853 | GₖₛAₖₛ^{m}Cₖₛ^{m}C_{ds}T_{ds}^{m}C_{ds}^{m}C_{ds}T_{ds}G_{ds}A_{ds}T_{ds}T_{ds}T_{ds}GₖₛAₖₛGₖ | 87.4 | 10765 | 10780 | 620 |
| 570854 | AₖₛAₖₛGₖₛA_{ds}^{m}C_{ds}^{m}C_{ds}T_{ds}^{m}C_{ds}^{m}C_{ds}T_{ds}G_{ds}A_{ds}T_{ds}TₖₛTₖₛGₖ | 60.3 | 10767 | 10782 | 621 |
| 570855 | ^{m}Cₖₛ^{m}CₖₛAₖₛA_{ds}G_{ds}A_{ds}^{m}C_{ds}^{m}C_{ds}T_{ds}^{m}C_{ds}^{m}C_{ds}T_{ds}G_{ds}AₖₛTₖₛTₖ | 61.4 | 10769 | 10784 | 622 |
| 570856 | ^{m}CₖₛTₖₛGₖₛ^{m}C_{ds}T_{ds}T_{ds}^{m}C_{ds}^{m}C_{ds}A_{ds}A_{ds}G_{ds}A_{ds}^{m}C_{ds}^{m}CₖₛTₖₛ^{m}Cₖ | 40.4 | 10775 | 10790 | 623 |
| 570857 | AₖₛGₖₛ^{m}CₖₛT_{ds}G_{ds}^{m}C_{ds}T_{ds}T_{ds}^{m}C_{ds}^{m}C_{ds}A_{ds}A_{ds}G_{ds}Aₖₛ^{m}Cₖₛ^{m}Cₖ | 48.5 | 10777 | 10792 | 624 |
| 570858 | Gₖₛ^{m}CₖₛAₖₛG_{ds}^{m}C_{ds}T_{ds}G_{ds}^{m}C_{ds}T_{ds}T_{ds}^{m}C_{ds}^{m}C_{ds}A_{ds}AₖₛGₖₛAₖ | 87.7 | 10779 | 10794 | 625 |
| 570859 | ^{m}CₖₛTₖₛGₖₛG_{ds}T_{ds}G_{ds}G_{ds}A_{ds}G_{ds}A_{ds}A_{ds}^{m}C_{ds}^{m}C_{ds}AₖₛGₖₛAₖ | 92.6 | 10816 | 10831 | 626 |
| 570860 | ^{m}CₖₛTₖₛ^{m}CₖₛT_{ds}G_{ds}G_{ds}T_{ds}G_{ds}G_{ds}A_{ds}G_{ds}A_{ds}A_{ds}^{m}Cₖₛ^{m}CₖₛAₖ | 86.6 | 10818 | 10833 | 627 |
| 570861 | TₖₛTₖₛ^{m}CₖₛT_{ds}^{m}C_{ds}T_{ds}G_{ds}G_{ds}T_{ds}G_{ds}G_{dsAds}G_{ds}AₖₛAₖₛ^{m}Cₖ | 82.6 | 10820 | 10835 | 628 |
| 570862 | GₖₛAₖₛTₖₛT_{ds}^{m}C_{ds}T_{ds}^{m}C_{ds}T_{ds}G_{ds}G_{ds}T_{ds}G_{ds}G_{ds}AₖₛGₖₛAₖ | 76.1 | 10822 | 10837 | 629 |
| 570863 | Aₖₛ^{m}CₖₛTₖₛT_{ds}A_{ds}^{m}C_{ds}T_{ds}G_{ds}T_{ds}T_{ds}T_{ds}^{m}C_{ds}A_{ds}Tₖₛ^{m}Cₖₛ^{m}Cₖ | 80.6 | 10981 | 10996 | 630 |
| 570864 | ^{m}CₖₛGₖₛGₖₛA_{ds}^{m}C_{ds}^{m}C_{ds}^{m}C_{ds}^{m}C_{ds}^{m}C_{ds}T_{ds}^{m}C_{ds}^{m}C_{ds}^{m}C_{ds}^{m}CₖₛTₖₛ^{m}Cₖ | 58.7 | 11002 | 11017 | 631 |
| 570865 | GₖₛAₖₛ^{m}CₖₛG_{ds}G_{ds}A_{ds}^{m}C_{ds}^{m}C_{ds}^{m}C_{ds}^{m}C_{ds}^{m}C_{ds}T_{ds}^{m}C_{ds}^{m}Cₖₛ^{m}Cₖₛ^{m}Cₖ | 61.5 | 11004 | 11019 | 632 |
| 570866 | ^{m}CₖₛTₖₛGₖₛA_{ds}^{m}C_{ds}G_{ds}G_{ds}A_{ds}^{m}C_{ds}^{m}C_{ds}^{m}C_{ds}^{m}C_{ds}^{m}C_{ds}Tₖₛ^{m}Cₖₛ^{m}Cₖ | 47.6 | 11006 | 11021 | 633 |
| 570867 | ^{m}Cₖₛ^{m}Cₖₛ^{m}CₖₛT_{ds}G_{ds}A_{ds}^{m}C_{ds}G_{ds}G_{ds}A_{ds}^{m}C_{ds}^{m}C_{ds}^{m}C_{ds}^{m}Cₖₛ^{m}CₖₛTₖ | 69.5 | 11008 | 11023 | 634 |
| 570868 | AₖₛAₖₛGₖₛ^{m}C_{ds}^{m}C_{ds}^{m}C_{ds}T_{ds}^{m}C_{ds}A_{ds}^{m}C_{ds}^{m}C_{ds}T_{ds}T_{ds}TₖₛTₖₛ^{m}Cₖ | 54 | 11036 | 11051 | 635 |
| 570869 | GₖₛGₖₛAₖₛA_{ds}G_{ds}^{m}C_{ds}^{m}C_{ds}^{m}C_{ds}T_{ds}^{m}C_{ds}A_{ds}^{m}C_{ds}^{m}C_{ds}TₖₛTₖₛTₖ | 37.5 | 11038 | 11053 | 636 |
| 570870 | ^{m}CₖₛGₖₛGₖₛG_{ds}A_{ds}A_{ds}G_{ds}^{m}C_{ds}^{m}C_{ds}^{m}C_{ds}T_{ds}^{m}C_{ds}A_{ds}^{m}Cₖₛ^{m}CₖₛTₖ | 70.7 | 11040 | 11055 | 637 |
| 570871 | ^{m}Cₖₛ^{m}Ck_{sc}^{m}CₖₛG_{ds}G_{ds}G_{ds}A_{ds}A_{ds}G_{ds}^{m}C_{ds}^{m}C_{ds}^{m}C_{ds}T_{ds}^{m}CₖₛAₖₛ^{m}Cₖ | 71.2 | 11042 | 11057 | 638 |
| 570872 | ^{m}CₖₛAₖₛ^{m}Cₖₛ^{m}C_{ds}^{m}C_{ds}G_{ds}G_{ds}G_{ds}A_{ds}A_{ds}G_{ds}^{m}C_{ds}^{m}C_{ds}^{m}CₖₛTₖₛ^{m}Cₖ | 51.6 | 11044 | 11059 | 639 |
| 570873 | Gₖₛ^{m}Cₖₛ^{m}CₖₛA_{ds}^{m}C_{ds}^{m}C_{ds}^{m}C_{ds}G_{ds}G_{ds}G_{ds}A_{ds}A_{ds}G_{ds}^{m}Cₖₛ^{m}Cₖₛ^{m}Cₖ | 45.8 | 11046 | 11061 | 640 |
| 570874 | Aₖₛ^{m}CₖₛGₖₛ^{m}C_{ds}^{m}C_{ds}A_{ds}^{m}C_{ds}^{m}C_{ds}^{m}C_{ds}G_{ds}G_{ds}G_{ds}A_{ds}AₖₛGₖₛ^{m}Cₖ | 31.8 | 11048 | 11063 | 641 |
| 570875 | ^{m}CₖₛTₖₛGₖₛT_{ds}T_{ds}^{m}C_{ds}A_{ds}G_{ds}G_{ds}A_{ds}A_{ds}G_{ds}T_{ds}^{m}Cₖₛ^{m}Cₖₛ^{m}Cₖ | 14.3 | 11082 | 11097 | 642 |
| 570876 | TₖₛTₖₛ^{m}CₖₛT_{ds}G_{ds}T_{ds}T_{ds}^{m}C_{ds}A_{ds}G_{ds}G_{ds}A_{ds}A_{ds}GₖₛTₖₛ^{m}Cₖ | 18 | 11084 | 11099 | 643 |
| 570877 | Gₖₛ^{m}CₖₛTₖₛT_{ds}^{m}C_{ds}T_{ds}G_{ds}T_{ds}T_{ds}^{m}C_{ds}A_{ds}G_{ds}G_{ds}AₖₛAₖₛGₖ | 44 | 11086 | 11101 | 644 |

**Table 15**

| Inhibition of human DMPK RNA transcript in HepG2 cells targeting SEQ ID NO: 2 | | | | | |
|---|---|---|---|---|---|
| ISIS No. | Sequence | % Target Expression | Start Site on Seq ID: 2 | Stop Site on Seq ID: 2 | Seq ID No. |
| UTC | N/A | 100 | N/A | N/A | |
| 445569 | ^{m}CₑₛGₑₛGₑₛAₑₛGₑₛ^{m}C_{ds}G_{ds}G_{ds}T_{ds}T_{ds}G_{ds}T_{ds}G_{ds}A_{ds}A_{ds}^{m}CₑₛTₑₛGₑₛGₑₛ^{m}Cₑ | 55 | 13226 | 13245 | 24 |
| 486178 | Aₖₛ^{m}CₖₛAₖₛA_{ds}T_{ds}A_{ds}A_{ds}A_{ds}T_{ds}A_{ds}^{m}C_{ds}^{m}C_{ds}G_{ds}AₖₛGₖₛGₖ | 33.9 | 13836 | 13851 | 23 |
| 570647 | Gₖₛ^{m}CₖₛTₖₛT_{ds}G_{ds}G_{ds}G_{ds}^{m}C_{ds}^{m}C_{ds}^{m}C_{ds}A_{ds}^{m}C_{ds}^{m}C_{ds}^{m}Cₖₛ^{m}CₖₛTₖ | 80.3 | 5718 | 5733 | 645 |
| 570648 | AₖₛGₖₛGₖₛ^{m}C_{ds}T_{ds}T_{ds}G_{ds}G_{ds}G_{ds}^{m}C_{ds}^{m}C_{ds}^{m}C_{ds}A_{ds}^{m}Cₖₛ^{m}Cₖₛ^{m}Cₖ | 92.3 | 5720 | 5735 | 646 |
| 570649 | ^{m}CₖₛGₖₛAₖₛG_{ds}G_{ds}^{m}C_{ds}T_{ds}T_{ds}G_{ds}G_{ds}G_{ds}^{m}C_{ds}^{m}C_{ds}^{m}CₖₛAₖₛ^{m}Cₖ | 100.7 | 5722 | 5737 | 647 |
| 570650 | AₖₛGₖₛ^{m}CₖₛG_{ds}A_{ds}G_{ds}G_{ds}^{m}C_{ds}T_{ds}T_{ds}G_{ds}G_{ds}G_{ds}^{m}Cₖₛ^{m}Cₖₛ^{m}Cₖ | 75.8 | 5724 | 5739 | 648 |
| 570651 | AₖₛGₖₛAₖₛG_{ds}^{m}C_{ds}G_{ds}A_{ds}G_{ds}G_{ds}^{m}C_{ds}T_{ds}T_{ds}G_{ds}GₖₛGₖₛ^{m}Cₖ | 99.8 | 5726 | 5741 | 649 |
| 570652 | Gₖₛ^{m}CₖₛAₖₛG_{ds}A_{ds}G_{ds}^{m}C_{ds}G_{ds}A_{ds}G_{ds}G_{ds}^{m}C_{ds}T_{ds}TₖₛGₖₛGₖ | 135.4 | 5728 | 5743 | 650 |
| 570653 | GₖₛAₖₛGₖₛ^{m}C_{ds}A_{ds}G_{ds}A_{ds}G_{ds}^{m}C_{ds}G_{ds}A_{ds}G_{ds}G_{ds}^{m}CₖₛTₖₛTₖ | 111.5 | 5730 | 5745 | 651 |
| 570654 | AₖₛAₖₛAₖₛG_{ds}G_{ds}A_{ds}G_{ds}^{m}C_{ds}A_{ds}G_{ds}A_{ds}G_{ds}^{m}C_{ds}GₖₛAₖₛGₖ | 87.5 | 5734 | 5749 | 652 |
| 570655 | ^{m}CₖₛAₖₛAₖₛA_{ds}A_{ds}G_{ds}G_{ds}A_{ds}G_{ds}^{m}C_{ds}A_{ds}G_{ds}A_{ds}Gₖₛ^{m}CₖₛGₖ | 94.5 | 5736 | 5751 | 653 |
| 570656 | TₖₛGₖₛGₖₛA_{ds} C_{ds} C_{ds}A_{ds}A_{ds}A_{ds}A_{ds}G_{ds}G_{ds}A_{ds}Gₖₛ CₖₛAₖ | 75.4 | 5741 | 5756 | 654 |
| 570657 | ^{m}Cₖₛ^{m}CₖₛTₖₛG_{ds}G_{ds}A_{ds}^{m}C_{ds}^{m}C_{ds}A_{ds}A_{ds}A_{ds}A_{ds}G_{ds}GₖₛAₖₛGₖ | 87.3 | 5743 | 5758 | 655 |
| 570658 | ^{m}CₖₛAₖₛ^{m}Cₖₛ^{m}C_{ds}T_{ds}G_{d}sG_{ds}A_{ds}^{m}C_{ds}^{m}C_{ds}A_{ds}A_{ds}A_{ds}AₖₛGₖₛGₖ | 93.2 | 5745 | 5760 | 656 |
| 570659 | ^{m}CₖₛGₖₛ^{m}CₖₛA_{ds}^{m}C_{ds}^{m}C_{ds}T_{ds}G_{ds}G_{ds}A_{ds}^{m}C_{ds}^{m}C_{ds}A_{ds}AₖₛAₖₛAₖ | 70 | 5747 | 5762 | 657 |
| 570660 | GₖₛAₖₛ^{m}Cₖₛ^{m}C_{ds}G_{ds}^{m}C_{ds}A_{ds}^{m}C_{ds}^{m}C_{ds}T_{ds}G_{ds}G_{ds}A_{ds}^{m}Cₖₛ^{m}CₖₛAₖ | 46.4 | 5750 | 5765 | 658 |
| 570661 | Aₖₛ^{m}Cₖₛ^{m}CₖₛT_{ds}T_{ds}G_{ds}T_{ds}A_{ds}G_{ds}T_{ds}G_{ds}G_{ds}A_{ds}^{m}CₖₛGₖₛAₖ | 44 | 5951 | 5966 | 659 |
| 570662 | Tₖₛ^{m}CₖₛAₖₛ^{m}C_{ds}^{m}C_{ds}T_{ds}T_{ds}G_{ds}T_{ds}A_{ds}G_{ds}T_{ds}G_{ds}GₖₛAₖₛ^{m}Cₖ | 76.8 | 5953 | 5968 | 660 |
| 570663 | Gₖₛ^{m}CₖₛTₖₛ^{m}C_{ds}A_{ds}^{m}C_{ds}^{m}C_{ds}T_{ds}T_{ds}G_{ds}T_{ds}A_{ds}G_{ds}TₖₛGₖₛGₖ | 69.5 | 5955 | 5970 | 661 |
| 570664 | GₖₛGₖₛAₖₛG_{ds}A_{ds}G_{d}sG_{ds}A_{ds}G_{ds}G_{ds}^{m}C_{ds}G_{ds}A_{ds}TₖₛAₖₛGₖ | 88.2 | 6015 | 6030 | 662 |
| 570665 | AₖₛGₖₛGₖₛG_{ds}A_{ds}G_{ds}A_{ds}G_{ds}G_{ds}A_{ds}G_{ds}G_{ds}^{m}C_{ds}GₖₛAₖₛTₖ | 96.9 | 6017 | 6032 | 663 |
| 570666 | ^{m}CₖₛTₖₛ^{m}Cₖₛ^{m}C_{ds}T_{ds}G_{ds}^{m}C_{ds}T_{ds}^{m}C_{ds}A_{ds}G_{dS}A_{ds}G_{ds}GₖₛGₖₛAₖ | 74.7 | 6028 | 6043 | 664 |
| 570667 | GₖₛTₖₛGₖₛ^{m}C_{ds}T_{ds}^{m}C_{ds}^{m}C_{ds}T_{ds}G_{ds}^{m}C_{ds}T_{ds}^{m}C_{ds}A_{ds}GₖₛAₖₛGₖ | 77.5 | 6031 | 6046 | 665 |
| 570668 | AₖₛGₖₛGₖₛT_{ds}G_{ds}^{m}C_{ds}T_{ds}^{m}C_{ds}^{m}C_{ds}T_{ds}G_{ds}^{m}C_{ds}T_{ds}^{m}CₖₛAₖₛGₖ | 76.7 | 6033 | 6048 | 666 |
| 570669 | AₖₛGₖₛAₖₛG_{ds}G_{ds}T_{ds}G_{ds}^{m}C_{ds}T_{ds}^{m}C_{ds}^{m}C_{ds}T_{ds}G_{ds}^{m}CₖₛTₖₛ^{m}Cₖ | 43.3 | 6035 | 6050 | 667 |
| 570670 | AₖₛGₖₛAₖₛG_{ds}A_{ds}G_{ds}G_{ds}T_{ds}G_{ds}^{m}C_{ds}T_{ds}^{m}C_{ds}^{m}C_{ds}TₖₛGₖₛ^{m}Cₖ | 27.1 | 6037 | 6052 | 668 |
| 570671 | Aₖₛ^{m}Cₖₛ^{m}Cₖₛ^{m}C_{ds}^{m}C_{ds}G_{ds}^{m}C_{ds}^{m}C_{ds}^{m}C_{ds}^{m}C_{ds}^{m}C_{ds}G_{ds}^{m}C_{ds}Tₖₛ^{m}CₖₛAₖ | 42.6 | 6291 | 6306 | 669 |
| 570672 | ^{m}CₖₛTₖₛAₖₛ^{m}C_{ds}^{m}C_{ds}^{m}C_{ds}^{m}C_{ds}G_{ds}^{m}C_{ds}^{m}C_{ds}^{m}C_{ds}^{m}C_{ds}^{m}C_{ds}Gₖₛ^{m}CₖₛTₖ | 44.9 | 6293 | 6308 | 670 |
| 570673 | Aₖₛ^{m}Cₖₛ^{m}CₖₛT_{ds}A_{ds}^{m}C_{ds}^{m}C_{ds}^{m}C_{ds}^{m}C_{ds}G_{ds}^{m}C_{ds}^{m}C_{ds}^{m}C_{ds}^{m}Cₖₛ^{m}CₖₛGₖ | 36.6 | 6295 | 6310 | 671 |
| 570674 | GₖₛTₖₛAₖₛ^{m}C_{ds}^{m}C_{ds}T_{ds}A_{ds}^{m}C_{ds}^{m}C_{ds}^{m}C_{ds}^{m}C_{ds}G_{ds}^{m}C_{ds}^{m}Cₖₛ^{m}Cₖₛ^{m}Cₖ | 52 | 6297 | 6312 | 672 |
| 570675 | AₖₛGₖₛGₖₛT_{ds}A_{ds}^{m}C_{ds}^{m}C_{ds}T_{ds}A_{ds}^{m}C_{ds}^{m}C_{ds}^{m}C_{ds}^{m}C_{ds}Gₖₛ^{m}Cₖₛ^{m}Cₖ | 56.4 | 6299 | 6314 | 673 |
| 570676 | GₖₛGₖₛGₖₛA_{ds}G_{ds}G_{dS}T_{ds}T_{ds}^{m}C_{ds}^{m}C_{ds}^{m}C_{ds}G_{ds}^{m}C_{ds}AₖₛGₖₛ^{m}Cₖ | 51.4 | 6329 | 6344 | 674 |
| 570677 | GₖₛTₖₛ^{m}Cₖₛ^{m}C_{ds}T_{ds}T_{ds}A_{ds}^{m}C_{ds}T_{ds}^{m}C_{ds}^{m}C_{ds}A_{ds}A_{ds}^{m}CₖₛTₖₛTₖ | 28 | 6360 | 6375 | 675 |
| 570678 | ^{m}CₖₛTₖₛGₖₛT_{ds}^{m}C_{ds}^{m}C_{ds}T_{ds}T_{ds}A_{ds}^{m}C_{ds}T_{ds}^{m}C_{ds}^{m}C_{ds}AₖₛAₖₛ^{m}Cₖ | 33.6 | 6362 | 6377 | 676 |
| 570679 | ^{m}CₖₛAₖₛ^{m}CₖₛT_{ds}G_{ds}T_{ds}^{m}C_{ds}^{m}C_{ds}T_{ds}T_{ds}A_{ds}^{m}C_{ds}T_{ds}^{m}Cₖₛ^{m}CₖₛAₖ | 7.9 | 6364 | 6379 | 677 |
| 570680 | GₖₛGₖₛ^{m}CₖₛA_{ds}^{m}C_{ds}T_{ds}G_{ds}T_{ds}^{m}C_{ds}^{m}C_{ds}T_{ds}T_{ds}A_{ds}^{m}CₖₛTₖₛ^{m}Cₖ | 20.2 | 6366 | 6381 | 678 |
| 570681 | TₖₛAₖₛGₖₛG_{ds}^{m}C_{ds}A_{ds}^{m}C_{ds}T_{ds}G_{ds}T_{ds}^{m}C_{ds}^{m}C_{ds}T_{ds}TₖₛAₖₛ^{m}Cₖ | 38.3 | 6368 | 6383 | 679 |
| 570682 | GₖₛGₖₛTₖₛA_{ds}G_{ds}G_{ds}^{m}C_{ds}A_{ds}^{m}C_{ds}T_{ds}G_{ds}T_{ds}^{m}C_{ds}^{m}CₖₛTₖₛTₖ | 13.9 | 6370 | 6385 | 680 |
| 570683 | GₖₛTₖₛ^{m}CₖₛA_{ds}^{m}C_{ds}T_{ds}G_{ds}^{m}C_{ds}T_{ds}G_{ds}G_{ds}G_{ds}T_{ds}^{m}Cₖₛ^{m}CₖₛTₖ | 29 | 6445 | 6460 | 681 |
| 570684 | GₖₛGₖₛTₖₛ^{m}C_{ds}A_{ds}^{m}C_{ds}T_{ds}G_{ds}^{m}C_{ds}T_{ds}G_{ds}G_{ds}G_{ds}Tₖₛ^{m}Cₖₛ^{m}Cₖ | 21.3 | 6446 | 6461 | 43 |
| 570685 | AₖₛGₖₛGₖₛT_{ds}^{m}C_{ds}A_{ds}^{m}C_{ds}T_{ds}G_{ds}^{m}C_{ds}T_{ds}G_{ds}G_{ds}GₖₛTₖₛ^{m}Cₖ | 16.9 | 6447 | 6462 | 682 |
| 570686 | ^{m}CₖₛTₖₛAₖₛG_{ds}G_{ds}T_{ds}^{m}C_{ds}A_{ds}^{m}C_{ds}T_{ds}G_{ds}^{m}C_{ds}T_{ds}GₖₛGₖₛGₖ | 19.6 | 6449 | 6464 | 683 |
| 570687 | GₖₛTₖₛ^{m}CₖₛT_{ds}A_{ds}G_{ds}G_{ds}T_{ds}^{m}C_{ds}A_{ds}^{m}C_{ds}T_{ds}G_{ds}^{m}CₖₛTₖₛGₖ | 15.7 | 6451 | 6466 | 684 |
| 570688 | AₖₛAₖₛGₖₛT_{ds}^{m}C_{ds}T_{ds}A_{ds}G_{ds}G_{ds}T_{ds}^{m}C_{ds}A_{ds}^{m}C_{ds}TₖₛGₖₛ^{m}Cₖ | 16.6 | 6453 | 6468 | 685 |
| 570689 | Gₖₛ^{m}CₖₛAₖₛ^{m}C_{ds}T_{ds}^{m}C_{ds}^{m}C_{ds}A_{ds}T_{ds}T_{ds}G_{ds}T_{ds}^{m}C_{ds}Tₖₛ^{m}CₖₛAₖ | 13.2 | 6530 | 6545 | 686 |
| 570690 | ^{m}CₖₛTₖₛGₖₛ^{m}C_{ds}A_{ds}^{m}C_{ds}T_{ds}^{m}C_{ds}^{m}C_{ds}A_{ds}T_{ds}T_{ds}G_{ds}Tₖₛ^{m}CₖₛTₖ | 50.1 | 6532 | 6547 | 687 |
| 570691 | ^{m}Cₖₛ^{m}Cₖₛ^{m}CₖₛT_{ds}G_{ds}^{m}C_{ds}A_{ds}^{m}C_{ds}T_{ds}^{m}C_{ds}^{m}C_{ds}A_{ds}T_{ds}TₖₛGₖₛTₖ | 48.4 | 6534 | 6549 | 688 |
| 570692 | ^{m}Cₖₛ^{m}Cₖₛ^{m}Cₖₛ^{m}C_{ds}^{m}C_{ds}T_{ds}G_{ds}^{m}C_{ds}A_{ds}^{m}C_{ds}T_{ds}^{m}C_{ds}^{m}C_{ds}AₖₛTₖₛTₖ | 74 | 6536 | 6551 | 689 |
| 570693 | ^{m}CₖₛTₖₛTₖₛG_{ds}^{m}C_{ds}T_{ds}G_{ds}A_{ds}G_{ds}T_{ds}^{m}C_{ds}A_{ds}G_{ds}GₖₛAₖₛGₖ | 25.3 | 6559 | 6574 | 690 |
| 570694 | Tₖₛ^{m}Cₖₛ^{m}CₖₛT_{ds}T_{ds}G_{ds}^{m}C_{ds}T_{ds}G_{ds}A_{ds}G_{ds}T_{ds}^{m}C_{ds}AₖₛGₖₛGₖ | 39.5 | 6561 | 6576 | 691 |
| 570695 | ^{m}CₖₛTₖₛTₖₛ^{m}C_{ds}^{m}C_{ds}T_{ds}T_{ds}G_{ds}^{m}C_{ds}T_{ds}G_{ds}A_{ds}G_{ds}Tₖₛ^{m}CₖₛAₖ | 22.9 | 6563 | 6578 | 692 |
| 570696 | Aₖₛ^{m}Cₖₛ^{m}CₖₛT_{ds}T_{ds}^{m}C_{ds}^{m}C_{ds}T_{ds}T_{ds}G_{ds}^{m}C_{ds}T_{ds}G_{ds}AₖₛGₖₛTₖ | 52.5 | 6565 | 6580 | 693 |
| 570697 | GₖₛGₖₛAₖₛ^{m}C_{ds}^{m}C_{ds}T_{ds}T_{ds}^{m}C_{ds}^{m}C_{ds}T_{ds}T_{ds}G_{ds}^{m}C_{ds}TₖₛGₖₛAₖ | 37.6 | 6567 | 6582 | 694 |
| 570698 | ^{m}CₖₛAₖₛGₖₛG_{ds}A_{ds}^{m}C_{ds}^{m}C_{ds}T_{ds}T_{ds}^{m}C_{ds}^{m}C_{ds}T_{ds}T_{ds}Gₖₛ^{m}CₖₛTₖ | 44.2 | 6569 | 6584 | 695 |
| 570699 | AₖₛGₖₛ^{m}Cₖₛ^{m}C_{ds}^{m}C_{ds}T_{ds}^{m}C_{ds}^{m}C_{ds}A_{ds}G_{ds}G_{ds}A_{ds}^{m}C_{ds}^{m}CₖₛTₖₛTₖ | 26.6 | 6576 | 6591 | 696 |
| 570700 | TₖₛAₖₛGₖₛ^{m}C_{ds}T_{ds}^{m}C_{ds}^{m}C_{ds}^{m}C_{ds}^{m}C_{ds}A_{ds}^{m}C_{ds}T_{ds}^{m}C_{ds}^{m}CₖₛAₖₛGₖ | 33.6 | 6594 | 6609 | 697 |
| 570701 | GₖₛAₖₛTₖₛA_{ds}G_{ds}^{m}C_{ds}T_{ds}^{m}C_{ds}^{m}C_{ds}^{m}C_{ds}^{m}C_{ds}A_{ds}^{m}C_{ds}Tₖₛ^{m}Cₖₛ^{m}Cₖ | 20.4 | 6596 | 6611 | 698 |
| 570702 | ^{m}CₖₛAₖₛGₖₛA_{ds}T_{ds}A_{ds}G_{ds}^{m}C_{ds}T_{ds}^{m}C_{ds}^{m}C_{ds}^{m}C_{ds}^{m}C_{ds}Aₖₛ^{m}CₖₛTₖ | 33.8 | 6598 | 6613 | 699 |
| 570703 | ^{m}CₖₛTₖₛ^{m}CₖₛA_{ds}G_{ds}A_{ds}T_{ds}A_{ds}G_{ds}^{m}C_{ds}T_{ds}^{m}C_{ds}^{m}C_{ds}^{m}Cₖₛ^{m}CₖₛAₖ | 25.8 | 6600 | 6615 | 700 |
| 570704 | AₖₛGₖₛ^{m}CₖₛT_{ds}^{m}C_{ds}A_{ds}G_{ds}A_{ds}T_{ds}A_{ds}G_{ds}^{m}C_{ds}T_{ds}^{m}Cₖₛ^{m}Cₖₛ^{m}Cₖ | 29.1 | 6602 | 6617 | 701 |
| 570705 | Tₖₛ^{m}CₖₛAₖₛG_{ds}^{m}C_{ds}T_{ds}^{m}C_{ds}A_{ds}G_{ds}A_{ds}T_{ds}A_{ds}G_{ds}^{m}CₖₛTₖₛ^{m}Cₖ | 47.4 | 6604 | 6619 | 702 |
| 570706 | Tₖₛ^{m}CₖₛTₖₛ^{m}C_{ds}A_{ds}G_{ds}^{m}C_{ds}T_{ds}^{m}C_{ds}A_{ds}G_{ds}A_{ds}T_{ds}AₖₛGₖₛ^{m}Cₖ | 33.4 | 6606 | 6621 | 703 |
| 570707 | GₖₛAₖₛGₖₛT_{ds}^{m}C_{ds}^{m}C_{ds}T_{ds}^{m}C_{ds}T_{ds}^{m}C_{ds}^{m}C_{ds}T_{ds}G_{ds}^{m}CₖₛTₖₛTₖ | 49 | 6636 | 6651 | 704 |
| 570708 | GₖₛGₖₛAₖₛG_{ds}G_{ds}A_{ds}G_{ds}T_{ds}^{m}C_{ds}^{m}C_{ds}T_{ds}^{m}C_{ds}T_{ds}^{m}Cₖₛ^{m}CₖₛTₖ | 79.2 | 6640 | 6655 | 705 |
| 570709 | GₖₛAₖₛGₖₛG_{ds}A_{ds}G_{ds}G_{ds}A_{ds}G_{ds}T_{ds}^{m}C_{ds}^{m}C_{ds}T_{ds}^{m}CₖₛTₖₛ^{m}Cₖ | 63.3 | 6642 | 6657 | 706 |
| 570710 | ^{m}CₖₛAₖₛAₖₛA_{ds}A_{ds}G_{ds}G_{ds}G_{ds}^{m}C_{ds}A_{ds}^{m}C_{ds}^{m}C_{ds}^{m}C_{ds}AₖₛGₖₛAₖ | 38.8 | 6713 | 6728 | 707 |
| 570711 | AₖₛGₖₛ^{m}CₖₛA_{ds}A_{ds}A_{ds}A_{ds}G_{ds}G_{ds}G_{ds}^{m}C_{ds}A_{ds}^{m}C_{ds}^{m}Cₖₛ^{m}CₖₛAₖ | 13.7 | 6715 | 6730 | 708 |
| 570712 | GₖₛGₖₛAₖₛT_{ds}^{m}C_{ds}^{m}C_{ds}^{m}C_{ds}^{m}C_{ds}A_{ds}G_{ds}T_{ds}A_{ds}T_{ds}TₖₛGₖₛTₖ | 45.8 | 6733 | 6748 | 709 |
| 570713 | ^{m}CₖₛTₖₛGₖₛG_{ds}A_{ds}T_{ds}^{m}C_{ds}^{m}C_{ds}^{m}C_{ds}^{m}C_{ds}A_{ds}G_{ds}T_{ds}AₖₛTₖₛTₖ | 45.6 | 6735 | 6750 | 710 |
| 570714 | TₖₛGₖₛ^{m}CₖₛT_{ds}G_{ds}G_{ds}A_{ds}T_{ds}^{m}C_{ds}^{m}C_{ds}^{m}C_{ds}^{m}C_{ds}A_{ds}GₖₛTₖₛAₖ | 43.6 | 6737 | 6752 | 711 |
| 570715 | AₖₛTₖₛTₖₛ^{m}C_{ds}T_{ds}^{m}C_{ds}T_{ds}A_{ds}G_{ds}A_{ds}^{m}C_{ds}T_{ds}G_{ds}^{m}CₖₛAₖₛAₖ | 18.3 | 6789 | 6804 | 712 |
| 570716 | TₖₛAₖₛAₖₛT_{ds}T_{ds}^{m}C_{ds}T_{ds}^{m}C_{ds}T_{ds}A_{ds}G_{ds}A_{ds}^{m}C_{ds}TₖₛGₖₛ^{m}Cₖ | 15.1 | 6791 | 6806 | 713 |
| 570717 | Tₖₛ^{m}CₖₛTₖₛA_{ds}A_{ds}T_{ds}T_{ds}^{m}C_{ds}T_{ds}^{m}C_{ds}T_{ds}A_{ds}G_{ds}Aₖₛ^{m}CₖₛTₖ | 49.9 | 6793 | 6808 | 714 |
| 570718 | Tₖₛ^{m}CₖₛTₖₛ^{m}C_{ds}T_{ds}A_{ds}A_{ds}T_{ds}T_{ds}^{m}C_{ds}T_{ds}^{m}C_{ds}T_{ds}AₖₛGₖₛAₖ | 77.6 | 6795 | 6810 | 715 |
| 570719 | ^{m}CₖₛTₖₛ^{m}Cₖₛ^{m}C_{ds}A_{ds}T_{ds}A_{ds}A_{ds}T_{ds}T_{ds}^{m}C_{ds}T_{ds}^{m}C_{ds}TₖₛAₖₛAₖ | 42 | 6804 | 6819 | 716 |
| 570720 | Aₖₛ^{m}CₖₛTₖₛ^{m}C_{ds}T_{ds}^{m}C_{ds}^{m}C_{ds}A_{ds}T_{ds}A_{ds}A_{ds}T_{ds}T_{ds}^{m}CₖₛTₖₛ^{m}Cₖ | 28.5 | 6807 | 6822 | 717 |
| 570721 | Aₖₛ^{m}CₖₛAₖₛ^{m}C_{ds}T_{ds}^{m}C_{ds}T_{ds}^{m}C_{ds}^{m}C_{ds}A_{ds}T_{ds}A_{ds}A_{ds}TₖₛTₖₛ^{m}Cₖ | 27.4 | 6809 | 6824 | 718 |
| 570722 | ^{m}Cₖₛ^{m}CₖₛAₖₛ^{m}C_{ds}A_{ds}^{m}C_{ds}T_{ds}^{m}C_{ds}T_{ds}^{m}C_{ds}^{m}C_{ds}A_{ds}T_{ds}AₖₛAₖₛTₖ | 35.4 | 6811 | 6826 | 719 |
| 570723 | TₖₛGₖₛ^{m}Cₖₛ^{m}C_{ds}A_{ds}^{m}C_{ds}A_{ds}^{m}C_{ds}T_{ds}^{m}C_{ds}T_{ds}^{m}C_{ds}^{m}C_{ds}AₖₛTₖₛAₖ | 45 | 6813 | 6828 | 720 |

**Table 16**

| Inhibition of human DMPK RNA transcript in HepG2 cells targeting SEQ ID NO: 2 | | | | | |
|---|---|---|---|---|---|
| ISIS No. | Sequence | % Target Expression | Start Site on Seq ID: 2 | Stop Site on Seq ID: 2 | Seq ID No. |
| UTC | N/A | 100 | N/A | N/A | |
| 445569 | ^{m}CₑₛGₑₛGₑₛAₑₛGₑₛ^{m}C_{ds}G_{ds}G_{ds}T_{ds}T_{ds}G_{ds}T_{ds}G_{ds}A_{ds}A_{ds}^{m}CₑₛTₑₛGₑₛGₑₛ^{m}Cₑ | 33.9 | 13226 | 13245 | 24 |
| 486178 | Aₖₛ^{m}CₖₛAₖₛA_{ds}T_{ds}A_{ds}A_{ds}A_{ds}T_{ds}A_{ds}^{m}C_{ds}^{m}C_{ds}G_{ds}AₖₛGₖₛGₖ | 21.5 | 13836 | 13851 | 23 |
| 570339 | ^{m}Cₖₛ^{m}Cₖₛ^{m}CₖₛA_{ds}T_{ds}G_{ds}^{m}C_{ds}^{m}C_{ds}^{m}C_{ds}A_{ds}T_{ds}^{m}C_{ds}^{m}C_{ds}TₖₛGₖₛ^{m}Cₖ | 56.2 | 1534 | 1549 | 721 |
| 570340 | GₖₛGₖₛAₖₛ^{m}C_{ds}A_{ds}G_{ds}A_{ds}G_{ds}A_{ds}A_{ds}A_{ds}T_{ds}G_{ds}TₖₛTₖₛGₖ | 46.7 | 1597 | 1612 | 722 |
| 570341 | GₖₛGₖₛ^{m}CₖₛA_{ds}T_{ds}A_{ds}G_{ds}G_{ds}A_{ds}^{m}C_{ds}A_{ds}G_{ds}A_{ds}GₖₛAₖₛAₖ | 35.6 | 1603 | 1618 | 723 |
| 570342 | GₖₛTₖₛGₖₛG_{ds}^{m}C_{ds}A_{ds}T_{ds}A_{ds}G_{ds}G_{ds}A_{ds}^{m}C_{ds}A_{ds}GₖₛAₖₛGₖ | 34.8 | 1605 | 1620 | 724 |
| 570343 | TₖₛGₖₛGₖₛT_{ds}G_{ds}G_{ds}^{m}C_{ds}A_{ds}T_{ds}A_{ds}G_{ds}G_{ds}A_{ds}^{m}CₖₛAₖₛGₖ | 60.3 | 1607 | 1622 | 725 |
| 570344 | ^{m}CₖₛTₖₛTₖₛA_{ds}^{m}C_{ds}T_{ds}^{m}CdₛT_{ds}G_{ds}^{m}C_{ds}^{m}C_{ds}^{m}C_{ds}^{m}C_{ds}Tₖₛ^{m}Cₖₛ^{m}Cₖ | 49.6 | 1627 | 1642 | 726 |
| 570345 | Aₖₛ^{m}Cₖₛ^{m}CₖₛT_{ds}T_{ds}A_{ds}^{m}C_{ds}T_{ds}^{m}C_{ds}T_{ds}G_{ds}^{m}C_{ds}^{m}C_{ds}^{m}Cₖₛ^{m}CₖₛTₖ | 48.6 | 1629 | 1644 | 727 |
| 570346 | TₖₛGₖsAₖₛ^{m}C_{ds}^{m}C_{ds}T_{ds}T_{ds}A_{ds}^{m}C_{ds}T_{ds}^{m}C_{ds}T_{ds}G_{ds}^{m}Cₖₛ^{m}Cₖₛ^{m}Cₖ | 36.8 | 1631 | 1646 | 728 |
| 570347 | Gₖₛ^{m}CₖₛTₖₛG_{ds}A_{ds}^{m}C_{ds}^{m}C_{ds}T_{ds}T_{ds}A_{ds}^{m}C_{ds}T_{ds}^{m}C_{ds}TₖₛGₖₛ^{m}Cₖ | 53.5 | 1633 | 1648 | 729 |
| 570348 | ^{m}CₖₛTₖₛGₖₛ^{m}C_{ds}T_{ds}G_{ds}A_{ds}^{m}C_{ds}^{m}C_{ds}T_{ds}T_{ds}A_{ds}^{m}C_{ds}Tₖₛ^{m}CₖₛTₖ | 59 | 1635 | 1650 | 730 |
| 570349 | ^{m}CₖₛTₖₛ^{m}CₖₛT_{ds}G_{ds}^{m}C_{ds}T_{ds}G_{ds}A_{ds}^{m}C_{ds}^{m}C_{ds}T_{ds}T_{ds}Aₖₛ^{m}CₖₛTₖ | 70.8 | 1637 | 1652 | 731 |
| 570350 | Gₖₛ^{m}Cₖₛ^{m}CₖₛT_{ds}^{m}C_{ds}T_{ds}G_{ds}^{m}C_{ds}T_{ds}G_{ds}A_{ds}^{m}C_{ds}^{m}C_{ds}TₖₛTₖₛAₖ | 54 | 1639 | 1654 | 732 |
| 570351 | ^{m}Cₖₛ^{m}CₖₛAₖₛT_{ds}G_{ds}G_{ds}^{m}C_{ds}T_{ds}^{m}C_{ds}T_{ds}G_{ds}A_{ds}G_{ds}Tₖₛ^{m}CₖₛAₖ | 52.6 | 1666 | 1681 | 733 |
| 570352 | AₖₛGₖₛ^{m}Cₖₛ^{m}C_{ds}A_{ds}T_{ds}G_{ds}G_{ds}^{m}C_{ds}T_{ds}^{m}C_{ds}T_{ds}G_{ds}AₖₛGₖₛTₖ | 60.7 | 1668 | 1683 | 734 |
| 570353 | TₖₛAₖₛAₖₛG_{ds}^{m}C_{ds}^{m}C_{ds}A_{ds}T_{ds}G_{ds}G_{ds}^{m}C_{ds}T_{ds}^{m}C_{ds}TₖₛGₖₛAₖ | 82.3 | 1670 | 1685 | 735 |
| 570354 | TₖₛAₖₛGₖₛ^{m}C_{ds}^{m}C_{ds}T_{ds}G_{ds}^{m}C_{ds}T_{ds}G_{ds}T_{ds}G_{ds}A_{ds}^{m}CₖₛTₖₛ^{m}Cₖ | 40.8 | 1687 | 1702 | 736 |
| 570355 | AₖₛTₖₛGₖₛG_{ds}G_{ds}A_{ds}G_{ds}G_{ds}^{m}C_{ds}T_{ds}G_{ds}T_{ds}T_{ds}GₖₛGₖₛ^{m}Cₖ | 90.7 | 1707 | 1722 | 737 |
| 570356 | ^{m}Cₖₛ^{m}CₖₛAₖₛT_{ds}G_{ds}G_{ds}G_{ds}A_{ds}G_{ds}G_{ds}^{m}C_{ds}T_{ds}G_{ds}TₖₛTₖₛGₖ | 73.9 | 1709 | 1724 | 738 |
| 570357 | GₖₛGₖₛ^{m}Cₖₛ^{m}C_{ds}A_{ds}T_{ds}G_{ds}G_{ds}G_{ds}A_{ds}G_{ds}G_{ds}^{m}C_{ds}TₖₛGₖₛTₖ | 94.9 | 1711 | 1726 | 739 |
| 570358 | GₖₛTₖₛGₖₛ^{m}C_{ds}A_{ds}G_{ds}A_{ds}G_{ds}A_{ds}G_{ds}G_{ds}^{m}C_{ds}^{m}C_{ds}AₖₛTₖₛGₖ | 73.5 | 1720 | 1735 | 740 |
| 570359 | GₖₛAₖₛGₖₛ^{m}C_{ds}T_{ds}^{m}C_{ds}^{m}C_{ds}^{m}C_{ds}A_{ds}G_{ds}^{m}C_{ds}A_{ds}T_{ds}GₖₛAₖₛ^{m}Cₖ | 70.2 | 1759 | 1774 | 741 |
| 570360 | AₖₛGₖₛGₖₛG_{ds}A_{ds}G_{ds}^{m}C_{ds}T_{ds}^{m}C_{ds}^{m}C_{ds}^{m}C_{ds}A_{ds}G_{ds}^{m}CₖₛAₖₛTₖ | 56.1 | 1762 | 1777 | 742 |
| 570361 | Gₖₛ^{m}Cₖₛ^{m}CₖₛA_{ds}T_{ds}A_{ds}G_{ds}A_{ds}G_{ds}^{m}C_{ds}^{m}C_{ds}^{m}C_{ds}A_{ds}^{m}CₖₛTₖₛTₖ | 54.9 | 1799 | 1814 | 743 |
| 570362 | GₖₛGₖₛGₖₛ^{m}C_{ds}^{m}C_{ds}A_{ds}T_{ds}A_{ds}G_{ds}A_{ds}G_{ds}^{m}C_{ds}^{m}C_{ds}^{m}CₖₛAₖₛ^{m}Cₖ | 78.1 | 1801 | 1816 | 744 |
| 570363 | AₖₛTₖₛGₖₛ^{m}C_{ds}T_{ds}G_{ds}G_{ds}^{m}C_{ds}^{m}C_{ds}^{m}C_{ds}T_{ds}^{m}C_{ds}^{m}C_{ds}TₖₛGₖₛGₖ | 76.2 | 1848 | 1863 | 745 |
| 570364 | AₖₛGₖₛ^{m}CₖₛT_{ds}G_{ds}^{m}C_{ds}^{m}C_{ds}^{m}C_{ds}^{m}C_{ds}A_{ds}T_{ds}G_{ds}^{m}C_{ds}TₖₛGₖₛGₖ | 92.6 | 1857 | 1872 | 746 |
| 570365 | ^{m}CₖₛGₖₛ^{m}Cₖₛ^{m}C_{ds}^{m}C_{ds}^{m}C_{ds}T_{ds}G_{ds}G_{ds}^{m}C_{ds}A_{ds}G_{ds}^{m}C_{ds}TₖₛGₖₛ^{m}Cₖ | 73.6 | 1867 | 1882 | 747 |
| 570366 | TₖₛGₖₛ^{m}CₖₛG_{ds}^{m}C_{ds}^{m}C_{ds}^{m}C_{ds}^{m}C_{ds}T_{ds}G_{ds}G_{ds}^{m}C_{ds}A_{ds}Gₖₛ^{m}CₖₛTₖ | 76.6 | 1869 | 1884 | 748 |
| 570367 | Gₖₛ^{m}CₖₛTₖₛG_{ds}^{m}C_{ds}G_{ds}^{m}C_{ds}^{m}C_{ds}^{m}C_{ds}^{m}C_{ds}T_{ds}G_{ds}G_{ds}^{m}CₖₛAₖₛGₖ | 79.1 | 1871 | 1886 | 749 |
| 570368 | ^{m}CₖₛGₖₛGₖₛ^{m}C_{ds}T_{ds}G_{ds}^{m}C_{ds}G_{ds}^{m}C_{ds}^{m}C_{ds}^{m}C_{ds}^{m}C_{ds}T_{ds}GₖₛGₖₛ^{m}Cₖ | 82.9 | 1873 | 1888 | 750 |
| 570369 | GₖₛTₖₛ^{m}CₖₛG_{ds}G_{ds}^{m}C_{ds}T_{ds}G_{ds}^{m}C_{ds}G_{ds}^{m}C_{ds}^{m}C_{ds}^{m}C_{ds}^{m}CₖₛTₖₛGₖ | 47.5 | 1875 | 1890 | 751 |
| 570370 | ^{m}CₖₛTₖₛGₖₛT_{ds}^{m}C_{ds}G_{ds}G_{ds}^{m}C_{ds}T_{ds}G_{ds}^{m}C_{ds}G_{ds}^{m}C_{ds}^{m}Cₖₛ^{m}Cₖₛ^{m}Cₖ | 79.6 | 1877 | 1892 | 752 |
| 570371 | Gₖₛ^{m}Cₖₛ^{m}CₖₛT_{ds}G_{ds}T_{ds}^{m}C_{ds}G_{ds}G_{ds}^{m}C_{ds}T_{ds}G_{ds}^{m}C_{ds}Gₖₛ^{m}Cₖₛ^{m}Cₖ | 58.4 | 1879 | 1894 | 753 |
| 570372 | ^{m}CₖₛTₖₛGₖₛ^{m}C_{ds}^{m}C_{ds}T_{ds}G_{ds}T_{ds}^{m}C_{ds}G_{ds}G_{ds}^{m}C_{ds}T_{ds}Gₖₛ^{m}CₖₛGₖ | 49.9 | 1881 | 1896 | 754 |
| 570373 | Aₖₛ^{m}Cₖₛ^{m}CₖₛT_{ds}G_{ds}^{m}C_{ds}^{m}C_{ds}T_{ds}G_{ds}T_{ds}^{m}C_{ds}G_{ds}G_{ds}^{m}CₖₛTₖₛGₖ | 27.4 | 1883 | 1898 | 755 |
| 570374 | Aₖₛ^{m}CₖₛAₖₛ^{m}C_{ds}^{m}C_{ds}T_{ds}G_{ds}^{m}C_{ds}^{m}C_{ds}T_{ds}G_{ds}T_{ds}^{m}C_{ds}GₖₛGₖₛ^{m}Cₖ | 54.3 | 1885 | 1900 | 756 |
| 570375 | GₖₛAₖₛAₖₛ^{m}C_{ds}A_{ds}^{m}C_{ds}^{m}C_{ds}T_{ds}G_{ds}^{m}C_{ds}^{m}C_{ds}T_{ds}G_{ds}Tₖₛ^{m}CₖₛGₖ | 50.5 | 1887 | 1902 | 757 |
| 570376 | ^{m}Cₖₛ^{m}CₖₛGₖₛA_{ds}A_{ds}^{m}C_{ds}A_{ds}^{m}C_{ds}^{m}C_{ds}T_{ds}G_{ds}^{m}C_{ds}^{m}C_{ds}TₖₛGₖₛTₖ | 57.7 | 1889 | 1904 | 758 |
| 570377 | ^{m}CₖₛGₖₛ^{m}Cₖₛ^{m}C_{ds}G_{ds}A_{ds}A_{ds}^{m}C_{ds}A_{ds}^{m}C_{ds}^{m}C_{ds}T_{ds}G_{ds}^{m}Cₖₛ^{m}CₖₛTₖ | 69.3 | 1891 | 1906 | 759 |
| 570378 | ^{m}Cₖₛ^{m}CₖₛTₖₛG_{ds}G_{ds}G_{ds}^{m}C_{ds}A_{ds}^{m}C_{ds}^{m}C_{ds}T_{ds}G_{ds}T_{ds}TₖₛGₖₛGₖ | 188.2 | 1925 | 1940 | 760 |
| 570379 | GₖₛTₖₛGₖₛ^{m}C_{ds}^{m}C_{ds}T_{ds}G_{ds}G_{ds}G_{ds}^{m}C_{ds}A_{ds}^{m}C_{ds}^{m}C_{ds}TₖₛGₖₛTₖ | 111.5 | 1928 | 1943 | 761 |
| 570380 | ^{m}CₖₛGₖₛ^{m}Cₖₛ^{m}C_{ds}^{m}C_{ds}T_{ds}^{m}C_{ds}^{m}C_{ds}^{m}C_{ds}A_{ds}G_{ds}T_{ds}G_{ds}^{m}Cₖₛ^{m}CₖₛTₖ | 78 | 1938 | 1953 | 762 |
| 570381 | Aₖₛ^{m}Cₖₛ^{m}CₖₛG_{ds}^{m}C_{ds}^{m}C_{ds}^{m}C_{ds}T_{ds}^{m}C_{ds}^{m}C_{ds}^{m}C_{ds}A_{ds}G_{ds}TₖₛGₖₛ^{m}Cₖ | 74.9 | 1940 | 1955 | 763 |
| 570382 | Tₖₛ^{m}CₖₛAₖₛ^{m}C_{ds}^{m}C_{ds}G_{ds}^{m}C_{ds}^{m}C_{ds}^{m}C_{ds}T_{ds}^{m}C_{ds}^{m}C_{ds}^{m}C_{ds}AₖₛGₖₛTₖ | 71.6 | 1942 | 1957 | 764 |
| 570383 | AₖₛGₖₛTₖₛ^{m}C_{ds}A_{ds}^{m}C_{ds}^{m}C_{ds}G_{ds}^{m}C_{ds}^{m}C_{ds}^{m}C_{ds}T_{ds}^{m}C_{ds}^{m}Cₖₛ^{m}CₖₛAₖ | 62.1 | 1944 | 1959 | 765 |
| 570384 | TₖₛGₖₛAₖₛG_{ds}T_{ds}^{m}C_{ds}A_{ds}^{m}C_{ds}^{m}C_{ds}G_{ds}^{m}C_{ds}^{m}C_{ds}^{m}C_{ds}Tₖₛ^{m}Cₖₛ^{m}Cₖ | 65.6 | 1946 | 1961 | 766 |
| 570385 | ^{m}CₖₛGₖₛTₖₛG_{ds}A_{ds}G_{ds}T_{ds}^{m}C_{ds}A_{ds}^{m}C_{ds}^{m}C_{ds}G_{ds}^{m}C_{ds}^{m}Cₖₛ^{m}CₖₛTₖ | 37.3 | 1948 | 1963 | 767 |
| 570386 | ^{m}CₖₛAₖₛAₖₛA_{ds}G_{ds}^{m}C_{ds}T_{ds}G_{ds}G_{ds}T_{ds}T_{ds}^{m}C_{ds}T_{ds}^{m}Cₖₛ^{m}Cₖₛ^{m}Cₖ | 30.5 | 1974 | 1989 | 768 |
| 570387 | TₖₛGₖₛ^{m}CₖₛA_{ds}A_{ds}A_{ds}G_{ds}^{m}C_{ds}T_{ds}G_{ds}G_{ds}T_{ds}T_{ds}^{m}CₖₛTₖₛ^{m}Cₖ | 35.8 | 1976 | 1991 | 769 |
| 570388 | Tₖₛ^{m}CₖₛTₖₛG_{ds}^{m}C_{ds}A_{ds}A_{ds}A_{ds}G_{ds}^{m}C_{ds}T_{ds}G_{ds}G_{ds}TₖₛTₖₛ^{m}Cₖ | 30.1 | 1978 | 1993 | 770 |
| 570389 | TₖₛGₖₛTₖₛ^{m}C_{ds}T_{ds}G_{ds}^{m}C_{ds}A_{ds}A_{ds}A_{ds}G_{ds}^{m}C_{ds}T_{ds}GₖₛGₖₛTₖ | 50.1 | 1980 | 1995 | 771 |
| 570390 | ^{m}Cₖₛ^{m}CₖₛTₖₛG_{ds}T_{ds}^{m}C_{ds}T_{ds}G_{ds}^{m}C_{ds}A_{ds}A_{ds}A_{ds}G_{ds}^{m}CₖₛTₖₛGₖ | 36 | 1982 | 1997 | 772 |
| 570391 | ^{m}CₖₛGₖₛ^{m}Cₖₛ^{m}C_{ds}T_{ds}G_{ds}T_{ds}^{m}C_{ds}T_{ds}G_{ds}^{m}C_{ds}A_{ds}A_{ds}AₖₛGₖₛ^{m}Cₖ | 31.1 | 1984 | 1999 | 773 |
| 570392 | TₖₛTₖₛGₖₛT_{ds}^{m}C_{ds}^{m}C_{ds}^{m}C_{ds}T_{ds}^{m}C_{ds}^{m}C_{ds}T_{ds}G_{ds}G_{ds}AₖₛTₖₛ^{m}Cₖ | 62.9 | 2022 | 2037 | 774 |
| 570393 | AₖₛGₖₛTₖₛT_{ds}G_{ds}T_{ds}^{m}C_{ds}^{m}C_{ds}^{m}C_{ds}T_{ds}^{m}C_{ds}^{m}C_{ds}T_{ds}GₖₛGₖₛAₖ | 57.1 | 2024 | 2039 | 775 |
| 570394 | AₖₛAₖₛAₖₛG_{ds}T_{ds}T_{ds}G_{ds}T_{ds}^{m}C_{ds}^{m}C_{ds}^{m}C_{ds}T_{ds}^{m}C_{ds}^{m}CₖₛTₖₛGₖ | 56.2 | 2026 | 2041 | 776 |
| 570395 | ^{m}Cₖₛ^{m}CₖₛAₖₛA_{ds}A_{ds}G_{ds}T_{ds}T_{ds}G_{ds}T_{ds}^{m}C_{ds}^{m}C_{ds}^{m}C_{ds}Tₖₛ^{m}Cₖₛ^{m}Cₖ | 48.9 | 2028 | 2043 | 777 |
| 570396 | Aₖₛ^{m}Cₖₛ^{m}Cₖₛ^{m}C_{ds}A_{ds}A_{dS}A_{ds}G_{ds}T_{ds}T_{ds}G_{ds}T_{ds}^{m}C_{ds}^{m}Cₖₛ^{m}CₖₛTₖ | 59.9 | 2030 | 2045 | 778 |
| 570397 | GₖₛAₖₛAₖₛ^{m}C_{ds}^{m}C_{ds}^{m}C_{ds}A_{ds}A_{ds}A_{ds}G_{ds}T_{ds}T_{ds}G_{ds}Tₖₛ^{m}Cₖₛ^{m}Cₖ | 47.9 | 2032 | 2047 | 779 |
| 570398 | GₖₛAₖₛAₖₛG_{ds}A_{ds}A_{ds}^{m}C_{ds}^{m}C_{ds}^{m}C_{ds}A_{ds}A_{ds}A_{ds}G_{ds}TₖₛTₖₛGₖ | 60 | 2035 | 2050 | 780 |
| 570399 | ^{m}Cₖₛ^{m}CₖₛAₖₛG_{ds}A_{ds}A_{ds}G_{ds}A_{ds}A_{ds}^{m}C_{ds}^{m}C_{ds}^{m}C_{ds}A_{ds}AₖₛAₖₛGₖ | 51.2 | 2038 | 2053 | 781 |
| 570400 | ^{m}CₖₛAₖₛ^{m}Cₖₛ^{m}C_{ds}^{m}C_{ds}A_{ds}G_{ds}A_{ds}A_{ds}G_{ds}A_{ds}A_{ds}^{m}C_{ds}^{m}Cₖₛ^{m}CₖₛAₖ | 51.1 | 2041 | 2056 | 782 |
| 570401 | Gₖₛ^{m}CₖₛAₖₛG_{ds}A_{ds}A_{ds}^{m}C_{ds}^{m}C_{ds}T_{ds}A_{ds}^{m}C_{ds}A_{ds}A_{ds}AₖₛAₖₛGₖ | 44.9 | 2066 | 2081 | 783 |
| 570402 | GₖₛTₖₛGₖₛ^{m}C_{ds}A_{ds}G_{ds}A_{dsAds}^{m}C_{ds}^{m}C_{ds}T_{ds}A_{ds}^{m}C_{ds}AₖₛAₖₛAₖ | 53 | 2068 | 2083 | 784 |
| 570403 | GₖₛGₖₛGₖₛT_{ds}G_{ds}^{m}C_{ds}A_{ds}G_{ds}A_{ds}A_{ds}^{m}C_{ds}^{m}C_{ds}T_{ds}Aₖₛ^{m}CₖₛAₖ | 51.5 | 2070 | 2085 | 785 |
| 570404 | GₖₛTₖₛGₖₛG_{ds}G_{ds}T_{ds}G_{ds}^{m}C_{ds}A_{ds}G_{ds}A_{ds}A_{ds}^{m}C_{ds}^{m}CₖₛTₖₛAₖ | 57.4 | 2072 | 2087 | 786 |
| 570405 | ^{m}Cₖₛ^{m}CₖₛAₖₛ^{m}C_{ds}A_{ds}^{m}C_{ds}G_{ds}G_{ds}^{m}C_{ds}T_{ds}^{m}C_{ds}A_{ds}T_{ds}AₖₛGₖₛGₖ | 54.3 | 2116 | 2131 | 787 |
| 570406 | Aₖₛ^{m}Cₖₛ^{m}Cₖₛ^{m}C_{ds}A_{ds}^{m}C_{ds}A_{ds}^{m}C_{ds}G_{ds}G_{ds}^{m}C_{ds}T_{ds}^{m}C_{ds}AₖₛTₖₛAₖ | 43.6 | 2118 | 2133 | 788 |
| 570407 | TₖₛGₖₛAₖₛ^{m}C_{ds}^{m}C_{ds}^{m}C_{ds}A_{ds}^{m}C_{ds}A_{ds}^{m}C_{ds}G_{ds}G_{ds}^{m}C_{ds}Tₖₛ^{m}CₖₛAₖ | 44 | 2120 | 2135 | 789 |
| 570408 | Gₖₛ^{m}CₖₛTₖₛG_{ds}A_{ds}^{m}C_{ds}^{m}C_{ds}^{m}C_{ds}A_{ds}^{m}C_{ds}A_{ds}^{m}C_{ds}G_{ds}Gₖₛ^{m}CₖₛTₖ | 56.5 | 2122 | 2137 | 790 |
| 570409 | TₖₛGₖₛGₖₛ^{m}C_{ds}T_{ds}G_{ds}A_{ds}^{m}C_{ds}^{m}C_{ds}^{m}C_{ds}A_{ds}^{m}C_{ds}A_{ds}^{m}CₖₛGₖₛGₖ | 54.8 | 2124 | 2139 | 791 |
| 570410 | GₖₛGₖₛTₖₛG_{ds}G_{ds}^{m}C_{ds}T_{ds}G_{ds}A_{ds}^{m}C_{ds}^{m}C_{ds}^{m}C_{ds}A_{ds}^{m}CₖₛAₖₛ^{m}Cₖ | 46.8 | 2126 | 2141 | 792 |
| 570411 | AₖₛTₖₛGₖₛG_{ds}T_{ds}G_{ds}G_{ds}^{m}C_{ds}T_{ds}G_{ds}A_{ds}^{m}C_{ds}^{m}C_{ds}^{m}CₖₛAₖₛ^{m}Cₖ | 73.8 | 2128 | 2143 | 793 |
| 570412 | GₖₛAₖₛAₖₛT_{ds}G_{ds}G_{ds}T_{ds}G_{ds}G_{ds}^{m}C_{ds}T_{ds}G_{ds}A_{ds}^{m}Cₖₛ^{m}Cₖₛ^{m}Cₖ | 43.5 | 2130 | 2145 | 794 |
| 570413 | ^{m}CₖₛTₖₛAₖₛA_{ds}A_{ds}G_{ds}G_{ds}A_{ds}^{m}C_{ds}G_{ds}^{m}C_{ds}A_{ds}G_{ds}GₖₛGₖₛAₖ | 54.4 | 2159 | 2174 | 795 |
| 570414 | AₖₛAₖₛ^{m}CₖₛT_{ds}A_{ds}A_{ds}A_{ds}G_{ds}G_{ds}A_{ds}^{m}C_{ds}G_{ds}^{m}C_{ds}AₖₛGₖₛGₖ | 49.1 | 2161 | 2176 | 796 |
| 570415 | GₖₛAₖₛGₖₛA_{ds}A_{ds}^{m}C_{ds}T_{ds}A_{ds}A_{ds}A_{ds}G_{ds}G_{ds}A_{ds}^{m}CₖₛGₖₛ^{m}Cₖ | 35.4 | 2164 | 2179 | 797 |

**Table 17**

| Inhibition of human DMPK RNA transcript in HepG2 cells targeting SEQ ID NO: 2 | | | | | |
|---|---|---|---|---|---|
| ISIS No. | Sequence | % Target Expression | Start Site on Seq ID: 2 | Stop Site on Seq ID: 2 | Seq ID No. |
| UTC | N/A | 100 | N/A | N/A | |
| 445569 | ^{m}CₑₛGₑₛGₑₛAₑₛGₑₛ^{m}C_{ds}G_{ds}G_{ds}T_{ds}T_{ds}G_{ds}T_{ds}G_{ds}A_{ds}A_{ds}^{m}CₑₛTₑₛGₑₛGₑₛ^{m}Cₑ | 41.4 | 13226 | 13245 | 24 |
| 486178 | Aₖₛ^{m}CₖₛAₖₛA_{ds}T_{ds}A_{ds}A_{ds}A_{ds}T_{ds}A_{ds}^{m}C_{ds}^{m}C_{ds}G_{ds}AₖₛGₖₛGₖ | 24 | 13836 | 13851 | 23 |
| 570493 | AₖₛTₖₛTₖₛG_{ds}G_{ds}T_{ds}^{m}C_{ds}^{m}C_{ds}^{m}C_{ds}A_{ds}A_{ds}G_{ds}^{m}C_{ds}^{m}Cₖₛ^{m}Cₖₛ^{m}Cₖ | 112.1 | 3973 | 3988 | 798 |
| 570494 | ^{m}Cₖₛ^{m}CₖₛAₖₛT_{ds}T_{ds}G_{ds}G_{ds}T_{ds}^{m}C_{ds}^{m}C_{ds}^{m}C_{ds}A_{ds}A_{ds}Gₖₛ^{m}Cₖₛ^{m}Cₖ | 91.3 | 3975 | 3990 | 799 |
| 570495 | Gₖₛ^{m}Cₖₛ^{m}Cₖₛ^{m}C_{ds}A_{ds}T_{ds}T_{ds}G_{ds}G_{ds}T_{ds}^{m}C_{ds}^{m}C_{ds}^{m}C_{ds}AₖₛAₖₛGₖ | 103.4 | 3977 | 3992 | 800 |
| 570496 | Aₖₛ^{m}CₖₛGₖₛ^{m}C_{ds}^{m}C_{ds}^{m}C_{ds}A_{ds}T_{ds}T_{ds}G_{ds}G_{ds}T_{ds}^{m}C_{ds}^{m}Cₖₛ^{m}CₖₛAₖ | 67.8 | 3979 | 3994 | 801 |
| 570497 | ^{m}Cₖₛ^{m}CₖₛAₖₛ^{m}C_{ds}G_{ds}^{m}C_{ds}^{m}C_{ds}^{m}C_{ds}A_{ds}T_{ds}T_{ds}G_{ds}G_{ds}Tₖₛ^{m}Cₖₛ^{m}Cₖ | 77.3 | 3981 | 3996 | 802 |
| 570498 | ^{m}CₖₛAₖₛ^{m}Cₖₛ^{m}C_{ds}A_{ds}^{m}C_{ds}G_{ds}^{m}C_{ds}^{m}C_{ds}^{m}C_{ds}A_{ds}T_{ds}T_{ds}GₖₛGₖₛTₖ | 98.3 | 3983 | 3998 | 803 |
| 570499 | AₖₛGₖₛAₖₛ^{m}C_{ds}^{m}C_{ds}^{m}C_{ds}A_{ds}A_{ds}^{m}C_{ds}T_{ds}^{m}C_{ds}^{m}C_{ds}A_{ds}^{m}Cₖₛ^{m}Cₖₛ^{m}Cₖ | 63.7 | 4036 | 4051 | 804 |
| 570500 | Tₖₛ^{m}CₖₛAₖₛ^{m}C_{ds}^{m}C_{ds}T_{ds}^{m}C_{ds}G_{ds}^{m}C_{ds}^{m}C_{ds}^{m}C_{ds}^{m}C_{ds}T_{ds}^{m}CₖₛTₖₛTₖ | 43 | 4181 | 4196 | 805 |
| 570501 | ^{m}Cₖₛ^{m}CₖₛTₖₛ^{m}C_{ds}A_{ds}^{m}C_{ds}^{m}C_{ds}T_{ds}^{m}C_{ds}G_{ds}^{m}C_{ds}^{m}C_{ds}^{m}C_{ds}^{m}CₖₛTₖₛ^{m}Cₖ | 38.1 | 4183 | 4198 | 806 |
| 570502 | AₖₛGₖₛ^{m}Cₖₛ^{m}C_{ds}^{m}C_{ds}^{m}C_{ds}T_{ds}^{m}C_{ds}A_{ds}^{m}C_{ds}^{m}C_{ds}T_{ds}^{m}C_{ds}Gₖₛ^{m}Cₖₛ^{m}Cₖ | 85.4 | 4187 | 4202 | 807 |
| 570503 | ^{m}CₖₛTₖₛ^{m}CₖₛA_{ds}A_{ds}A_{ds}G_{ds}^{m}C_{ds}^{m}C_{ds}^{m}C_{ds}^{m}C_{ds}^{m}C_{ds}^{m}C_{ds}Aₖₛ^{m}CₖₛGₖ | 115.8 | 4210 | 4225 | 808 |
| 570504 | AₖₛTₖₛ^{m}Cₖₛ^{m}C_{ds}T_{ds}^{m}C_{ds}A_{ds}A_{ds}A_{ds}G_{ds}^{m}C_{ds}^{m}C_{ds}^{m}C_{ds}^{m}Cₖₛ^{m}Cₖₛ^{m}Cₖ | 114.5 | 4213 | 4228 | 809 |
| 570505 | GₖₛGₖₛAₖₛT_{ds}^{m}C_{ds}^{m}C_{ds}T_{ds}^{m}C_{ds}A_{ds}A_{ds}A_{ds}G_{ds}^{m}C_{ds}^{m}Cₖₛ^{m}Cₖₛ^{m}Cₖ | 88.1 | 4215 | 4230 | 810 |
| 570506 | Gₖₛ^{m}CₖₛGₖₛG_{ds}A_{ds}T_{ds}^{m}C_{ds}^{m}C_{ds}T_{ds}^{m}C_{ds}A_{ds}A_{ds}A_{ds}Gₖₛ^{m}Cₖₛ^{m}Cₖ | 93.1 | 4217 | 4232 | 811 |
| 570507 | Gₖₛ^{m}CₖₛGₖₛ^{m}C_{ds}G_{ds}G_{ds}A_{ds}T_{ds}^{m}C_{ds}^{m}C_{ds}T_{ds}^{m}C_{ds}A_{ds}AₖₛAₖₛGₖ | 102.9 | 4219 | 4234 | 812 |
| 570508 | GₖₛGₖₛGₖₛ^{m}C_{ds}G_{ds}^{m}C_{ds}G_{ds}G_{ds}A_{ds}T_{ds}^{m}C_{ds}^{m}C_{ds}T_{ds}^{m}CₖₛAₖₛAₖ | 78.5 | 4221 | 4236 | 813 |
| 570509 | GₖₛAₖₛGₖₛ^{m}C_{ds}T_{ds}G_{ds}^{m}C_{ds}A_{ds}G_{ds}^{m}C_{ds}^{m}C_{ds}G_{ds}G_{ds}AₖₛGₖₛAₖ | 192.2 | 4239 | 4254 | 814 |
| 570510 | AₖₛGₖₛGₖₛA_{ds}G_{ds}^{m}C_{ds}T_{ds}G_{ds}^{m}C_{ds}A_{ds}G_{ds}^{m}C_{ds}^{m}C_{ds}GₖₛGₖₛAₖ | 219.8 | 4241 | 4256 | 815 |
| 570511 | ^{m}CₖₛGₖₛGₖₛA_{ds}G_{ds}G_{ds}A_{ds}G_{ds}^{m}C_{ds}T_{ds}G_{ds}^{m}C_{ds}A_{ds}Gₖₛ^{m}Cₖₛ^{m}Cₖ | 128.6 | 4244 | 4259 | 816 |
| 570512 | Aₖₛ^{m}Cₖₛ^{m}Cₖₛ^{m}C_{ds}G_{ds}G_{ds}A_{ds}G_{ds}G_{ds}A_{ds}G_{ds}^{m}C_{ds}T_{ds}Gₖₛ^{m}CₖₛAₖ | 89.9 | 4247 | 4262 | 817 |
| 570513 | Gₖₛ^{m}CₖₛAₖₛ^{m}C_{ds}^{m}C_{ds}^{m}C_{ds}G_{ds}G_{ds}A_{ds}G_{ds}G_{ds}A_{ds}G_{ds}^{m}CₖₛTₖₛGₖ | 96.1 | 4249 | 4264 | 818 |
| 570514 | GₖₛGₖₛGₖₛ^{m}C_{ds}A_{ds}^{m}C_{ds}^{m}C_{ds}^{m}C_{ds}G_{ds}G_{ds}A_{ds}G_{ds}G_{ds}AₖₛGₖₛ^{m}Cₖ | 67.8 | 4251 | 4266 | 819 |
| 570515 | ^{m}CₖₛAₖₛGₖₛG_{ds}G_{ds}^{m}C_{ds}A_{ds}^{m}C_{ds}^{m}C_{ds}^{m}C_{ds}G_{ds}G_{ds}A_{ds}GₖₛGₖₛAₖ | 64.2 | 4253 | 4268 | 820 |
| 570516 | TₖₛGₖₛ^{m}CₖₛA_{ds}G_{ds}G_{ds}G_{ds}^{m}C_{ds}A_{ds}^{m}C_{ds}^{m}C_{ds}^{m}C_{ds}G_{ds}GₖₛAₖₛGₖ | 62.2 | 4255 | 4270 | 821 |
| 570517 | ^{m}Cₖₛ^{m}CₖₛTₖₛG_{ds}^{m}C_{ds}A_{ds}G_{ds}G_{ds}G_{ds}^{m}C_{ds}A_{ds}^{m}C_{ds}^{m}C_{ds}^{m}CₖₛGₖₛGₖ | 77.7 | 4257 | 4272 | 822 |
| 570518 | ^{m}CₖₛGₖₛAₖₛ^{m}C_{ds}A_{ds}^{m}C_{ds}^{m}C_{ds}T_{ds}G_{ds}^{m}C_{ds}A_{ds}G_{ds}G_{ds}Gₖₛ^{m}CₖₛAₖ | 79 | 4262 | 4277 | 823 |
| 570519 | ^{m}CₖₛAₖₛ^{m}CₖₛG_{ds}A_{ds}^{m}C_{ds}A_{ds}^{m}C_{ds}^{m}C_{ds}T_{ds}G_{ds}^{m}C_{ds}A_{ds}GₖₛGₖₛGₖ | 68.5 | 4264 | 4279 | 824 |
| 570520 | AₖₛGₖₛ^{m}CₖₛA_{ds}^{m}C_{ds}G_{ds}A_{ds}^{m}C_{ds}A_{ds}^{m}C_{ds}^{m}C_{ds}T_{ds}G_{ds}^{m}CₖₛAₖₛGₖ | 39.8 | 4266 | 4281 | 825 |
| 570521 | GₖₛAₖₛAₖₛG_{ds}^{m}C_{ds}A_{ds}^{m}C_{ds}G_{ds}A_{ds}^{m}C_{ds}A_{ds}^{m}C_{ds}^{m}C_{ds}TₖₛGₖₛ^{m}Cₖ | 32.4 | 4268 | 4283 | 826 |
| 570522 | ^{m}Cₖₛ^{m}CₖₛAₖₛG_{ds}G_{ds}T_{ds}A_{ds}G_{ds}T_{ds}T_{ds}^{m}C_{ds}T_{ds}^{m}C_{ds}AₖₛTₖₛ^{m}Cₖ | 41 | 4353 | 4368 | 827 |
| 570523 | ^{m}CₖₛAₖₛ^{m}Cₖₛ^{m}C_{ds}A_{ds}G_{ds}G_{ds}T_{ds}A_{ds}G_{ds}T_{ds}T_{ds}^{m}C_{ds}Tₖₛ^{m}CₖₛAₖ | 71.9 | 4355 | 4370 | 828 |
| 570524 | ^{m}CₖₛTₖₛ^{m}CₖₛA_{ds}^{m}C_{ds}^{m}C_{ds}A_{ds}G_{ds}G_{ds}T_{ds}A_{ds}G_{ds}T_{ds}Tₖₛ^{m}CₖₛTₖ | 105.9 | 4357 | 4372 | 829 |
| 570525 | AₖₛGₖₛ^{m}CₖₛT_{ds}^{m}C_{ds}A_{ds}^{m}C_{ds}^{m}C_{ds}A_{ds}G_{ds}G_{ds}T_{ds}A_{ds}GₖₛTₖₛTₖ | 99.3 | 4359 | 4374 | 830 |
| 570526 | GₖₛGₖₛAₖₛG_{ds}^{m}C_{ds}T_{ds}^{m}C_{ds}A_{ds}^{m}C_{ds}^{m}C_{ds}A_{ds}G_{ds}G_{ds}TₖₛAₖₛGₖ | 85.2 | 4361 | 4376 | 831 |
| 570527 | ^{m}Cₖₛ^{m}CₖₛGₖₛG_{ds}A_{ds}G_{ds}^{m}C_{ds}T_{ds}^{m}C_{ds}A_{ds}^{m}C_{ds}^{m}C_{ds}A_{ds}GₖₛGₖₛTₖ | 82.5 | 4363 | 4378 | 832 |
| 570528 | Gₖₛ^{m}Cₖₛ^{m}Cₖₛ^{m}C_{ds}G_{ds}G_{ds}A_{ds}G_{ds}^{m}C_{ds}T_{ds}^{m}C_{ds}A_{ds}^{m}C_{ds}^{m}CₖₛAₖₛGₖ | 60.5 | 4365 | 4380 | 833 |
| 570529 | TₖₛAₖₛGₖₛA_{ds}G_{ds}^{m}C_{ds}T_{ds}T_{ds}^{m}C_{ds}^{m}C_{ds}T_{ds}^{m}C_{ds}T_{ds}^{m}Cₖₛ^{m}Cₖₛ^{m}Cₖ | 35.4 | 4435 | 4450 | 834 |
| 570530 | ^{m}Cₖₛ^{m}CₖₛTₖₛA_{ds}G_{ds}A_{ds}G_{ds}^{m}C_{ds}T_{ds}T_{ds}^{m}C_{ds}^{m}C_{ds}T_{ds}^{m}CₖₛTₖₛ^{m}Cₖ | 29.4 | 4437 | 4452 | 835 |
| 570531 | AₖₛTₖₛ^{m}Cₖₛ^{m}C_{ds}T_{ds}A_{ds}G_{ds}A_{ds}G_{ds}^{m}C_{ds}T_{ds}T_{ds}^{m}C_{ds}^{m}CₖₛTₖₛ^{m}Cₖ | 30.4 | 4439 | 4454 | 836 |
| 570532 | ^{m}CₖₛAₖₛAₖₛT_{ds}^{m}C_{ds}^{m}C_{ds}T_{ds}A_{dS}G_{ds}A_{ds}G_{ds}^{m}C_{ds}T_{ds}Tₖₛ^{m}Cₖₛ^{m}Cₖ | 30.3 | 4441 | 4456 | 837 |
| 570533 | ^{m}Cₖₛ^{m}Cₖₛ^{m}CₖₛA_{ds}A_{ds}T_{ds}^{m}C_{ds}^{m}C_{ds}T_{ds}A_{ds}G_{ds}A_{ds}G_{ds}^{m}CₖₛTₖₛTₖ | 54.1 | 4443 | 4458 | 838 |
| 570534 | ^{m}Cₖₛ^{m}Cₖₛ^{m}Cₖₛ^{m}C_{ds}^{m}C_{ds}A_{ds}A_{ds}T_{ds}^{m}C_{ds}^{m}C_{ds}T_{ds}A_{ds}G_{ds}AₖₛGₖₛ^{m}Cₖ | 60.1 | 4445 | 4460 | 839 |
| 570535 | ^{m}CₖₛAₖₛ^{m}Cₖₛ^{m}C_{ds}^{m}C_{ds}^{m}C_{ds}^{m}C_{ds}A_{ds}A_{ds}T_{ds}^{m}C_{ds}^{m}C_{ds}T_{ds}AₖₛGₖₛAₖ | 68.5 | 4447 | 4462 | 840 |
| 570536 | AₖₛGₖₛ^{m}CₖₛA_{ds}^{m}C_{ds}^{m}C_{ds}^{m}C_{ds}^{m}C_{ds}^{m}C_{ds}A_{ds}A_{ds}T_{ds}^{m}C_{ds}^{m}CₖₛTₖₛAₖ | 37.5 | 4449 | 4464 | 841 |
| 570537 | Gₖₛ^{m}CₖₛAₖₛG_{ds}^{m}C_{ds}A_{ds}^{m}C_{ds}^{m}C_{ds}^{m}C_{ds}^{m}C_{ds}^{m}C_{ds}A_{ds}A_{ds}Tₖₛ^{m}Cₖₛ^{m}Cₖ | 50.9 | 4451 | 4466 | 842 |
| 570538 | GₖₛGₖₛGₖₛ^{m}C_{ds}A_{ds}G_{ds}^{m}C_{ds}A_{ds}^{m}C_{ds}^{m}C_{ds}^{m}C_{ds}^{m}C_{ds}^{m}C_{ds}AₖₛAₖₛTₖ | 67.7 | 4453 | 4468 | 843 |
| 570539 | TₖₛGₖₛAₖₛ^{m}C_{ds}A_{ds}^{m}C_{ds}A_{ds}^{m}C_{ds}^{m}C_{ds}^{m}C_{ds}T_{ds}^{m}C_{ds}T_{ds}TₖₛAₖₛ^{m}Cₖ | 55.9 | 4498 | 4513 | 844 |
| 570540 | ^{m}Cₖₛ^{m}CₖₛTₖₛG_{ds}A_{ds}^{m}C_{ds}A_{ds}^{m}C_{ds}A_{ds}^{m}C_{ds}^{m}C_{ds}^{m}C_{ds}T_{ds}^{m}CₖₛTₖₛTₖ | 45.1 | 4500 | 4515 | 845 |
| 570541 | ^{m}CₖₛAₖₛ^{m}Cₖₛ^{m}C_{ds}T_{ds}G_{ds}A_{ds}^{m}C_{ds}A_{ds}^{m}C_{ds}A_{ds}^{m}C_{ds}^{m}C_{ds}^{m}CₖₛTₖₛ^{m}Cₖ | 30.9 | 4502 | 4517 | 846 |
| 570542 | Tₖₛ^{m}Cₖₛ^{m}CₖₛA_{ds}^{m}C_{ds}^{m}C_{ds}T_{ds}G_{ds}A_{ds}^{m}C_{ds}A_{ds}^{m}C_{ds}A_{ds}^{m}Cₖₛ^{m}Cₖₛ^{m}Cₖ | 35 | 4504 | 4519 | 847 |
| 570543 | ^{m}CₖₛAₖₛTₖₛ^{m}C_{ds}^{m}C_{ds}A_{ds}^{m}C_{ds}^{m}C_{ds}T_{ds}G_{ds}A_{ds}^{m}C_{ds}A_{ds}^{m}CₖₛAₖₛ^{m}Cₖ | 48 | 4506 | 4521 | 848 |
| 570544 | ^{m}CₖₛTₖₛ^{m}CₖₛA_{ds}T_{ds}^{m}C_{ds}^{m}C_{ds}A_{ds}^{m}C_{ds}^{m}C_{ds}T_{ds}G_{ds}A_{ds}^{m}CₖₛAₖₛ^{m}Cₖ | 37.1 | 4508 | 4523 | 849 |
| 570545 | ^{m}Cₖₛ^{m}Cₖₛ^{m}CₖₛT_{ds}^{m}C_{ds}A_{ds}T_{ds}^{m}C_{ds}^{m}C_{ds}A_{ds}^{m}C_{ds}^{m}C_{ds}T_{ds}GₖₛAₖₛ^{m}Cₖ | 46 | 4510 | 4525 | 850 |
| 570546 | Gₖₛ^{m}Cₖₛ^{m}Cₖₛ^{m}C_{ds}^{m}C_{ds}T_{ds}^{m}C_{ds}A_{ds}T_{ds}^{m}C_{ds}^{m}C_{ds}A_{ds}^{m}C_{ds}^{m}CₖₛTₖₛGₖ | 79.2 | 4512 | 4527 | 851 |
| 570547 | AₖₛGₖₛGₖₛ^{m}C_{ds}^{m}C_{ds}^{m}C_{ds}^{m}C_{ds}T_{ds}^{m}C_{ds}A_{ds}T_{ds}^{m}C_{ds}^{m}C_{ds}Aₖₛ^{m}Cₖₛ^{m}Cₖ | 40.7 | 4514 | 4529 | 852 |
| 570548 | GₖₛAₖₛAₖₛG_{ds}G_{ds}^{m}C_{ds}^{m}C_{ds}^{m}C_{ds}^{m}C_{ds}T_{ds}^{m}C_{ds}A_{ds}T_{ds}^{m}Cₖₛ^{m}CₖₛAₖ | 35.9 | 4516 | 4531 | 853 |
| 570549 | AₖₛGₖₛGₖₛT_{ds}A_{ds}A_{ds}G_{ds}A_{ds}G_{ds}A_{ds}^{m}C_{ds}^{m}C_{ds}^{m}C_{ds}^{m}Cₖₛ^{m}Cₖₛ^{m}Cₖ | 18.8 | 4613 | 4628 | 854 |
| 570550 | ^{m}Cₖₛ^{m}CₖₛAₖₛG_{ds}G_{ds}T_{ds}A_{ds}A_{ds}G_{ds}A_{ds}G_{ds}A_{ds}^{m}C_{ds}^{m}Cₖₛ^{m}Cₖₛ^{m}Cₖ | 16.2 | 4615 | 4630 | 855 |
| 570551 | TₖₛTₖₛ^{m}Cₖₛ^{m}C_{ds}A_{ds}G_{ds}G_{ds}T_{dsAds}A_{ds}G_{ds}A_{ds}G_{ds}Aₖₛ^{m}Cₖₛ^{m}Cₖ | 38.9 | 4617 | 4632 | 856 |
| 570552 | ^{m}Cₖₛ^{m}CₖₛAₖₛT_{ds}T_{ds}^{m}C_{ds}^{m}C_{ds}A_{ds}G_{ds}G_{ds}T_{ds}A_{ds}A_{ds}GₖₛAₖₛGₖ | 28.6 | 4620 | 4635 | 857 |
| 570553 | Tₖₛ^{m}Cₖₛ^{m}Cₖₛ^{m}C_{ds}A_{ds}T_{ds}T_{ds}^{m}C_{ds}^{m}C_{ds}A_{ds}G_{ds}G_{dS}T_{ds}AₖₛAₖₛGₖ | 42.6 | 4622 | 4637 | 858 |
| 570554 | TₖₛAₖₛTₖₛ^{m}C_{ds}^{m}C_{ds}^{m}C_{ds}A_{ds}T_{ds}T_{ds}^{m}C_{ds}^{m}C_{ds}A_{ds}G_{ds}GₖₛTₖₛAₖ | 31.8 | 4624 | 4639 | 859 |
| 570555 | ^{m}Cₖₛ^{m}CₖₛTₖₛA_{ds}T_{ds}^{m}C_{ds}^{m}C_{ds}^{m}C_{ds}A_{ds}T_{ds}T_{ds}^{m}C_{ds}^{m}C_{ds}AₖₛGₖₛGₖ | 62 | 4626 | 4641 | 860 |
| 570556 | GₖₛAₖₛ^{m}Cₖₛ^{m}C_{ds}T_{ds}A_{ds}T_{ds}^{m}C_{ds}^{m}C_{ds}^{m}C_{ds}A_{ds}T_{ds}T_{ds}^{m}Cₖₛ^{m}CₖₛAₖ | 20 | 4628 | 4643 | 861 |
| 570557 | AₖₛAₖₛGₖₛA_{ds}^{m}C_{ds}^{m}C_{ds}T_{ds}A_{ds}T_{ds}^{m}C_{ds}^{m}C_{ds}^{m}C_{ds}A_{ds}TₖₛTₖₛ^{m}Cₖ | 29.8 | 4630 | 4645 | 862 |
| 570558 | TₖₛGₖₛAₖₛA_{ds}G_{ds}A_{ds}^{m}C_{ds}^{m}C_{ds}T_{ds}A_{ds}T_{ds}^{m}C_{ds}^{m}C_{ds}^{m}CₖₛAₖₛTₖ | 45.5 | 4632 | 4647 | 863 |
| 570559 | TₖₛGₖₛGₖₛ^{m}C_{ds}^{m}C_{ds}^{m}C_{ds}^{m}C_{ds}G_{ds}T_{ds}T_{ds}A_{ds}G_{ds}A_{ds}AₖₛTₖₛTₖ | 72.7 | 4650 | 4665 | 864 |
| 570560 | AₖₛGₖₛTₖₛG_{ds}G_{ds}^{m}C_{ds}^{m}C_{ds}^{m}C_{ds}^{m}C_{ds}G_{ds}T_{ds}T_{ds}A_{ds}GₖₛAₖₛAₖ | 33.7 | 4652 | 4667 | 865 |
| 570561 | Gₖₛ^{m}CₖₛAₖₛG_{ds}T_{ds}G_{ds}G_{ds}^{m}C_{ds}^{m}C_{ds}^{m}C_{ds}^{m}C_{ds}G_{ds}T_{ds}TₖₛAₖₛGₖ | 17.5 | 4654 | 4669 | 866 |
| 570562 | AₖₛGₖₛGₖₛ^{m}C_{ds}A_{ds}G_{ds}T_{ds}G_{ds}G_{ds}^{m}C_{ds}^{m}C_{ds}^{m}C_{ds}^{m}C_{ds}GₖₛTₖₛTₖ | 27.9 | 4656 | 4671 | 867 |
| 570563 | ^{m}CₖₛTₖₛAₖₛG_{ds}G_{ds}^{m}C_{ds}A_{ds}G_{ds}T_{ds}G_{ds}G_{ds}^{m}C_{ds}^{m}C_{ds}^{m}Cₖₛ^{m}CₖₛGₖ | 31.3 | 4658 | 4673 | 868 |
| 570564 | ^{m}Cₖₛ^{m}Cₖₛ^{m}CₖₛT_{ds}A_{ds}G_{ds}G_{ds}^{m}C_{ds}A_{ds}G_{ds}T_{ds}G_{ds}G_{ds}^{m}Cₖₛ^{m}Cₖₛ^{m}Cₖ | 23.8 | 4660 | 4675 | 869 |
| 570565 | AₖₛGₖₛGₖₛT_{ds}^{m}C_{ds}^{m}C_{ds}^{m}C_{ds}A_{ds}G_{ds}A_{ds}^{m}C_{ds}A_{ds}^{m}C_{ds}Tₖₛ^{m}Cₖₛ^{m}Cₖ | 17.2 | 4678 | 4693 | 870 |
| 570566 | AₖₛTₖₛAₖₛG_{ds}G_{ds}T_{ds}^{m}C_{ds}^{m}C_{ds}^{m}C_{ds}A_{ds}G_{ds}A_{ds}^{m}C_{ds}Aₖₛ^{m}CₖₛTₖ | 33.1 | 4680 | 4695 | 871 |
| 570567 | GₖₛAₖₛAₖₛT_{ds}A_{ds}G_{ds}G_{ds}T_{ds}^{m}C_{ds}^{m}C_{ds}^{m}C_{ds}A_{ds}G_{ds}Aₖₛ^{m}CₖₛAₖ | 51.8 | 4682 | 4697 | 872 |
| 570568 | GₖₛAₖₛGₖₛA_{ds}A_{ds}T_{ds}A_{ds}G_{ds}G_{ds}T_{ds}^{m}C_{ds}^{m}C_{ds}^{m}C_{ds}AₖₛGₖₛAₖ | 20.3 | 4684 | 4699 | 873 |
| 570569 | ^{m}CₖₛAₖₛGₖₛA_{ds}G_{ds}A_{ds}A_{ds}T_{ds}A_{ds}G_{ds}G_{ds}T_{ds}^{m}C_{ds}^{m}Cₖₛ^{m}CₖₛAₖ | 19 | 4686 | 4701 | 874 |

**Table 18**

| Inhibition of human DMPK RNA transcript in HepG2 cells targeting SEQ ID NO: 2 | | | | | |
|---|---|---|---|---|---|
| ISIS No. | Sequence | % Target Expression | Start Site on Seq ID: 2 | Stop Site on Seq ID: 2 | Seq ID No. |
| UTC | N/A | 100 | N/A | N/A | |
| 445569 | ^{m}CₑₛGₑₛGₑₛAₑₛGₑₛ^{m}C_{ds}G_{ds}G_{ds}T_{ds}T_{ds}G_{ds}T_{ds}G_{ds}A_{ds}A_{ds}^{m}CₑₛTₑₛGₑₛGₑₛ^{m}Cₑ | 33.8 | 13226 | 13245 | 24 |
| 486178 | Aₖₛ^{m}CₖₛAₖₛA_{ds}T_{ds}A_{ds}A_{ds}A_{ds}T_{ds}A_{ds}^{m}C_{ds}^{m}C_{ds}G_{ds}AₖₛGₖₛGₖ | 24.4 | 13836 | 13851 | 23 |
| 570647 | Gₖₛ^{m}CₖₛTₖₛT_{ds}G_{ds}G_{ds}G_{ds}^{m}C_{ds}^{m}C_{ds}^{m}C_{ds}A_{ds}^{m}C_{ds}^{m}C_{ds}^{m}Cₖₛ^{m}CₖₛTₖ | 60.6 | 5718 | 5733 | 645 |
| 570648 | AₖₛGₖₛGₖₛ^{m}C_{ds}T_{ds}T_{ds}G_{ds}G_{ds}G_{ds}^{m}C_{ds}^{m}C_{ds}^{m}C_{ds}A_{ds}^{m}Cₖₛ^{m}Cₖₛ^{m}Cₖ | 82 | 5720 | 5735 | 646 |
| 570649 | ^{m}CₖₛGₖₛAₖₛG_{ds}G_{ds}^{m}C_{ds}T_{ds}T_{ds}G_{ds}G_{ds}G_{ds}^{m}C_{ds}^{m}C_{ds}^{m}CₖₛAₖₛ^{m}Cₖ | 133.4 | 5722 | 5737 | 647 |
| 570650 | AₖₛG_{kS}^{m}CₖₛG_{ds}A_{ds}G_{ds}G_{ds}^{m}C_{ds}T_{ds}T_{ds}G_{ds}G_{ds}G_{ds}^{m}Cₖₛ^{m}Cₖₛ^{m}Cₖ | 54.1 | 5724 | 5739 | 648 |
| 570651 | AₖₛGₖₛAₖₛG_{ds}^{m}C_{ds}G_{ds}A_{ds}G_{ds}G_{ds}^{m}C_{ds}T_{ds}T_{ds}G_{ds}GₖₛGₖₛ^{m}Cₖ | 88.5 | 5726 | 5741 | 649 |
| 570652 | Gₖₛ^{m}CₖₛAₖₛG_{ds}A_{ds}G_{ds}^{m}C_{ds}G_{ds}A_{ds}G_{ds}G_{ds}^{m}C_{ds}T_{ds}TₖₛGₖₛGₖ | 162.9 | 5728 | 5743 | 650 |
| 570653 | GₖₛAₖₛGₖₛ^{m}C_{ds}A_{ds}G_{ds}A_{ds}G_{ds}^{m}C_{ds}G_{ds}A_{ds}G_{ds}G_{ds}^{m}CₖₛTₖₛTₖ | 130 | 5730 | 5745 | 651 |
| 570654 | AₖₛAₖₛAₖₛG_{ds}G_{ds}A_{ds}G_{ds}^{m}C_{ds}A_{ds}G_{ds}A_{ds}G_{ds}C_{ds}GₖₛAₖₛGₖ | 66.5 | 5734 | 5749 | 652 |
| 570655 | ^{m}CₖₛAₖₛAₖₛA_{ds}A_{ds}G_{ds}G_{d}A_{ds}G_{ds}^{m}C_{ds}A_{ds}G_{d}A_{ds}GₖₛCₖₛGₖ | 79 | 5736 | 5751 | 653 |
| 570656 | TₖₛGₖₛGₖₛA_{ds}C_{ds}^{m}C_{ds}^{m}A_{ds}A_{ds}A_{ds}A_{ds}G_{ds}G_{ds}A_{ds}GₖₛCₖₛAₖ | 57.4 | 5741 | 5756 | 654 |
| 570657 | ^{m}Cₖₛ^{m}CₖₛTₖₛG_{ds}G_{ds}A_{ds}^{m}C_{ds}^{m}C_{ds}A_{ds}A_{d}A_{ds}A_{ds}G_{ds}GₖₛAₖₛGₖ | 129.2 | 5743 | 5758 | 655 |
| 570658 | ^{m}CₖₛAₖₛ^{m}Cₖₛ^{m}C_{ds}T_{ds}G_{ds}G_{d}A_{ds}^{m}C_{ds}^{m}C_{ds}A_{ds}A_{ds}A_{d}AₖₛGₖₛGₖ | 66.3 | 5745 | 5760 | 656 |
| 570659 | ^{m}CₖₛGₖₛ^{m}CₖₛA_{ds}^{m}C_{ds}^{m}C_{ds}T_{ds}G_{ds}G_{ds}A_{ds}^{m}C_{ds}^{m}C_{ds}A_{ds}AₖₛAₖₛAₖ | 58.7 | 5747 | 5762 | 657 |
| 570660 | GₖₛAₖₛ^{m}Cₖₛ^{m}C_{ds}G_{ds}^{m}C_{ds}A_{ds}^{m}C_{ds}^{m}C_{ds}T_{ds}G_{ds}G_{ds}A_{ds}^{m}Cₖₛ^{m}CₖₛAₖ | 55.4 | 5750 | 5765 | 658 |
| 570661 | Aₖₛ^{m}Cₖₛ^{m}CₖₛT_{ds}T_{ds}G_{ds}T_{ds}A_{ds}G_{ds}T_{ds}G_{ds}G_{ds}A_{ds}^{m}CₖₛGₖₛAₖ | 45.4 | 5951 | 5966 | 659 |
| 570662 | Tₖₛ^{m}CₖₛAₖₛ^{m}C_{ds}^{m}C_{ds}T_{ds}T_{ds}G_{ds}T_{ds}A_{ds}G_{ds}T_{ds}G_{ds}GₖₛAₖₛ^{m}Cₖ | 63.5 | 5953 | 5968 | 660 |
| 570663 | Gₖₛ^{m}CₖₛTₖₛ^{m}C_{ds}A_{ds}^{m}C_{ds}^{m}C_{ds}T_{ds}T_{ds}G_{ds}T_{ds}A_{ds}G_{ds}TₖₛGₖₛGₖ | 56.6 | 5955 | 5970 | 661 |
| 570664 | GₖₛGₖₛAₖₛG_{ds}A_{ds}G_{d}sG_{ds}A_{ds}G_{ds}G_{ds}^{m}C_{ds}G_{ds}A_{ds}TₖₛAₖₛGₖ | 125.6 | 6015 | 6030 | 662 |
| 570665 | AₖₛGₖₛGₖₛG_{ds}A_{ds}G_{ds}A_{ds}G_{ds}G_{ds}A_{ds}G_{ds}G_{ds}^{m}C_{ds}GₖₛAₖₛTₖ | 64.2 | 6017 | 6032 | 663 |
| 570666 | ^{m}CₖₛTₖₛ^{m}Cₖₛ^{m}C_{ds}T_{ds}G_{ds}^{m}C_{ds}T_{ds}^{m}C_{ds}A_{ds}G_{ds}A_{ds}G_{ds}GₖₛGₖₛAₖ | 59 | 6028 | 6043 | 664 |
| 570667 | GₖₛTₖₛGₖₛ^{m}C_{ds}T_{ds}^{m}C_{ds}^{m}C_{ds}T_{ds}G_{ds}^{m}C_{ds}T_{ds}^{m}C_{ds}A_{ds}GₖₛAₖₛGₖ | 82.3 | 6031 | 6046 | 665 |
| 570668 | AₖₛGₖₛGₖₛT_{ds}G_{ds}^{m}C_{ds}T_{ds}^{m}C_{ds}^{m}C_{ds}T_{ds}G_{ds}^{m}C_{ds}T_{ds}^{m}CₖₛAₖₛGₖ | 96.2 | 6033 | 6048 | 666 |
| 570669 | AₖₛGₖₛAₖₛG_{ds}G_{ds}T_{ds}G_{ds}^{m}C_{ds}T_{ds}^{m}C_{ds}^{m}C_{ds}T_{ds}G_{ds}^{m}CₖₛTₖₛ^{m}Cₖ | 26.2 | 6035 | 6050 | 667 |
| 570670 | AₖₛGₖₛAₖₛG_{ds}A_{ds}G_{ds}G_{ds}T_{ds}G_{ds}^{m}C_{ds}T_{ds}^{m}C_{ds}^{m}C_{ds}TₖₛGₖₛ^{m}Cₖ | 18.2 | 6037 | 6052 | 668 |
| 570671 | Aₖₛ^{m}Cₖₛ^{m}Cₖₛ^{m}C_{ds}^{m}C_{ds}G_{ds}^{m}C_{ds}^{m}C_{ds}^{m}C_{ds}^{m}C_{ds}^{m}C_{ds}G_{ds}^{m}C_{ds}Tₖₛ^{m}CₖₛAₖ | 29.2 | 6291 | 6306 | 669 |
| 570672 | ^{m}CₖₛTₖₛAₖₛ^{m}C_{ds}^{m}C_{ds}^{m}C_{ds}^{m}C_{ds}G_{ds}^{m}C_{ds}^{m}C_{ds}^{m}C_{ds}^{m}C_{ds}^{m}C_{ds}Gₖₛ^{m}CₖₛTₖ | 50.3 | 6293 | 6308 | 670 |
| 570673 | Aₖₛ^{m}Cₖₛ^{m}CₖₛT_{ds}A_{ds}^{m}C_{ds}^{m}C_{ds}^{m}C_{ds}^{m}C_{ds}G_{ds}^{m}C_{ds}^{m}C_{ds}^{m}C_{ds}^{m}Cₖₛ^{m}CₖₛGₖ | 26.8 | 6295 | 6310 | 671 |
| 570674 | GₖₛTₖₛAₖₛ^{m}C_{ds}^{m}C_{ds}T_{ds}A_{ds}^{m}C_{ds}^{m}C_{ds}^{m}C_{ds}^{m}C_{ds}G_{ds}^{m}C_{ds}^{m}Cₖₛ^{m}Cₖₛ^{m}Cₖ | 40.8 | 6297 | 6312 | 672 |
| 570675 | AₖₛGₖₛGₖₛT_{ds}A_{ds}^{m}C_{ds}^{m}C_{ds}T_{ds}A_{ds}^{m}C_{ds}^{m}C_{ds}^{m}C_{ds}^{m}C_{ds}Gₖₛ^{m}Cₖₛ^{m}Cₖ | 56.1 | 6299 | 6314 | 673 |
| 570676 | GₖₛGₖₛGₖₛA_{ds}G_{ds}G_{ds}T_{ds}T_{ds}^{m}C_{ds}^{m}C_{ds}^{m}C_{ds}G_{ds}^{m}C_{ds}AₖₛGₖₛ^{m}Cₖ | 95 | 6329 | 6344 | 674 |
| 570677 | GₖₛTₖₛ^{m}Cₖₛ^{m}C_{ds}T_{ds}T_{ds}A_{ds}^{m}C_{ds}T_{ds}^{m}C_{ds}^{m}C_{ds}A_{ds}A_{ds}^{m}CₖₛTₖₛTₖ | 23 | 6360 | 6375 | 675 |
| 570678 | ^{m}CₖₛTₖₛGₖₛT_{ds}^{m}C_{ds}^{m}C_{ds}T_{ds}T_{ds}A_{ds}^{m}C_{ds}T_{ds}^{m}C_{ds}^{m}C_{ds}AₖₛAₖₛ^{m}Cₖ | 23.4 | 6362 | 6377 | 676 |
| 570679 | ^{m}CₖₛAₖₛ^{m}CₖₛT_{ds}G_{ds}T_{ds}^{m}C_{ds}^{m}C_{ds}T_{ds}T_{ds}A_{ds}^{m}C_{ds}T_{ds}^{m}Cₖₛ^{m}CₖₛAₖ | 7.4 | 6364 | 6379 | 677 |
| 570680 | GₖₛGₖₛ^{m}CₖₛA_{ds}^{m}C_{ds}T_{ds}G_{ds}T_{ds}^{m}C_{ds}^{m}C_{ds}T_{ds}T_{ds}A_{ds}^{m}CₖₛTₖₛ^{m}Cₖ | 20.6 | 6366 | 6381 | 678 |
| 570681 | TₖₛAₖₛGₖₛG_{ds}^{m}C_{ds}A_{ds}^{m}C_{ds}T_{ds}G_{ds}T_{ds}^{m}C_{ds}^{m}C_{ds}T_{ds}TₖₛAₖₛ^{m}Cₖ | 29 | 6368 | 6383 | 679 |
| 570682 | GₖₛGₖₛTₖₛA_{ds}G_{ds}G_{ds}^{m}C_{ds}A_{ds}^{m}C_{ds}T_{ds}G_{ds}T_{ds}^{m}C_{ds}^{m}CₖₛTₖₛTₖ | 10.5 | 6370 | 6385 | 680 |
| 570683 | GₖₛTₖₛ^{m}CₖₛA_{ds}^{m}C_{ds}T_{ds}G_{ds}^{m}C_{ds}T_{ds}G_{ds}G_{ds}G_{ds}T_{ds}^{m}Cₖₛ^{m}CₖₛTₖ | 23 | 6445 | 6460 | 681 |
| 570684 | GₖₛGₖₛTₖₛ^{m}C_{ds}A_{ds}^{m}C_{ds}T_{ds}G_{ds}^{m}C_{ds}T_{ds}G_{ds}G_{ds}G_{ds}Tₖₛ^{m}Cₖₛ^{m}Cₖ | 22.5 | 6446 | 6461 | 433 |
| 570685 | AₖₛGₖₛGₖₛT_{ds}^{m}C_{ds}A_{ds}^{m}C_{ds}T_{ds}G_{ds}^{m}C_{ds}T_{ds}G_{ds}G_{ds}GₖₛTₖₛ^{m}Cₖ | 10.2 | 6447 | 6462 | 682 |
| 570686 | ^{m}CₖₛTₖₛAₖₛG_{ds}G_{ds}T_{ds}^{m}C_{ds}A_{ds}^{m}C_{ds}T_{ds}G_{ds}^{m}C_{ds}T_{dS}GₖₛGₖₛGₖ | 11.1 | 6449 | 6464 | 683 |
| 570687 | GₖₛTₖₛ^{m}CₖₛT_{ds}A_{ds}G_{ds}G_{ds}T_{ds}^{m}C_{ds}A_{ds}^{m}C_{ds}T_{ds}G_{ds}^{m}CₖₛTₖₛGₖ | 11.7 | 6451 | 6466 | 684 |
| 570688 | AₖₛAₖₛGₖₛT_{ds}^{m}C_{ds}T_{ds}A_{ds}G_{ds}G_{ds}T_{ds}^{m}C_{ds}A_{ds}^{m}C_{ds}TₖₛGₖₛ^{m}Cₖ | 14.6 | 6453 | 6468 | 685 |
| 570689 | Gₖₛ^{m}CₖₛAₖₛ^{m}C_{ds}T_{ds}^{m}C_{ds}^{m}C_{ds}A_{ds}T_{ds}T_{ds}G_{ds}T_{ds}^{m}C_{ds}Tₖₛ^{m}CₖₛAₖ | 10.1 | 6530 | 6545 | 686 |
| 570690 | ^{m}CₖₛTₖₛGₖₛ^{m}C_{ds}A_{ds}^{m}C_{ds}T_{ds}^{m}C_{ds}^{m}C_{ds}A_{ds}T_{ds}T_{ds}G_{ds}Tₖₛ^{m}CₖₛTₖ | 35.4 | 6532 | 6547 | 687 |
| 570691 | ^{m}Cₖₛ^{m}Cₖₛ^{m}CₖₛT_{ds}G_{ds}^{m}C_{ds}A_{ds}^{m}C_{ds}T_{ds}^{m}C_{ds}^{m}C_{ds}A_{ds}T_{ds}TₖₛGₖₛTₖ | 33.6 | 6534 | 6549 | 688 |
| 570692 | ^{m}Cₖₛ^{m}Cₖₛ^{m}Cₖₛ^{m}C_{ds}^{m}C_{ds}T_{ds}G_{ds}^{m}C_{ds}A_{ds}^{m}C_{ds}T_{ds}^{m}C_{ds}^{m}C_{ds}AₖₛTₖₛTₖ | 77.3 | 6536 | 6551 | 689 |
| 570693 | ^{m}CₖₛTₖₛTₖₛG_{ds}^{m}C_{ds}T_{ds}G_{ds}A_{ds}G_{ds}T_{ds}^{m}C_{ds}A_{ds}G_{ds}GₖₛAₖₛGₖ | 18.9 | 6559 | 6574 | 690 |
| 570694 | Tₖₛ^{m}Cₖₛ^{m}CₖₛT_{ds}T_{ds}G_{ds}^{m}C_{ds}T_{ds}G_{ds}A_{ds}G_{ds}T_{ds}^{m}C_{ds}AₖₛGₖₛGₖ | 30.9 | 6561 | 6576 | 691 |
| 570695 | ^{m}CₖₛTₖₛTₖₛ^{m}C_{ds}^{m}C_{ds}T_{ds}T_{ds}G_{ds}^{m}C_{ds}T_{ds}G_{ds}A_{ds}G_{ds}Tₖₛ^{m}CₖₛAₖ | 21 | 6563 | 6578 | 692 |
| 570696 | Aₖₛ^{m}Cₖₛ^{m}CₖₛT_{ds}T_{ds}^{m}C_{ds}^{m}C_{ds}T_{ds}T_{ds}G_{ds}^{m}C_{ds}T_{ds}G_{ds}AₖₛGₖₛTₖ | 50.3 | 6565 | 6580 | 693 |
| 570697 | GₖₛGₖₛAₖₛ^{m}C_{ds}^{m}C_{ds}T_{ds}T_{ds}^{m}C_{ds}^{m}C_{ds}T_{ds}T_{ds}G_{ds}^{m}C_{ds}TₖₛGₖₛAₖ | 28.3 | 6567 | 6582 | 694 |
| 570698 | ^{m}CₖₛAₖₛGₖₛG_{ds}A_{ds}^{m}C_{ds}^{m}C_{ds}T_{ds}T_{ds}^{m}C_{ds}^{m}C_{ds}T_{ds}T_{ds}Gₖₛ^{m}CₖₛTₖ | 47.6 | 6569 | 6584 | 695 |
| 570699 | AₖₛGₖₛ^{m}Cₖₛ^{m}C_{ds}^{m}C_{ds}T_{ds}^{m}C_{ds}^{m}C_{ds}A_{ds}G_{ds}G_{ds}A_{ds}^{m}C_{ds}^{m}CₖₛTₖₛTₖ | 17.9 | 6576 | 6591 | 696 |
| 570700 | TₖₛAₖₛGₖₛ^{m}C_{ds}T_{ds}^{m}C_{ds}^{m}C_{ds}^{m}C_{ds}^{m}C_{ds}A_{ds}^{m}C_{ds}T_{ds}^{m}C_{ds}^{m}CₖₛAₖₛGₖ | 24.1 | 6594 | 6609 | 697 |
| 570701 | GₖₛAₖₛTₖₛA_{ds}G_{ds}^{m}C_{ds}T_{ds}^{m}C_{ds}^{m}C_{ds}^{m}C_{ds}^{m}C_{ds}A_{ds}^{m}C_{ds}Tₖₛ^{m}Cₖₛ^{m}Cₖ | 12.9 | 6596 | 6611 | 698 |
| 570702 | ^{m}CₖₛAₖₛGₖₛA_{ds}T_{ds}A_{ds}G_{ds}^{m}C_{ds}T_{ds}^{m}C_{ds}^{m}C_{ds}^{m}C_{ds}^{m}C_{ds}Aₖₛ^{m}CₖₛTₖ | 24 | 6598 | 6613 | 699 |
| 570703 | ^{m}CₖₛTₖₛ^{m}CₖₛA_{ds}G_{ds}A_{ds}T_{ds}A_{ds}G_{ds}^{m}C_{ds}T_{ds}^{m}C_{ds}^{m}C_{ds}^{m}Cₖₛ^{m}CₖₛAₖ | 22.3 | 6600 | 6615 | 700 |
| 570704 | AₖₛGₖₛ^{m}CₖₛT_{ds}^{m}C_{ds}A_{ds}G_{ds}A_{ds}T_{ds}A_{ds}G_{ds}^{m}C_{ds}T_{ds}^{m}Cₖₛ^{m}Cₖₛ^{m}Cₖ | 31.8 | 6602 | 6617 | 701 |
| 570705 | Tₖₛ^{m}CₖₛAₖₛG_{ds}^{m}C_{ds}T_{ds}^{m}C_{ds}A_{ds}G_{ds}A_{ds}T_{ds}A_{ds}G_{ds}^{m}CₖₛTₖₛ^{m}Cₖ | 33.9 | 6604 | 6619 | 702 |
| 570706 | Tₖₛ^{m}CₖₛTₖₛ^{m}C_{ds}A_{ds}G_{ds}^{m}C_{ds}T_{ds}^{m}C_{ds}A_{ds}G_{ds}A_{ds}T_{ds}AₖₛGₖₛ^{m}Cₖ | 28.1 | 6606 | 6621 | 703 |
| 570707 | GₖₛAₖₛGₖₛT_{ds}^{m}C_{ds}^{m}C_{ds}T_{ds}^{m}C_{ds}T_{ds}^{m}C_{ds}^{m}C_{ds}T_{ds}G_{ds}^{m}CₖₛTₖₛTₖ | 37.2 | 6636 | 6651 | 704 |
| 570708 | GₖₛGₖₛAₖₛG_{ds}G_{ds}A_{ds}G_{ds}T_{ds}^{m}C_{ds}^{m}C_{ds}T_{ds}^{m}C_{ds}T_{ds}^{m}Cₖₛ^{m}CₖₛTₖ | 66.3 | 6640 | 6655 | 705 |
| 570709 | GₖₛAₖₛGₖₛG_{ds}A_{ds}G_{ds}G_{ds}A_{ds}G_{ds}T_{ds}^{m}C_{ds}^{m}C_{ds}T_{ds}^{m}CₖₛTₖₛ^{m}Cₖ | 52.7 | 6642 | 6657 | 706 |
| 570710 | ^{m}CₖₛAₖₛAₖₛA_{ds}A_{ds}G_{ds}G_{ds}G_{ds}^{m}C_{ds}A_{ds}^{m}C_{ds}^{m}C_{ds}^{m}C_{ds}AₖₛGₖₛAₖ | 31.8 | 6713 | 6728 | 707 |
| 570711 | AₖₛGₖₛ^{m}CₖₛA_{ds}A_{ds}A_{ds}A_{ds}G_{ds}G_{ds}G_{ds}^{m}C_{ds}A_{ds}^{m}C_{ds}^{m}Cₖₛ^{m}CₖₛAₖ | 12.3 | 6715 | 6730 | 708 |
| 570712 | GₖₛGₖₛAₖₛT_{ds}^{m}C_{ds}^{m}C_{ds}^{m}C_{ds}^{m}C_{ds}A_{ds}G_{ds}T_{ds}A_{ds}T_{ds}TₖₛGₖₛTₖ | 37.1 | 6733 | 6748 | 709 |
| 570713 | ^{m}CₖₛTₖₛGₖₛG_{ds}A_{ds}T_{ds}^{m}C_{ds}^{m}C_{ds}^{m}C_{ds}^{m}C_{ds}A_{ds}G_{ds}T_{ds}AₖₛTₖₛTₖ | 42.4 | 6735 | 6750 | 710 |
| 570714 | TₖₛGₖₛ^{m}CₖₛT_{ds}G_{ds}G_{ds}A_{ds}T_{ds}^{m}C_{ds}^{m}C_{ds}^{m}C_{ds}^{m}C_{ds}A_{ds}GₖₛTₖₛAₖ | 31.4 | 6737 | 6752 | 711 |
| 570715 | AₖₛTₖₛTₖₛ^{m}C_{ds}T_{ds}^{m}C_{ds}T_{ds}A_{ds}G_{ds}A_{ds}^{m}C_{ds}T_{ds}G_{ds}^{m}CₖₛAₖₛAₖ | 12.1 | 6789 | 6804 | 712 |
| 570716 | TₖₛAₖₛAₖₛT_{ds}T_{ds}^{m}C_{ds}T_{ds}^{m}C_{ds}T_{ds}A_{ds}G_{ds}A_{ds}^{m}C_{ds}TₖₛGₖₛ^{m}Cₖ | 9 | 6791 | 6806 | 713 |
| 570717 | Tₖₛ^{m}CₖₛTₖₛA_{ds}A_{ds}T_{ds}T_{ds}^{m}C_{ds}T_{ds}^{m}C_{ds}T_{ds}A_{ds}G_{ds}Aₖₛ^{m}CₖₛTₖ | 32.1 | 6793 | 6808 | 714 |
| 570718 | Tₖₛ^{m}CₖₛTₖₛ^{m}C_{ds}T_{ds}A_{ds}A_{ds}T_{ds}T_{ds}^{m}C_{ds}T_{ds}^{m}C_{ds}T_{ds}AₖₛGₖₛAₖ | 71.4 | 6795 | 6810 | 715 |
| 570719 | ^{m}CₖₛTₖₛ^{m}Cₖₛ^{m}C_{ds}A_{ds}T_{ds}A_{ds}A_{ds}T_{ds}T_{ds}^{m}C_{dS}T_{ds}^{m}C_{dS}T_{b}AₖₛAₖ | 36.9 | 6804 | 6819 | 716 |
| 570720 | Aₖₛ^{m}CₖₛTₖₛ^{m}C_{ds}T_{ds}^{m}C_{ds}^{m}C_{ds}A_{ds}T_{ds}A_{ds}A_{ds}T_{ds}T_{ds}^{m}CₖₛTₖₛ^{m}Cₖ | 17.1 | 6807 | 6822 | 717 |
| 570721 | Aₖₛ^{m}CₖₛAₖₛ^{m}C_{ds}T_{ds}^{m}C_{ds}T_{ds}^{m}C_{ds}^{m}C_{ds}A_{ds}T_{ds}A_{ds}A_{ds}TₖₛTₖₛ^{m}Cₖ | 23.7 | 6809 | 6824 | 718 |
| 570722 | ^{m}Cₖₛ^{m}CₖₛAₖₛ^{m}C_{ds}A_{ds}^{m}C_{ds}T_{ds}^{m}C_{ds}T_{ds}^{m}C_{ds}^{m}C_{ds}A_{ds}T_{ds}AₖₛAₖₛTₖ | 34.4 | 6811 | 6826 | 719 |
| 570723 | TₖₛGₖₛ^{m}Cₖₛ^{m}C_{ds}A_{ds}^{m}C_{ds}A_{ds}^{m}C_{ds}T_{ds}^{m}C_{ds}T_{ds}^{m}C_{ds}^{m}C_{ds}AₖₛTₖₛAₖ | 38.7 | 6813 | 6828 | 720 |

### Example 10: Dose response studies with antisense oligonucleotides targeting human dystrophia myotonica-protein kinase (DMPK) in HepG2 Cells

Antisense oligonucleotides targeted to a human DMPK nucleic acid were tested for their effect on human DMPK RNA transcript *in vitro.* Cultured HepG2 cells at a density of 20,000 cells per well were transfected using electroporation with 61.7 nM, 185.2 nM, 555.6 nM, 1666.7 nM, 5000.0 nM, and 15000.0 nM concentrations of each antisense oligonucleotide. After approximately 24 hours, RNA was isolated from the cells and DMPK RNA transcript levels were measured by quantitative real-time PCR using primer probe set RTS3164 (forward sequence AGCCTGAGCCGGGAGATG, designated herein as SEQ ID NO: 20; reverse sequence GCGTAGTTGACTGGCGAAGTT, designated herein as SEQ ID NO: 21; probe sequence AGGCCATCCGCACGGACAACCX, designated herein as SEQ ID NO: 22). Human DMPK RNA transcript levels were adjusted according to total RNA content, as measured by RIBOGREEN®. Results are presented as percent expression of human DMPK, relative to untreated control (UTC) cells. For example, if the UTC is 100 and a dose of 5000 nM of ISIS No. 445569 yields a % Expression of human DMPK of 35 then the 5000 nM dose of ISIS reduced expression of human DMPK by 65% relative to the UTC. The half maximal inhibitory concentration (IC₅₀) of each oligonucleotide is presented in the table below and was calculated by plotting the concentrations of oligonucleotides used versus the percent inhibition of human DMPK mRNA expression achieved at each concentration, and noting the concentration of oligonucleotide at which 50% inhibition of human DMPK mRNA expression was achieved compared to the control. The results are presented in Table 19.

The tested antisense oligonucleotide sequences demonstrated dose-dependent inhibition of human DMPK mRNA levels under the conditions specified above.

**Table 19**

| Dose response studies for with antisense oligonucleotides targeting hDMPK in HepG2 Cells | | | |
|---|---|---|---|
| **ISIS No.** | **Dose (nM)** | **% Expression of human DMPK** | **IC₅₀** |
| UTC | ND | 100 | ND |
| 445569 | 61.7 | 115.3 | 2.3 |
| | 185.2 | 87.9 | |
| | 555.6 | 69.0 | |
| | 1666.7 | 57.2 | |
| | 5000.0 | 35.0 | |
| | 15000.0 | 22.6 | |
| 512497 | 61.7 | 108.6 | 2 |
| | 185.2 | 98.4 | |
| | 555.6 | 77.9 | |
| | 1666.7 | 57.2 | |
| | 5000.0 | 28.0 | |
| | 15000.0 | 12.8 | |
| 486178 | 61.7 | 88.2 | 0.7 |
| | 185.2 | 67.1 | |
| | 555.6 | 49.4 | |
| | 1666.7 | 32.8 | |
| | 5000.0 | 26.7 | |
| | 15000.0 | 11.8 | |
| 569473 | 61.7 | 107.9 | 0.6 |
| | 185.2 | 66.5 | |
| | 555.6 | 33.6 | |
| | 1666.7 | 23.5 | |
| | 5000.0 | 12.8 | |
| | 15000.0 | 9.2 | |
| 570808 | 61.7 | 77.2 | 0.2 |
| | 185.2 | 52.7 | |
| | 555.6 | 20.6 | |
| | 1666.7 | 8.1 | |
| | 5000.0 | 7.2 | |
| | 15000.0 | 5.4 | |
| 594292 | 61.7 | 96.2 | 5.5 |
| | 185.2 | 99.6 | |
| | 555.6 | 80.0 | |
| | 1666.7 | 59.0 | |
| | 5000.0 | 45.5 | |
| | 15000.0 | 42.8 | |
| 594300 | 61.7 | 101.7 | >15 |
| | 185.2 | 104.3 | |
| | 555.6 | 101.6 | |
| | 1666.7 | 93.6 | |
| | 5000.0 | 74.9 | |
| | 15000.0 | 66.8 | |
| 598768 | 61.7 | 95.5 | 1.2 |
| | 185.2 | 83.6 | |
| | 555.6 | 70.6 | |
| | 1666.7 | 40.7 | |
| | 5000.0 | 22.2 | |
| | 15000.0 | 7.3 | |
| 598769 | 61.7 | 103.9 | 1.9 |
| | 185.2 | 105.3 | |
| | 555.6 | 76.1 | |
| | 1666.7 | 50.4 | |
| | 5000.0 | 29.8 | |
| | 15000.0 | 12.1 | |
| 598777 | 61.7 | 96.4 | 0.9 |
| | 185.2 | 69.4 | |
| | 555.6 | 41.8 | |
| | 1666.7 | 42.8 | |
| | 5000.0 | 16.4 | |
| | 15000.0 | 27.1 | |

### Example 11: Dose response studies with antisense oligonucleotides targeting human dystrophia myotonica-protein kinase (hDMPK) in Steinert DM1 Myoblast Cells

Antisense oligonucleotides targeted to a human DMPK nucleic acid were tested for their effect on human DMPK RNA transcript *in vitro.* Cultured Steinert DM1 myoblast cells at a density of 20,000 cells per well were transfected using electroporation with 61.7 nM, 185.2 nM, 555.6 nM, 1666.7 nM, 5000.0 nM, and 15000.0 nM concentrations of each antisense oligonucleotide. After approximately 24 hours, RNA was isolated from the cells and DMPK RNA transcript levels were measured by quantitative real-time PCR using primer probe set RTS3164 described above. Human DMPK RNA transcript levels were adjusted according to total RNA content, as measured by RIBOGREEN®. Results are presented as percent (%) expression of human DMPK, relative to untreated control (UTC) cells. The half maximal inhibitory concentration (IC₅₀) of each oligonucleotide is presented in the table below and was calculated by plotting the concentrations of oligonucleotides used versus the percent inhibition of human DMPK mRNA expression achieved at each concentration, and noting the concentration of oligonucleotide at which 50% inhibition of human DMPK mRNA expression was achieved compared to the control. The results are presented in Table 20.

The tested antisense oligonucleotide sequences demonstrated dose-dependent inhibition of human DMPK mRNA levels under the conditions specified above.

**Table 20**

| Dose response studies for with antisense oligonucleotides targeting hDMPK in Steinert DM1 Cells | | | |
|---|---|---|---|
| **ISIS No.** | **Dose (nM)** | **% Expression of human DMPK** | **IC₅₀** |
| UTC | ND | 100 | ND |
| 445569 | 61.7 | 58.3 | 0.4 |
| | 185.2 | 56.7 | |
| | 555.6 | 58.5 | |
| | 1666.7 | 40.9 | |
| | 5000.0 | 26.0 | |
| | 15000.0 | 23.5 | |
| 512497 | 61.7 | 78.1 | 5.1 |
| | 185.2 | 77.5 | |
| | 555.6 | 98.8 | |
| | 1666.7 | 71.2 | |
| | 5000.0 | 51.3 | |
| | 15000.0 | 22.8 | |
| 486178 | 61.7 | 78.0 | 0.5 |
| | 185.2 | 61.3 | |
| | 555.6 | 43.3 | |
| | 1666.7 | 27.4 | |
| | 5000.0 | 24.6 | |
| | 15000.0 | 16.9 | |
| 569473 | 61.7 | 83.3 | 0.6 |
| | 185.2 | 54.8 | |
| | 555.6 | 64.5 | |
| | 1666.7 | 26.1 | |
| | 5000.0 | 19.4 | |
| | 15000.0 | 15.4 | |
| 570808 | 61.7 | 103.6 | 0.9 |
| | 185.2 | 77.8 | |
| | 555.6 | 46.7 | |
| | 1666.7 | 25.2 | |
| | 5000.0 | 20.8 | |
| | 15000.0 | 19.3 | |
| 594292 | 61.7 | 100.1 | 5.6 |
| | 185.2 | 109.7 | |
| | 555.6 | 72.6 | |
| | 1666.7 | 66.2 | |
| | 5000.0 | 39.5 | |
| | 15000.0 | 45.7 | |
| 594300 | 61.7 | 96.2 | 5.6 |
| | 185.2 | 87.1 | |
| | 555.6 | 70.3 | |
| | 1666.7 | 66.4 | |
| | 5000.0 | 58.1 | |
| | 15000.0 | 33.2 | |
| 598768 | 61.7 | 77.0 | 0.7 |
| | 185.2 | 62.9 | |
| | 555.6 | 62.0 | |
| | 1666.7 | 35.6 | |
| | 5000.0 | 24.5 | |
| | 15000.0 | 21.0 | |
| 598769 | 61.7 | 70.3 | 0.4 |
| | 185.2 | 49.2 | |
| | 555.6 | 55.3 | |
| | 1666.7 | 33.2 | |
| | 5000.0 | 27.1 | |
| | 15000.0 | 13.4 | |
| 598777 | 61.7 | 87.7 | 1 |
| | 185.2 | 61.7 | |
| | 555.6 | 57.3 | |
| | 1666.7 | 37.9 | |
| | 5000.0 | 30.0 | |
| | 15000.0 | 29.7 | |

### Example 12: Dose response studies with antisense oligonucleotides targeting rhesus monkey dystrophia myotonica-protein kinase (DMPK) in cynomolgus monkey primary hepatocytes

Antisense oligonucleotides targeted to a rhesus monkey DMPK nucleic acid were tested for their effect on rhesus monkey DMPK RNA transcript *in vitro.* Cultured cynomolgus monkey primary hepatocytes cells at a density of 20,000 cells per well were transfected using electroporation with 61.7 nM, 185.2 nM, 555.6 nM, 1666.7 nM, 5000.0 nM, and 15000.0 nM concentrations of each antisense oligonucleotide. After approximately 24 hours, RNA was isolated from the cells and DMPK RNA transcript levels were measured by quantitative real-time PCR using primer probe set RTS3164 described above. Rhesus monkey DMPK RNA transcript levels were adjusted according to total RNA content, as measured by RIBOGREEN®. Results are presented as percent (%) expression of rhesus monkey DMPK, relative to untreated control (UTC) cells. The half maximal inhibitory concentration (IC₅₀) of each oligonucleotide is presented in the table below and was calculated by plotting the concentrations of oligonucleotides used versus the percent inhibition of rhesus monkey DMPK mRNA expression achieved at each concentration, and noting the concentration of oligonucleotide at which 50% inhibition of rhesus monkey DMPK mRNA expression was achieved compared to the control.

The tested antisense oligonucleotide sequences demonstrated dose-dependent inhibition of rhesus monkey DMPK mRNA levels under the conditions specified above.

**Table 21**

| Dose response studies for with antisense oligonucleotides targeting rhesus monkey DMPK in cynomolgus monkey primary hepatocytes | | | |
|---|---|---|---|
| **ISIS No.** | **Dose (nM)** | **% Expression of human DMPK** | **IC₅₀** |
| UTC | ND | 100 | ND |
| 445569 | 61.7 | 79.7 | 1.4 |
| | 185.2 | 41.1 | |
| | 555.6 | 58.1 | |
| | 1666.7 | 33.5 | |
| | 5000.0 | 46.9 | |
| | 15000.0 | 50.0 | |
| 512497 | 61.7 | 123.4 | 1.5 |
| | 185.2 | 63.7 | |
| | 555.6 | 44.8 | |
| | 1666.7 | 34.1 | |
| | 5000.0 | 51.2 | |
| | 15000.0 | 23.5 | |
| 486178 | 61.7 | 51.1 | <.06 |
| | 185.2 | 30.6 | |
| | 555.6 | 22.0 | |
| | 1666.7 | 23.5 | |
| | 5000.0 | 9.8 | |
| | 15000.0 | 19.2 | |
| 569473 | 61.7 | 82.1 | .2 |
| | 185.2 | 39.4 | |
| | 555.6 | 17.7 | |
| | 1666.7 | 28.5 | |
| | 5000.0 | 20.0 | |
| | 15000.0 | 15.6 | |
| 570808 | 61.7 | 74.6 | 0.1 |
| | 185.2 | 27.6 | |
| | 555.6 | 16.4 | |
| | 1666.7 | 25.6 | |
| | 5000.0 | 8.8 | |
| | 15000.0 | 21.9 | |
| 594292 | 61.7 | 93.0 | >15 |
| | 185.2 | 82.1 | |
| | 555.6 | 106.0 | |
| | 1666.7 | 91.1 | |
| | 5000.0 | 62.2 | |
| | 15000.0 | 70.4 | |
| 594300 | 61.7 | 105.5 | >15 |
| | 185.2 | 91.8 | |
| | 555.6 | 114.9 | |
| | 1666.7 | 65.7 | |
| | 5000.0 | 110.2 | |
| | 15000.0 | 118.8 | |
| 598768 | 61.7 | 70.3 | 0.4 |
| | 185.2 | 57.8 | |
| | 555.6 | 58.5 | |
| | 1666.7 | 16.5 | |
| | 5000.0 | 24.0 | |
| | 15000.0 | 13.4 | |
| 598769 | 61.7 | 76.5 | 1.1 |
| | 185.2 | 65.1 | |
| | 555.6 | 64.0 | |
| | 1666.7 | 34.4 | |
| | 5000.0 | 60.9 | |
| | 15000.0 | 8.6 | |
| 598777 | 61.7 | 161.4 | 2.1 |
| | 185.2 | 51.7 | |
| | 555.6 | 47.5 | |
| | 1666.7 | 34.6 | |
| | 5000.0 | 27.8 | |
| | 15000.0 | 52.9 | |

### Example 13: In vivo antisense inhibition of hDMPK in DMSXL transgenic mice

To test the effect of antisense inhibition for the treatment of myotonic dystrophy type 1 (DM1), an appropriate mouse model was required. The transgenic mouse model, DMSXL carrying the hDMPK gene with large expansions of over 1000 CTG repeats was generated (Huguet et al., PLOS Genetics, 2012, 8(11), e1003034-e1003043). These DMSXL mice express the mutant hDMPK allele and display muscle weakness phenotype similar to that seen in DM1 patients.

ISIS 486178 from Table 1 was selected and tested for antisense inhibition of hDMPK transcript *in vivo.* ISIS 445569 was included in the study for comparison.

### Treatment

DMSXL mice were maintained on a 12-hour light/dark cycle and fed *ad libitum* normal Purina mouse chow. Animals were acclimated for at least 7 days in the research facility before initiation of the experiment. Antisense oligonucleotides (ASOs) were prepared in PBS and sterilized by filtering through a 0.2 micron filter. ASOs were dissolved in 0.9% PBS for injection.

DMSXL mice received subcutaneous injections of ISIS 445569 at 50 mg/kg or ISIS 486178 at 25 mg/kg twice per week for 4 weeks. The control group received subcutaneous injections of PBS twice weekly for 4 weeks. Each treatment group consisted of 4 animals.

### Inhibition of hDMPK mRNA levels

Twenty four hours after the final dose, the mice were sacrificed and tissues were collected. mRNA was isolated for real-time PCR analysis of hDMPK and normalized to 18s RNA. Human primer probe set RTS3164 was used to measure mRNA levels. The results are expressed as the average percent of hDMPK mRNA levels for each treatment group, relative to PBS control.

Human primer probe set RTS3164 (forward sequence AGCCTGAGCCGGGAGATG, designated herein as SEQ ID NO: 20; reverse sequence GCGTAGTTGACTGGCGAAGTT, designated herein as SEQ ID NO: 21; probe sequence AGGCCATCCGCACGGACAACCX, designated herein as SEQ ID NO: 22).

As presented in Table 22 below, treatment with antisense oligonucleotides reduced hDMPK transcript expression. The results indicate that treatment with ISIS 445569 and 486178 resulted in reduction of hDMPK mRNA levels in DMSXL mice.

**Table 22**

| Effect of antisense oligonucleotides on hDMPK inhibition in DMSXL mice | | | | |
|---|---|---|---|---|
| ISIS No. | Dosage (mg/kg) | Tissue Type | hDMPK mRNA levels (% PBS) | Motif/Length |
| PBS | 0 | | | |
| 486178 | 25 | Tibialis Anterior | 70.7 | kkk-d10-kkk (16 mer) |
| | | Soleus | 67.3 | |
| | | Quadriceps | 73.9 | |
| | | Latissiumus grand dorsi | 71.0 | |
| | | Triceps | 67.1 | |
| | | Diaphragm | 68.9 | |
| | | Heart | 30.8 | |
| | | Brain | 11.8 | |
| 445569 | 50 | Tibialis Anterior | 38.4 | e5-d10-e5 (20 mer) |
| | | Soleus | 47.5 | |
| | | Quadriceps | 41.3 | |
| | | Latissiumus grand dorsi | 35.7 | |
| | | Triceps | 30.5 | |
| | | Diaphragm | 44.7 | |
| | | Heart | 7.6 | |
| | | Brain | 13.1 | |

### Example 14: Effect of ASO treatment on muscle strength in DMSXL mice targeting hDMPK

### Griptest

Mice were assessed for grip strength performance in wild-type (WT) and DMSXL forelimb using a commercial grip strength dynamometer as described in the literature ((Huguet et al., PLOS Genetics, 2012, 8(11), e1003034-e1003043).

DMSXL mice received subcutaneous injections of ISIS 486178 at 25 mg/kg or ISIS 445569 at 50 mg/kg twice per week for 4 weeks. The control DMSXL group received subcutaneous injections of PBS twice weekly for 4 weeks. Each treatment group consisted of 4 animals. The forelimb force for each treatment group and WT was measured at day 0, 30, and 60 using the griptest. The grip strength performance was determined by measuring the force difference between day 60 and day 0. Results are presented as the average forelimb force from each group.

As illustrated in Table 23, below, treatment with ASOs targeting hDMPK improved muscle strength in DMSXL mice compared to untreated control. ISIS 486178, an ASO with cEt modifications, demonstrated substantial improvement in the forelimb strength (+3.4) compared to ISIS 445569 with MOE modifications (+0.38).

**Table 23**

| Effect of ASO treatment on muscle strength in DMSXL mice targeting hDMPK | | | | |
|---|---|---|---|---|
| Treatment group | Forelimb force (g) | | | |
| | Day 0 | Day 30 | Day 60 | Δ = Day 60 - Day 0 |
| Untreated control | 72.2 | 70.2 | 67.5 | -4.6 |
| ASO 486178 | 62.3 | 65.7 | 65.6 | +3.4 |
| ASO 445569 | 64.3 | 68 | 64.7 | +0.38 |
| Wild type (WT) | 75.2 | 76.5 | 78.4 | +3.2 |

### Example 15: Effect of ASO treatment on muscle fiber distribution in DMSXL mice targeting hDMPK

The muscle fiber distribution in DMSXL mice targeting hDMPK in the presence and absence of ISIS 445569 and 486178 was also assessed. Both ASOs were previously described in Table 1, above.

DMSXL mice received subcutaneous injections of ISIS 486178 at 25 mg/kg or ISIS 445569 at 50 mg/kg twice per week for 4 weeks. The control DMSXL group received subcutaneous injections of PBS twice weekly for 4 weeks. Each treatment group consisted of 4 animals. The muscle fiber distribution was assessed and the results are presented Table 44, below.

As illustrated, treatment with ASOs targeting hDMPK decreased the distribution of DM1 Associated Type 2c muscle fiber in the tibialis anterior (TA) of DMSXL mice compared to untreated control. The results demonstrated that normal pattern of fiber distribution in the skeletal muscles can be restored with ASO treatment. ISIS 445569 demonstrated an improvement in the muscle fiber distribution as compared to the untreated control; however ISIS 486178, an ASO with cEt modifications, demonstrated muscle fiber distribution that was more consistent with the muscle fiber distribution found in the wild-type mice.

**Table 24**

| Effect of ASO treatment on muscle fiber distribution in DMSXL mice targeting hDMPK | | | |
|---|---|---|---|
| Treatment group | Fiber Type Distribution in TA muscle | | |
| | Fiber 1 | Fiber 2a | Fiber 2c |
| Untreated control | 4% | 25% | 5.90% |
| ASO 486178 | 3.10% | 15% | 0.70% |
| ASO 445569 | 4% | 21% | 2% |
| Wild type (WT) | 3.30% | 15% | 0.00% |

### Example 16: Dose-dependent antisense inhibition of hDMPK in DMSXL transgenic mice

The newly designed ASOs from Table 1, above, were further evaluated in a dose-response study for antisense inhibition of hDMPK transcript *in vivo.* ISIS 445569 was included in the study for comparison.

### Treatment

DMSXL mice were maintained on a 12-hour light/dark cycle and fed *ad libitum* normal Purina mouse chow. Animals were acclimated for at least 7 days in the research facility before initiation of the experiment. Antisense oligonucleotides (ASOs) were prepared in PBS and sterilized by filtering through a 0.2 micron filter. ASOs were dissolved in 0.9% PBS for injection.

DMSXL mice received subcutaneous injections of PBS or ASOs from Table 1, above, targeting hDMPK. The ASO was dosed twice per week for 4 weeks at the indicated doses in Table 25, below. The control group received subcutaneous injections of PBS twice weekly for 4 weeks. Each treatment group consisted of 4 animals.

### Inhibition of hDMPK mRNA levels

Forty eight hours after the final dose, the mice were sacrificed and tissue from the tibialis anterior muscles, quadriceps muscles (left), gastrocnemius muscles, heart and diaphragm was isolated. mRNA was isolated for real-time PCR analysis of hDMPK and normalized to RIBOGREEN®. Human primer probe set RTS3164 was used to measure mRNA levels. The results summarized in Table 25, below, were independently generated from various dose-response studies. The results are presented as the average percent of hDMPK mRNA expression levels for each treatment group, relative to PBS control.

As presented, treatment with antisense oligonucleotides reduced hDMPK transcript expression in a dose-dependent manner.

**Table 25**

| Dose-dependent inhibition of hDMPK mRNA levels in DMSXL mice | | | | | | |
|---|---|---|---|---|---|---|
| ISIS No. | mg/kg/wk | hDMPK mRNA levels (% PBS) | | | | |
| | | TA | Quad (Left) | Gastroc | Heart | Diaphragm |
| PBS | 0 | 100 | 100 | 100 | 100 | 100 |
| 445569 | 50 | 54.7 | 80.3 | 97.1 | 55.4 | 21.7 |
| | 100 | 28.3 | 42.1 | 71.3 | 48.9 | 19.7 |
| | 200 | 22.2 | 33.9 | 45.2 | 34.2 | 10.0 |
| 512497 | 50 | 23.8 | 48.9 | 52.9 | 44.4 | 35.0 |
| | 100 | 9.7 | 28.7 | 24.8 | 43.8 | 24.2 |
| | 200 | 11.4 | 22.4 | 16.4 | 42.0 | 15.2 |
| 486178 | 25 | 59.1 | 56.1 | 63.1 | 75.3 | 39.1 |
| | 50 | 33.8 | 61.9 | 58.7 | 59.2 | 32.5 |
| | 100 | 36.6 | 65.8 | 51.6 | 47.3 | 26.2 |
| 570808 | 25 | 26.3 | 41.1 | 39.8 | 44.9 | 17.3 |
| | 50 | 12.2 | 13.0 | 36.3 | 18.4 | 8.1 |
| | 100 | 6.1 | 5.4 | 7.9 | 10.2 | 3.0 |
| 594292 | 25 | 48.8 | 32.2 | 68.8 | 70.6 | 72.7 |
| | 50 | 32.0 | 30.4 | 41.1 | 85.1 | 48.3 |
| | 100 | 31.6 | 39.6 | 53.3 | 63.9 | 40.2 |
| 598768 | 25 | 16.9 | 27.1 | 27.5 | 56.3 | 26.9 |
| | 50 | 10.2 | 33.6 | 24.1 | 30.8 | 20.2 |
| | 100 | 6.8 | 22.0 | 25.5 | 22.6 | 13.1 |
| 598769 | 25 | 21.6 | 50.8 | 48.1 | 61.0 | 30.3 |
| | 50 | 12.7 | 25.1 | 42.3 | 36.4 | 16.7 |
| | 100 | 12.8 | 18.4 | 33.2 | 32.0 | 20.2 |
| 569473 | 25 | 42.0 | 21.8 | 48.9 | 51.8 | 34.8 |
| | 50 | 41.6 | 16.2 | 47.6 | 55.6 | 23.6 |
| | 100 | 31.9 | 19.2 | 31.9 | 35.6 | 20.5 |
| 594300 | 25 | 114.5 | 56.7 | 96.2 | 91.0 | 62.6 |
| | 50 | 44.3 | 22.3 | 52.8 | 69.3 | 54.7 |
| | 100 | 73.0 | 22.6 | 56.6 | 78.3 | 44.5 |
| 598777 | 25 | 49.4 | 28.8 | 76.1 | 97.1 | 58.7 |
| | 50 | 44.8 | 13.6 | 36.5 | 87.4 | 40.8 |
| | 100 | 31.8 | 10.1 | 22.5 | 86.8 | 33.6 |

| | | | | | | |
|---|---|---|---|---|---|---|
| TA = Tibialis Anterior; Quad = Quadriceps; Gastroc = Gastrocnemius | | | | | | |

### Example 17: Six Week in vivo Tolerability Study in CD-1 Mice

The newly designed ASOs from Table 1, above, were further evaluated in a 6 week study to assess plasma chemistry, body/organ weights and histology. Groups of CD-1 mice were administered 100 mg/kg/wk of ISIS 445569 or ISIS 512497. Further groups of CD-1 mice were administered 50 mg/kg/wk of ISIS 486178, ISIS 570808, ISIS 594292, ISIS 598768, ISIS 598769, ISIS 569473, ISIS 594300, and ISIS 598777. After six weeks and two days after each group of mice received the last dose, the mice were sacrificed and tissues were collected for analysis. For each group of mice, analysis to measure alanine transaminase levels, aspartate aminotransferase levels, blood urea nitrogen (BUN) levels, albumin levels, total bilirubin, and creatine levels was measured. Additionally, organ weights were also measured, the results of which are presented in the tables below.

**Table 26**

| Plasma Chemistry in CD-1 mice | | | | | | |
|---|---|---|---|---|---|---|
| **ISIS No.** | **ALT (U/L)** | **AST (U/L)** | **BUN (mg/dL)** | **Albumin (g/dL)** | **T. Bil (mg/dL)** | **Creatinine (mg/dL)** |
| PBS | 31.75 | 60.75 | 32.73 | 2.99 | 0.23 | 0.16 |
| 486178 | 65.00 | 103.00 | 27.18 | 2.90 | 0.19 | 0.13 |
| 445569 | 162.75 | 195.25 | 29.70 | 3.38 | 0.26 | 0.14 |
| 570808 | 313.50 | 332.50 | 32.40 | 2.81 | 0.28 | 0.15 |
| 594292 | 58.75 | 133.00 | 28.15 | 2.94 | 0.21 | 0.13 |
| 598768 | 45.50 | 92.00 | 26.85 | 2.90 | 0.21 | 0.11 |
| 598769 | 69.25 | 94.25 | 32.73 | 2.89 | 0.18 | 0.13 |
| 512497 | 101.25 | 144.50 | 26.90 | 2.90 | 0.19 | 0.12 |
| 569473 | 75.75 | 137.00 | 28.98 | 3.05 | 0.26 | 0.13 |
| 594300 | 46.00 | 76.75 | 24.70 | 2.94 | 0.18 | 0.11 |
| 598777 | 186.50 | 224.25 | 24.68 | 2.97 | 0.30 | 0.11 |

**Table 27**

| Body & Organ Weights in CD-1 mice | | | |
|---|---|---|---|
| **ISIS No.** | ***Kidney % BW** | ***Liver % BW** | ***Spleen % BW** |
| PBS | 1.00 | 1.00 | 1.00 |
| 486178 | 1.05 | 1.05 | 1.03 |
| 445569 | 1.07 | 1.09 | 1.23 |
| 570808 | 0.94 | 1.27 | 1.43 |
| 594292 | 1.03 | 1.03 | 1.16 |
| 598768 | 1.14 | 1.08 | 0.97 |
| 598769 | 0.97 | 1.05 | 1.04 |
| 512497 | 0.99 | 1.17 | 1.38 |
| 569473 | 1.02 | 1.01 | 1.09 |
| 594300 | 1.14 | 1.07 | 1.02 |
| 598777 | 1.05 | 1.20 | 1.01 |

| | | | |
|---|---|---|---|
| * Fold change over Saline control group | | | |

### Example 18: Six Week in vivo Tolerability Study in Sprague-Dawley Rats

The newly designed ASOs from Table 1, above, were further evaluated in a 6 week study to assess plasma chemistry, body/organ weights and histology. Groups of Sprague-Dawley rats were administered 100 mpk/wk of ISIS 445569 or ISIS 512497. Further groups of Groups of Sprague-Dawley rats were administered 50 mpk/wk of ISIS 486178, ISIS 570808, ISIS 594292, ISIS 598768, ISIS 598769, ISIS 569473, ISIS 594300, and ISIS 598777. After six weeks and two days after each group of mice received the last dose, the mice were sacrificed and tissues were collected for analysis. For each group of mice, analysis to measure alanine transaminase levels, aspartate aminotransferase levels, blood urea nitrogen (BUN) levels, albumin levels, total bilirubin, creatine levels, and urinary creatine levels was measured. Additionally, organ weights were also measured, the results of which are presented in the tables below.

**Table 28**

| Plasma Chemistry & Urine Analysis in Sprague-Dawley Rats | | | | | | | |
|---|---|---|---|---|---|---|---|
| **ISIS No.** | **ALT (U/L)** | **AST (U/L)** | **BUN (mg/dl)** | **Total protein (mg/dl)** | **T.Bil (mg/dl)** | **Creatinine (mg/dl)** | **Urine MTP/Creatine** |
| **Saline** | 59.25 | 100.35 | 18.05 | 3.47 | 0.158 | 0.30 | 1.09 |
| 569473 | 101 | 198.25 | 25.9 | 2.74 | 0.195 | 0.4025 | 4.59 |
| 512497 | 211 | 240.25 | 19.32 | 3.58 | 0.17 | 0.39 | 6.18 |
| 598768 | 78.2 | 103.5 | 20.6 | 3.36 | 0.14 | 0.38 | 3.85 |
| 598769 | 84.5 | 104.5 | 18.6 | 3.52 | 0.15 | 0.34 | 3.02 |
| 570808 | 82 | 141 | 23.8 | 3.08 | 0.21 | 0.4 | 2.71 |
| 598777 | 109 | 119.5 | 21.65 | 3.79 | 0.22 | 0.37 | 2.56 |
| 445569 | 117.5 | 163.2 | 22.45 | 3.86 | 0.18 | 0.47 | 6.4 |
| 594300 | 66 | 80.75 | 17.53 | 3.59 | 0.12 | 0.29 | 4.72 |
| 486178 | 56.8 | 80.75 | 23.3 | 5.28 | 0.08 | 3.0 | 4.5 |
| 594292 | 64.5 | 80.5 | 19.62 | 3.38 | 0.098 | 0.29 | 5.17 |

**Table 29**

| Plasma Chemistry & Urine Analysis in Sprague-Dawley Rats | | | |
|---|---|---|---|
| **ISIS No.** | **Kidney (fold) *** | **Liver (fold) *** | **Spleen (fold) *** |
| Saline | 1 | 1 | 1 |
| 569473 | 1.46 | 1.20 | 0.82 |
| 512497 | 1.03 | 1.22 | 1.94 |
| 598768 | 0.92 | 0.92 | 1.49 |
| 598769 | 0.93 | 1.04 | 0.98 |
| 570808 | 1.18 | 0.98 | 2.43 |
| 598777 | 1.07 | 0.93 | 2.31 |
| 445569 | 1 | 1.13 | 3.25 |
| 594300 | 1.03 | 1.04 | 1.94 |
| 486178 | 0.87 | 0.89 | 1.45 |
| 594292 | 1.08 | 1.01 | 2.04 |

| | | | |
|---|---|---|---|
| * Fold change over Saline control group | | | |

### Example 19: Thirteen (13) Week in vivo Study in Cynomolgus Monkeys

Groups of 4 cynomolgus male monkeys were administered 40 mg/kg/wk of ISIS 445569, ISIS 512497, ISIS 486178, ISIS 570808, ISIS 594292, ISIS 598768, ISIS 598769, ISIS 569473, ISIS 594300, and ISIS 598777 via subcutaneous injection. Thirteen weeks after the first dose, the animals were sacrificed and tissue analysis was performed. mRNA was isolated for real-time PCR analysis of rhesus monkey DMPK and normalized to RIBOGREEN®. Primer probe set RTS3164 (described above) was used to measure mRNA levels and the results are shown in Table 30 below. Additionally, further mRNA was isolated for real-time PCR analysis of rhesus monkey DMPK and normalized to RIBOGREEN® using primer probe set RTS4447 and the results are shown in Table 31 below. RTS4447 (forward sequence AGCCTGAGCCGGGAGATG, designated herein as SEQ ID NO: 20; reverse sequence GCGTAGTTGACTGGCAAAGTT, designated herein as SEQ ID NO: 21; probe sequence AGGCCATCCGCATGGCCAACC, designated herein as SEQ ID NO: 22).

**Table 30**

| Dose-dependent inhibition of DMPK mRNA levels in Cynomolgus Monkeys using Primer Probe Set RTS3164 | | | | | | | |
|---|---|---|---|---|---|---|---|
| ISIS No. | mg/kg/wk | hDMPK mRNA levels (% PBS) | | | | | |
| | | TA | Quad (Left) | Gastroc | Kidney | Heart | Liver |
| PBS | 0 | 100 | 100 | 100 | 100 | 100 | 100 |
| 486178 | 40 | 26.1 | 30.8 | 49.3 | 55.3 | 45.8 | 44.9 |
| 445569 | 40 | 68.5 | 82.2 | 128.9 | 65.6 | 91.2 | 113.5 |
| 512497 | 40 | 60.3 | 58.7 | 66.7 | 61.9 | 74.2 | 68.1 |
| 598768 | 40 | 69.1 | 64.9 | 80.7 | 58.1 | 70.6 | 100.8 |
| 594300 | 40 | 73.6 | 80.2 | 106.0 | 57.9 | 97.5 | 91.6 |
| 594292 | 40 | 55.6 | 52.0 | 71.9 | 46.2 | 72.1 | 81.6 |
| 569473 | 40 | 44.8 | 31.7 | 61.6 | 44.0 | 58.7 | 28.0 |
| 598769 | 40 | 31.7 | 28.9 | 49.7 | 26.8 | 45.0 | 38.6 |
| 570808 | 40 | 2.5 | 4.4 | 6.4 | 29.7 | 17.5 | 7.2 |
| 598777 | 40 | 53.3 | 31.8 | 76.4 | 42.7 | 44.6 | 111.6 |

**Table 31**

| Dose-dependent inhibition of DMPK mRNA levels in Cynomolgus Monkeys using Primer Probe Set RTS4447 | | | | | | | |
|---|---|---|---|---|---|---|---|
| ISIS No. | mg/kg/wk | hDMPK mRNA levels (% PBS) | | | | | |
| | | TA | Quad (Left) | Gastroc | Kidney | Heart | Liver |
| PBS | 0 | 100.0 | 100.0 | 100.0 | 100.0 | 100.0 | 100.0 |
| 486178 | 40 | 26.7 | 29.0 | 32.9 | 57.0 | 49.4 | 58.1 |
| 445569 | 40 | 85.4 | 87.4 | 147.1 | 77.1 | 97.2 | 93.6 |
| 512497 | 40 | 66.4 | 70.4 | 94.2 | 81.9 | 87.6 | 79.5 |
| 598768 | 40 | 48.3 | 76.4 | 106.7 | 73.7 | 81.0 | 85.1 |
| 594300 | 40 | 100.9 | 113.5 | 219.6 | 96.9 | 131.0 | 118.9 |
| 594292 | 40 | 76.5 | 75.7 | 151.7 | 86.6 | 107.1 | 108.6 |
| 569473 | 40 | 52.6 | 51.7 | 114.2 | 72.9 | 87.2 | 53.7 |
| 598769 | 40 | 45.2 | 57.6 | 86.3 | 56.6 | 65.4 | 72.5 |
| 570808 | 40 | 6.6 | 8.3 | 14.8 | 60.7 | 27.9 | 35.0 |
| 598777 | 40 | 55.1 | 56.8 | 124.1 | 78.6 | 88.9 | 131.2 |

### Example 20: Thirteen (13) Week in vivo Tolerability Study in Cynomolgus Monkeys

Groups of cynomolgus male monkeys were administered 40 mg/kg of ISIS 445569, ISIS 512497, ISIS 486178, ISIS 570808, ISIS 594292, ISIS 598768, ISIS 598769, ISIS 569473, ISIS 594300, and ISIS 598777 via subcutaneous injection on days 1, 3, 5, and 7. Following administration on day 7, each monkey was administered 40 mg/kg/wk of ISIS 445569, ISIS 512497, ISIS 486178, ISIS 570808, ISIS 594292, ISIS 598768, ISIS 598769, ISIS 569473, ISIS 594300, and ISIS 598777 via subcutaneous injection.

48 hours after each monkey received a subcutaneous dose on days 28 and 91, blood and urine samples were taken for analysis. Some of the monkeys had blood and urine taken 48 hours after the dose given on day 56. Alanine aminotransferase (ALT), aspartate aminotransferase (AST), lactate dehydrogenase (LDH), and creatine kinase (CK) were measured for each animal in a treatment group and the average values are presented in the table below. Day of Sample values with a negative represent time point before treatment began. For example, a Day of Treatment value of -7 represents a sample taken 7 days before the first dose. Thirteen weeks after the first dose, the animals were sacrificed and tissue analysis was performed.

**Table 32**

| Plasma Chemistry & Urine Analysis in Cynomolgus Monkeys | | | | | |
|---|---|---|---|---|---|
| **ISIS No.** | **Day of Sample** | **ALT (U/L)** | **AST (U/L)** | **LDH (mg/dl)** | **CK (mg/dl)** |
| Saline | -14 | 34.2 | 25.9 | 604.0 | 160.8 |
| | -7 | 38.8 | 27.8 | 861.3 | 249.0 |
| | 30 | 43.0 | 34.4 | 1029.0 | 300.0 |
| | 93 | 66.1 | 43.0 | 1257.3 | 898.8 |
| 486178 | -14 | 37.6 | 40.5 | 670.0 | 236.8 |
| | -7 | 49.8 | 55.0 | 1039.8 | 380.8 |
| | 30 | 47.0 | 41.2 | 875.4 | 415.0 |
| | 93 | 59.7 | 43.6 | 960.6 | 809.6 |
| 594292 | -14 | 38.9 | 32.0 | 776.3 | 375.8 |
| | -7 | 37.8 | 38.4 | 877.3 | 210.0 |
| | 30 | 35.4 | 39.6 | 666.0 | 93.8 |
| | 93 | 49.8 | 46.3 | 958.5 | 339.0 |
| 569473 | -14 | 49.4 | 49.8 | 1185.3 | 365.3 |
| | -7 | 50.4 | 59.7 | 1609.5 | 261.0 |
| | 30 | 46.7 | 52.5 | 1390.8 | 107.8 |
| | 93 | 56.3 | 49.8 | 1483.3 | 524.5 |
| 570808 | -14 | 47.1 | 46.8 | 896.0 | 448.3 |
| | -7 | 44.4 | 63.6 | 913.3 | 257.3 |
| | 30 | 47.1 | 57.7 | 660.5 | 125.0 |
| | 93 | 79.8 | 92.2 | 813.5 | 294.0 |
| 598768 | -14 | 37.9 | 41.6 | 666.3 | 253.8 |
| | -7 | 41.4 | 53.5 | 754.0 | 231.5 |
| | 30 | 37.2 | 38.9 | 652.3 | 106.3 |
| | 93 | 45.8 | 41.5 | 721.3 | 238.3 |
| 598769 | -14 | 44.2 | 36.1 | 1106.8 | 456.8 |
| | -7 | 45.7 | 41.5 | 1323.3 | 214.0 |
| | 30 | 40.3 | 42.0 | 981.0 | 147.8 |
| | 58 | 56.7 | 49.9 | 1101.5 | 552.3 |
| | 93 | 69.0 | 50.3 | 1167.3 | 749.5 |
| 512497 | -14 | 31.5 | 34.3 | 689.3 | 293.8 |
| | -7 | 39.0 | 45.4 | 1110.3 | 286.0 |
| | 30 | 47.2 | 60.2 | 960.5 | 202.5 |
| | 93 | 69.6 | 87.1 | 997.0 | 1118.5 |
| 594300 | -14 | 42.0 | 34.0 | 935.5 | 459.5 |
| | -7 | 42.1 | 53.6 | 1020.5 | 272.0 |
| | 30 | 28.0 | 34.6 | 620.8 | 124.5 |
| | 58 | 42.9 | 48.5 | 883.5 | 169.8 |
| | 93 | 45.7 | 45.7 | 835.5 | 252.3 |
| 598777 | -14 | 45.6 | 37.7 | 707.0 | 558.5 |
| | -7 | 43.3 | 50.0 | 705.8 | 200.3 |
| | 30 | 50.2 | 47.3 | 585.3 | 159.3 |
| | 93 | 79.2 | 56.1 | 1029.0 | 785.0 |
| 445569 | -14 | 40.2 | 44.2 | 835.8 | 404.0 |
| | -7 | 41.0 | 46.1 | 1074.3 | 305.5 |
| | 30 | 45.9 | 61.7 | 994.8 | 283.0 |
| | 58 | 51.6 | 85.1 | 739.0 | 117.8 |
| | 93 | 99.3 | 97.5 | 1583.5 | 2114.0 |

## Claims

1. A compound comprising a modified oligonucleotide consisting of 10-30 linked nucleosides and having a nucleobase sequence comprising a portion of at least 8 contiguous nucleobases 100% complementary to an equal length portion of nucleobases 1343-1368 of SEQ ID NO: 1 and/or nucleobases 8603-8619 of SEQ ID NO: 2, wherein the nucleobase sequence of the modified oligonucleotide is at least 90% complementary to SEQ ID NO: 1 and/or SEQ ID NO: 2 as measured over the entirety of the modified oligonucleotide and wherein the compound is capable of inhibiting DMPK expression.

2. A compound comprising a modified oligonucleotide consisting of 10-30 linked nucleosides wherein the modified oligonucleotide has a nucleobase sequence comprising a portion of at least 8 contiguous nucleobases of any of SEQ ID NOs: 30, 25, 26, 123, 124, 125, 126, 127, 128, 378, 379, 380, 381, 382, 383 and 384, wherein the nucleobase sequence of the modified oligonucleotide is at least 90% complementary to SEQ ID NO: 1 and/or SEQ ID NO: 2 as measured over the entirety of the modified oligonucleotide and wherein the compound is capable of inhibiting DMPK expression.

3. The compound of claim 1 or claim 2, wherein the modified oligonucleotide is a single-stranded oligonucleotide.

4. The compound of any one of claims 1-3, wherein at least one nucleoside comprises a modified nucleobase, wherein optionally the modified nucleobase is a 5-methylcytosine.

5. The compound of any one of claims 1-4, wherein at least one internucleoside linkage is a modified internucleoside linkage, wherein optionally each internucleoside linkage is a phosphorothioate internucleoside linkage.

6. The compound of any one of claims 1-5, wherein at least one nucleoside comprises a modified sugar.

7. The compound of claim 6, wherein at least two nucleosides comprise a modified sugar, wherein optionally each of the modified sugars have the same modification, or at least one of the modified sugars has a different modification.

8. The compound of claim 6 or claim 7, wherein the modified sugar is a bicyclic sugar, wherein optionally the bicyclic sugar is selected from among cEt, LNA, α-L-LNA, ENA, and 2'-thio LNA.

9. The compound of claim 6, wherein the at least one nucleoside comprising a modified sugar is a 2'-substituted nucleoside, wherein optionally the 2'-substituted nucleoside is selected from among: 2'-OCH3, 2'-F, and 2'-O-methoxyethyl.

10. The compound of any one of claims 1-9, wherein
(a) the modified oligonucleotide comprises:
i. a gap segment consisting of linked deoxynucleosides;
ii. a 5' wing segment consisting of liked nucleosides;
iii. a 3' wing segment consisting of linked nucleosides;
iv. wherein the gap segment is positioned between the 5' wing segment and the 3' wing segment and wherein each nucleoside of each wing segment comprises a modified sugar; and/or
(b) the modified oligonucleotide consists of 16, 17, 18, 19, or 20 linked nucleosides.

11. A modified oligonucleotide having a nucleobase sequence as set forth in SEQ ID NO: 30, wherein the modified oligonucleotide consists of 16 linked nucleosides and comprises:
a. a gap segment consisting of eight linked deoxynucleosides;
b. a 5' wing segment consisting of four linked nucleosides and having a E-E-K-K 5'-wing motif;
c. a 3' wing segment consisting of four linked nucleosides and having a K-K-E-E 3'-wing motif;
d. wherein the gap segment is positioned between the 5' wing segment and the 3' wing segment;
e. wherein each E represents 2'-O-methoxyethyl sugar and each K represents a cEt sugar;
f. wherein each internucleoside linkage is a phosphorothioate internucleoside linkage; and
g. wherein each cytosine residue is a 5-methyl cytosine.

12. The compound of claim 2, wherein
(a) the compound has a nucleobase sequence as set forth in SEQ ID NO: 25, wherein the modified oligonucleotide consists of 20 linked nucleosides and comprises:
v. a gap segment consisting of ten linked deoxynucleosides;
vi. a 5' wing segment consisting of five linked nucleosides;
vii. a 3' wing segment consisting of five linked nucleosides;
viii. wherein the gap segment is positioned between the 5' wing segment and the 3' wing segment;
ix. wherein each nucleoside of each wing segment comprises a 2'-O-methoxyethyl sugar;
x. wherein each internucleoside linkage is a phosphorothioate internucleoside linkage; and
xi. wherein each cytosine residue is a 5-methylcytosine; or
(b) the compound has a nucleobase sequence as set forth in SEQ ID NO: 26, wherein the modified oligonucleotide consists of 16 linked nucleosides and comprises:
i. a gap segment consisting of eight linked deoxynucleosides;
ii. a 5' wing segment consisting of four linked nucleosides and having a E-E-K-K 5'-wing motif;
iii. a 3' wing segment consisting of four linked nucleosides and having a K-K-E-E 3'-wing motif;
iv. wherein the gap segment is positioned between the 5' wing segment and the 3' wing segment;
v. wherein each E represents 2'-O-methoxyethyl sugar and each K represents a cEt sugar;
vi. wherein each internucleoside linkage is a phosphorothioate internucleoside linkage; and
vii. wherein each cytosine residue is a 5-methylcytosine.

13. The compound of any one of claims 1-12, wherein the compound is a conjugated compound.

14. A composition comprising the compound of any one of claims 1-12 or a salt thereof and a pharmaceutically acceptable carrier or diluent.

15. A compound of any one of claims 1-13 or a composition of claim 14 for use in a method of treating type 1 myotonic dystrophy in an animal.

## Patentansprüche

1. Verbindung, umfassend ein modifiziertes Oligonukleotid, das aus 10-30 verknüpften Nukleosiden besteht und eine Nukleobasensequenz hat, die einen Abschnitt von mindestens 8 aufeinanderfolgenden Nukleobasen umfasst, die zu 100 % komplementär zu einem Abschnitt gleicher Länge der Nukleobasen 1343-1368 von SEQ ID NO: 1 und/oder der Nukleobasen 8603-8619 von SEQ ID NO: 2 sind, wobei die Nukleobasensequenz des modifizierten Oligonukleotids, über das gesamte modifizierte Oligonukleotid gemessen, mindestens 90 % komplementär zu SEQ ID NO: 1 und/oder SEQ ID NO: 2 ist und wobei die Verbindung in der Lage ist, die DMPK-Expression zu hemmen.

2. Verbindung, umfassend ein modifiziertes Oligonukleotid, das aus 10-30 verknüpften Nukleosiden besteht, wobei das modifizierte Oligonukleotid eine Nukleobasensequenz hat, die einen Abschnitt von mindestens 8 aufeinanderfolgenden Nukleobasen von einer der SEQ ID NO: 30, 25, 26, 123, 124, 125, 126, 127, 128, 378, 379, 380, 381, 382, 383 und 384 umfasst, wobei die Nukleobasensequenz des modifizierten Oligonukleotids, über das gesamte modifizierte Oligonukleotid gemessen, mindestens 90 % komplementär zu SEQ ID NO: 1 und/oder SEQ ID NO: 2 ist und wobei die Verbindung in der Lage ist, die DMPK-Expression zu hemmen.

3. Verbindung nach Anspruch 1 oder Anspruch 2, wobei das modifizierte Oligonukleotid ein einzelsträngiges Oligonukleotid ist.

4. Verbindung nach einem der Ansprüche 1-3, wobei mindestens ein Nukleosid eine modifizierte Nukleobase umfasst, wobei optional die modifizierte Nukleobase ein 5-Methylcytosin ist.

5. Verbindung nach einem der Ansprüche 1-4, wobei mindestens eine Internukleosidbindung eine modifizierte Internukleosidbindung ist, wobei optional jede Internukleosidbindung eine Phosphorthioat-Internukleosidbindung ist.

6. Verbindung nach einem der Ansprüche 1-5, wobei mindestens ein Nukleosid einen modifizierten Zucker umfasst.

7. Verbindung nach Anspruch 6, wobei mindestens zwei Nukleoside einen modifizierten Zucker umfassen, wobei optional jeder der modifizierten Zucker dieselbe Modifikation aufweist oder mindestens einer der modifizierten Zucker eine andere Modifikation aufweist.

8. Verbindung nach Anspruch 6 oder Anspruch 7, wobei der modifizierte Zucker ein bizyklischer Zucker ist, wobei optional der bizyklische Zucker aus cEt, LNA, α-L-LNA, ENA und 2'-Thio-LNA ausgewählt ist.

9. Verbindung nach Anspruch 6, wobei das mindestens eine Nukleosid, das einen modifizierten Zucker umfasst, ein 2'-substituiertes Nukleosid ist, wobei optional das 2'-substituierte Nukleosid aus den Folgenden ausgewählt ist: 2'-OCH3, 2'-F und 2'-O-Methoxyethyl.

10. Verbindung nach einem der Ansprüche 1-9, wobei
(a) das modifizierte Oligonukleotid Folgendes umfasst:
i. ein Lücken (Gap)-Segment, das aus verknüpften Desoxynukleosiden besteht,
ii. ein 5'-Flügel(Wing)-Segment, das aus verknüpften Nukleosiden besteht,
iii. ein 3'-Flügel(Wing)-Segment, das aus verknüpften Nukleosiden besteht,
iv. wobei das Lücken-Segment zwischen dem 5'-Flügel-Segment und dem 3'-Flügel-Segment positioniert ist und wobei jedes Nukleosid jedes Flügel-Segments einen modifizierten Zucker umfasst, und/oder
(b) das modifizierte Oligonukleotid aus 16, 17, 18, 19 oder 20 verknüpften Nukleosiden besteht.

11. Modifiziertes Oligonukleotid mit einer Nukleobasensequenz wie in SEQ ID NO: 30 dargestellt, wobei das modifizierte Oligonukleotid aus 16 verknüpften Nukleosiden besteht und Folgendes umfasst:
a. ein Lücken-Segment, das aus acht verknüpften Desoxynukleosiden besteht,
b. ein 5'-Flügel-Segment, das aus vier verknüpften Nukleosiden besteht und ein E-E-K-5'-Flügelmotiv aufweist,
c. ein 3'-Flügel-Segment, das aus vier verknüpften Nukleosiden besteht und ein K-K-E-E-3'-Flügelmotiv aufweist,
d. wobei das Lücken-Segment zwischen dem 5'-Flügel-Segment und dem 3'-Flügel-Segment positioniert ist,
e. wobei jedes E einen 2'-O-Methoxyethylzucker darstellt und jedes K einen cEt-Zucker darstellt,
f. wobei jede Internukleosidbindung eine Phosphorthioat-Internukleosidbindung ist und
g. wobei jeder Cytosinrest ein 5-Methylcytosin ist.

12. Verbindung nach Anspruch 2, wobei
(a) die Verbindung eine Nukleobasensequenz wie in SEQ ID NO: 25 dargestellt besitzt, wobei das modifizierte Oligonukleotid aus 20 verknüpften Nukleosiden besteht und Folgendes umfasst:
v. ein Lücken-Segment, das aus zehn verknüpften Desoxynukleosiden besteht,
vi. ein 5'-Flügel-Segment, das aus fünf verknüpften Nukleosiden besteht,
vii. ein 3'-Flügel-Segment, das aus fünf verknüpften Nukleosiden besteht,
viii. wobei das Lücken-Segment zwischen dem 5'-Flügel-Segment und dem 3'-Flügel-Segment positioniert ist,
ix. wobei jedes Nukleosid jedes Flügel-Segments einen 2'-O-Methoxyethylzucker umfasst,
x. wobei jede Internukleosidbindung eine Phosphorthioat-Internukleosidbindung ist und
xi. wobei jeder Cytosinrest ein 5-Methylcytosin ist, oder
(b) die Verbindung eine Nukleobasensequenz wie in SEQ ID NO: 26 dargestellt besitzt, wobei das modifizierte Oligonukleotid aus 16 verknüpften Nukleosiden besteht und Folgendes umfasst:
i. ein Lücken-Segment, das aus acht verknüpften Desoxynukleosiden besteht,
ii. ein 5'-Flügel-Segment, das aus vier verknüpften Nukleosiden besteht und ein E-E-K-5'-Flügelmotiv aufweist,
iii. ein 3'-Flügel-Segment, das aus vier verknüpften Nukleosiden besteht und ein K-K-E-E-3'-Flügelmotiv aufweist,
iv. wobei das Lücken-Segment zwischen dem 5'-Flügel-Segment und dem 3'-Flügel-Segment positioniert ist,
v. wobei jedes E einen 2'-O-Methoxyethylzucker darstellt und jedes K einen cEt-Zucker darstellt,
vi. wobei jede Internukleosidbindung eine Phosphorthioat-Internukleosidbindung ist und
vii. wobei jeder Cytosinrest ein 5-Methylcytosin ist.

13. Verbindung nach einem der Ansprüche 1-12, wobei die Verbindung eine konjugierte Verbindung ist.

14. Zusammensetzung, umfassend die Verbindung nach einem der Ansprüche 1-12 oder ein Salz davon und einen pharmazeutisch verträglichen Träger oder ein pharmazeutisch verträgliches Verdünnungsmittel.

15. Verbindung nach einem der Ansprüche 1-13 oder Zusammensetzung nach Anspruch 14 zur Verwendung bei einem Verfahren zur Behandlung von myotoner Dystrophie Typ 1 bei einem Tier.

## Revendications

1. Composé comprenant un oligonucléotide modifié constitué par 10-30 nucléosides liés et ayant une séquence de nucléobases comprenant une partie d'au moins 8 nucléobases contigües étant 100% complémentaires à une partie de longueur égale des nucléobases 1343-1368 de la SEQ ID n° : 1 et/ou des nucléobases 8603-8619 de la SEQ ID n° : 2, dans lequel la séquence de nucléobases de l'oligonucléotide modifié est complémentaire au moins à 90% à la SEQ ID n° : 1 et/ou la SEQ ID n° : 2, tel que mesuré sur la longueur totale de l'oligonucléotide modifié, et le composé étant capable d'inhiber l'expression d'une DMPK.

2. Composé comprenant un oligonucléotide modifié constitué par 10-30 nucléosides, dans lequel l'oligonucléotide modifié a une séquence de nucléobases comprenant une partie d'au moins 8 nucléobases contigües de l'une quelconque des SEQ ID n° : 30, 25, 26, 123, 124, 125, 126, 127, 128, 378, 379, 380, 381, 382, 383 et 384, dans lequel la séquence de nucléobases de l'oligonucléotide modifié est complémentaire au moins à 90% à la SEQ ID n° : 1 et/ou la SEQ ID n° : 2, tel que mesuré sur la longueur totale de l'oligonucléotide modifié, et le composé étant capable d'inhiber l'expression d'une DMPK.

3. Composé de la revendication 1 ou la revendication 2, dans lequel l'oligonucléotide modifié est un oligonucléotide en simple brin.

4. Composé de l'une quelconque des revendications 1 à 3, dans lequel au moins un nucléoside comprend une nucléobase modifiée, dans lequel en option la nucléobase modifiée est une 5-méthylcytosine.

5. Composé de l'une quelconque des revendications 1 à 4, dans lequel au moins une liaison inter-nucléosidique est une liaison inter-nucléosidique modifiée, dans lequel en option chaque liaison inter-nucléosidique est une liaison inter-nucléosidique de phosphorothioate.

6. Composé de l'une quelconque des revendications 1 à 5, dans lequel au moins un nucléoside comprend un sucre modifié.

7. Composé de la revendication 6, dans lequel au moins deux nucléosides comprennent un sucre modifié, dans lequel en option chacun des sucres modifiés a la même modification ou au moins l'un des sucres modifiés a une modification différente.

8. Composé de la revendication 6 ou la revendication 7, dans lequel le sucre modifié est un sucre bicyclique, dans lequel en option le sucre bicyclique est choisi parmi les cEt, LNA, α-L-LNA, ENA et 2'-thio LNA.

9. Composé de la revendication 6, dans lequel l'au moins un nucléoside comprenant un sucre modifié est un nucléoside substitué en 2', dans lequel en option le nucléoside substitué en 2' est choisi parmi les : 2'-OCH3, 2'-F et 2'-O-méthoxyéthyle.

10. Composé de l'une quelconque des revendications 1 à 9, dans lequel
(a) l'oligonucléotide modifié comprend :
i. un segment de brèche constitué par des désoxynucléosides liés ;
ii. un segment d'aile en 5' constitué par des nucléosides liés ;
iii. un segment d'aile en 3' constitué par des nucléosides liés ;
iv. dans lequel le segment de brèche est positionné entre le segment d'aile en 5' et le segment d'aile en 3', et dans lequel chaque nucléoside de chaque segment d'aile comprend un sucre modifié ; et/ou
(b) l'oligonucléotide modifié est constitué par 16, 17, 18, 19 ou 20 nucléosides liés.

11. Oligonucléotide modifié ayant une séquence de nucléobases telle qu'indiquée dans la SEQ ID n° : 30, l'oligonucléotide modifié étant constitué par 16 nucléosides liés et comprenant :
a. un segment de brèche constitué par huit désoxynucléosides liés ;
b. un segment d'aile en 5' constitué par quatre nucléosides liés et ayant un motif d'aile en 5' E-E-K-K ;
c. un segment d'aile en 3' constitué par quatre nucléosides liés et ayant un motif d'aile en 3' K-K-E-E ;
d. dans lequel le segment de brèche est positionné entre le segment d'aile en 5' et le segment d'aile en 3' ;
e. dans lequel chaque E représente un sucre à 2'-O-méthoxyéthyle et chaque K représente un sucre à cEt ;
f. dans lequel chaque liaison inter-nucléosidique est une liaison inter-nucléosidique de phosphorothioate ; et
g. dans lequel chaque résidu de cytosine est une 5-méthyl cytosine.

12. Composé de la revendication 2, le composé ayant
(a) une séquence de nucléobases telle qu'indiquée dans la SEQ ID n° : 25, dans lequel l'oligonucléotide modifié est constitué par 20 nucléosides liés et comprend :
v. un segment de brèche constitué par dix désoxynucléosides liés ;
vi. un segment d'aile en 5' constitué par cinq nucléosides liés ;
vii. un segment d'aile en 3' constitué par cinq nucléosides liés ;
viii. dans lequel le segment de brèche est positionné entre le segment d'aile en 5' et le segment d'aile en 3' ;
ix. dans lequel chaque nucléoside de chaque segment d'aile comprend un sucre à 2'-O-méthoxyéthyle ;
x. dans lequel chaque liaison inter-nucléosidique est une liaison inter-nucléosidique de phosphorothioate ; et
xi. dans lequel chaque résidu de cytosine est une 5-méthylcytosine ; ou
(b) une séquence de nucléobases telle qu'indiquée dans la SEQ ID n° : 26, dans lequel l'oligonucléotide modifié est constitué par 16 nucléosides liés et comprend :
i. un segment de brèche constitué par huit désoxynucléosides liés ;
ii. un segment d'aile en 5' constitué par quatre nucléosides liés et ayant un motif d'aile en 5' E-E-K-K ;
iii. un segment d'aile en 3' constitué par quatre nucléosides liés et ayant un motif d'aile en 3' K-K-E-E ;
iv. dans lequel le segment de brèche est positionné entre le segment d'aile en 5' et le segment d'aile en 3' ;
v. dans lequel chaque E représente un sucre à 2'-O-méthoxyéthyle et chaque K représente un sucre à cEt ;
vi. dans lequel chaque liaison inter-nucléosidique est une liaison inter-nucléosidique de phosphorothioate ; et
vii. dans lequel chaque résidu de cytosine est une 5-méthylcytosine.

13. Composé de l'une quelconque des revendications 1 à 12, le composé étant un composé conjugué.

14. Composition comprenant le composé de l'une quelconque des revendications 1 à 12, ou un sel de celui-ci, et un véhicule ou un diluant acceptable d'un point de vue pharmaceutique.

15. Composé de l'une quelconque des revendications 1 à 13 ou composition de la revendication 14 destiné(e) à être utilisé(e) dans un procédé de traitement d'une dystrophie myotonique de type 1 chez un animal.


### SEQUENCE LISTING

<110> Isis Pharmaceuticals, Inc.
<120> COMPOUNDS AND METHODS FOR MODULATION OF DYSTROPHIA MYOTONICA-PROTEIN KINASE (DMPK) EXPRESSION
<130> BIOL0171WO
<150> 61/864,439
   <151> 2013-08-09
<150> 61/889,337
   <151> 2013-10-10
<160> 874
<170> PatentIn version 3.5
<210> 1
   <211> 2877
   <212> DNA
   <213> homo sapiens
<220>
   <221> CDS
   <222> (206)..(2080)
<400> 1
   aggggggctg gaccaagggg tggggagaag gggaggaggc ctcggccggc cgcagagaga        60
   agtggccaga gaggcccagg ggacagccag ggacaggcag acatgcagcc agggctccag       120
   ggcctggaca ggggctgcca ggccctgtga caggaggacc ccgagccccc ggcccgggga       180
   ggggccatgg tgctgcctgt ccaac atg tca gcc gag gtg cgg ctg agg cgg        232
                               Met Ser Ala Glu Val Arg Leu Arg Arg
                               1               5
   ctc cag cag ctg gtg ttg gac ccg ggc ttc ctg ggg ctg gag ccc ctg        280
   Leu Gln Gln Leu Val Leu Asp Pro Gly Phe Leu Gly Leu Glu Pro Leu
   10                  15                  20                  25
   ctc gac ctt ctc ctg ggc gtc cac cag gag ctg ggc gcc tcc gaa ctg        328
   Leu Asp Leu Leu Leu Gly Val His Gln Glu Leu Gly Ala Ser Glu Leu
                   30                  35                  40
   gcc cag gac aag tac gtg gcc gac ttc ttg cag tgg gcg gag ccc atc        376
   Ala Gln Asp Lys Tyr Val Ala Asp Phe Leu Gln Trp Ala Glu Pro Ile
               45                  50                  55
   gtg gtg agg ctt aag gag gtc cga ctg cag agg gac gac ttc gag att        424
   Val Val Arg Leu Lys Glu Val Arg Leu Gln Arg Asp Asp Phe Glu Ile
           60                  65                  70
   ctg aag gtg atc gga cgc ggg gcg ttc agc gag gta gcg gta gtg aag        472
   Leu Lys Val Ile Gly Arg Gly Ala Phe Ser Glu Val Ala Val Val Lys
       75                  80                  85
   atg aag cag acg ggc cag gtg tat gcc atg aag atc atg aac aag tgg        520
   Met Lys Gln Thr Gly Gln Val Tyr Ala Met Lys Ile Met Asn Lys Trp
   90                  95                  100                 105
   gac atg ctg aag agg ggc gag gtg tcg tgc ttc cgt gag gag agg gac         568
   Asp Met Leu Lys Arg Gly Glu Val Ser Cys Phe Arg Glu Glu Arg Asp
                   110                 115                 120
   gtg ttg gtg aat ggg gac cgg cgg tgg atc acg cag ctg cac ttc gcc         616
   Val Leu Val Asn Gly Asp Arg Arg Trp Ile Thr Gln Leu His Phe Ala
               125                 130                 135
   ttc cag gat gag aac tac ctg tac ctg gtc atg gag tat tac gtg ggc         664
   Phe Gln Asp Glu Asn Tyr Leu Tyr Leu Val Met Glu Tyr Tyr Val Gly
           140                 145                 150
   ggg gac ctg ctg aca ctg ctg agc aag ttt ggg gag cgg att ccg gcc         712
   Gly Asp Leu Leu Thr Leu Leu Ser Lys Phe Gly Glu Arg Ile Pro Ala
       155                 160                 165
   gag atg gcg cgc ttc tac ctg gcg gag att gtc atg gcc ata gac tcg         760
   Glu Met Ala Arg Phe Tyr Leu Ala Glu Ile Val Met Ala Ile Asp Ser
   170                 175                 180                 185
   gtg cac cgg ctt ggc tac gtg cac agg gac atc aaa ccc gac aac atc         808
   Val His Arg Leu Gly Tyr Val His Arg Asp Ile Lys Pro Asp Asn Ile
                   190                 195                 200
   ctg ctg gac cgc tgt ggc cac atc cgc ctg gcc gac ttc ggc tct tgc         856
   Leu Leu Asp Arg Cys Gly His Ile Arg Leu Ala Asp Phe Gly Ser Cys
               205                 210                 215
   ctc aag ctg cgg gca gat gga acg gtg cgg tcg ctg gtg gct gtg ggc         904
   Leu Lys Leu Arg Ala Asp Gly Thr Val Arg Ser Leu Val Ala Val Gly
           220                 225                 230
   acc cca gac tac ctg tcc ccc gag atc ctg cag gct gtg ggc ggt ggg         952
   Thr Pro Asp Tyr Leu Ser Pro Glu Ile Leu Gln Ala Val Gly Gly Gly
       235                 240                 245
   cct ggg aca ggc agc tac ggg ccc gag tgt gac tgg tgg gcg ctg ggt        1000
   Pro Gly Thr Gly Ser Tyr Gly Pro Glu Cys Asp Trp Trp Ala Leu Gly
   250                 255                 260                 265
   gta ttc gcc tat gaa atg ttc tat ggg cag acg ccc ttc tac gcg gat        1048
   Val Phe Ala Tyr Glu Met Phe Tyr Gly Gln Thr Pro Phe Tyr Ala Asp
                   270                 275                 280
   tcc acg gcg gag acc tat ggc aag atc gtc cac tac aag gag cac ctc        1096
   Ser Thr Ala Glu Thr Tyr Gly Lys Ile Val His Tyr Lys Glu His Leu
               285                 290                 295
   tct ctg ccg ctg gtg gac gaa ggg gtc cct gag gag gct cga gac ttc        1144
   Ser Leu Pro Leu Val Asp Glu Gly Val Pro Glu Glu Ala Arg Asp Phe
           300                 305                 310
   att cag cgg ttg ctg tgt ccc ccg gag aca cgg ctg ggc cgg ggt gga        1192
   Ile Gln Arg Leu Leu Cys Pro Pro Glu Thr Arg Leu Gly Arg Gly Gly
       315                 320                 325
   gca ggc gac ttc cgg aca cat ccc ttc ttc ttt ggc ctc gac tgg gat        1240
   Ala Gly Asp Phe Arg Thr His Pro Phe Phe Phe Gly Leu Asp Trp Asp
   330                 335                 340                 345
   ggt ctc cgg gac agc gtg ccc ccc ttt aca ccg gat ttc gaa ggt gcc        1288
   Gly Leu Arg Asp Ser Val Pro Pro Phe Thr Pro Asp Phe Glu Gly Ala
                   350                 355                 360
   acc gac aca tgc aac ttc gac ttg gtg gag gac ggg ctc act gcc atg      1336
   Thr Asp Thr Cys Asn Phe Asp Leu Val Glu Asp Gly Leu Thr Ala Met
               365                 370                 375
   gag aca ctg tcg gac att cgg gaa ggt gcg ccg cta ggg gtc cac ctg      1384
   Glu Thr Leu Ser Asp Ile Arg Glu Gly Ala Pro Leu Gly Val His Leu
           380                 385                 390
   cct ttt gtg ggc tac tcc tac tcc tgc atg gcc ctc agg gac agt gag      1432
   Pro Phe Val Gly Tyr Ser Tyr Ser Cys Met Ala Leu Arg Asp Ser Glu
       395                 400                 405
   gtc cca ggc ccc aca ccc atg gaa ctg gag gcc gag cag ctg ctt gag      1480
   Val Pro Gly Pro Thr Pro Met Glu Leu Glu Ala Glu Gln Leu Leu Glu
   410                 415                 420                 425
   cca cac gtg caa gcg ccc agc ctg gag ccc tcg gtg tcc cca cag gat      1528
   Pro His Val Gln Ala Pro Ser Leu Glu Pro Ser Val Ser Pro Gln Asp
                   430                 435                 440
   gaa aca gct gaa gtg gca gtt cca gcg gct gtc cct gcg gca gag gct      1576
   Glu Thr Ala Glu Val Ala Val Pro Ala Ala Val Pro Ala Ala Glu Ala
               445                 450                 455
   gag gcc gag gtg acg ctg cgg gag ctc cag gaa gcc ctg gag gag gag      1624
   Glu Ala Glu Val Thr Leu Arg Glu Leu Gln Glu Ala Leu Glu Glu Glu
           460                 465                 470
   gtg ctc acc cgg cag agc ctg agc cgg gag atg gag gcc atc cgc acg      1672
   Val Leu Thr Arg Gln Ser Leu Ser Arg Glu Met Glu Ala Ile Arg Thr
       475                 480                 485
   gac aac cag aac ttc gcc agt caa cta cgc gag gca gag gct cgg aac      1720
   Asp Asn Gln Asn Phe Ala Ser Gln Leu Arg Glu Ala Glu Ala Arg Asn
   490                 495                 500                 505
   cgg gac cta gag gca cac gtc cgg cag ttg cag gag cgg atg gag ttg      1768
   Arg Asp Leu Glu Ala His Val Arg Gln Leu Gln Glu Arg Met Glu Leu
                   510                 515                 520
   ctg cag gca gag gga gcc aca gct gtc acg ggg gtc ccc agt ccc cgg      1816
   Leu Gln Ala Glu Gly Ala Thr Ala Val Thr Gly Val Pro Ser Pro Arg
               525                 530                 535
   gcc acg gat cca cct tcc cat cta gat ggc ccc ccg gcc gtg gct gtg      1864
   Ala Thr Asp Pro Pro Ser His Leu Asp Gly Pro Pro Ala Val Ala Val
           540                 545                 550
   ggc cag tgc ccg ctg gtg ggg cca ggc ccc atg cac cgc cgc cac ctg      1912
   Gly Gln Cys Pro Leu Val Gly Pro Gly Pro Met His Arg Arg His Leu
       555                 560                 565
   ctg ctc cct gcc agg gtc cct agg cct ggc cta tcg gag gcg ctt tcc      1960
   Leu Leu Pro Ala Arg Val Pro Arg Pro Gly Leu Ser Glu Ala Leu Ser
   570                 575                 580                 585
   ctg ctc ctg ttc gcc gtt gtt ctg tct cgt gcc gcc gcc ctg ggc tgc      2008
   Leu Leu Leu Phe Ala Val Val Leu Ser Arg Ala Ala Ala Leu Gly Cys
                   590                 595                 600
   att ggg ttg gtg gcc cac gcc ggc caa ctc acc gca gtc tgg cgc cgc      2056
   Ile Gly Leu Val Ala His Ala Gly Gln Leu Thr Ala Val Trp Arg Arg
                   605                 610                 615
   cca gga gcc gcc cgc gct ccc tga accctagaac tgtcttcgac tccggggccc      2110
   Pro Gly Ala Ala Arg Ala Pro
           620
   cgttggaaga ctgagtgccc ggggcacggc acagaagccg cgcccaccgc ctgccagttc     2170
   acaaccgctc cgagcgtggg tctccgccca gctccagtcc tgtgatccgg gcccgccccc     2230
   tagcggccgg ggagggaggg gccgggtccg cggccggcga acggggctcg aagggtcctt     2290
   gtagccggga atgctgctgc tgctgctgct gctgctgctg ctgctgctgc tgctgctgct     2350
   gctgctgctg ctggggggat cacagaccat ttctttcttt cggccaggct gaggccctga      2410
   cgtggatggg caaactgcag gcctgggaag gcagcaagcc gggccgtccg tgttccatcc     2470
   tccacgcacc cccacctatc gttggttcgc aaagtgcaaa gctttcttgt gcatgacgcc     2530
   ctgctctggg gagcgtctgg cgcgatctct gcctgcttac tcgggaaatt tgcttttgcc     2590
   aaacccgctt tttcggggat cccgcgcccc cctcctcact tgcgctgctc tcggagcccc     2650
   agccggctcc gcccgcttcg gcggtttgga tatttattga cctcgtcctc cgactcgctg     2710
   acaggctaca ggacccccaa caaccccaat ccacgttttg gatgcactga gaccccgaca     2770
   ttcctcggta tttattgtct gtccccacct aggaccccca cccccgaccc tcgcgaataa     2830
   aaggccctcc atctgcccaa aaaaaaaaaa aaaaaaaaaa aaaaaaa                   2877
<210> 2
   <211> 14411
   <212> DNA
   <213> homo sapiens
<400> 2
   ctcccagccc agcgcctccc accccttttc atagcaggaa aagccggagc ccagggaggg        60
   aacggacctg cgagtcacac aactggtgac ccacaccagc ggctggagca ggaccctctt       120
   ggggagaaga gcatcctgcc cgcagccagg gcccctcatc aaagtcctcg gtgtttttta       180
   aattatcaga actgcccagg accacgtttc ccaggccctg cccagctggg actcctcggt       240
   ccttgcctcc tagtttctca ggcctggccc tctcaaggcc caggcacccc aggccggttg       300
   gaggccccga cttccactct ggagaaccgt ccaccctgga aagaagagct cagattcctc       360
   ttggctctcg gagccgcagg gagtgtgtct tcccgcgcca ccctccaccc cccgaaatgt       420
   ttctgtttct aatcccagcc tgggcaggaa tgtggctccc cggccagggg ccaaggagct       480
   attttggggt ctcgtttgcc cagggagggc ttggctccac cactttcctc ccccagcctt       540
   tgggcagcag gtcacccctg ttcaggctct gagggtgccc cctcctggtc ctgtcctcac       600
   caccccttcc ccacctcctg ggaaaaaaaa aaaaaaaaaa aaaaaaagct ggtataaagc      660
   agagagcctg agggctaaat ttaactgtcc gagtcggaat ccatctctga gtcacccaag       720
   aagctgccct ggcctcccgt ccccttccca ggcctcaacc cctttctccc acccagcccc 780
   aacccccagc cctcaccccc tagcccccag ttctggagct tgtcgggagc aagggggtgg 840
   ttgctactgg gtcactcagc ctcaattggc cctgtttcag caatgggcag gttcttcttg 900
   aaattcatca cacctgtggc ttcctctgtg ctctaccttt ttattggggt gacagtgtga 960
   cagctgagat tctccatgca ttccccctac tctagcactg aagggttctg aagggccctg 1020
   gaaggaggga gcttgggggg ctggcttgtg aggggttaag gctgggaggc gggagggggg 1080
   ctggaccaag gggtggggag aaggggagga ggcctcggcc ggccgcagag agaagtggcc 1140
   agagaggccc aggggacagc cagggacagg cagacatgca gccagggctc cagggcctgg 1200
   acaggggctg ccaggccctg tgacaggagg accccgagcc cccggcccgg ggaggggcca 1260
   tggtgctgcc tgtccaacat gtcagccgag gtgcggctga ggcggctcca gcagctggtg 1320
   ttggacccgg gcttcctggg gctggagccc ctgctcgacc ttctcctggg cgtccaccag 1380
   gagctgggcg cctccgaact ggcccaggac aagtacgtgg ccgacttctt gcagtggggt 1440
   gagtgcctac cctcggggct cctgcagatg gggtgggggt ggggcaggag acaggtctgg 1500
   gcacagaggc ctggctgttg ggggggcagg atggcaggat gggcatgggg agatcctccc 1560
   atcctggggc tcagagtgtg gacctgggcc ctggggcaac atttctctgt cctatgccac 1620
   cactctggag gggcagagta aggtcagcag aggctagggt ggctgtgact cagagccatg 1680
   gcttaggagt cacagcaggc taggctgcca acagcctccc atggcctctc tgcaccccgc 1740
   ctcagggtca gggtcagggt catgctggga gctccctctc ctaggaccct ccccccaaaa 1800
   gtgggctcta tggccctctc ccctggtttc ctgtggcctg gggcaagcca ggagggccag 1860
   catggggcag ctgccagggg cgcagccgac aggcaggtgt tcggcgccag cctctccagc 1920
   tgccccaaca ggtgcccagg cactgggagg gcggtgactc acgcgggccc tgtgggagaa 1980
   ccagctttgc agacaggcgc caccagtgcc ccctcctctg cgatccagga gggacaactt 2040
   tgggttcttc tgggtgtgtc tccttctttt gtaggttctg cacccacccc cacccccagc 2100
   cccaaagtct cggttcctat gagccgtgtg ggtcagccac cattcccgcc accccgggtc 2160
   cctgcgtcct ttagttctcc tggcccaggg cctccaacct tccagctgtc ccacaaaacc 2220
   ccttcttgca agggctttcc agggcctggg gccagggctg gaaggaggat gcttccgctt 2280
   ctgccagctg ccttgtctgc ccacctcctc cccaagccca ggactcgggc tcactggtca 2340
   ctggtttctt tcattcccag caccctgccc ctctggccct catatgtctg gccctcagtg 2400
   actggtgttt ggtttttggc ctgtgtgtaa caaactgtgt gtgacacttg tttcctgttt 2460
   ctccgccttc ccctgcttcc tcttgtgtcc atctctttct gacccaggcc tggttccttt 2520
   ccctcctcct cccatttcac agatgggaag gtggaggcca agaagggcca ggccattcag 2580
   cctctggaaa aaccttctcc caacctccca cagcccctaa tgactctcct ggcctccctt 2640
   tagtagagga tgaagttggg ttggcagggt aaactgagac cgggtggggt aggggtctgg 2700
   cgctcccggg aggagcactc cttttgtggc ccgagctgca tctcgcggcc cctcccctgc 2760
   caggcctggg gcgggggagg gggccagggt tcctgctgcc ttaaaagggc tcaatgtctt 2820
   ggctctctcc tccctccccc gtcctcagcc ctggctggtt cgtccctgct ggcccactct 2880
   cccggaaccc cccggaaccc ctctctttcc tccagaaccc actgtctcct ctccttccct 2940
   cccctcccat acccatccct ctctccatcc tgcctccact tcttccaccc ccgggagtcc 3000
   aggcctccct gtccccacag tccctgagcc acaagcctcc accccagctg gtcccccacc 3060
   caggctgccc agtttaacat tcctagtcat aggaccttga cttctgagag gcctgattgt 3120
   catctgtaaa taaggggtag gactaaagca ctcctcctgg aggactgaga gatgggctgg 3180
   accggagcac ttgagtctgg gatatgtgac catgctacct ttgtctccct gtcctgttcc 3240
   ttcccccagc cccaaatcca gggttttcca aagtgtggtt caagaaccac ctgcatctga 3300
   atctagaggt actggataca accccacgtc tgggccgtta cccaggacat tctacatgag 3360
   aacgtggggg tggggccctg gctgcacctg aactgtcacc tggagtcagg gtggaaggtg 3420
   gaagaactgg gtcttatttc cttctcccct tgttctttag ggtctgtcct tctgcagact 3480
   ccgttacccc accctaacca tcctgcacac ccttggagcc ctctgggcca atgccctgtc 3540
   ccgcaaaggg cttctcaggc atctcacctc tatgggaggg catttttggc ccccagaacc 3600
   ttacacggtg tttatgtggg gaagcccctg ggaagcagac agtcctaggg tgaagctgag 3660
   aggcagagag aaggggagac agacagaggg tggggctttc cccttgtctc ccagtgccct 3720
   ttctggtgac cctcggttct tttcccccac caccccccca gcggagccca tcgtggtgag 3780
   gcttaaggag gtccgactgc agagggacga cttcgagatt ctgaaggtga tcggacgcgg 3840
   ggcgttcagc gaggtaagcc gaaccgggcg ggagcctgac ttgactcgtg gtgggcgggg 3900
   cataggggtt ggggcggggc cttagaaatt gatgaatgac cgagccttag aacctagggc 3960
   tgggctggag gcggggcttg ggaccaatgg gcgtggtgtg gcaggtgggg cggggccacg 4020
   gctgggtgca gaagcgggtg gagttgggtc tgggcgagcc cttttgtttt cccgccgtct 4080
   ccactctgtc tcactatctc gacctcaggt agcggtagtg aagatgaagc agacgggcca 4140
   ggtgtatgcc atgaagatca tgaacaagtg ggacatgctg aagaggggcg aggtgagggg 4200
   ctgggcggac gtggggggct ttgaggatcc gcgccccgtc tccggctgca gctcctccgg 4260
   gtgccctgca ggtgtcgtgc ttccgtgagg agagggacgt gttggtgaat ggggaccggc 4320
   ggtggatcac gcagctgcac ttcgccttcc aggatgagaa ctacctggtg agctccgggc 4380
   cggggtgact aggaagaggg acaagagccc gtgctgtcac tggacgagga ggtggggaga 4440
   ggaagctcta ggattggggg tgctgcccgg aaacgtctgt gggaaagtct gtgtgcggta 4500
   agagggtgtg tcaggtggat gaggggcctt ccctatctga gacggggatg gtgtccttca 4560
   ctgcccgttt ctggggtgat ctgggggact cttataaaga tgtctctgtt gcggggggtc 4620
   tcttacctgg aatgggatag gtcttcagga attctaacgg ggccactgcc tagggaagga 4680
   gtgtctggga cctattctct gggtgttggg tggcctctgg gttctctttc ccagaacatc 4740
   tcagggggag tgaatctgcc cagtgacatc ccaggaaagt ttttttgttt gtgttttttt 4800
   ttgaggggcg ggggcggggg ccgcaggtgg tctctgattt ggcccggcag atctctatgg 4860
   ttatctctgg gctggggctg caggtctctg cccaaggatg gggtgtctct gggaggggtt 4920
   gtcccagcca tccgtgatgg atcagggcct caggggacta ccaaccaccc atgacgaacc 4980
   ccttctcagt acctggtcat ggagtattac gtgggcgggg acctgctgac actgctgagc 5040
   aagtttgggg agcggattcc ggccgagatg gcgcgcttct acctggcgga gattgtcatg 5100
   gccatagact cggtgcaccg gcttggctac gtgcacaggt gggtgcagca tggccgaggg 5160
   gatagcaagc ttgttccctg gccgggttct tggaaggtca gagcccagag aggccagggc 5220
   ctggagaggg accttcttgg ttggggccca ccggggggtg cctgggagta ggggtcagaa 5280
   ctgtagaagc cctacagggg cggaacccga ggaagtgggg tcccaggtgg cactgcccgg 5340
   aggggcggag cctggtggga ccacagaagg gaggttcatt tatcccaccc ttctcttttc 5400
   ctccgtgcag ggacatcaaa cccgacaaca tcctgctgga ccgctgtggc cacatccgcc 5460
   tggccgactt cggctcttgc ctcaagctgc gggcagatgg aacggtgagc cagtgccctg 5520
   gccacagagc aactggggct gctgatgagg gatggaaggc acagagtgtg ggagcgggac 5580
   tggatttgga ggggaaaaga ggtggtgtga cccaggctta agtgtgcatc tgtgtggcgg 5640
   agtattagac caggcagagg gaggggctaa gcatttgggg agtggttgga aggagggccc 5700
   agagctggtg ggcccagagg ggtgggccca agcctcgctc tgctcctttt ggtccaggtg 5760
   cggtcgctgg tggctgtggg caccccagac tacctgtccc ccgagatcct gcaggctgtg 5820
   ggcggtgggc ctgggacagg cagctacggg cccgagtgtg actggtgggc gctgggtgta 5880
   ttcgcctatg aaatgttcta tgggcagacg cccttctacg cggattccac ggcggagacc 5940
   tatggcaaga tcgtccacta caaggtgagc acggccgcag ggagacctgg cctctcccgg 6000
   taggcgctcc caggctatcg cctcctctcc ctctgagcag gagcacctct ctctgccgct 6060
   ggtggacgaa ggggtccctg aggaggctcg agacttcatt cagcggttgc tgtgtccccc 6120
   ggagacacgg ctgggccggg gtggagcagg cgacttccgg acacatccct tcttctttgg 6180
   cctcgactgg gatggtctcc gggacagcgt gccccccttt acaccggatt tcgaaggtgc 6240
   caccgacaca tgcaacttcg acttggtgga ggacgggctc actgccatgg tgagcggggg 6300
   cggggtaggt acctgtggcc cctgctcggc tgcgggaacc tccccatgct ccctccataa 6360
   agttggagta aggacagtgc ctaccttctg gggtcctgaa tcactcattc cccagagcac 6420
   ctgctctgtg cccatctact actgaggacc cagcagtgac ctagacttac agtccagtgg 6480
   gggaacacag agcagtcttc agacagtaag gccccagagt gatcagggct gagacaatgg 6540
   agtgcagggg gtgggggact cctgactcag caaggaaggt cctggagggc tttctggagt 6600
   ggggagctat ctgagctgag acttggaggg atgagaagca ggagaggact cctcctccct 6660
   taggccgtct ctcttcaccg tgtaacaagc tgtcatggca tgcttgctcg gctctgggtg 6720
   cccttttgct gaacaatact ggggatccag cacggaccag atgagctctg gtccctgccc 6780
   tcatccagtt gcagtctaga gaattagaga attatggaga gtgtggcagg tgccctgaag 6840
   ggaagcaaca ggatacaaga aaaaatgatg gggccaggca cggtggctca cgcctgtaac 6900
   cccagcaatt tggcaggccg aagtgggtgg attgcttgag cccaggagtt cgagaccagc 6960
   ctgggcaatg tggtgagacc cccgtctcta caaaaatgtt ttaaaaattg gttgggcgtg 7020
   gtggcgcatg cctgtatact cagctactag ggtggccgac gtgggcttga gcccaggagg 7080
   tcaaggctgc agtgagctgt gattgtgcca ctgcactcca gcctgggcaa cggagagaga 7140
   ctctgtctca aaaataagat aaactgaaat taaaaaatag gctgggctgg ccgggcgtgg 7200
   tggctcacgc ctgtaatctc agcactttgg gaggccgagg cgggtggatc acgaggtcag 7260
   gagatcgaga ccatcttggc taacacggtg aaaccccatc tctcctaaaa atacaaaaaa 7320
   ttagccaggc gtggtggcgg gcgcctgtag tcccagctac tcaggaggct gaggcaggag 7380
   aatggcgtga acccgggagg cagagtttgc agtgagccga gatcgtgcca ctgcactcca 7440
   gcctgggcga cagagcgaga ctctgtctca gaaaaaaaaa aaaaaaaaaa aaaaaatagg 7500
   ctggaccgcg gccgggcgct gtggctcatg cctgtaatcc cagcactttg ggagtccaag 7560
   gccggtgggt catgagatca ggagttttga gactaggctg gccaacacgg tgaaaccccg 7620
   tctctactaa aaatacaaga aaattagctg ggtgtggtct cgggtgcctg taattccagt 7680
   tactggggaa gctgaggcag gagaattgct tgaacctggg aggcagagtt tgcagtgagc 7740
   caagatcatg ccactacact ccagtctggg tgacagagtg agactctgtc tcaaaaaaaa 7800
   aaaaaaaaaa aagggttggg caaggtggtt cacgcctgta atcccagaac tttgggaggc 7860
   tgaggcaggc agatcactgg aagtcaggag ttcaagacca gcctggccaa catggtgaaa 7920
   ccctgtgtct actaaaaata caaaatttag ccaggcttgg tggcgtatgc ctgtaatgcc 7980
   agctactcag gaggctgagg caggagaatc gcttgattga acctgggagg cagagtttgc 8040
   agtgggctgg ggttgtgcca ctgcactcta ggctgggaga cagcaagact ccatctaaaa 8100
   aaaaaaaaca gaactgggct gggcacagtg gcttatattt gtaatcccag cactttggga 8160
   ggctgaggtt ggaggactgc ttgagcccag agtttgggac tacaacagct gaggtaggcg 8220
   gatcacttga ggtcagaaga tggagaccag cctggccagc gtggcgaaac cccgtctcta 8280
   ccaaaaatat aaaaaattag ccaggcgtgg tagagggcgc ctgtaatctc agctactcag 8340
   gacgctgagg caggagaatc gcctgaacct gggaggcgga ggttgcagtg agctgagatt 8400
   gcaccactgc actccagcct gggtaacaga gcgagactcc gtatcaaaga aaaagaaaaa 8460
   agaaaaaatg ctggaggggc cactttagat aagccctgag ttggggctgg tttgggggga 8520
   acatgtaagc caagatcaaa aagcagtgag gggcccgccc tgacgactgc tgctcacatc 8580
   tgtgtgtctt gcgcaggaga cactgtcgga cattcgggaa ggtgcgccgc taggggtcca 8640
   cctgcctttt gtgggctact cctactcctg catggccctc aggtaagcac tgccctggac 8700
   ggcctccagg ggccacgagg ctgcttgagc ttcctgggtc ctgctccttg gcagccaatg 8760
   gagttgcagg atcagtcttg gaaccttact gttttgggcc caaagactcc taagaggcca 8820
   gagttggagg accttaaatt ttcagatcta tgtacttcaa aatgttagat tgaattttaa 8880
   aacctcagag tcacagactg ggcttcccag aatcttgtaa ccattaactt ttacgtctgt 8940
   agtacacaga gccacaggac ttcagaactt ggaaaatatg aagtttagac ttttacaatc 9000
   agttgtaaaa gaatgcaaat tctttgaatc agccatataa caataaggcc atttaaaagt 9060
   attaatttag gcgggccgcg gtggctcacg cctgtaatcc tagcactttg ggaggccaag 9120
   gcaggtggat catgaggtca ggagatcgag accatcctgg ctaacacggt gaaaccccgt 9180
   ctctactaaa aatacaaaaa aattagccgg gcatggtggc gggcgcttgc ggtcccagct 9240
   acttgggagg cgaggcagga gaatggcatg aacccgggag gcggagcttg cagtgagccg 9300
   agatcatgcc actgcactcc agcctgggcg acagagcaag actccgtctc aaaaaaaaaa 9360
   aaaaaaaagt atttatttag gccgggtgtg gtggctcacg cctgtaattc cagtgctttg 9420
   ggaggatgag gtgggtggat cacctgaggt caggagttcg agaccagcct gaccaacgtg 9480
   gagaaacctc atctctacta aaaaacaaaa ttagccaggc gtggtggcat atacctgtaa 9540
   tcccagctac tcaggaggct gaggcaggag aatcagaacc caggaggggg aggttgtggt 9600
   gagctgagat cgtgccattg cattccagcc tgggcaacaa gagtgaaact tcatctcaaa 9660
   aaaaaaaaaa aaaaagtact aatttacagg ctgggcatgg tggctcacgc ttggaatccc 9720
   agcactttgg gaggctgaag tggacggatt gcttcagccc aggagttcaa gaccagcctg 9780
   agcaacataa tgagaccctg tctctacaaa aaattgaaaa aatcgtgcca ggcatggtgg 9840
   tctgtgcctg cagtcctagc tactcaggag tctgaagtag gagaatcact tgagcctgga 9900
   gtttgaggct tcagtgagcc atgatagatt ccagcctagg caacaaagtg agacctggtc 9960
   tcaacaaaag tattaattac acaaataatg cattgcttat cacaagtaaa ttagaaaata 10020
   cagataagga aaaggaagtt gatatctcgt gagctcacca gatggcagtg gtccctggct 10080
   cacacgtgta ctgacacatg tttaaatagt ggagaacagg tgtttttttg gtttgttttt 10140
   ttccccttcc tcatgctact ttgtctaaga gaacagttgg ttttctagtc agcttttatt 10200
   actggacaac attacacata ctatacctta tcattaatga actccagctt gattctgaac 10260
   cgctgcgggg cctgaacggt gggtcaggat tgaacccatc ctctattaga acccaggcgc 10320
   atgtccagga tagctaggtc ctgagccgtg ttcccacagg agggactgct gggttggagg 10380
   ggacagccac ttcatacccc agggaggagc tgtccccttc ccacagctga gtggggtgtg 10440
   ctgacctcaa gttgccatct tggggtccca tgcccagtct taggaccaca tctgtggagg 10500
   tggccagagc caagcagtct ccccatcagg tcggcctccc tgtcctgagg ccctgagaag 10560
   aggggtctgc agcggtcaca tgtcaaggga ggagatgagc tgaccctaga acatgggggt 10620
   ctggacccca agtccctgca gaaggtttag aaagagcagc tcccaggggc ccaaggccag 10680
   gagaggggca gggcttttcc taagcagagg aggggctatt ggcctacctg ggactctgtt 10740
   ctcttcgctc tgctgctccc cttcctcaaa tcaggaggtc ttggaagcag ctgcccctac 10800
   ccacaggcca gaagttctgg ttctccacca gagaatcagc attctgtctc cctccccact 10860
   ccctcctcct ctccccaggg acagtgaggt cccaggcccc acacccatgg aactggaggc 10920
   cgagcagctg cttgagccac acgtgcaagc gcccagcctg gagccctcgg tgtccccaca 10980
   ggatgaaaca gtaagttggt ggaggggagg gggtccgtca gggacaattg ggagagaaaa 11040
   ggtgagggct tcccgggtgg cgtgcactgt agagccctct agggacttcc tgaacagaag 11100
   cagacagaaa ccacggagag acgaggttac ttcagacatg ggacggtctc tgtagttaca 11160
   gtggggcatt aagtaagggt gtgtgtgttg ctggggatct gagaagtcga tctttgagct 11220
   gagcgctggt gaaggagaaa caagccatgg aaggaaaggt gccaagtggt caggcgagag 11280
   cctccagggc aaaggccttg ggcaggtggg aatcctgatt tgttcctgaa aggtagtttg 11340
   gctgaatcat tcctgagaag gctggagagg ccagcaggaa acaaaaccca gcaaggcctt 11400
   ttgtcgtgag ggcattaggg agctggaggg attttgagca gcagagggac ataggttgtg 11460
   ttagtgtttg agcaccagcc ctctggtccc tgtgtagatt tagaggacca gactcaggga 11520
   tggggctgag ggaggtaggg aagggagggg gcttggatca ttgcaggagc tatggggatt 11580
   ccagaaatgt tgaggggacg gaggagtagg ggataaacaa ggattcctag cctggaacca 11640
   gtgcccaagt cctgagtctt ccaggagcca caggcagcct taagcctggt ccccatacac 11700
   aggctgaagt ggcagttcca gcggctgtcc ctgcggcaga ggctgaggcc gaggtgacgc 11760
   tgcgggagct ccaggaagcc ctggaggagg aggtgctcac ccggcagagc ctgagccggg 11820
   agatggaggc catccgcacg gacaaccaga acttcgccag gtcgggatcg gggccggggc 11880
   cggggccggg atgcgggccg gtggcaaccc ttggcatccc ctctcgtccg gcccggacgg 11940
   actcaccgtc cttacctccc cacagtcaac tacgcgaggc agaggctcgg aaccgggacc 12000
   tagaggcaca cgtccggcag ttgcaggagc ggatggagtt gctgcaggca gagggagcca 12060
   caggtgagtc cctcatgtgt ccccttcccc ggaggaccgg gaggaggtgg gccgtctgct 12120
   ccgcggggcg tgtatagaca cctggaggag ggaagggacc cacgctgggg cacgccgcgc 12180
   caccgccctc cttcgcccct ccacgcgccc tatgcctctt tcttctcctt ccagctgtca 12240
   cgggggtccc cagtccccgg gccacggatc caccttccca tgtaagaccc ctctctttcc 12300
   cctgcctcag acctgctgcc cattctgcag atcccctccc tggctcctgg tctccccgtc 12360
   cagatatagg gctcacccta cgtctttgcg actttagagg gcagaagccc tttattcagc 12420
   cccagatctc cctccgttca ggcctcacca gattccctcc gggatctccc tagataacct 12480
   ccccaacctc gattcccctc gctgtctctc gccccaccgc tgagggctgg gctgggctcc 12540
   gatcgggtca cctgtccctt ctctctccag ctagatggcc ccccggccgt ggctgtgggc 12600
   cagtgcccgc tggtggggcc aggccccatg caccgccgcc acctgctgct ccctgccagg 12660
   gtacgtccgg ctgcccacgc ccccctccgc cgtcgcgccc cgcgctccac ccgccccttg 12720
   ccacccgctt agctgcgcat ttgcggggct gggcccacgg caggagggcg gatcttcggg 12780
   cagccaatca acacaggccg ctaggaagca gccaatgacg agttcggacg ggattcgagg 12840
   cgtgcgagtg gactaacaac agctgtaggc tgttggggcg ggggcggggc gcagggaaga 12900
   gtgcgggccc acctatgggc gtaggcgggg cgagtcccag gagccaatca gaggcccatg 12960
   ccgggtgttg acctcgccct ctccccgcag gtccctaggc ctggcctatc ggaggcgctt 13020
   tccctgctcc tgttcgccgt tgttctgtct cgtgccgccg ccctgggctg cattgggttg 13080
   gtggcccacg ccggccaact caccgcagtc tggcgccgcc caggagccgc ccgcgctccc 13140
   tgaaccctag aactgtcttc gactccgggg ccccgttgga agactgagtg cccggggcac 13200
   ggcacagaag ccgcgcccac cgcctgccag ttcacaaccg ctccgagcgt gggtctccgc 13260
   ccagctccag tcctgtgatc cgggcccgcc ccctagcggc cggggaggga ggggccgggt 13320
   ccgcggccgg cgaacggggc tcgaagggtc cttgtagccg ggaatgctgc tgctgctgct 13380
   gctgctgctg ctgctgctgc tgctgctgct gctgctgctg ctgctggggg gatcacagac 13440
   catttctttc tttcggccag gctgaggccc tgacgtggat gggcaaactg caggcctggg 13500
   aaggcagcaa gccgggccgt ccgtgttcca tcctccacgc acccccacct atcgttggtt 13560
   cgcaaagtgc aaagctttct tgtgcatgac gccctgctct ggggagcgtc tggcgcgatc 13620
   tctgcctgct tactcgggaa atttgctttt gccaaacccg ctttttcggg gatcccgcgc 13680
   ccccctcctc acttgcgctg ctctcggagc cccagccggc tccgcccgct tcggcggttt 13740
   ggatatttat tgacctcgtc ctccgactcg ctgacaggct acaggacccc caacaacccc 13800
   aatccacgtt ttggatgcac tgagaccccg acattcctcg gtatttattg tctgtcccca 13860
   cctaggaccc ccacccccga ccctcgcgaa taaaaggccc tccatctgcc caaagctctg 13920
   gactccacag tgtccgcggt ttgcgttgtg ggccggaggc tccgcagcgg gccaatccgg 13980
   aggcgtgtgg aggcggccga aggtctggga ggagctagcg ggatgcgaag cggccgaatc 14040
   agggttgggg gaggaaaagc cacggggcgg ggctttggcg tccggccaat aggagggcga 14100
   gcgggccacc cggaggcacc gcccccgccc agctgtggcc cagctgtgcc accgagcgtc 14160
   gagaagaggg ggctgggctg gcagcgcgcg cggccatcct ccttccactg cgcctgcgca 14220
   cgccacgcgc atccgctcct gggacgcaag ctcgagaaaa gttgctgcaa actttctagc 14280
   ccgttccccg cccctcctcc cggccagacc cgccccccct gcggagccgg gaattccgag 14340
   gggcggagcg caggccgaga tggggaatgt gggggcctgc agaggaccct ggagacggag 14400
   gcgtgcagaa g 14411
<210> 3
   <211> 15000
   <212> DNA
   <213> mus musculus
<400> 3
   cagtgtcccc actgcccaag gctggctcca tcacgtaccg ctttggctca gctggccagg 60
   acacacagtt ctgcctgtgg gacctcacag aagatgtgct ctcccctcat ccgtctctgg     120
   cccgtacccg cacccttccg ggcacacctg gtgccacccc accagcttct ggtagttctc     180
   gggccggaga gacaggtgca ggccccctgc cccgctccct gtctcgttcc aacagtctcc 240
   cacacccagc tggtggtggc aaggctggtg ggcctagtgc atcgatggag cctggcatac 300
   cattcagcat tggccgcttt gccacactga ccctgcagga gcggcgggac cggggagctg 360
   agaaggaaca caaacgctac catagcctgg gaaacatcag ccgcggtggc agtgggggca 420
   atagcagcaa tgacaagctc agtggtcctg ccccccgaag ccgattggac ccagctaagg 480
   tgctgggcac ggcactgtgc cctcggatcc atgaggtgcc actgctggag cctctcgtgt 540
   gcaagaagat tgctcaggaa cgcctgaccg tgctactgtt cctggaggat tgtatcatca 600
   ctgcctgcca agagggcctc atctgcacct gggcccggcc aggcaaggcg gtgagtccgc 660
   acctgcccaa gcgctgaggg gcaccagttc tgtccctacc ggatgccagt tatccgtcag 720
   cagaaaggtc aggtatagga gacagaatgg ggggaaccac agctaacgtc tttagagcct 780
   ctgctggccc atatggctca tccttagtac ttcacactca aggcagaacc tgtgtttata 840
   ggaaatctga agtgtagatg gtgaaacttt attcaggtct agggatgtga ttgagctggg 900
   ggcccacttc tggcctgcct cttagacact gtttctgagc cagctgctga aggcctggat 960
   gggaattagc cagggtccag gcctgcactt cctcttgctg ctgtgtggtc ctggtcattg 1020
   ggtctcacag atgggctgtg cagtggctgt gctcttagtt ggtgaggtgc aggcctgtca 1080
   cctggtcagg cttgagcatg tggtctcagt gtctaggacc ctactctgcc ctcagtcctt 1140
   cagtcccttg ctttggaagg ctagagtcca gaagccttag aacgtcaggc agttgcagag 1200
   ccactgccag gctagtaggg ctgcgggagt tgactgagtt ctcacagaca cccctctgtc 1260
   tccctagttc acagacgagg agaccgaggc ccaggcaggg caagcaagtt ggcccaggtc 1320
   acccagcaag tcagttgtag aggtaggaca acccctgaag ctgcaagtgg accccagttt 1380
   cttttctctc cactgtcgtc ccctgtatgc ccaggacacc tggggccaca ttactgtgga 1440
   agtgctactc tgggtcagtg gagacggccg agctgtttgt tcctagctag gacagcagct 1500
   ttaggcctgg ggggcagatc ccagctgggg cagcagctcc aaggcctttg ggtggctcct 1560
   tctccgggtt ctggcagaag cccaggtgct gtctaatcca cctttctcct cttgttctcc 1620
   ccagggcatc tcctcccaac caggcagctc ccccagtggc actgtggtgt gaaatgtgga 1680
   tgtcccatgt tcccggcctc ctagccataa ccctccccgc tgacctcaag aatcactgta 1740
   ttaacaagac taatcatgat ggaaggactg ctccaagccc cacgctgcac acatactggg 1800
   ggtcccctag gttggcccag ccatggggat gtagtgtcct gtgtggcctt ggccctgtcc 1860
   tccacccact gccaagtaca atgacctgtt ctctgaaaca tcagtgttaa ccatatccct 1920
   gtcccagcat gtgactgttc actcctggga gagacttagc ccacagtacc cctgggtgag 1980
   agggcagggc aggggccatc cccactcctg cccaaactcc accccttgct atggtctgtg 2040
   attttgaaag tgttaaatta tggaagccct gagggccctc cttgttcccc tggacctctt 2100
   atttatacta aagtccttgt ttgcacagtg tttctgttcc ctggggcagg gtagggtggg 2160
   ggttgcagta cttggcctcc aagctgtgct ctgaccaaag gaagcccaat cttagctgtt 2220
   tccccatccc tagccccgag cagagagccc tctgaaagat gagtctcgac ccccaaagtc 2280
   aagaggctga gatggccttc ctactaggtc cttggagatg tttgaaactt gttttaaaca 2340
   ccaggactat ccaagcatgc tctccttggg gagaggagga tgctggaatt gactgcactc 2400
   cctgcctcct ctgaacatgc ctttgcagtc tgctgcccct ggcccattta tgactggcca 2460
   tctagtgcca gctggaggtc atgatttcct ccccagagaa ctggccaccc tagaaagaag 2520
   ctaacttgtc gcctggcttg ctgtccaggc agctccgccc tcaaccccta aaatgtttct 2580
   gtctctaatc ctagcccagg caggaatgtg gctgccccgg cctgtggcca aggagctatt 2640
   ttggggttct cttttgctta aggagggcct ggatccacca cttgcctccc ccaggctggg 2700
   gccagcaggt cacccctggc cctggcggct gagcaaactc tctcctgatc ttccttctac 2760
   ctcctgccaa aaaatggggg ggcgggtaat acagcaggca caggggctaa atttaactgt 2820
   cccaaagtcg gaatccattg ctgagtcacg aagaagctgc ccctggcctt tgcccccccc 2880
   actaccccct caccccctgt tgcccaggca tcagcccttt cccccaaccc ctcccagctc 2940
   tgagtctata gactggctct cctgggcact gacacctccc acctgtaact ccctgtgctc 3000
   tctttatggg tgggtagagt caatgggggg gggcaaccct ggagtattac tctgtcccct 3060
   gacattgggc tctgaagagt tttgaggggc cctggaagaa gggagttggg gtgttggctc 3120
   aggaggggtt aaaaactggg aggcgggagg ggggctgggc caaggggtgg agaaaagagg 3180
   aggaggcctt aagcatagaa ctggccagag agacccaagg gatagtcagg gacgggcaga 3240
   catgcagcta gggttctggg gcctggacag gggcagccag gccctgtgac gggaagaccc 3300
   cgagctccgg cccggggagg ggccatggtg ttgcctgccc aacatgtcag ccgaagtgcg 3360
   gctgaggcag ctccagcagc tggtgctgga cccaggcttc ctgggactgg agcccctgct 3420
   cgaccttctc ctgggcgtcc accaggagct gggtgcctct cacctagccc aggacaagta 3480
   tgtggccgac ttcttgcagt ggggtgagta tggataggaa gcctggggtt gggtgcaagg 3540
   cagaggtggg tctacagggc aagaatgggc tatggagggg caggagggcc tggaaagggc 3600
   tttttgtaag ggagccaagc agagctcatg acctgacccc aagctcccct ggtgaggcac 3660
   cagggtcagt gaggccacct atgactcagc cagtgcaggc tggggtgggc atagcctcct 3720
   gctatctcag cacccacact aggacctggc agctttctct tttaggaccc ttggctcctc 3780
   aaactggctt catagccctc cccagtttcc cagagtgtgg ggagggacag cgtggggcag 3840
   ctgccagggt gtggcccata ggcaggtgtt tggcgtctgc ctccccagct gccctgacag 3900
   gtgtccagga gctatgaggg cactgtgact cacagaggcc ctgggggaga accagcccgg 3960
   cagacaggcg ccaccgagca ccctttctgt tccccaaatt aagaggaagg aacaacttca 4020
   gcttctgagt gtgcccatcc ctagcactct gatcccgccc agcctttgtg ggccagattg 4080
   gtcatccctc ctggcttctc atctgctttt gtggttctag ctcaagacct ctaattcctc 4140
   tgctgactta aatgcccttc cccagaggtc ttctcaggcc tagtggacaa gcttggagcc 4200
   ttatctgctc ctgcccaaca ttgagccaaa gctccagctt accccagctt ccttacaagt 4260
   aacgacctgt tttgttgctc tgtgcctatt attaagggtc caggtcttga ttcttggctg 4320
   tctgcccatg tgtgtgaccc tagtgcattc tcccctcctc ccccgtttca cagatggaaa 4380
   ggttgaggcc atcggttaga ctgctaagcc tgtgaaagac tttttctcct ctccagtctt 4440
   tagtgtctcc ctcaaccttt cttttgaagg atggggtttg ggctggcagg gtaaactgag 4500
   aactggggtg ggggcagggg gtctgaccct ctgggaagga gcagtccttt tgtggcctga 4560
   gcagcatcct gtgggcccct cccctgccag gcctgggcgg gggagggggc ctgggttccc 4620
   gctgccttaa aagggctcaa cgccttggct ctctcctcct ccccaccccc cagccttggc 4680
   cctagctgta tcttccccgg ctgcccactt tcccaaaccc ctttcttctc tgtgacccca 4740
   tctccccgct tccccacacg tccctcctcc atccttactc cccggcctta gaacttccct 4800
   aagggagatc tgacctccct ctgcccaccc cgcaccccca gtcgccagcc tcagacctag 4860
   ctgctctccc ctctggctga accaccctag cacaggacct tataccctgg agctttggtt 4920
   ataagaagac tctccttcac cctttggaaa ccaagaaagc ccttccaaca gtgtccagga 4980
   tgctggaggg cagtgaccct cccccacttc ttcttcgtgc tggctgtgct gacacagctc 5040
   cagttcgagg ttgtggcccg agacattaag tgagagcccc gggtgacctg acttagcacc 5100
   ctgatcatca catgggagtg aaaggcctga tgcgccagct tctcccactg cctccctttc 5160
   tgccctgcaa ccctgtggaa acaggcagtt ctgggtccca caaacatcac agaggttttg 5220
   aaagcagaat cctaaagccg atttaagggg cagaaggaag gaggctataa agtcactacc 5280
   cttaccgcta gtgttctgat gacccttggt tcttcttccc ccacccccgc ccagtggagc 5340
   ccattgcagc aaggcttaag gaggtccgac tgcagaggga tgattttgag attttgaagg 5400
   tgatcgggcg tggggcgttc agcgaggtga gtcttcagtg gcctgggaat ggaactttac 5460
   ttgatgtggg tggggcataa cagctggggc agagccttaa aaattgatga atgagcttga 5520
   atttaaggct ggaggggtgg gggcggagct tgtggtcagt gggcggtgtg cacgtgaggg 5580
   cggggctaag gttgggtgga gataagggtg gagtcctgtc tgggtgagcc ttgctggttt 5640
   tccctgccac ctcttgctgt catctcggtt ccgtatttag gtagcggtgg tgaagatgaa 5700
   acagacgggc caagtgtatg ccatgaagat tatgaataag tgggacatgc tgaagagagg 5760
   cgaggtgagg gccagggatt agggcagcgc cctcatctct ccaactcacc tcctgtagct 5820
   tctctcctac ctcacaggtg tcgtgcttcc gggaagaaag ggatgtatta gtgaaagggg 5880
   accggcgctg gatcacacag ctgcactttg ccttccagga tgagaactac ctggtaagct 5940
   ccgggttcag gtgactagga aagagtgaca gttacatcgc cccaagtcaa gaaggctgga 6000
   gaagggagaa gctgctgtag atcggggggg tgggggtggg ggggacacac acaggggatg 6060
   ggggacgggg gtaggattgt gtctcaagta taggagagac cttccttgag acaggagtga 6120
   tatctggttt ggcctttgga tggggcgctc tctcactgtg cgggggtcct ctgtgcttgg 6180
   gaacggggtg tctttgggag tcttgggggc taccaaaccc ctgtgacaca cccgctccca 6240
   gtacctggtc atggaatact acgtgggcgg ggacctgcta acgctgctga gcaagtttgg 6300
   ggagcggatc cccgccgaga tggctcgctt ctacctggcc gagattgtca tggccataga 6360
   ctccgtgcac cggctgggct acgtgcacag gtgggcgtgg cggggccctt ggagggttag 6420
   cagaatttgt gtgggaagga agggtacctg aaggtcagat cccattgggg acagaatcgg 6480
   ggtctagaat tgtagaatcc tgggtggggt ggaagtggat cgagctgacg ggccctaaga 6540
   gggaaggttt tcaagaaagc acactttccc tcttctctct atgcacaggg acatcaaacc 6600
   agataacatt ctgctggacc gatgtgggca cattcgcctg gcagacttcg gctcctgcct 6660
   caaactgcag cctgatggaa tggtaagaag agcctggcga aactctcctc attggtgaag 6720
   gaccggatta gggggcgggg ctgggttgag gagcaggagg ggagcttggt ctgggatgtc 6780
   ctgcgcacca tatttggaca gtcaagggaa aggttttaag cattcaggtc tgattggcac 6840
   aggtgaggtc gctggtggct gtgggcaccc cggactacct gtctcctgag attctgcagg 6900
   ccgttggtgg agggcctggg gcaggcagct acgggccaga gtgtgactgg tgggcactgg 6960
   gcgtgttcgc ctatgagatg ttctatgggc agaccccctt ctacgcggac tccacagccg 7020
   agacatatgc caagattgtg cactacaggg tgagcacaag caccatgcag gggggctgac 7080
   ttagtggctt gtgctcccag actgtctttt ttaaaagata tttatttata tgtgtgtgtt 7140
   ttctgtgtat gtatatctgt gcactgagta ggtgtgcgaa ggtcagaggg catgggatcc 7200
   cctggaactg gagtcacaga ctattgtgtg ctgccatgct gagtgctggg aaacagaacc 7260
   ttgatcactc tgcaagagca gccagtgcac tgaaacgaca gagccagctc tgcagcccag 7320
   ggctaactgt tgcttttctt tctaaatagg aacacttgtc gctgccgctg gcagacacag 7380
   ttgtccccga ggaagctcag gacctcattc gtgggctgct gtgtcctgct gagataaggc 7440
   taggtcgagg tggggcaggt gatttccaga aacatccttt cttctttggc cttgattggg 7500
   agggtctccg agacagtgta ccccccttta caccagactt cgagggtgcc acggacacat 7560
   gcaatttcga tgtggtggag gaccggctca ctgccatggt gagcgggggc ggggtacgta 7620
   cctgcagttc ctgatccgtt gaggggactt ccctagcctc ttccataaaa ttggggtgat 7680
   tggccaggtg tggtggtgca tacctttaat cgtagaactt cataggcaga ggcaggtggc 7740
   tctctggtaa atcaaggcca tcttggtcta catagtgact tctaggccag tcaggagtga 7800
   gatcctccct tgaaaaataa aaaagggggt gttgaccttc ctgggtccca aattattatc 7860
   ctagagcact gctatgtatc cactcaggta tgaggacaca caggtgacca gtcccaaaga 7920
   cagtgagtga ggcctcactc ttggcagtac taaaattgat tgtagggggc tgggctcttg 7980
   acccagcctg gaaagtgctg gagggcttcc tggaggagga gactagctga gcccagaagg 8040
   atgcaggaga tcctttctcg ggtgagtgct ctcagcattt taacaagctc taggccctgc 8100
   agagagaagt ctggtgtggg cagagcccca atagaaagca acaagataga agagaaaatg 8160
   gtggagtttg ttagtggggg cagttatgcc gtgaacatag aggggcgaag ggccatctcg 8220
   gataactgct agccacaaga gccctgtctg tcttcctagg agacgctgtc agacatgcag 8280
   gaagacatgc cccttggggt gcgcctgccc ttcgtgggct actcctactg ctgcatggcc 8340
   ttcaggtgag cacgactgcc ccctgctggg gcctgtgtgc aggcccacca cagccactca 8400
   attgaaggct cagtcttcaa accaagtatt cctaggagct gtctaagtta ggctttctgc 8460
   tgctgcgatg aaccctgact aaaagcaagc tggggaggaa aaggcttatc gggcttacgt 8520
   ttccacatgg gagcccatca ctgaaggaag ccaggacagg aactcacagc ggggcaggaa 8580
   cgtggagctg atgcagaggc aatggagggg agctgcttac tgacttgatc cttatgtctt 8640
   cctcagcctg tttccttgta gagcccagga ccaccaggcc agtgagggct ccactcacaa 8700
   tgggctgagc tctcatctat gatcactagt tatgaaaatg cccgataggc ttgcctgcag 8760
   cttcagtttt tgaggcactt tccttccttc cttccttcct tccttccttc cttccttcct 8820
   ttctttcttt ctttctttct ttctttcttt ctttctttct ttctttcttt ctttctttct 8880
   tagtctttta gagacagggt ctttctatgt agctctggct gtcttggaat tcattctgta 8940
   gaccaggctg gtcttattta tttattttat gtatgtgagt ccactatcac tgtcctcaga 9000
   cacaccagaa gagggcatca gatcccatta cagatggctg tgagccacca tgtggttgct 9060
   gggaattgaa ctcaggacct ctggaagagc agccagtgct cctgccctgt agaggcattt 9120
   tcttcatgaa ggctgtctcc tctctgatga cttgatgact ctagcttgtt gtgtcaagtg 9180
   gacataagac taggaaagca gctacacatg cactttgttt atttttgttt tgctttttga 9240
   gactgggtct ctccatctca tagctctggc catcctgcct ggtgacattc cagtccagtt 9300
   gtataaccta agaatctgag actcagcctt gcagaatcct gctattaacg ggtctaggac 9360
   actccataga atccaggatc ttagaaaaac aaacctgaag tgtgacagtt tattttaaga 9420
   acacaattgg agcacataac aataatacaa cttttcagtt ttaaaaagtt ttctgtcttg 9480
   ttttttgagg caggagctcc ttaatatagt ctaagccgcc ctgcgagtgc tgtgattgat 9540
   gggcatgtac caccatgcct agtcaataaa gcctttaaaa agcatccgtt atgctggctg 9600
   tggtgccaca aacctgtaat cccagcactt agaaggtaga ggcaagatta tcagaaattc 9660
   aaggccatcc tgggctatac agtaatctaa ggctagcctg gtctacaaga gactctgtct 9720
   aaagaaacaa aagataaata gcacccacta ttgctaggca atataaccct ataaccccac 9780
   cattgaggag gctgaggctg gagcatcact gcaaatttga ggccaggatg gtcaacaaat 9840
   aagtcccaga gctggcatag aggaactctg tctcaacaat aaagagaact tatctagcat 9900
   ttatgagggt aaataaaaat ttaccattgc cacaaaaaat gtaaatgaag agactgcttt 9960
   taggagtgaa ctgggaagca gggaacactt agaggatgct cactcacaca ggtatccacc 10020
   atcaggcatg cctcaggcct gcacagggaa ggacaacttg tttcatgatt tgcaagcagc 10080
   atcccatgct ccttagagcg ggttgggccc agcccaccct ctgtggagtt atcgctcagc 10140
   caggcagcaa ggcagccaag gtgctgaggc cctggcagtc tgctctcttc tctgctctga 10200
   acctccttta gctttagcct aggagcctgg cctggtgccc acaggctagg gagtccctag 10260
   cctcttcctc ttctcagaga caatcaggtc ccggacccca cccctatgga actagaggcc 10320
   ctgcagttgc ctgtgtcaga cttgcaaggg cttgacttgc agcccccagt gtccccaccg 10380
   gatcaagtgg tgagtagact gagaggtggg caaagcttcc tgggtgggtg tacctgcagt 10440
   gccaactgcc aggctgttaa ttcagtagga cactgtcccc aactggccca actgcacatc 10500
   ctgtagtcag gaggcacagg cagaaaaatc ccaaattcaa ggcttgctcc cgttatgtaa 10560
   tgagatcctg tcttggagta aaaaacaaag aagagaacta gggatagctc agaggtagat 10620
   gctctcctgg catgggggtg gggtcagaaa gcaacaccaa ccggggcctg ggagggaggg 10680
   actgccaacc acctggagga gtctggggta gacttggtga acaaagttca gaggccatca 10740
   ggtgggatgc tggtttctta aaagccacag ataggtgggt agcattggaa agaggagtgg 10800
   ggggttgcag aaagtgacaa gacacaaact ggggaggcct aagggtaaag ccagggttgt 10860
   ctgaagcact gtggagctgg gaggaacacg ctaaacttct gacttcagcc cttcagttcc 10920
   cctgttgact acactgtccc cagggaccca gggatgggga gaggtggacg ggggagggaa 10980
   gtacgggact gatccagctc caggtcccaa ctctgatccc caccgacagg ctgaagaggc 11040
   tgacctagtg gctgtccctg cccctgtggc tgaggcagag accacggtaa cgctgcagca 11100
   gctccaggaa gccctggaag aagaggttct cacccggcag agcctgagcc gcgagctgga 11160
   ggccatccgg accgccaacc agaacttctc caggtcaggg tcacagtgct ggggtgaggg 11220
   gagaggagag cagcaaccct cgcagtctcc tcaccgatag gtcggctcac tcccctatct 11280
   ttcccagcca actacaggag gccgaggtcc gaaaccgaga cctggaggcg catgttcggc 11340
   agctacagga acggatggag atgctgcagg ccccaggagc cgcaggcgag tccctcacct 11400
   gcttccagcc aagggggcac tgggtggaga tggggggcat gttgggtgtg tgaaccctcg 11460
   gggcagggga ggagtccagg ctggggcacc gcagccgcgc cactgccttt ctcctccatc 11520
   ctccacactc catacacctc tctcttctcc ttccagccat cacgggggtc cccagtcccc 11580
   gggccacgga tccaccttcc catgtaagac ccctctctcc cctccccgat ccccatctta 11640
   gatatgctac ccacagccct tctcccgtcc acgtttaggg tccattctcc ttgggggttc 11700
   cagaagaaag ctgcccttca ctcatccatt cagcatgcac tatctaccag ctctccctcg 11760
   tttcaggctt ctcgccaaat cctccccaag ggaactccct atactcccgt tctggcctcg 11820
   actagattcc cgcactgcct ctcgccctgc tgctgggctc cgatcgggtc acctgtccct 11880
   tctctctcca gctagatggc cccccggccg tggctgtggg ccagtgcccg ctggtggggc 11940
   caggccccat gcaccgccgt cacctgctgc tccctgccag ggtatgtccc acgtccgccc 12000
   accacgggcc tctgcctagc tctgcccact gagtgtcacc actgcttgct gtgcctctgt 12060
   ggagctcggc ccaccgcagg gagggggggt attcgggcgg ccaatcaaca caggctgctg 12120
   ctaagtagcc aatgacgagt tccaacagga gctctttctt gcgagcagac caactttagc 12180
   tgcgggctgt ggggaccaga gatgcgctca gaggcccatc tatgggtata ggctgggcgg 12240
   ctcccaggag ccagtgggcc cctgtagcct agtgctaatc caaccttctc tcctgcagat 12300
   ccctaggcct ggcctatccg aggcgcgttg cctgctcctg ttcgccgctg ctctggctgc 12360
   tgccgccaca ctgggctgca ctgggttggt ggcctatacc ggcggtctca ccccagtctg 12420
   gtgtttcccg ggagccacct tcgccccctg aaccctaaga ctccaagcca tctttcattt 12480
   aggcctccta ggaaggtcga gcgaccaggg agcgacccaa agcgtctctg tgcccatcgc 12540
   gccccccccc cccccccacc gctccgctcc acacttctgt gagcctgggt ccccacccag 12600
   ctccgctcct gtgatccagg cctgccacct ggcggccggg gagggaggaa cagggctcgt 12660
   gcccagcacc cctggttcct gcagagctgg tagccaccgc tgctgcagca gctgggcatt 12720
   cgccgacctt gctttactca gccccgacgt ggatgggcaa actgctcagc tcatccgatt 12780
   tcactttttc actctcccag ccatcagtta caagccataa gcatgagccc cctatttcca 12840
   gggacatccc attcccatag tgatggatca gcaagacctc tgccagcaca cacggagtct 12900
   ttggcttcgg acagcctcac tcctgggggt tgctgcaact ccttccccgt gtacacgtct 12960
   gcactctaac aacggagcca cagctgcact cccccctccc ccaaagcagt gtgggtattt 13020
   attgatcttg ttatctgact cactgacaga ctccgggacc cacgttttag atgcattgag 13080
   actcgacatt cctcggtatt tattgtctgt ccccacctac gacctccact cccgaccctt 13140
   gcgaataaaa tacttctggt ctgccctaaa tcccgcgcaa tatctctgtt gtggaaagga 13200
   aaccgccccg caggccaatg gagagtccaa tagagacaac caatggcttg agtgggagct 13260
   agaggggagg caaagcgcac gaatcaggtt gaagggtggg gcttaggcat ccagccagta 13320
   ggagagaagc aacaagccac cagagacacc accgcccccc accctccccc ccagctgtga 13380
   cccagctgtg ccactcaagt ttggaaaaaa gtagggggtt gggccagcag cgggcacacc 13440
   atcttcccac tgcgcctgcg caagccacgc gcatccgctt tttggaccga cactccagaa 13500
   aagttgctgc aaactttcta gcgcgattcc ccgcccctcc tcccagctag atccaccgcc 13560
   tacccgcggg gccgggaatt ccgaggggcg gagcacggcg cggagatggg aagggagggg 13620
   gcccttcaag ggacccggga gatgggagcg gcttcgcgcc cttaaccctc cggacggccc 13680
   attaccttct ccgttgctct gatagggaaa ctgaggccct gagtcagagg cacacaaggg 13740
   gggaaggcca aaagcgcggc cagagacgga gggaaaacaa agaatcctga cagcccggga 13800
   ggggggcgga cacacaggga caaggacaga cccgagtgca gagctgggtc tagtctttgg 13860
   gagggggcca gaagactgca aggggaccgg gggggggggc ggcgaggagg actgggcgga 13920
   ggagggggct ggggaagccc gcgggaggcg gcaaaggagg gaggaacttt ccaaagttgc 13980
   caaacatggc tacctcgcct gcggagccga gcgcggggcc cgcggctcgg ggggaggcgg 14040
   cggcggcgac cgaggagcag gaggaggaag cgcgccagct tctgcagact ctgcaggcag 14100
   ccgaggggga ggcggcggcg gccggggcgg gagatgcggc ggcggcggcg gactctgggt 14160
   ccccgagtgg cccggggtct ccccgggaga ccgtgaccga ggtgcccact ggccttcgct 14220
   tctcgcccga acaggtggca tgcgtgtgcg aggcgctgct gcaggcgggc cacgccggcc 14280
   gcttgagccg cttcctgggc gcgctgcccc cggccgagcg cctacgtggc agcgatccgg 14340
   tgctgcgcgc gcgggcccta gtggccttcc agcggggtga atacgccgag ctctaccaac 14400
   ttctcgagag ccgccctttc cccgccgccc accacgcctt cctgcaggac ctctacctgc 14460
   gcgcgcgcta ccacgaggcc gagcgggccc gtggccgtgc gctgggcgct gtggacaaat 14520
   accggctgcg caagaagttc cctctgccca agaccatctg ggatggcgag gagaccgtct 14580
   attgcttcaa ggagcgctcg cgagcggcgc tcaaggcctg ctaccgcggc aaccgctatc 14640
   ccacgcctga cgagaagcgc cgcctggcca cgctcaccgg cctctcgctt acacaggtca 14700
   gcaactggtt caagaaccgg cgacagcgcg accgcactgg gaccggcggt ggagcgcctt 14760
   gcaaaaggtg aggggggaac cgaccctcct tcctcggtgg ccgctggagt ctgcgcaagt 14820
   gacccttcac atccctcttc ggtggcgtcg gcgagtgcat aggctgagcg tggagagacc 14880
   aggcacaccc tgggttctct gggcatcact gcctcagggg cagaggttgt tccagctact 14940
   tctaagctgg gaacgcagtg ccaggaatgg gggggggggc gggggcggga cgggcagtga 15000
<210> 4
   <211> 1896
   <212> DNA
   <213> mus musculus
<220>
   <221> CDS
   <222> (1)..(1896)
<400> 4
   atg tca gcc gaa gtg cgg ctg agg cag ctc cag cag ctg gtg ctg gac        48
   Met Ser Ala Glu Val Arg Leu Arg Gln Leu Gln Gln Leu Val Leu Asp
   1               5                   10                  15
   cca ggc ttc ctg gga ctg gag ccc ctg ctc gac ctt ctc ctg ggc gtc        96
   Pro Gly Phe Leu Gly Leu Glu Pro Leu Leu Asp Leu Leu Leu Gly Val
               20                  25                  30
   cac cag gag ctg ggt gcc tct cac cta gcc cag gac aag tat gtg gcc       144
   His Gln Glu Leu Gly Ala Ser His Leu Ala Gln Asp Lys Tyr Val Ala
           35                  40                  45
   gac ttc ttg cag tgg gtg gag ccc att gca gca agg ctt aag gag gtc       192
   Asp Phe Leu Gln Trp Val Glu Pro Ile Ala Ala Arg Leu Lys Glu Val
       50                  55                  60
   cga ctg cag agg gat gat ttt gag att ttg aag gtg atc ggg cgt ggg       240
   Arg Leu Gln Arg Asp Asp Phe Glu Ile Leu Lys Val Ile Gly Arg Gly
   65                  70                  75                  80
   gcg ttc agc gag gta gcg gtg gtg aag atg aaa cag acg ggc caa gtg       288
   Ala Phe Ser Glu Val Ala Val Val Lys Met Lys Gln Thr Gly Gln Val
                   85                  90                  95
   tat gcc atg aag att atg aat aag tgg gac atg ctg aag aga ggc gag       336
   Tyr Ala Met Lys Ile Met Asn Lys Trp Asp Met Leu Lys Arg Gly Glu
               100                 105                 110
   gtg tcg tgc ttc cgg gaa gaa agg gat gta tta gtg aaa ggg gac cgg       384
   Val Ser Cys Phe Arg Glu Glu Arg Asp Val Leu Val Lys Gly Asp Arg
           115                 120                 125
   cgc tgg atc aca cag ctg cac ttt gcc ttc cag gat gag aac tac ctg      432
   Arg Trp Ile Thr Gln Leu His Phe Ala Phe Gln Asp Glu Asn Tyr Leu
       130                 135                 140
   tac ctg gtc atg gaa tac tac gtg ggc ggg gac ctg cta acg ctg ctg      480
   Tyr Leu Val Met Glu Tyr Tyr Val Gly Gly Asp Leu Leu Thr Leu Leu
   145                 150                 155                 160
   agc aag ttt ggg gag cgg atc ccc gcc gag atg gct cgc ttc tac ctg      528
   Ser Lys Phe Gly Glu Arg Ile Pro Ala Glu Met Ala Arg Phe Tyr Leu
                   165                 170                 175
   gcc gag att gtc atg gcc ata gac tcc gtg cac cgg ctg ggc tac gtg      576
   Ala Glu Ile Val Met Ala Ile Asp Ser Val His Arg Leu Gly Tyr Val
               180                 185                 190
   cac agg gac atc aaa cca gat aac att ctg ctg gac cga tgt ggg cac      624
   His Arg Asp Ile Lys Pro Asp Asn Ile Leu Leu Asp Arg Cys Gly His
           195                 200                 205
   att cgc ctg gca gac ttc ggc tcc tgc ctc aaa ctg cag cct gat gga      672
   Ile Arg Leu Ala Asp Phe Gly Ser Cys Leu Lys Leu Gln Pro Asp Gly
       210                 215                 220
   atg gtg agg tcg ctg gtg gct gtg ggc acc ccg gac tac ctg tct cct      720
   Met Val Arg Ser Leu Val Ala Val Gly Thr Pro Asp Tyr Leu Ser Pro
   225                 230                 235                 240
   gag att ctg cag gcc gtt ggt gga ggg cct ggg gca ggc agc tac ggg      768
   Glu Ile Leu Gln Ala Val Gly Gly Gly Pro Gly Ala Gly Ser Tyr Gly
                   245                 250                 255
   cca gag tgt gac tgg tgg gca ctg ggc gtg ttc gcc tat gag atg ttc      816
   Pro Glu Cys Asp Trp Trp Ala Leu Gly Val Phe Ala Tyr Glu Met Phe
               260                 265                 270
   tat ggg cag acc ccc ttc tac gcg gac tcc aca gcc gag aca tat gcc      864
   Tyr Gly Gln Thr Pro Phe Tyr Ala Asp Ser Thr Ala Glu Thr Tyr Ala
           275                 280                 285
   aag att gtg cac tac agg gaa cac ttg tcg ctg ccg ctg gca gac aca      912
   Lys Ile Val His Tyr Arg Glu His Leu Ser Leu Pro Leu Ala Asp Thr
       290                 295                 300
   gtt gtc ccc gag gaa gct cag gac ctc att cgt ggg ctg ctg tgt cct      960
   Val Val Pro Glu Glu Ala Gln Asp Leu Ile Arg Gly Leu Leu Cys Pro
   305                 310                 315                 320
   gct gag ata agg cta ggt cga ggt ggg gca ggt gat ttc cag aaa cat     1008
   Ala Glu Ile Arg Leu Gly Arg Gly Gly Ala Gly Asp Phe Gln Lys His
                   325                 330                 335
   cct ttc ttc ttt ggc ctt gat tgg gag ggt ctc cga gac agt gta ccc     1056
   Pro Phe Phe Phe Gly Leu Asp Trp Glu Gly Leu Arg Asp Ser Val Pro
               340                 345                 350
   ccc ttt aca cca gac ttc gag ggt gcc acg gac aca tgc aat ttc gat     1104
   Pro Phe Thr Pro Asp Phe Glu Gly Ala Thr Asp Thr Cys Asn Phe Asp
           355                 360                 365
   gtg gtg gag gac cgg ctc act gcc atg gtg agc ggg ggc ggg gag acg     1152
   Val Val Glu Asp Arg Leu Thr Ala Met Val Ser Gly Gly Gly Glu Thr
       370                 375                 380
   ctg tca gac atg cag gaa gac atg ccc ctt ggg gtg cgc ctg ccc ttc      1200
   Leu Ser Asp Met Gln Glu Asp Met Pro Leu Gly Val Arg Leu Pro Phe
   385                 390                 395                 400
   gtg ggc tac tcc tac tgc tgc atg gcc ttc aga gac aat cag gtc ccg      1248
   Val Gly Tyr Ser Tyr Cys Cys Met Ala Phe Arg Asp Asn Gln Val Pro
                   405                 410                 415
   gac ccc acc cct atg gaa cta gag gcc ctg cag ttg cct gtg tca gac      1296
   Asp Pro Thr Pro Met Glu Leu Glu Ala Leu Gln Leu Pro Val Ser Asp
               420                 425                 430
   ttg caa ggg ctt gac ttg cag ccc cca gtg tcc cca ccg gat caa gtg      1344
   Leu Gln Gly Leu Asp Leu Gln Pro Pro Val Ser Pro Pro Asp Gln Val
           435                 440                 445
   gct gaa gag gcc gac cta gtg gct gtc cct gcc cct gtg gct gag gca      1392
   Ala Glu Glu Ala Asp Leu Val Ala Val Pro Ala Pro Val Ala Glu Ala
       450                 455                 460
   gag acc acg gta acg ctg cag cag ctc cag gaa gcc ctg gaa gaa gag      1440
   Glu Thr Thr Val Thr Leu Gln Gln Leu Gln Glu Ala Leu Glu Glu Glu
   465                 470                 475                 480
   gtt ctc acc cgg cag agc ctg agc cgc gag ctg gag gcc atc cgg acc      1488
   Val Leu Thr Arg Gln Ser Leu Ser Arg Glu Leu Glu Ala Ile Arg Thr
                   485                 490                 495
   gcc aac cag aac ttc tcc agc caa cta cag gag gcc gag gtc cga aac      1536
   Ala Asn Gln Asn Phe Ser Ser Gln Leu Gln Glu Ala Glu Val Arg Asn
               500                 505                 510
   cga gac ctg gag gcg cat gtt cgg cag cta cag gaa cgg atg gag atg      1584
   Arg Asp Leu Glu Ala His Val Arg Gln Leu Gln Glu Arg Met Glu Met
           515                 520                 525
   ctg cag gcc cca gga gcc gca gcc atc acg ggg gtc ccc agt ccc cgg      1632
   Leu Gln Ala Pro Gly Ala Ala Ala Ile Thr Gly Val Pro Ser Pro Arg
       530                 535                 540
   gcc acg gat cca cct tcc cat cta gat ggc ccc ccg gcc gtg gct gtg      1680
   Ala Thr Asp Pro Pro Ser His Leu Asp Gly Pro Pro Ala Val Ala Val
   545                 550                 555                 560
   ggc cag tgc ccg ctg gtg ggg cca ggc ccc atg cac cgc cgt cac ctg      1728
   Gly Gln Cys Pro Leu Val Gly Pro Gly Pro Met His Arg Arg His Leu
                   565                 570                 575
   ctg ctc cct gcc agg atc cct agg cct ggc cta tcc gag gcg cgt tgc      1776
   Leu Leu Pro Ala Arg Ile Pro Arg Pro Gly Leu Ser Glu Ala Arg Cys
               580                 585                 590
   ctg ctc ctg ttc gcc gct gct ctg gct gct gcc gcc aca ctg ggc tgc      1824
   Leu Leu Leu Phe Ala Ala Ala Leu Ala Ala Ala Ala Thr Leu Gly Cys
           595                 600                 605
   act ggg ttg gtg gcc tat acc ggc ggt ctc acc cca gtc tgg tgt ttc      1872
   Thr Gly Leu Val Ala Tyr Thr Gly Gly Leu Thr Pro Val Trp Cys Phe
       610                 615                 620
   ccg gga gcc acc ttc gcc ccc tga                                      1896
   Pro Gly Ala Thr Phe Ala Pro
   625                 630
<210> 5
   <211> 771
   <212> DNA
   <213> mus musculus
<220>
   <221> misc_feature
   <222> (89)..(89)
   <223> n is a, c, g, or t
<220>
   <221> misc_feature
   <222> (238)..(238)
   <223> n is a, c, g, or t
<220>
   <221> misc_feature
   <222> (506)..(506)
   <223> n is a, c, g, or t
<400> 5
   cctgcccctg tggctgaggc agagaccacg gtaacgctgc agcagctcca ggaagccctg      60
   gaagaagagg ttctcacccg gcagagctng agccgcgagc tggaggccat ccggaccgcc     120
   aaccagaact tctccagcca actacaggag gccgaggtcc gaaaccgaga cctggaggcg     180
   catgttcggc agctacagga acggatggag atgctgcagg ccccaggagc cgccggantc     240
   cctcacctgc ttccagccaa gggggcactg ggtggagatg gggggcatgt tgggtgtgtg     300
   aaccctcggg gcaggggagg agtccaggct ggggcaccgc gccgcgccac tgcctttctc     360
   ctccatcctc cacactccat acacctctct cttctccttc cagccatcac gggggtccca     420
   gtccccgggc cacggatcca ccttcccatc tagatggccc cccggcggtg gctgtgggcc     480
   agtgcccgct ggtggggcca ggacantgtc accgccgtca cctgctgctc cctgccagga     540
   ttcctaggcc tggctatccg aggcgcgttg ctgctcctgt tcgccgctgc tctggctgct     600
   gcgccacact gggctgcact gggttggttg gctataccgg cggtcttcac ccagtctggt     660
   gtttcccgtg agcacccttc gcccctgaaa cctaagactt caagccatct ttcatttagg     720
   ccttctagga aggtcgagcg acaggggagc gacccaaagc gtctctgtgc c              771
<210> 6
   <211> 434
   <212> **DNA**
   <213> mus musculus
<400> 6
   gagagaccca aggggtagtc agggacgggc agacatgcag ctagggttct ggggcctgga      60
   caggggcagc caggccctgt gacgggaaga ccccgagctc cggcccgggg aggggccatg     120
   gtgttgcctg cccaacatgt cagccgaagt gcggctgagg cagctccagc agctggtgct     180
   ggacccaggc ttcctgggac tggagcccct gctcgacctt ctcctgggcg tccaccagga     240
   gctgggtgcc tctcacctag cccaggacaa gtatgtggcc gacttcttgc agtgggtgga     300
   gcccattgca gcaaggctta aggaggtccg actgcagagg gatgattttg agattttgaa     360
   ggtgatcggg cgtggggcgt tcagcgaggt agcggtggtg aagatgaaac acacggagtc     420
   tttggcttcg gaca                                                       434
<210> 7
   <211> 2688
   <212> DNA
   <213> mus musculus
<220>
   <221> CDS
   <222> (134)..(1747)
<400> 7
   ccacgcgtcc gcccacgcgt ccggggcaga catgcagcta gggttctggg gcctggacag        60
   gggcagccag gccctgtgac gggaagaccc cgagctccgg cccggggagg ggccatggtg       120
   ttgcctgccc aac atg tca gcc gaa gtg cgg ctg agg cag ctc cag cag          169
                  Met Ser Ala Glu Val Arg Leu Arg Gln Leu Gln Gln
                  1               5                   10
   ctg gtg ctg gac cca ggc ttc ctg gga ctg gag ccc ctg ctc gac ctt         217
   Leu Val Leu Asp Pro Gly Phe Leu Gly Leu Glu Pro Leu Leu Asp Leu
           15                  20                  25
   ctc ctg ggc gtc cac cag gag ctg ggt gcc tct cac cta gcc cag gac         265
   Leu Leu Gly Val His Gln Glu Leu Gly Ala Ser His Leu Ala Gln Asp
       30                  35                  40
   aag tat gtg gcc gac ttc ttg cag tgg gtg gag ccc att gca gca agg         313
   Lys Tyr Val Ala Asp Phe Leu Gln Trp Val Glu Pro Ile Ala Ala Arg
   45                  50                  55                  60
   ctt aag gag gtc cga ctg cag agg gat gat ttt gag att ttg aag gtg         361
   Leu Lys Glu Val Arg Leu Gln Arg Asp Asp Phe Glu Ile Leu Lys Val
                   65                  70                  75
   atc ggg cgt ggg gcg ttc agc gag gta gcg gtg gtg aag atg aaa cag         409
   Ile Gly Arg Gly Ala Phe Ser Glu Val Ala Val Val Lys Met Lys Gln
               80                  85                  90
   acg ggc caa gtg tat gcc atg aag att atg aat aag tgg gac atg ctg         457
   Thr Gly Gln Val Tyr Ala Met Lys Ile Met Asn Lys Trp Asp Met Leu
           95                  100                 105
   aag aga ggc gag gtg tcg tgc ttc cgg gaa gaa agg gat gta tta gtg         505
   Lys Arg Gly Glu Val Ser Cys Phe Arg Glu Glu Arg Asp Val Leu Val
       110                 115                 120
   aaa ggg gac cgg cgc tgg atc aca cag ctg cac ttt gcc ttc cag gat         553
   Lys Gly Asp Arg Arg Trp Ile Thr Gln Leu His Phe Ala Phe Gln Asp
   125                 130                 135                 140
   gag aac tac ctg tac ctg gtc atg gaa tac tac gtg ggc ggg gac ctg       601
   Glu Asn Tyr Leu Tyr Leu Val Met Glu Tyr Tyr Val Gly Gly Asp Leu
                   145                 150                 155
   cta acg ctg ctg agc aag ttt ggg gag cgg atc ccc gcc gag atg gct       649
   Leu Thr Leu Leu Ser Lys Phe Gly Glu Arg Ile Pro Ala Glu Met Ala
               160                 165                 170
   cgc ttc tac ctg gcc gag att gtc atg gcc ata gac tcc gtg cac cgg       697
   Arg Phe Tyr Leu Ala Glu Ile Val Met Ala Ile Asp Ser Val His Arg
           175                 180                 185
   ctg ggc tac gtg cac agg gac atc aaa cca gat aac att ctg ctg gac       745
   Leu Gly Tyr Val His Arg Asp Ile Lys Pro Asp Asn Ile Leu Leu Asp
       190                 195                 200
   cga tgt ggg cac att cgc ctg gca gac ttc ggc tcc tgc ctc aaa ctg       793
   Arg Cys Gly His Ile Arg Leu Ala Asp Phe Gly Ser Cys Leu Lys Leu
   205                 210                 215                 220
   cag cct gat gga atg gtg agg tcg ctg gtg gct gtg ggc acc ccg gac       841
   Gln Pro Asp Gly Met Val Arg Ser Leu Val Ala Val Gly Thr Pro Asp
                   225                 230                 235
   tac ctg tct cct gag att ctg cag gcc gtt ggt gga ggg cct ggg gca       889
   Tyr Leu Ser Pro Glu Ile Leu Gln Ala Val Gly Gly Gly Pro Gly Ala
               240                 245                 250
   ggc agc tac ggg cca gag tgt gac tgg tgg gca ctg ggc gtg ttc acc       937
   Gly Ser Tyr Gly Pro Glu Cys Asp Trp Trp Ala Leu Gly Val Phe Thr
           255                 260                 265
   tat gag atg ttc tat ggg cag acc ccc ttc tac gcg gac tcc aca gcc       985
   Tyr Glu Met Phe Tyr Gly Gln Thr Pro Phe Tyr Ala Asp Ser Thr Ala
       270                 275                 280
   gag aca tat gcc aag att gtg cac tac agg gaa cac ttg tcg ctg ccg      1033
   Glu Thr Tyr Ala Lys Ile Val His Tyr Arg Glu His Leu Ser Leu Pro
   285                 290                 295                 300
   ctg gca gac aca gtt gtc ccc gag gaa gct cag gac ctc att cgt ggg      1081
   Leu Ala Asp Thr Val Val Pro Glu Glu Ala Gln Asp Leu Ile Arg Gly
                   305                 310                 315
   ctg ctg tgt cct gct gag ata agg cta ggt cga ggt ggg gca ggt gat      1129
   Leu Leu Cys Pro Ala Glu Ile Arg Leu Gly Arg Gly Gly Ala Gly Asp
               320                 325                 330
   ttc cag aaa cat cct ttc ttc ttt ggc ctt gat tgg gag ggt ctc cga      1177
   Phe Gln Lys His Pro Phe Phe Phe Gly Leu Asp Trp Glu Gly Leu Arg
           335                 340                 345
   gac agt gta ccc ccc ttt aca cca gac ttc gag ggt gcc acg gac aca      1225
   Asp Ser Val Pro Pro Phe Thr Pro Asp Phe Glu Gly Ala Thr Asp Thr
       350                 355                 360
   tgc aat ttc gat gtg gtg gag gac cgg ctc act gcc atg gtg agc ggg      1273
   Cys Asn Phe Asp Val Val Glu Asp Arg Leu Thr Ala Met Val Ser Gly
   365                 370                 375                 380
   ggc ggg gag acg ctg tca gac atg cag gaa gac atg ccc ctt ggg gtg      1321
   Gly Gly Glu Thr Leu Ser Asp Met Gln Glu Asp Met Pro Leu Gly Val
   385 390 395
   cgc ctg ccc ttc gtg ggc tac tcc tac tgc tgc atg gcc ttc aga gac 1369
   Arg Leu Pro Phe Val Gly Tyr Ser Tyr Cys Cys Met Ala Phe Arg Asp
   400 405 410
   aat cag gtc ccg gac ccc acc cct atg gaa cta gag gcc ctg cag ttg 1417
   Asn Gln Val Pro Asp Pro Thr Pro Met Glu Leu Glu Ala Leu Gln Leu
   415 420 425
   cct gtg tca gac ttg caa ggg ctt gac ttg cag ccc cca gtg tcc cca 1465
   Pro Val Ser Asp Leu Gln Gly Leu Asp Leu Gln Pro Pro Val Ser Pro
   430 435 440
   ccg gat caa gtg gct gaa gag gct gac cta gtg gct gtc cct gcc cct 1513
   Pro Asp Gln Val Ala Glu Glu Ala Asp Leu Val Ala Val Pro Ala Pro
   445 450 455 460
   gtg gct gag gca gag acc acg gta acg ctg cag cag ctc cag gaa gcc 1561
   Val Ala Glu Ala Glu Thr Thr Val Thr Leu Gln Gln Leu Gln Glu Ala
   465 470 475
   ctg gaa gaa gag gtt ctc acc cgg cag agc ctg agc cgc gag ctg gag 1609
   Leu Glu Glu Glu Val Leu Thr Arg Gln Ser Leu Ser Arg Glu Leu Glu
   480 485 490
   gcc atc cgg acc gcc aac cag aac ttc tcc agc caa cta cag gag gcc 1657
   Ala Ile Arg Thr Ala Asn Gln Asn Phe Ser Ser Gln Leu Gln Glu Ala
   495 500 505
   gag gtc cga aac cga gac ctg gag gcg cat gtt cgg cag cta cag gaa 1705
   Glu Val Arg Asn Arg Asp Leu Glu Ala His Val Arg Gln Leu Gln Glu
   510 515 520
   cgg atg gag atg ctg cag gcc cca gga gcc gca gat ccc tag 1747
   Arg Met Glu Met Leu Gln Ala Pro Gly Ala Ala Asp Pro
   525 530 535
   gcctggccta tccgaggcgc gttgcctgct cctgttcgcc gctgctctgg ctgctgccgc 1807
   cacactgggc tgcactgggt tggtggccta taccggcggt ctcaccccag tctggtgttt 1867
   cccgggagcc accttcgccc cctgaaccct aagactccaa gccatctttc atttaggcct 1927
   cctaggaagg tcgagcgacc agggagcgac ccaaagcgtc tctgtgccca tcgccccccc 1987
   cccccccccc accgctccgc tccacacttc tgtgagcctg ggtccccacc cagctccgct 2047
   cctgtgatcc aggcctgcca cctggcggcc ggggagggag gaacagggct cgtgcccagc 2107
   acccctggtt cctgcagagc tggtagccac cgctgctgca gcagctgggc attcgccgac 2167
   cttgctttac tcagccccga cgtggatggg caaactgctc agctcatccg atttcacttt 2227
   ttcactctcc cagccatcag ttacaagcca taagcatgag ccccctattt ccagggacat 2287
   cccattccca tagtgatgga tcagcaagac ctctgccagc acacacggag tctttggctt 2347
   cggacagcct cactcctggg ggttgctgca actccttccc cgtgtacacg tctgcactct 2407
   aacaacggag ccacagctgc actcccccct cccccaaagc agtgtgggta tttattgatc 2467
   ttgttatctg actcactgac agactccggg acccacgttt tagatgcatt gagactcgac 2527
   attcctcggt atttattgtc tgtccccacc tacgacctcc actcccgacc cttgcgaata 2587
   aaatacttct ggtctgccct aaaaaaaaaa aaaaaaaaaa aaaaaaaaaa aaaaaaaaaa 2647
   aaaaaaaaaa aaaaaaaaaa aaaaaaaaaa aaaaaaaaaa a 2688
<210> 8
   <211> 2862
   <212> DNA
   <213> mus musculus
<400> 8
   gggatagtca gggacgggca gacatgcagc tagggttctg gggcctggac aggggcagcc      60
   aggccctgtg acgggaagac cccgagctcc ggcccgggga ggggccatgg tgttgcctgc     120
   ccaacatgtc agccgaagtg cggctgaggc agctccagca gctggtgctg gacccaggct     180
   tcctgggact ggagcccctg ctcgaccttc tcctgggcgt ccaccaggag ctgggtgcct     240
   ctcacctagc ccaggacaag tatgtggccg acttcttgca gtgggtggag cccattgcag     300
   caaggcttaa ggaggtccga ctgcagaggg atgattttga gattttgaag gtgatcgggc     360
   gtggggcgtt cagcgaggta gcggtggtga agatgaaaca gacgggccaa gtgtatgcca     420
   tgaagattac gaataagtgg gacatgctga agagaggcga ggtgtcgtgc ttccgggaag     480
   aaagggatgt attagtgaaa ggggaccggc gctggatcac acagctgcac tttgccttcc     540
   aggatgagaa ctacctgtac ctggtcatgg aatactacgt gggcggggac ctgctaacgc     600
   tgctgagcaa gtttggggag cggatccccg ccgagatggc tcgcttctac ctggccgaga     660
   ttgtcatggc catagactcc gtgcaccggc tgggctacgt gcacagggac atcaaaccag     720
   ataacattct gctggaccga tgtgggcaca ttcgcctggc agacttcggc tcctgcctca     780
   aactgcagcc tgatggaatg gtgaggtcgc tggtggctgt gggcaccccg gactacctgt     840
   ctcctgagat tctgcaggcc gttggtggag ggcctggggc aggcagctac gggccagagt     900
   gtgactggtg ggcactgggc gtgttcacct atgagatgtt ctatgggcag acccccttct    960
   acgcggactc cacagccgag acatatgcca agattgtgca ctacagggaa cacttgtcgc    1020
   tgccgctggc agacacagtt gtccccgagg aagctcagga cctcattcgt gggctgctgt    1080
   gtcctgctga gataaggcta ggtcgaggtg gggcaggtga tttccagaaa catcctttct    1140
   tctttggcct tgattgggag ggtctccgag acagtgtacc cccctttaca ccagacttcg    1200
   agggtgccac ggacacatgc aatttcgatg tggtggagga ccggctcact gccatggaga    1260
   cgctgtcaga catgcaggaa gacatgcccc ttggggtgcg cctgcccttc gtgggctact    1320
   cctactgctg catggccttc agagacaatc aggtcccgga ccccacccct atggaactag    1380
   aggccctgca gttgcctgtg tcagacttgc aagggcttga cttgcagccc ccagtgtccc    1440
   caccggatca agtggctgaa gaggctgacc tagtggctgt ccctgcccct gtggctgagg    1500
   cagagaccac ggtaacgctg cagcagctcc aggaagccct ggaagaagag gttctcaccc 1560
   ggcagagcct gagccgcgag ctggaggcca tccggaccgc caaccagaac ttctccagcc 1620
   aactacagga ggccgaggtc cgaaaccgag acctggaggc gcatgttcgg cagctacagg 1680
   aacggatgga gatgctgcag gccccaggag ccgcagccat cacgggggtc cccagtcccc 1740
   gggccacgga tccaccttcc catgcttctc gccaaatcct ccccaaggga actccctaga 1800
   ctcccgttct ggcctcgact agattcccgc actgcctctc gccctgctgc tgggctccga 1860
   tcgggtcacc tgtcccttct ctctccagct agatggcccc ccggccgtgg ctgtgggcca 1920
   gtgcccgctg gtggggccag gccccatgca ccgccgtcac ctgctgctcc ctgccaggat 1980
   ccctaggcct ggcctatccg aggcgcgttg cctgctcctg ttcgccgctg ctctggctgc 2040
   tgccgccaca ctgggctgca ctgggttggt ggcctatacc ggcggtctca ccccagtctg 2100
   gtgtttcccg ggagccacct tcgccccctg aaccctaaga ctccaagcca tctttcattt 2160
   aggcctccta ggaaggtcga gcgaccaggg agcgacccaa agcgtctctg tgcccatcgc 2220
   cccccccccc ccccccaccg ctccgctcca cacttctgtg agcctgggtc cccacccagc 2280
   tccgctcctg tgatccaggc ctgccacctg gcggccgggg agggaggaac agggctcgtg 2340
   cccagcaccc ctggttcctg cagagctggt agccaccgct gctgcagcag ctgggcattc 2400
   gccgaccttg ctttactcag ccccgacgtg gatgggcaaa ctgctcagct catccgattt 2460
   cactttttca ctctcccagc catcagttac aagccataag catgagcccc ctatttccag 2520
   ggacatccca ttcccatagt gatggatcag caagacctct gccagcacac acggagtctt 2580
   tggcttcgga cagcctcact cctgggggtt gctgcaactc cttccccgtg tacacgtctg 2640
   cactctaaca acggagccac agctgcactc ccccctcccc caaagcagtg tgggtattta 2700
   ttgatcttgt tatctgactc actgacagac tccgggaccc acgttttaga tgcattgaga 2760
   ctcgacattc ctcggtattt attgtctgtc cccacctacg acctccactc ccgacccttg 2820
   cgaataaaat acttctggtc tgccctaaaa aaaaaaaaaa aa 2862
<210> 9
   <211> 2611
   <212> DNA
   <213> mus musculus
<220>
   <221> CDS
   <222> (55)..(1872)
<400> 9
   cgggaagacc ccgagctccg gcccggggag gggccatggt gttgcctgcc caac atg 57
   Met
   1
   tca gcc gaa gtg cgg ctg agg cag ctc cag cag ctg gtg ctg gac cca        105
   Ser Ala Glu Val Arg Leu Arg Gln Leu Gln Gln Leu Val Leu Asp Pro
               5                  10                  15
   ggc ttc ctg gga ctg gag ccc ctg ctc gac ctt ctc ctg ggc gtc cac        153
   Gly Phe Leu Gly Leu Glu Pro Leu Leu Asp Leu Leu Leu Gly Val His
           20                  25                  30
   cag gag ctg ggt gcc tct cac cta gcc cag gac aag tat gtg gcc gac        201
   Gln Glu Leu Gly Ala Ser His Leu Ala Gln Asp Lys Tyr Val Ala Asp
       35                  40                  45
   ttc ttg cag tgg gtg gag ccc att gca gca agg ctt aag gag gtc cga        249
   Phe Leu Gln Trp Val Glu Pro Ile Ala Ala Arg Leu Lys Glu Val Arg
   50                  55                  60                  65
   ctg cag agg gat gat ttt gag att ttg aag gtg atc ggg cgt ggg gcg        297
   Leu Gln Arg Asp Asp Phe Glu Ile Leu Lys Val Ile Gly Arg Gly Ala
                   70                  75                  80
   ttc agc gag gta gcg gtg gtg aag atg aaa cag acg ggc caa gtg tat        345
   Phe Ser Glu Val Ala Val Val Lys Met Lys Gln Thr Gly Gln Val Tyr
               85                  90                  95
   gcc atg aag att atg aat aag tgg gac atg ctg aag aga ggc gag gtg        393
   Ala Met Lys Ile Met Asn Lys Trp Asp Met Leu Lys Arg Gly Glu Val
           100                 105                 110
   tcg tgc ttc cgg gaa gaa agg gat gta tta gtg aaa ggg gac cgg cgc        441
   Ser Cys Phe Arg Glu Glu Arg Asp Val Leu Val Lys Gly Asp Arg Arg
       115                 120                 125
   tgg atc aca cag ctg cac ttt gcc ttc cag gat gag aac tac ctg tac        489
   Trp Ile Thr Gln Leu His Phe Ala Phe Gln Asp Glu Asn Tyr Leu Tyr
   130                 135                 140                 145
   ctg gtc atg gaa tac tac gtg ggc ggg gac ctg cta acg ctg ctg agc        537
   Leu Val Met Glu Tyr Tyr Val Gly Gly Asp Leu Leu Thr Leu Leu Ser
                   150                 155                 160
   aag ttt ggg gag cgg atc ccc gcc gag atg gct cgc ttc tac ctg gcc        585
   Lys Phe Gly Glu Arg Ile Pro Ala Glu Met Ala Arg Phe Tyr Leu Ala
               165                 170                 175
   gag att gtc atg gcc ata gac tcc gtg cac cgg ctg ggc tac gtg cac        633
   Glu Ile Val Met Ala Ile Asp Ser Val His Arg Leu Gly Tyr Val His
           180                 185                 190
   agg gac atc aaa cca gat aac att ctg ctg gac cga tgt ggg cac att        681
   Arg Asp Ile Lys Pro Asp Asn Ile Leu Leu Asp Arg Cys Gly His Ile
       195                 200                 205
   cgc ctg gca gac ttc ggc tcc tgc ctc aaa ctg cag cct gat gga atg        729
   Arg Leu Ala Asp Phe Gly Ser Cys Leu Lys Leu Gln Pro Asp Gly Met
   210                 215                 220                 225
   gtg agg tcg ctg gtg gct gtg ggc acc ccg gac tac ctg tct cct gag        777
   Val Arg Ser Leu Val Ala Val Gly Thr Pro Asp Tyr Leu Ser Pro Glu
                   230                 235                 240
   att ctg cag gcc gtt ggt gga ggg cct ggg gca ggc agc tac ggg cca        825
   Ile Leu Gln Ala Val Gly Gly Gly Pro Gly Ala Gly Ser Tyr Gly Pro
               245                 250                 255
   gag tgt gac tgg tgg gca ctg ggc gtg ttc acc tat gag atg ttc tat        873
   Glu Cys Asp Trp Trp Ala Leu Gly Val Phe Thr Tyr Glu Met Phe Tyr
           260                 265                 270
   ggg cag acc ccc ttc tac gcg gac tcc aca gcc gag aca tat gcc aag        921
   Gly Gln Thr Pro Phe Tyr Ala Asp Ser Thr Ala Glu Thr Tyr Ala Lys
       275                 280                 285
   att gtg cac tac agg gaa cac ttg tcg ctg ccg ctg gca gac aca gtt        969
   Ile Val His Tyr Arg Glu His Leu Ser Leu Pro Leu Ala Asp Thr Val
   290                 295                 300                 305
   gtc ccc gag gaa gct cag gac ctc att cgt ggg ctg ctg tgt cct gct       1017
   Val Pro Glu Glu Ala Gln Asp Leu Ile Arg Gly Leu Leu Cys Pro Ala
                   310                 315                 320
   gag ata agg cta ggt cga ggt ggg gca gac ttc gag ggt gcc acg gac       1065
   Glu Ile Arg Leu Gly Arg Gly Gly Ala Asp Phe Glu Gly Ala Thr Asp
               325                 330                 335
   aca tgc aat ttc gat gtg gtg gag gac cgg ctc act gcc atg gtg agc       1113
   Thr Cys Asn Phe Asp Val Val Glu Asp Arg Leu Thr Ala Met Val Ser
           340                 345                 350
   ggg ggc ggg gag acg ctg tca gac atg cag gaa gac atg ccc ctt ggg       1161
   Gly Gly Gly Glu Thr Leu Ser Asp Met Gln Glu Asp Met Pro Leu Gly
       355                 360                 365
   gtg cgc ctg ccc ttc gtg ggc tac tcc tac tgc tgc atg gcc ttc aga       1209
   Val Arg Leu Pro Phe Val Gly Tyr Ser Tyr Cys Cys Met Ala Phe Arg
   370                 375                 380                 385
   gac aat cag gtc ccg gac ccc acc cct atg gaa cta gag gcc ctg cag       1257
   Asp Asn Gln Val Pro Asp Pro Thr Pro Met Glu Leu Glu Ala Leu Gln
                   390                 395                 400
   ttg cct gtg tca gac ttg caa ggg ctt gac ttg cag ccc cca gtg tcc       1305
   Leu Pro Val Ser Asp Leu Gln Gly Leu Asp Leu Gln Pro Pro Val Ser
               405                 410                 415
   cca ccg gat caa gtg gct gaa gag gct gac cta gtg gct gtc cct gcc       1353
   Pro Pro Asp Gln Val Ala Glu Glu Ala Asp Leu Val Ala Val Pro Ala
           420                 425                 430
   cct gtg gct gag gca gag acc acg gta acg ctg cag cag ctc cag gaa       1401
   Pro Val Ala Glu Ala Glu Thr Thr Val Thr Leu Gln Gln Leu Gln Glu
       435                 440                 445
   gcc ctg gaa gaa gag gtt ctc acc cgg cag agc ctg agc cgc gag ctg       1449
   Ala Leu Glu Glu Glu Val Leu Thr Arg Gln Ser Leu Ser Arg Glu Leu
   450                 455                 460                 465
   gag gcc atc cgg acc gcc aac cag aac ttc tcc agc caa cta cag gag       1497
   Glu Ala Ile Arg Thr Ala Asn Gln Asn Phe Ser Ser Gln Leu Gln Glu
                   470                 475                 480
   gcc gag gtc cga aac cga gac ctg gag gcg cat gtt cgg cag cta cag       1545
   Ala Glu Val Arg Asn Arg Asp Leu Glu Ala His Val Arg Gln Leu Gln
               485                 490                 495
   gaa cgg atg gag atg ctg cag gcc cca gga gcc gca gcc atc acg ggg       1593
   Glu Arg Met Glu Met Leu Gln Ala Pro Gly Ala Ala Ala Ile Thr Gly
           500                 505                 510
   gtc ccc agt ccc cgg gcc acg gat cca cct tcc cat cta gat ggc ccc      1641
   Val Pro Ser Pro Arg Ala Thr Asp Pro Pro Ser His Leu Asp Gly Pro
       515                 520                 525
   ccg gcc gtg gct gtg ggc cag tgc ccg ctg gtg ggg cca ggc ccc atg      1689
   Pro Ala Val Ala Val Gly Gln Cys Pro Leu Val Gly Pro Gly Pro Met
   530                 535                 540                 545
   cac cgc cgt cac ctg ctg ctc cct gcc agg atc cct agg cct ggc cta      1737
   His Arg Arg His Leu Leu Leu Pro Ala Arg Ile Pro Arg Pro Gly Leu
                   550                 555                 560
   tcc gag gcg cgt tgc ctg ctc ctg ttc gcc gct gct ctg gct gct gcc      1785
   Ser Glu Ala Arg Cys Leu Leu Leu Phe Ala Ala Ala Leu Ala Ala Ala
               565                 570                 575
   gcc aca ctg ggc tgc act ggg ttg gtg gcc tat acc ggc ggt ctc acc      1833
   Ala Thr Leu Gly Cys Thr Gly Leu Val Ala Tyr Thr Gly Gly Leu Thr
           580                 585                 590
   cca gtc tgg tgt ttc ccg gga gcc acc ttc gcc ccc tga accctaagac       1882
   Pro Val Trp Cys Phe Pro Gly Ala Thr Phe Ala Pro
       595                 600                 605
   tccaagccat ctttcattta ggcctcctag gaaggtcgag cgaccaggga gcgacccaaa    1942
   gcgtctctgt gcccatcgcc cccccccccc cccccaccgc tccgctccac acttctgtga    2002
   gcctgggtcc ccacccagct ccgctcctgt gatccaggcc tgccacctgg cggccgggga    2062
   gggaggaaca gggctcgtgc ccagcacccc tggttcctgc agagctggta gccaccgctg    2122
   ctgcagcagc tgggcattcg ccgaccttgc tttactcagc cccgacgtgg atgggcaaac    2182
   tgctcagctc atccgatttc actttttcac tctcccagcc atcagttaca agccataagc    2242
   atgagccccc tatttccagg gacatcccat tcccatagtg atggatcagc aagacctctg    2302
   ccagcacaca cggagtcttt ggcttcggac agcctcactc ctgggggttg ctgcaactcc    2362
   ttccccgtgt acacgtctgc actctaacaa cggagccaca gctgcactcc cccctccccc    2422
   aaagcagtgt gggtatttat tgatcttgtt atctgactca ctgacagact ccgggaccca    2482
   cgttttagat gcattgagac tcgacattcc tcggtattta ttgtctgtcc ccacctacga    2542
   cctccactcc cgacccttgc gaataaaata cttctggtct gccctaaaaa aaaaaaaaaa    2602
   aaaaaaaaa                                                            2611
<210> 10
   <211> 988
   <212> DNA
   <213> mus musculus
<220>
   <221> misc_feature
   <222> (531)..(531)
   <223> n is a, c, g, or t
<220>
   <221> misc_feature
   <222> (942)..(942)
   <223> n is a, c, g, or t
<400> 10
   gctggaccgg tccggaattc tccggatcgc cagcctttgt gggccatatt cgtcatccct      60
   cctggcttct catctgcttt tgtggtccta gctcaagacc tctaattcct ctgctgactt     120
   aaatgccctt ccccagaggt cttctcaggc ctagtggaca agcttggagc cttatctgct     180
   cctgcccaac attgagccaa agctccagct taccccagct tccttacaat ggaccccatt     240
   gcagcaaggc ttaaggaggt ccgactgcag agggatgatt ttgagatttt gaaggtgatc     300
   gggcgtgggg cgttcagcga ggtagcggtg gtgaagatga aacagacggg ccaagtgtat     360
   gccatgaaga ttatgaataa gtgggacatg ctgaagagag gcgaggtgtc gtgcttccgg     420
   gaagaaaggg atgtattagt gaaaggggac cggcgctgga tcacacagct gcactttgcc     480
   ttccaggatg agaactacct gtacctggtc atggaatact acgtgggcgg ngacctgcta     540
   acgctgctga gcaagttttg gggagcggat ccccgccgag atggctcgct tctacctggc     600
   cgagattgtc atggccatag actccgtgca ccggctgggc tacgtgcaca gggacatcaa     660
   accagataac attctgctgg accgatgtgg gcacattcgc ctggcagact tcggctcctg     720
   gcctcaactg cagcctgatg gaatggtgga gtcccctggt ggctgtgggc acccccggac     780
   tacctgtctc ctgaaattct gcagggcctt ggtgggaggc cctggggaag gcaactacgg     840
   gccaaaagtt ggaagggggg ggcctggggg gggttcccct atgaaaagtt ctatggggag     900
   gacccccttt aagcggaatc ccaggccgaa aaatatgccc angattgggc cctaacaggg     960
   aaaacttttc ccctgcccct gggacaat                                        988
<210> 11
   <211> 649
   <212> **DNA**
   <213> mus musculus
<400> 11
   ggcgtgttcg cctatgagat gttctatggg cagaccccct tctacgcgga ctccacagcc 60
   gagacatatg ccaagattgt gcactacagg gaacacttgt cgctgccgct ggcagacaca 120
   gttgtccccg aggaagctca ggacctcatt cgtgggctgc tgtgtcctgc tgagataagg 180
   ctaggtcgag gtggggcagg tgatttccag aaacatcctt tcttctttgg ccttgattgg 240
   gagggtctcc gagacagtgt accccccttt acaccagact tcgagggtgc cacggacaca 300
   tgcaatttcg atgtggtgga ggaccggctc actgccatgg agacgctgtc agacatgcag 360
   gaagacatgc cccttggggt gcgcctgccc ttcgtgggct actcctactg ctgcatggcc 420
   ttcagagaca atcaggtccc ggaccccacc cctatggaac tagaggccct gcagttgcct 480
   gtgtcagact tgcaagggct tgacttgcag cccccagtgt ccccaccgga tcaagtggtc 540
   ccaactctga tccccaccga caggctgaag aggctgacct agtggctgtc cctgcccctg 600
   tggctgaggc agagccacgg taacgctgca gcagctccag gaagccctg 649
<210> 12
   <211> 527
   <212> DNA
   <213> mus musculus
<400> 12
   atttcgatgt ggtggaggac cggctcactg ccatggtgag cgggggcggg gagacgctgt 60
   cagacatgca ggaagacatg ccccttgggg tgcgcctgcc cttcgtgggc tactcctact 120
   gctgcatggc cttcagagac aatcaggtcc cggaccccac ccctatggaa ctagaggccc 180
   tgcagttgcc tgtgtcagac ttgcaagggc ttgacttgca gcccccagtg tccccaccgg 240
   atcaagtggc tgaagaggct gacctagtgg ctgtccctgc ccctgtggct gaggcagaga 300
   ccacggtaac gctgcagcag ctccaggaag ccctggaaga agaggttctc acccggcaga 360
   gcctgagccg cgagctggag gccatccgga ccgccaacca gaacttctcc aggaggccga 420
   ggtccgaaac cgagacctgg aggcgcatgt tcggcagcta caggaacgga tggagatgct 480
   gcaggcccca ggaaccgcag ccatcacggg ggtccccagt cccccgg 527
<210> 13
   <211> 567
   <212> DNA
   <213> mus musculus
<400> 13
   atggtgaggt cgctggtggc tgtgggcacc ccggactacc tgtctcctga gattctgcag      60
   gccgttggtg gagggcctgg ggcaggcagc tacgggccag agtgtgactg gtgggcactg     120
   ggcgtgttcg cctatgagat gttctatggg cagaccccct tctacgcgga ctccacagcc    180
   gagacatatg ccaagattgt gcactacagg gaacacttgt cgctgccgct ggcagacaca     240
   gttgtccccg aggaagctca ggacctcatt cgtgggctgc tgtgtcctgc tgagataagg     300
   ctaggtcgag gtggggcagg tgatttccag aaacatcctt tcttctttgg ccttgattgg     360
   gagggtctcc gagacagtgt accccccttt acaccagact tcgagggtgc cacggacaca      420
   tgcaatttcg atgtggtgga ggaccggctc actgccatgg tgagcggggg cggggtatga     480
   ggacacacag gtgaccagtc cccaagacag tgagtgaggc ttcactcttg gcagtactaa     540
   aattgaatgt agggggctgg gctcttg                                         567
<210> 14
   <211> 2474
   <212> DNA
   <213> mus musculus
<400> 14
   ccgggaagaa agggatgtat tagtgaaagg ggaccggcgc tggatcacac agctgcactt 60
   tgccttccag gatgagaact acctgtacct ggtcatggaa tactacgtgg gcggggacct 120
   gctaacgctg ctgagcaagt ttggggagcg gatccccgcc gagatggctc gcttctacct 180
   ggccgagatt gtcatggcca tagactccgt gcaccggctg ggctacgtgc acagggacat 240
   caaaccagat aacattctgc tggaccgatg tgggcacatt cgcctggcag acttcggctc 300
   ctgcctcaaa ctgcagcctg atggaatggt gaggtcgctg gtggctgtgg gcaccccgga 360
   ctacctgtct cctgagattc tgcaggccgt tggtggaggg cctggggcag gcagctacgg 420
   gccagagtgt gactggtggg cactgggcgt gttcgcctat gagatgttct atgggcagac 480
   ccccttctac gcggactcca cagccgagac atatgccaag attgtgcact acagggaaca 540
   cttgtcgctg ccgctggcag acacagttgt ccccgaggaa gctcaggacc tcattcgtgg 600
   gctgctgtgt cctgctgaga taaggctagg tcgagacttc gagggtgcca cggacacatg 660
   caatttcgat gtggtggagg accggctcac tgccatggtg agcgggggcg gggagacgct 720
   gtcagacatg caggaagaca tgccccttgg ggtgcgcctg cccttcgtgg gctactccta 780
   ctgctgcatg gccttcagag acaatcaggt cccggacccc acccctatgg aactagaggc 840
   cctgcagttg cctgtgtcag acttgcaagg gcttgacttg cagcccccag tgtccccacc 900
   ggatcaagtg gctgaagagg ccgacctagt ggctgtccct gcccctgtgg ctgaggcaga 960
   gaccacggta acgctgcagc agctccagga agccctggaa gaagaggttc tcacccggca 1020
   gagcctgagc cgcgagctgg aggccatccg gaccgccaac cagaacttct ccagccaact 1080
   acaggaggcc gaggtccgaa accgagacct ggaggcgcat gttcggcagc tacaggaacg 1140
   gatggagatg ctgcaggccc caggagccgc aggcgagtcc ctcacctgct tccagccaag 1200
   ggggcactgg gtggagatgg ggggcatgtt gggtgtgtga accctcgggg caggggagga 1260
   gtccaggctg gggcaccgca gccgcgccac tgcctttctc ctccatcctc cacactccat 1320
   acacctctct cttctccttc cagccatcac gggggtcccc agtccccggg ccacggatcc 1380
   accttcccat gcttctcgcc aaatcctccc caagggaact ccctagactc ccgttctggc 1440
   ctcgactaga ttcccgcact gcctctcgcc ctgctgctgg gctccgatcg ggtcacctgt 1500
   cccttctctc tccagctaga tggccccccg gccgtggctg tgggccagtg cccgctggtg 1560
   gggccaggcc ccatgcaccg ccgtcacctg ctgctccctg ccaggatccc taggcctggc 1620
   ctatccgagg cgcgttgcct gctcctgttc gccgctgctc tggctgctgc cgccacactg 1680
   ggctgcactg ggttggtggc ctataccggc ggtctcaccc cagtctggtg tttcccggga 1740
   gccaccttcg ccccctgaac cctaagactc caagccatct ttcatttagg cctcctagga 1800
   agatcgagcg accagggagc gacccaaagc gtctctgtgc ccatcgcccc cccccccccc 1860
   cccaccgctc cgctccacac ttctgtgagc ctgggtcccc acccagctcc gctcctgtga 1920
   tccaggcctg ccacctggcg gccggggagg gaggaacagg gctcgtgccc agcacccctg 1980
   gttcctgcag agctggtagc caccgctgct gcagcagctg ggcattcgcc gaccttgctt 2040
   tactcagccc tgacgtggat gggctaactg ctcagctcat ccgatttcac tttttcactc 2100
   tcccagccat cagttacaag ccataagcat gagcccccta tttccaggga catcccattc 2160
   ccatagtgat ggatcagcaa gacctctgcc agcacacacg gagtctttgg cttcggacag 2220
   cctcactcct gggggttgct gcaactcctt ccccgtgtac acgtctgcac tctaacaacg 2280
   gagccacagc tgcactcccc cctcccccaa agcagtgtgg gtatttattg atcttgttat 2340
   ctgactcact gacagactcc gggacccacg ttttagatgc attgagactc gacattcctc 2400
   ggtatttatt gtctgtcccc acctacgacc tccactcccg acccttgcga ataaaatact 2460
   tctggtctgc ccta 2474
<210> 15
   <211> 2135
   <212> DNA
   <213> mus musculus
<400> 15
   ccgggaagaa agggatgtat tagtgaaagg ggaccggcgc tggatcacac agctgcactt      60
   tgccttccag gatgagaact acctgtacct ggtcatggaa tactacgtgg gcggggacct     120
   gctaacgctg ctgagcaagt ttggggagcg gatccccgcc gagatggctc gcttctacct     180
   ggccgagatt gtcatggcca tagactccgt gcaccggctg ggctacgtgc acagggacat     240
   caaaccagat aacattctgc tggaccgatg tgggcacatt cgcctggcag acttcggctc     300
   ctgcctcaaa ctgcagcctg atggaatggt gaggtcgctg gtggctgtgg gcaccccgga     360
   ctacctgtct cctgagattc tgcaggccgt tggtggaggg cctggggcag gcagctacgg     420
   gccagagtgt gactggtggg cactgggcgt gttcgcctat gagatgttct atgggcagac     480
   ccccttctac gcggactcca cagccgagac atatgccaag attgtgcact acagggaaca     540
   cttgtcgctg ccgctggcag acacagttgt ccccgaggaa gctcaggacc tcattcgtgg     600
   gctgctgtgt cctgctgaga taaggctagg tcgaggtggg gcaggtgatt tccagaaaca     660
   tcctttcttc tttggccttg attgggaggg tctccgagac agtgtacccc cctttacacc     720
   agacttcgag ggtgccacgg acacatgcaa tttcgatgtg gtggaggacc ggctcactgc     780
   catggagacg ctgtcagaca tgcaggaaga catgcccctt ggggtgcgcc tgcccttcgt     840
   gggctactcc tactgctgca tggccttcag agctgaagag gccgacctag tggctgtccc     900
   tgcccctgtg gctgaggcag agaccacggt aacgctgcag cagctccagg aagccctgga     960
   agaagaggtt ctcacccggc agagcctgag ccgcgagctg gaggccatcc ggaccgccaa 1020
   ccagaacttc tccagccaac tacaggaggc cgaggtccga aaccgagacc tggaggcgca 1080
   tgttcggcag ctacaggaac ggatggagat gctgcaggcc ccaggagccg cagccatcac 1140
   gggggtcccc agtccccggg ccacggatcc accttcccat atggcccccc ggccgtggct 1200
   gtgggccagt gcccgctggt ggggccaggc cccatgcacc gccgtcacct gctgctccct 1260
   gccaggatcc ctaggcctgg cctatccgag gcgcgttgcc tgctcctgtt cgccgctgct 1320
   ctggctgctg ccgccacact gggctgcact gggttggtgg cctataccgg cggtctcacc 1380
   ccagtctggt gtttcccggg agccaccttc gccccctgaa ccctaagact ccaagccatc 1440
   tttcatttag gcctcctagg aagatcgagc gaccagggag cgacccaaag cgtctctgtg 1500
   cccatcgccc cccccccccc ccccaccgct ccgctccaca cttctgtgag cctgggtccc 1560
   cacccagctc cgctcctgtg atccaggcct gccacctggc ggccggggag ggaggaacag 1620
   ggctcgtgcc cagcacccct ggttcctgca gagctggtag ccaccgctgc tgcagcagct 1680
   gggcattcgc cgaccttgct ttactcagcc ctgacgtgga tgggctaact gctcagctca 1740
   tccgatttca ctttttcact ctcccagcca tcagttacaa gccataagca tgagccccct 1800
   atttccaggg acatcccatt cccatagtga tggatcagca agacctctgc cagcacacac 1860
   ggagtctttg gcttcggaca gcctcactcc tgggggttgc tgcaactcct tccccgtgta 1920
   cacgtctgca ctctaacaac ggagccacag ctgcactccc ccctccccca aagcagtgtg 1980
   ggtatttatt gatcttgtta tctgactcac tgacagactc cgggacccac gttttagatg 2040
   cattgagact cgacattcct cggtatttat tgtctgtccc cacctacgac ctccactccc 2100
   gacccttgcg aataaaatac ttctggtctg cccta 2135
<210> 16
   <211> 2873
   <212> DNA
   <213> homo sapiens
<220>
   <221> CDS
   <222> (206)..(2083)
<400> 16
   aggggggctg gaccaagggg tggggagaag gggaggaggc ctcggccggc cgcagagaga 60
   agtggccaga gaggcccagg ggacagccag ggacaggcag acatgcagcc agggctccag 120
   ggcctggaca ggggctgcca ggccctgtga caggaggacc ccgagccccc ggcccgggga 180
   ggggccatgg tgctgcctgt ccaac atg tca gcc gag gtg cgg ctg agg cgg 232
   Met Ser Ala Glu Val Arg Leu Arg Arg
   1 5
   ctc cag cag ctg gtg ttg gac ccg ggc ttc ctg ggg ctg gag ccc ctg         280
   Leu Gln Gln Leu Val Leu Asp Pro Gly Phe Leu Gly Leu Glu Pro Leu
   10                  15                  20                  25
   ctc gac ctt ctc ctg ggc gtc cac cag gag ctg ggc gcc tcc gaa ctg         328
   Leu Asp Leu Leu Leu Gly Val His Gln Glu Leu Gly Ala Ser Glu Leu
                   30                  35                  40
   gcc cag gac aag tac gtg gcc gac ttc ttg cag tgg gcg gag ccc atc         376
   Ala Gln Asp Lys Tyr Val Ala Asp Phe Leu Gln Trp Ala Glu Pro Ile
               45                  50                  55
   gtg gtg agg ctt aag gag gtc cga ctg cag agg gac gac ttc gag att         424
   Val Val Arg Leu Lys Glu Val Arg Leu Gln Arg Asp Asp Phe Glu Ile
           60                  65                  70
   ctg aag gtg atc gga cgc ggg gcg ttc agc gag gta gcg gta gtg aag         472
   Leu Lys Val Ile Gly Arg Gly Ala Phe Ser Glu Val Ala Val Val Lys
       75                  80                  85
   atg aag cag acg ggc cag gtg tat gcc atg aag atc atg aac aag tgg         520
   Met Lys Gln Thr Gly Gln Val Tyr Ala Met Lys Ile Met Asn Lys Trp
   90                  95                  100                 105
   gac atg ctg aag agg ggc gag gtg tcg tgc ttc cgt gag gag agg gac         568
   Asp Met Leu Lys Arg Gly Glu Val Ser Cys Phe Arg Glu Glu Arg Asp
                   110                 115                 120
   gtg ttg gtg aat ggg gac cgg cgg tgg atc acg cag ctg cac ttc gcc         616
   Val Leu Val Asn Gly Asp Arg Arg Trp Ile Thr Gln Leu His Phe Ala
               125                 130                 135
   ttc cag gat gag aac tac ctg tac ctg gtc atg gag tat tac gtg ggc         664
   Phe Gln Asp Glu Asn Tyr Leu Tyr Leu Val Met Glu Tyr Tyr Val Gly
           140                 145                 150
   ggg gac ctg ctg aca ctg ctg agc aag ttt ggg gag cgg att ccg gcc         712
   Gly Asp Leu Leu Thr Leu Leu Ser Lys Phe Gly Glu Arg Ile Pro Ala
       155                 160                 165
   gag atg gcg cgc ttc tac ctg gcg gag att gtc atg gcc ata gac tcg         760
   Glu Met Ala Arg Phe Tyr Leu Ala Glu Ile Val Met Ala Ile Asp Ser
   170                 175                 180                 185
   gtg cac cgg ctt ggc tac gtg cac agg gac atc aaa ccc gac aac atc         808
   Val His Arg Leu Gly Tyr Val His Arg Asp Ile Lys Pro Asp Asn Ile
                   190                 195                 200
   ctg ctg gac cgc tgt ggc cac atc cgc ctg gcc gac ttc ggc tct tgc         856
   Leu Leu Asp Arg Cys Gly His Ile Arg Leu Ala Asp Phe Gly Ser Cys
               205                 210                 215
   ctc aag ctg cgg gca gat gga acg gtg cgg tcg ctg gtg gct gtg ggc         904
   Leu Lys Leu Arg Ala Asp Gly Thr Val Arg Ser Leu Val Ala Val Gly
           220                 225                 230
   acc cca gac tac ctg tcc ccc gag atc ctg cag gct gtg ggc ggt ggg         952
   Thr Pro Asp Tyr Leu Ser Pro Glu Ile Leu Gln Ala Val Gly Gly Gly
       235                 240                 245
   cct ggg aca ggc agc tac ggg ccc gag tgt gac tgg tgg gcg ctg ggt        1000
   Pro Gly Thr Gly Ser Tyr Gly Pro Glu Cys Asp Trp Trp Ala Leu Gly
   250                 255                 260                 265
   gta ttc gcc tat gaa atg ttc tat ggg cag acg ccc ttc tac gcg gat        1048
   Val Phe Ala Tyr Glu Met Phe Tyr Gly Gln Thr Pro Phe Tyr Ala Asp
                   270                 275                 280
   tcc acg gcg gag acc tat ggc aag atc gtc cac tac aag gag cac ctc        1096
   Ser Thr Ala Glu Thr Tyr Gly Lys Ile Val His Tyr Lys Glu His Leu
               285                 290                 295
   tct ctg ccg ctg gtg gac gaa ggg gtc cct gag gag gct cga gac ttc        1144
   Ser Leu Pro Leu Val Asp Glu Gly Val Pro Glu Glu Ala Arg Asp Phe
           300                 305                 310
   att cag cgg ttg ctg tgt ccc ccg gag aca cgg ctg ggc cgg ggt gga        1192
   Ile Gln Arg Leu Leu Cys Pro Pro Glu Thr Arg Leu Gly Arg Gly Gly
       315                 320                 325
   gca ggc gac ttc cgg aca cat ccc ttc ttc ttt ggc ctc gac tgg gat        1240
   Ala Gly Asp Phe Arg Thr His Pro Phe Phe Phe Gly Leu Asp Trp Asp
   330                 335                 340                 345
   ggt ctc cgg gac agc gtg ccc ccc ttt aca ccg gat ttc gaa ggt gcc        1288
   Gly Leu Arg Asp Ser Val Pro Pro Phe Thr Pro Asp Phe Glu Gly Ala
                   350                 355                 360
   acc gac aca tgc aac ttc gac ttg gtg gag gac ggg ctc act gcc atg        1336
   Thr Asp Thr Cys Asn Phe Asp Leu Val Glu Asp Gly Leu Thr Ala Met
               365                 370                 375
   gag aca ctg tcg gac att cgg gaa ggt gcg ccg cta ggg gtc cac ctg        1384
   Glu Thr Leu Ser Asp Ile Arg Glu Gly Ala Pro Leu Gly Val His Leu
           380                 385                 390
   cct ttt gtg ggc tac tcc tac tcc tgc atg gcc ctc agg gac agt gag        1432
   Pro Phe Val Gly Tyr Ser Tyr Ser Cys Met Ala Leu Arg Asp Ser Glu
       395                 400                 405
   gtc cca ggc ccc aca ccc atg gaa ctg gag gcc gag cag ctg ctt gag        1480
   Val Pro Gly Pro Thr Pro Met Glu Leu Glu Ala Glu Gln Leu Leu Glu
   410                 415                 420                 425
   cca cac gtg caa gcg ccc agc ctg gag ccc tcg gtg tcc cca cag gat        1528
   Pro His Val Gln Ala Pro Ser Leu Glu Pro Ser Val Ser Pro Gln Asp
                   430                 435                 440
   gaa aca gct gaa gtg gca gtt cca gcg gct gtc cct gcg gca gag gct        1576
   Glu Thr Ala Glu Val Ala Val Pro Ala Ala Val Pro Ala Ala Glu Ala
               445                 450                 455
   gag gcc gag gtg acg ctg cgg gag ctc cag gaa gcc ctg gag gag gag        1624
   Glu Ala Glu Val Thr Leu Arg Glu Leu Gln Glu Ala Leu Glu Glu Glu
           460                 465                 470
   gtg ctc acc cgg cag agc ctg agc cgg gag atg gag gcc atc cgc acg        1672
   Val Leu Thr Arg Gln Ser Leu Ser Arg Glu Met Glu Ala Ile Arg Thr
       475                 480                 485
   gac aac cag aac ttc gcc agt caa cta cgc gag gca gag gct cgg aac        1720
   Asp Asn Gln Asn Phe Ala Ser Gln Leu Arg Glu Ala Glu Ala Arg Asn
   490                 495                 500                 505
   cgg gac cta gag gca cac gtc cgg cag ttg cag gag cgg atg gag ttg        1768
   Arg Asp Leu Glu Ala His Val Arg Gln Leu Gln Glu Arg Met Glu Leu
                   510                 515                 520
   ctg cag gca gag gga gcc aca gct gtc acg ggg gtc ccc agt ccc cgg        1816
   Leu Gln Ala Glu Gly Ala Thr Ala Val Thr Gly Val Pro Ser Pro Arg
               525                 530                 535
   gcc acg gat cca cct tcc cat atg gcc ccc cgg ccg tgg ctg tgg gcc        1864
   Ala Thr Asp Pro Pro Ser His Met Ala Pro Arg Pro Trp Leu Trp Ala
           540                 545                 550
   agt gcc cgc tgg tgg ggc cag gcc cca tgc acc gcc gcc acc tgc tgc        1912
   Ser Ala Arg Trp Trp Gly Gln Ala Pro Cys Thr Ala Ala Thr Cys Cys
       555                 560                 565
   tcc ctg cca ggg tcc cta ggc ctg gcc tat cgg agg cgc ttt ccc tgc        1960
   Ser Leu Pro Gly Ser Leu Gly Leu Ala Tyr Arg Arg Arg Phe Pro Cys
   570                 575                 580                 585
   tcc tgt tcg ccg ttg ttc tgt ctc gtg ccg ccg ccc tgg gct gca ttg        2008
   Ser Cys Ser Pro Leu Phe Cys Leu Val Pro Pro Pro Trp Ala Ala Leu
                   590                 595                 600
   ggt tgg tgg ccc acg ccg gcc aac tca ccg cag tct ggc gcc gcc cag        2056
   Gly Trp Trp Pro Thr Pro Ala Asn Ser Pro Gln Ser Gly Ala Ala Gln
               605                 610                 615
   gag ccg ccc gcg ctc cct gaa ccc tag aactgtcttc gactccgggg              2103
   Glu Pro Pro Ala Leu Pro Glu Pro
           620                 625
   ccccgttgga agactgagtg cccggggcac ggcacagaag ccgcgcccac cgcctgccag      2163
   ttcacaaccg ctccgagcgt gggtctccgc ccagctccag tcctgtgatc cgggcccgcc      2223
   ccctagcggc cggggaggga ggggccgggt ccgcggccgg cgaacggggc tcgaagggtc      2283
   cttgtagccg ggaatgctgc tgctgctgct gctgctgctg ctgctgctgc tgctgctgct      2343
   gctgctgctg ctgctggggg gatcacagac catttctttc tttcggccag gctgaggccc      2403
   tgacgtggat gggcaaactg caggcctggg aaggcagcaa gccgggccgt ccgtgttcca      2463
   tcctccacgc acccccacct atcgttggtt cgcaaagtgc aaagctttct tgtgcatgac      2523
   gccctgctct ggggagcgtc tggcgcgatc tctgcctgct tactcgggaa atttgctttt      2583
   gccaaacccg ctttttcggg gatcccgcgc ccccctcctc acttgcgctg ctctcggagc      2643
   cccagccggc tccgcccgct tcggcggttt ggatatttat tgacctcgtc ctccgactcg      2703
   ctgacaggct acaggacccc caacaacccc aatccacgtt ttggatgcac tgagaccccg      2763
   acattcctcg gtatttattg tctgtcccca cctaggaccc ccacccccga ccctcgcgaa      2823
   taaaaggccc tccatctgcc caaaaaaaaa aaaaaaaaaa aaaaaaaaaa                 2873
<210> 17
   <211> 1509
   <212> DNA
   <213> homo sapiens
<220>
   <221> CDS
   <222> (106)..(1239)
<400> 17
   ccaccgcagc ggacagcgcc aagtgaagcc tcgcttcccc tccgcggcga ccagggcccg      60
   agccgagagt agcagttgta gctacccgcc cagaaactag acaca atg tgc gac gaa     117
                                                     Met Cys Asp Glu
                                                     1
   gac gag acc acc gcc ctc gtg tgc gac aat ggc tcc ggc ctg gtg aaa       165
   Asp Glu Thr Thr Ala Leu Val Cys Asp Asn Gly Ser Gly Leu Val Lys
   5                   10                  15                  20
   gcc ggc ttc gcc ggg gat gac gcc cct agg gcc gtg ttc ccg tcc atc       213
   Ala Gly Phe Ala Gly Asp Asp Ala Pro Arg Ala Val Phe Pro Ser Ile
                   25                  30                  35
   gtg ggc cgc ccc cga cac cag ggc gtc atg gtc ggt atg ggt cag aaa       261
   Val Gly Arg Pro Arg His Gln Gly Val Met Val Gly Met Gly Gln Lys
               40                  45                  50
   gat tcc tac gtg ggc gac gag gct cag agc aag aga ggt atc ctg acc       309
   Asp Ser Tyr Val Gly Asp Glu Ala Gln Ser Lys Arg Gly Ile Leu Thr
           55                  60                  65
   ctg aag tac cct atc gag cac ggc atc atc acc aac tgg gat gac atg       357
   Leu Lys Tyr Pro Ile Glu His Gly Ile Ile Thr Asn Trp Asp Asp Met
       70                  75                  80
   gag aag atc tgg cac cac acc ttc tac aac gag ctt cgc gtg gct ccc       405
   Glu Lys Ile Trp His His Thr Phe Tyr Asn Glu Leu Arg Val Ala Pro
   85                  90                  95                  100
   gag gag cac ccc acc ctg ctc acc gag gcc ccc ctc aat ccc aag gcc       453
   Glu Glu His Pro Thr Leu Leu Thr Glu Ala Pro Leu Asn Pro Lys Ala
                   105                 110                 115
   aac cgc gag aag atg acc cag atc atg ttt gag acc ttc aac gtg ccc       501
   Asn Arg Glu Lys Met Thr Gln Ile Met Phe Glu Thr Phe Asn Val Pro
               120                 125                 130
   gcc atg tac gtg gcc atc cag gcc gtg ctg tcc ctc tac gcc tcc ggc       549
   Ala Met Tyr Val Ala Ile Gln Ala Val Leu Ser Leu Tyr Ala Ser Gly
           135                 140                 145
   agg acc acc ggc atc gtg ctg gac tcc ggc gac ggc gtc acc cac aac       597
   Arg Thr Thr Gly Ile Val Leu Asp Ser Gly Asp Gly Val Thr His Asn
       150                 155                 160
   gtg ccc att tat gag ggc tac gcg ctg ccg cac gcc atc atg cgc ctg       645
   Val Pro Ile Tyr Glu Gly Tyr Ala Leu Pro His Ala Ile Met Arg Leu
   165                 170                 175                 180
   gac ctg gcg ggc cgc gat ctc acc gac tac ctg atg aag atc ctc act       693
   Asp Leu Ala Gly Arg Asp Leu Thr Asp Tyr Leu Met Lys Ile Leu Thr
                   185                 190                 195
   gag cgt ggc tac tcc ttc gtg acc aca gct gag cgc gag atc gtg cgc       741
   Glu Arg Gly Tyr Ser Phe Val Thr Thr Ala Glu Arg Glu Ile Val Arg
               200                 205                 210
   gac atc aag gag aag ctg tgc tac gtg gcc ctg gac ttc gag aac gag       789
   Asp Ile Lys Glu Lys Leu Cys Tyr Val Ala Leu Asp Phe Glu Asn Glu
           215                 220                 225
   atg gcg acg gcc gcc tcc tcc tcc tcc ctg gaa aag agc tac gag ctg       837
   Met Ala Thr Ala Ala Ser Ser Ser Ser Leu Glu Lys Ser Tyr Glu Leu
       230                 235                 240
   cca gac ggg cag gtc atc acc atc ggc aac gag cgc ttc cgc tgc ccg       885
   Pro Asp Gly Gln Val Ile Thr Ile Gly Asn Glu Arg Phe Arg Cys Pro
   245                 250                 255                 260
   gag acg ctc ttc cag ccc tcc ttc atc ggt atg gag tcg gcg ggc att       933
   Glu Thr Leu Phe Gln Pro Ser Phe Ile Gly Met Glu Ser Ala Gly Ile
                   265                 270                 275
   cac gag acc acc tac aac agc atc atg aag tgt gac atc gac atc agg       981
   His Glu Thr Thr Tyr Asn Ser Ile Met Lys Cys Asp Ile Asp Ile Arg
               280                 285                 290
   aag gac ctg tat gcc aac aac gtc atg tcg ggg ggc acc acg atg tac      1029
   Lys Asp Leu Tyr Ala Asn Asn Val Met Ser Gly Gly Thr Thr Met Tyr
           295                 300                 305
   cct ggg atc gct gac cgc atg cag aaa gag atc acc gcg ctg gca ccc      1077
   Pro Gly Ile Ala Asp Arg Met Gln Lys Glu Ile Thr Ala Leu Ala Pro
       310                 315                 320
   agc acc atg aag atc aag atc atc gcc ccg ccg gag cgc aaa tac tcg      1125
   Ser Thr Met Lys Ile Lys Ile Ile Ala Pro Pro Glu Arg Lys Tyr Ser
   325                 330                 335                 340
   gtg tgg atc ggc ggc tcc atc ctg gcc tcg ctg tcc acc ttc cag cag      1173
   Val Trp Ile Gly Gly Ser Ile Leu Ala Ser Leu Ser Thr Phe Gln Gln
                   345                 350                 355
   atg tgg atc acc aag cag gag tac gac gag gcc ggc cct tcc atc gtc      1221
   Met Trp Ile Thr Lys Gln Glu Tyr Asp Glu Ala Gly Pro Ser Ile Val
               360                 365                 370
   cac cgc aaa tgc ttc tag acacactcca cctccagcac gcgacttctc             1269
   His Arg Lys Cys Phe
           375
   aggacgacga atcttctcaa tgggggggcg gctgagctcc agccaccccg cagtcacttt      1329
   ctttgtaaca acttccgttg ctgccatcgt aaactgacac agtgtttata acgtgtacat      1389
   acattaactt attacctcat tttgttattt ttcgaaacaa agccctgtgg aagaaaatgg      1449
   aaaacttgaa gaagcattaa agtcattctg ttaagctgcg taaaaaaaaa aaaaaaaaaa      1509
<210> 18
<400> 18
   000
<210> 19
<400> 19
   000
<210> 20
   <211> 18
   <212> DNA
   <213> Artificial sequence
<220>
   <223> Primer
<400> 20
   agcctgagcc gggagatg 18
<210> 21
   <211> 21
   <212> DNA
   <213> Artificial sequence
<220>
   <223> Primer
<400> 21
   gcgtagttga ctggcgaagt t 21
<210> 22
   <211> 21
   <212> DNA
   <213> Artificial sequence
<220>
   <223> Probe
<400> 22
   aggccatccg cacggacaac c 21
<210> 23
   <211> 16
   <212> DNA
   <213> Artificial sequence
<220>
   <223> Synthetic oligonucleotide
<400> 23
   acaataaata ccgagg 16
<210> 24
   <211> 20
   <212> DNA
   <213> Artificial sequence
<220>
   <223> Synthetic oligonucleotide
<400> 24
   cggagcggtt gtgaactggc 20
<210> 25
   <211> 20
   <212> DNA
   <213> Artificial sequence
<220>
   <223> Synthetic oligonucleotide
<400> 25
   gcgcaccttc ccgaatgtcc 20
<210> 26
   <211> 16
   <212> DNA
   <213> Artificial sequence
<220>
   <223> Synthetic oligonucleotide
<400> 26
   cccgaatgtc cgacag 16
<210> 27
   <211> 17
   <212> DNA
   <213> Artificial sequence
<220>
   <223> Synthetic oligonucleotide
<400> 27
   cggagcggtt gtgaact 17
<210> 28
   <211> 17
   <212> DNA
   <213> Artificial sequence
<220>
   <223> Synthetic oligonucleotide
<400> 28
   acaataaata ccgagga 17
<210> 29
   <211> 16
   <212> DNA
   <213> Artificial sequence
<220>
   <223> Synthetic oligonucleotide
<400> 29
   gacaatctcc gccagg 16
<210> 30
   <211> 16
   <212> DNA
   <213> Artificial sequence
<220>
   <223> Synthetic oligonucleotide
<400> 30
   tcccgaatgt ccgaca 16
<210> 31
   <211> 16
   <212> DNA
   <213> Artificial sequence
<220>
   <223> Synthetic oligonucleotide
<400> 31
   tgtaatgttg tccagt 16
<210> 32
   <211> 16
   <212> DNA
   <213> Artificial sequence
<220>
   <223> Synthetic oligonucleotide
<400> 32
   gtgtaatgtt gtccag 16
<210> 33
   <211> 20
   <212> DNA
   <213> Artificial sequence
<220>
   <223> Synthetic oligonucleotide
<400> 33
   ttgcactttg cgaaccaacg 20
<210> 34
   <211> 20
   <212> DNA
   <213> Artificial sequence
<220>
   <223> Synthetic oligonucleotide
<400> 34
   cgacacctcg cccctcttca 20
<210> 35
   <211> 20
   <212> DNA
   <213> Artificial sequence
<220>
   <223> Synthetic oligonucleotide
<400> 35
   acgacacctc gcccctcttc 20
<210> 36
   <211> 20
   <212> DNA
   <213> Artificial sequence
<220>
   <223> Synthetic oligonucleotide
<400> 36
   cacgacacct cgcccctctt 20
<210> 37
   <211> 20
   <212> DNA
   <213> Artificial sequence
<220>
   <223> Synthetic oligonucleotide
<400> 37
   gcacgacacc tcgcccctct 20
<210> 38
   <211> 20
   <212> DNA
   <213> Artificial sequence
<220>
   <223> Synthetic oligonucleotide
<400> 38
   agcacgacac ctcgcccctc 20
<210> 39
   <211> 20
   <212> DNA
   <213> Artificial sequence
<220>
   <223> Synthetic oligonucleotide
<400> 39
   aagcacgaca cctcgcccct 20
<210> 40
   <211> 20
   <212> DNA
   <213> Artificial sequence
<220>
   <223> Synthetic oligonucleotide
<400> 40
   gaagcacgac acctcgcccc 20
<210> 41
   <211> 20
   <212> DNA
   <213> Artificial sequence
<220>
   <223> Synthetic oligonucleotide
<400> 41
   ggaagcacga cacctcgccc 20
<210> 42
   <211> 20
   <212> DNA
   <213> Artificial sequence
<220>
   <223> Synthetic oligonucleotide
<400> 42
   cggaagcacg acacctcgcc 20
<210> 43
   <211> 20
   <212> DNA
   <213> Artificial sequence
<220>
   <223> Synthetic oligonucleotide
<400> 43
   acggaagcac gacacctcgc 20
<210> 44
   <211> 20
   <212> DNA
   <213> Artificial sequence
<220>
   <223> Synthetic oligonucleotide
<400> 44
   cacggaagca cgacacctcg 20
<210> 45
   <211> 20
   <212> DNA
   <213> Artificial sequence
<220>
   <223> Synthetic oligonucleotide
<400> 45
   tcacggaagc acgacacctc 20
<210> 46
   <211> 20
   <212> DNA
   <213> Artificial sequence
<220>
   <223> Synthetic oligonucleotide
<400> 46
   ctcacggaag cacgacacct 20
<210> 47
   <211> 20
   <212> DNA
   <213> Artificial sequence
<220>
   <223> Synthetic oligonucleotide
<400> 47
   cctcacggaa gcacgacacc 20
<210> 48
   <211> 20
   <212> DNA
   <213> Artificial sequence
<220>
   <223> Synthetic oligonucleotide
<400> 48
   tcctcacgga agcacgacac 20
<210> 49
   <211> 20
   <212> DNA
   <213> Artificial sequence
<220>
   <223> Synthetic oligonucleotide
<400> 49
   ctcctcacgg aagcacgaca 20
<210> 50
   <211> 20
   <212> DNA
   <213> Artificial sequence
<220>
   <223> Synthetic oligonucleotide
<400> 50
   tctcctcacg gaagcacgac 20
<210> 51
   <211> 20
   <212> DNA
   <213> Artificial sequence
<220>
   <223> Synthetic oligonucleotide
<400> 51
   ctctcctcac ggaagcacga 20
<210> 52
   <211> 20
   <212> DNA
   <213> Artificial sequence
<220>
   <223> Synthetic oligonucleotide
<400> 52
   cctctcctca cggaagcacg 20
<210> 53
   <211> 20
   <212> DNA
   <213> Artificial sequence
<220>
   <223> Synthetic oligonucleotide
<400> 53
   ccctctcctc acggaagcac 20
<210> 54
   <211> 20
   <212> DNA
   <213> Artificial sequence
<220>
   <223> Synthetic oligonucleotide
<400> 54
   tccctctcct cacggaagca 20
<210> 55
   <211> 20
   <212> DNA
   <213> Artificial sequence
<220>
   <223> Synthetic oligonucleotide
<400> 55
   gtccctctcc tcacggaagc 20
<210> 56
   <211> 20
   <212> DNA
   <213> Artificial sequence
<220>
   <223> Synthetic oligonucleotide
<400> 56
   cgtccctctc ctcacggaag 20
<210> 57
   <211> 20
   <212> DNA
   <213> Artificial sequence
<220>
   <223> Synthetic oligonucleotide
<400> 57
   ggtccccatt caccaacacg 20
<210> 58
   <211> 20
   <212> DNA
   <213> Artificial sequence
<220>
   <223> Synthetic oligonucleotide
<400> 58
   cggtccccat tcaccaacac 20
<210> 59
   <211> 20
   <212> DNA
   <213> Artificial sequence
<220>
   <223> Synthetic oligonucleotide
<400> 59
   ccggtcccca ttcaccaaca 20
<210> 60
   <211> 20
   <212> DNA
   <213> Artificial sequence
<220>
   <223> Synthetic oligonucleotide
<400> 60
   gccggtcccc attcaccaac 20
<210> 61
   <211> 20
   <212> DNA
   <213> Artificial sequence
<220>
   <223> Synthetic oligonucleotide
<400> 61
   cgccggtccc cattcaccaa 20
<210> 62
   <211> 20
   <212> DNA
   <213> Artificial sequence
<220>
   <223> Synthetic oligonucleotide
<400> 62
   ccgccggtcc ccattcacca 20
<210> 63
   <211> 20
   <212> DNA
   <213> Artificial sequence
<220>
   <223> Synthetic oligonucleotide
<400> 63
   accgccggtc cccattcacc 20
<210> 64
   <211> 20
   <212> DNA
   <213> Artificial sequence
<220>
   <223> Synthetic oligonucleotide
<400> 64
   caccgccggt ccccattcac 20
<210> 65
   <211> 20
   <212> DNA
   <213> Artificial sequence
<220>
   <223> Synthetic oligonucleotide
<400> 65
   ccaccgccgg tccccattca 20
<210> 66
   <211> 20
   <212> DNA
   <213> Artificial sequence
<220>
   <223> Synthetic oligonucleotide
<400> 66
   tccaccgccg gtccccattc 20
<210> 67
   <211> 20
   <212> DNA
   <213> Artificial sequence
<220>
   <223> Synthetic oligonucleotide
<400> 67
   atccaccgcc ggtccccatt 20
<210> 68
   <211> 20
   <212> DNA
   <213> Artificial sequence
<220>
   <223> Synthetic oligonucleotide
<400> 68
   gatccaccgc cggtccccat 20
<210> 69
   <211> 20
   <212> DNA
   <213> Artificial sequence
<220>
   <223> Synthetic oligonucleotide
<400> 69
   tgatccaccg ccggtcccca 20
<210> 70
   <211> 20
   <212> DNA
   <213> Artificial sequence
<220>
   <223> Synthetic oligonucleotide
<400> 70
   gtgatccacc gccggtcccc 20
<210> 71
   <211> 20
   <212> DNA
   <213> Artificial sequence
<220>
   <223> Synthetic oligonucleotide
<400> 71
   cgtgatccac cgccggtccc 20
<210> 72
   <211> 20
   <212> DNA
   <213> Artificial sequence
<220>
   <223> Synthetic oligonucleotide
<400> 72
   ttctcatcct ggaaggcgaa 20
<210> 73
   <211> 20
   <212> DNA
   <213> Artificial sequence
<220>
   <223> Synthetic oligonucleotide
<400> 73
   gttctcatcc tggaaggcga 20
<210> 74
   <211> 20
   <212> DNA
   <213> Artificial sequence
<220>
   <223> Synthetic oligonucleotide
<400> 74
   agttctcatc ctggaaggcg 20
<210> 75
   <211> 20
   <212> DNA
   <213> Artificial sequence
<220>
   <223> Synthetic oligonucleotide
<400> 75
   gtagttctca tcctggaagg 20
<210> 76
   <211> 20
   <212> DNA
   <213> Artificial sequence
<220>
   <223> Synthetic oligonucleotide
<400> 76
   ggtagttctc atcctggaag 20
<210> 77
   <211> 20
   <212> DNA
   <213> Artificial sequence
<220>
   <223> Synthetic oligonucleotide
<400> 77
   aggtagttct catcctggaa 20
<210> 78
   <211> 20
   <212> DNA
   <213> Artificial sequence
<220>
   <223> Synthetic oligonucleotide
<400> 78
   caggtagttc tcatcctgga 20
<210> 79
   <211> 20
   <212> DNA
   <213> Artificial sequence
<220>
   <223> Synthetic oligonucleotide
<400> 79
   tacaggtagt tctcatcctg 20
<210> 80
   <211> 20
   <212> DNA
   <213> Artificial sequence
<220>
   <223> Synthetic oligonucleotide
<400> 80
   gtacaggtag ttctcatcct 20
<210> 81
   <211> 20
   <212> DNA
   <213> Artificial sequence
<220>
   <223> Synthetic oligonucleotide
<400> 81
   ggtacaggta gttctcatcc 20
<210> 82
   <211> 20
   <212> DNA
   <213> Artificial sequence
<220>
   <223> Synthetic oligonucleotide
<400> 82
   aggtacaggt agttctcatc 20
<210> 83
   <211> 20
   <212> DNA
   <213> Artificial sequence
<220>
   <223> Synthetic oligonucleotide
<400> 83
   ccaggtacag gtagttctca 20
<210> 84
   <211> 20
   <212> DNA
   <213> Artificial sequence
<220>
   <223> Synthetic oligonucleotide
<400> 84
   accaggtaca ggtagttctc 20
<210> 85
   <211> 20
   <212> DNA
   <213> Artificial sequence
<220>
   <223> Synthetic oligonucleotide
<400> 85
   gaccaggtac aggtagttct 20
<210> 86
   <211> 20
   <212> DNA
   <213> Artificial sequence
<220>
   <223> Synthetic oligonucleotide
<400> 86
   tgaccaggta caggtagttc 20
<210> 87
   <211> 20
   <212> DNA
   <213> Artificial sequence
<220>
   <223> Synthetic oligonucleotide
<400> 87
   catgaccagg tacaggtagt 20
<210> 88
   <211> 20
   <212> DNA
   <213> Artificial sequence
<220>
   <223> Synthetic oligonucleotide
<400> 88
   ccatgaccag gtacaggtag 20
<210> 89
   <211> 20
   <212> DNA
   <213> Artificial sequence
<220>
   <223> Synthetic oligonucleotide
<400> 89
   tccatgacca ggtacaggta 20
<210> 90
   <211> 20
   <212> DNA
   <213> Artificial sequence
<220>
   <223> Synthetic oligonucleotide
<400> 90
   ctccatgacc aggtacaggt 20
<210> 91
   <211> 20
   <212> DNA
   <213> Artificial sequence
<220>
   <223> Synthetic oligonucleotide
<400> 91
   actccatgac caggtacagg 20
<210> 92
   <211> 20
   <212> DNA
   <213> Artificial sequence
<220>
   <223> Synthetic oligonucleotide
<400> 92
   tactccatga ccaggtacag 20
<210> 93
   <211> 20
   <212> DNA
   <213> Artificial sequence
<220>
   <223> Synthetic oligonucleotide
<400> 93
   atactccatg accaggtaca 20
<210> 94
   <211> 20
   <212> DNA
   <213> Artificial sequence
<220>
   <223> Synthetic oligonucleotide
<400> 94
   aatactccat gaccaggtac 20
<210> 95
   <211> 20
   <212> DNA
   <213> Artificial sequence
<220>
   <223> Synthetic oligonucleotide
<400> 95
   taatactcca tgaccaggta 20
<210> 96
   <211> 20
   <212> DNA
   <213> Artificial sequence
<220>
   <223> Synthetic oligonucleotide
<400> 96
   cgtaatactc catgaccagg 20
<210> 97
   <211> 20
   <212> DNA
   <213> Artificial sequence
<220>
   <223> Synthetic oligonucleotide
<400> 97
   agcagtgtca gcaggtcccc 20
<210> 98
   <211> 20
   <212> DNA
   <213> Artificial sequence
<220>
   <223> Synthetic oligonucleotide
<400> 98
   cagcagtgtc agcaggtccc 20
<210> 99
   <211> 20
   <212> DNA
   <213> Artificial sequence
<220>
   <223> Synthetic oligonucleotide
<400> 99
   tcagcagtgt cagcaggtcc 20
<210> 100
   <211> 20
   <212> DNA
   <213> Artificial sequence
<220>
   <223> Synthetic oligonucleotide
<400> 100
   ctcagcagtg tcagcaggtc 20
<210> 101
   <211> 20
   <212> DNA
   <213> Artificial sequence
<220>
   <223> Synthetic oligonucleotide
<400> 101
   gctcagcagt gtcagcaggt 20
<210> 102
   <211> 20
   <212> DNA
   <213> Artificial sequence
<220>
   <223> Synthetic oligonucleotide
<400> 102
   tgctcagcag tgtcagcagg 20
<210> 103
   <211> 20
   <212> DNA
   <213> Artificial sequence
<220>
   <223> Synthetic oligonucleotide
<400> 103
   ttgctcagca gtgtcagcag 20
<210> 104
   <211> 20
   <212> DNA
   <213> Artificial sequence
<220>
   <223> Synthetic oligonucleotide
<400> 104
   cttgctcagc agtgtcagca 20
<210> 105
   <211> 20
   <212> DNA
   <213> Artificial sequence
<220>
   <223> Synthetic oligonucleotide
<400> 105
   acttgctcag cagtgtcagc 20
<210> 106
   <211> 20
   <212> DNA
   <213> Artificial sequence
<220>
   <223> Synthetic oligonucleotide
<400> 106
   aacttgctca gcagtgtcag 20
<210> 107
   <211> 20
   <212> DNA
   <213> Artificial sequence
<220>
   <223> Synthetic oligonucleotide
<400> 107
   aaacttgctc agcagtgtca 20
<210> 108
   <211> 20
   <212> DNA
   <213> Artificial sequence
<220>
   <223> Synthetic oligonucleotide
<400> 108
   caaacttgct cagcagtgtc 20
<210> 109
   <211> 20
   <212> DNA
   <213> Artificial sequence
<220>
   <223> Synthetic oligonucleotide
<400> 109
   ccaaacttgc tcagcagtgt 20
<210> 110
   <211> 20
   <212> DNA
   <213> Artificial sequence
<220>
   <223> Synthetic oligonucleotide
<400> 110
   cccaaacttg ctcagcagtg 20
<210> 111
   <211> 20
   <212> DNA
   <213> Artificial sequence
<220>
   <223> Synthetic oligonucleotide
<400> 111
   gtgagcccgt cctccaccaa 20
<210> 112
   <211> 20
   <212> DNA
   <213> Artificial sequence
<220>
   <223> Synthetic oligonucleotide
<400> 112
   agtgagcccg tcctccacca 20
<210> 113
   <211> 20
   <212> DNA
   <213> Artificial sequence
<220>
   <223> Synthetic oligonucleotide
<400> 113
   cagtgagccc gtcctccacc 20
<210> 114
   <211> 20
   <212> DNA
   <213> Artificial sequence
<220>
   <223> Synthetic oligonucleotide
<400> 114
   gcagtgagcc cgtcctccac 20
<210> 115
   <211> 20
   <212> DNA
   <213> Artificial sequence
<220>
   <223> Synthetic oligonucleotide
<400> 115
   ggcagtgagc ccgtcctcca 20
<210> 116
   <211> 20
   <212> DNA
   <213> Artificial sequence
<220>
   <223> Synthetic oligonucleotide
<400> 116
   tggcagtgag cccgtcctcc 20
<210> 117
   <211> 20
   <212> DNA
   <213> Artificial sequence
<220>
   <223> Synthetic oligonucleotide
<400> 117
   catggcagtg agcccgtcct 20
<210> 118
   <211> 20
   <212> DNA
   <213> Artificial sequence
<220>
   <223> Synthetic oligonucleotide
<400> 118
   ccatggcagt gagcccgtcc 20
<210> 119
   <211> 20
   <212> DNA
   <213> Artificial sequence
<220>
   <223> Synthetic oligonucleotide
<400> 119
   tccatggcag tgagcccgtc 20
<210> 120
   <211> 20
   <212> DNA
   <213> Artificial sequence
<220>
   <223> Synthetic oligonucleotide
<400> 120
   ctccatggca gtgagcccgt 20
<210> 121
   <211> 20
   <212> DNA
   <213> Artificial sequence
<220>
   <223> Synthetic oligonucleotide
<400> 121
   tctccatggc agtgagcccg 20
<210> 122
   <211> 20
   <212> DNA
   <213> Artificial sequence
<220>
   <223> Synthetic oligonucleotide
<400> 122
   gtctccatgg cagtgagccc 20
<210> 123
   <211> 20
   <212> DNA
   <213> Artificial sequence
<220>
   <223> Synthetic oligonucleotide
<400> 123
   ccttcccgaa tgtccgacag 20
<210> 124
   <211> 20
   <212> DNA
   <213> Artificial sequence
<220>
   <223> Synthetic oligonucleotide
<400> 124
   accttcccga atgtccgaca 20
<210> 125
   <211> 20
   <212> DNA
   <213> Artificial sequence
<220>
   <223> Synthetic oligonucleotide
<400> 125
   caccttcccg aatgtccgac 20
<210> 126
   <211> 20
   <212> DNA
   <213> Artificial sequence
<220>
   <223> Synthetic oligonucleotide
<400> 126
   gcaccttccc gaatgtccga 20
<210> 127
   <211> 20
   <212> DNA
   <213> Artificial sequence
<220>
   <223> Synthetic oligonucleotide
<400> 127
   cgcaccttcc cgaatgtccg 20
<210> 128
   <211> 20
   <212> DNA
   <213> Artificial sequence
<220>
   <223> Synthetic oligonucleotide
<400> 128
   ggcgcacctt cccgaatgtc 20
<210> 129
   <211> 20
   <212> DNA
   <213> Artificial sequence
<220>
   <223> Synthetic oligonucleotide
<400> 129
   acaaaaggca ggtggacccc 20
<210> 130
   <211> 20
   <212> DNA
   <213> Artificial sequence
<220>
   <223> Synthetic oligonucleotide
<400> 130
   cacaaaaggc aggtggaccc 20
<210> 131
   <211> 20
   <212> DNA
   <213> Artificial sequence
<220>
   <223> Synthetic oligonucleotide
<400> 131
   ccacaaaagg caggtggacc 20
<210> 132
   <211> 20
   <212> DNA
   <213> Artificial sequence
<220>
   <223> Synthetic oligonucleotide
<400> 132
   cccacaaaag gcaggtggac 20
<210> 133
   <211> 20
   <212> DNA
   <213> Artificial sequence
<220>
   <223> Synthetic oligonucleotide
<400> 133
   gcccacaaaa ggcaggtgga 20
<210> 134
   <211> 20
   <212> DNA
   <213> Artificial sequence
<220>
   <223> Synthetic oligonucleotide
<400> 134
   agcccacaaa aggcaggtgg 20
<210> 135
   <211> 20
   <212> DNA
   <213> Artificial sequence
<220>
   <223> Synthetic oligonucleotide
<400> 135
   tagcccacaa aaggcaggtg 20
<210> 136
   <211> 20
   <212> DNA
   <213> Artificial sequence
<220>
   <223> Synthetic oligonucleotide
<400> 136
   gtagcccaca aaaggcaggt 20
<210> 137
   <211> 20
   <212> DNA
   <213> Artificial sequence
<220>
   <223> Synthetic oligonucleotide
<400> 137
   agtagcccac aaaaggcagg 20
<210> 138
   <211> 20
   <212> DNA
   <213> Artificial sequence
<220>
   <223> Synthetic oligonucleotide
<400> 138
   gagtagccca caaaaggcag 20
<210> 139
   <211> 20
   <212> DNA
   <213> Artificial sequence
<220>
   <223> Synthetic oligonucleotide
<400> 139
   ggagtagccc acaaaaggca 20
<210> 140
   <211> 20
   <212> DNA
   <213> Artificial sequence
<220>
   <223> Synthetic oligonucleotide
<400> 140
   aggagtagcc cacaaaaggc 20
<210> 141
   <211> 20
   <212> DNA
   <213> Artificial sequence
<220>
   <223> Synthetic oligonucleotide
<400> 141
   taggagtagc ccacaaaagg 20
<210> 142
   <211> 20
   <212> DNA
   <213> Artificial sequence
<220>
   <223> Synthetic oligonucleotide
<400> 142
   gtaggagtag cccacaaaag 20
<210> 143
   <211> 20
   <212> DNA
   <213> Artificial sequence
<220>
   <223> Synthetic oligonucleotide
<400> 143
   agtaggagta gcccacaaaa 20
<210> 144
   <211> 20
   <212> DNA
   <213> Artificial sequence
<220>
   <223> Synthetic oligonucleotide
<400> 144
   gagtaggagt agcccacaaa 20
<210> 145
   <211> 20
   <212> DNA
   <213> Artificial sequence
<220>
   <223> Synthetic oligonucleotide
<400> 145
   ggagtaggag tagcccacaa 20
<210> 146
   <211> 20
   <212> DNA
   <213> Artificial sequence
<220>
   <223> Synthetic oligonucleotide
<400> 146
   aggagtagga gtagcccaca 20
<210> 147
   <211> 20
   <212> DNA
   <213> Artificial sequence
<220>
   <223> Synthetic oligonucleotide
<400> 147
   caggagtagg agtagcccac 20
<210> 148
   <211> 20
   <212> DNA
   <213> Artificial sequence
<220>
   <223> Synthetic oligonucleotide
<400> 148
   gcaggagtag gagtagccca 20
<210> 149
   <211> 20
   <212> DNA
   <213> Artificial sequence
<220>
   <223> Synthetic oligonucleotide
<400> 149
   tgcaggagta ggagtagccc 20
<210> 150
   <211> 20
   <212> DNA
   <213> Artificial sequence
<220>
   <223> Synthetic oligonucleotide
<400> 150
   atgcaggagt aggagtagcc 20
<210> 151
   <211> 20
   <212> DNA
   <213> Artificial sequence
<220>
   <223> Synthetic oligonucleotide
<400> 151
   catgcaggag taggagtagc 20
<210> 152
   <211> 20
   <212> DNA
   <213> Artificial sequence
<220>
   <223> Synthetic oligonucleotide
<400> 152
   ccatgcagga gtaggagtag 20
<210> 153
   <211> 20
   <212> DNA
   <213> Artificial sequence
<220>
   <223> Synthetic oligonucleotide
<400> 153
   gccatgcagg agtaggagta 20
<210> 154
   <211> 20
   <212> DNA
   <213> Artificial sequence
<220>
   <223> Synthetic oligonucleotide
<400> 154
   ggccatgcag gagtaggagt 20
<210> 155
   <211> 20
   <212> DNA
   <213> Artificial sequence
<220>
   <223> Synthetic oligonucleotide
<400> 155
   gggccatgca ggagtaggag 20
<210> 156
   <211> 20
   <212> DNA
   <213> Artificial sequence
<220>
   <223> Synthetic oligonucleotide
<400> 156
   agggccatgc aggagtagga 20
<210> 157
   <211> 20
   <212> DNA
   <213> Artificial sequence
<220>
   <223> Synthetic oligonucleotide
<400> 157
   gagggccatg caggagtagg 20
<210> 158
   <211> 20
   <212> DNA
   <213> Artificial sequence
<220>
   <223> Synthetic oligonucleotide
<400> 158
   ctgagggcca tgcaggagta 20
<210> 159
   <211> 20
   <212> DNA
   <213> Artificial sequence
<220>
   <223> Synthetic oligonucleotide
<400> 159
   cctgagggcc atgcaggagt 20
<210> 160
   <211> 20
   <212> DNA
   <213> Artificial sequence
<220>
   <223> Synthetic oligonucleotide
<400> 160
   ccctgagggc catgcaggag 20
<210> 161
   <211> 20
   <212> DNA
   <213> Artificial sequence
<220>
   <223> Synthetic oligonucleotide
<400> 161
   tccctgaggg ccatgcagga 20
<210> 162
   <211> 20
   <212> DNA
   <213> Artificial sequence
<220>
   <223> Synthetic oligonucleotide
<400> 162
   gtccctgagg gccatgcagg 20
<210> 163
   <211> 20
   <212> DNA
   <213> Artificial sequence
<220>
   <223> Synthetic oligonucleotide
<400> 163
   tgtccctgag ggccatgcag 20
<210> 164
   <211> 20
   <212> DNA
   <213> Artificial sequence
<220>
   <223> Synthetic oligonucleotide
<400> 164
   ctgtccctga gggccatgca 20
<210> 165
   <211> 20
   <212> DNA
   <213> Artificial sequence
<220>
   <223> Synthetic oligonucleotide
<400> 165
   actgtccctg agggccatgc 20
<210> 166
   <211> 20
   <212> DNA
   <213> Artificial sequence
<220>
   <223> Synthetic oligonucleotide
<400> 166
   cactgtccct gagggccatg 20
<210> 167
   <211> 20
   <212> DNA
   <213> Artificial sequence
<220>
   <223> Synthetic oligonucleotide
<400> 167
   tcactgtccc tgagggccat 20
<210> 168
   <211> 20
   <212> DNA
   <213> Artificial sequence
<220>
   <223> Synthetic oligonucleotide
<400> 168
   ctcactgtcc ctgagggcca 20
<210> 169
   <211> 20
   <212> DNA
   <213> Artificial sequence
<220>
   <223> Synthetic oligonucleotide
<400> 169
   cctcactgtc cctgagggcc 20
<210> 170
   <211> 20
   <212> DNA
   <213> Artificial sequence
<220>
   <223> Synthetic oligonucleotide
<400> 170
   acctcactgt ccctgagggc 20
<210> 171
   <211> 20
   <212> DNA
   <213> Artificial sequence
<220>
   <223> Synthetic oligonucleotide
<400> 171
   gacctcactg tccctgaggg 20
<210> 172
   <211> 20
   <212> DNA
   <213> Artificial sequence
<220>
   <223> Synthetic oligonucleotide
<400> 172
   ggacctcact gtccctgagg 20
<210> 173
   <211> 20
   <212> DNA
   <213> Artificial sequence
<220>
   <223> Synthetic oligonucleotide
<400> 173
   gggacctcac tgtccctgag 20
<210> 174
   <211> 20
   <212> DNA
   <213> Artificial sequence
<220>
   <223> Synthetic oligonucleotide
<400> 174
   cctccagttc catgggtgtg 20
<210> 175
   <211> 20
   <212> DNA
   <213> Artificial sequence
<220>
   <223> Synthetic oligonucleotide
<400> 175
   gcctccagtt ccatgggtgt 20
<210> 176
   <211> 20
   <212> DNA
   <213> Artificial sequence
<220>
   <223> Synthetic oligonucleotide
<400> 176
   ggcctccagt tccatgggtg 20
<210> 177
   <211> 20
   <212> DNA
   <213> Artificial sequence
<220>
   <223> Synthetic oligonucleotide
<400> 177
   cggcctccag ttccatgggt 20
<210> 178
   <211> 20
   <212> DNA
   <213> Artificial sequence
<220>
   <223> Synthetic oligonucleotide
<400> 178
   tcggcctcca gttccatggg 20
<210> 179
   <211> 20
   <212> DNA
   <213> Artificial sequence
<220>
   <223> Synthetic oligonucleotide
<400> 179
   ctcggcctcc agttccatgg 20
<210> 180
   <211> 20
   <212> DNA
   <213> Artificial sequence
<220>
   <223> Synthetic oligonucleotide
<400> 180
   gctcggcctc cagttccatg 20
<210> 181
   <211> 20
   <212> DNA
   <213> Artificial sequence
<220>
   <223> Synthetic oligonucleotide
<400> 181
   tgctcggcct ccagttccat 20
<210> 182
   <211> 20
   <212> DNA
   <213> Artificial sequence
<220>
   <223> Synthetic oligonucleotide
<400> 182
   ctgctcggcc tccagttcca 20
<210> 183
   <211> 20
   <212> DNA
   <213> Artificial sequence
<220>
   <223> Synthetic oligonucleotide
<400> 183
   gctgctcggc ctccagttcc 20
<210> 184
   <211> 20
   <212> DNA
   <213> Artificial sequence
<220>
   <223> Synthetic oligonucleotide
<400> 184
   agctgctcgg cctccagttc 20
<210> 185
   <211> 20
   <212> DNA
   <213> Artificial sequence
<220>
   <223> Synthetic oligonucleotide
<400> 185
   cagctgctcg gcctccagtt 20
<210> 186
   <211> 20
   <212> DNA
   <213> Artificial sequence
<220>
   <223> Synthetic oligonucleotide
<400> 186
   gcagctgctc ggcctccagt 20
<210> 187
   <211> 20
   <212> DNA
   <213> Artificial sequence
<220>
   <223> Synthetic oligonucleotide
<400> 187
   agcagctgct cggcctccag 20
<210> 188
   <211> 20
   <212> DNA
   <213> Artificial sequence
<220>
   <223> Synthetic oligonucleotide
<400> 188
   aagcagctgc tcggcctcca 20
<210> 189
   <211> 20
   <212> DNA
   <213> Artificial sequence
<220>
   <223> Synthetic oligonucleotide
<400> 189
   caagcagctg ctcggcctcc 20
<210> 190
   <211> 20
   <212> DNA
   <213> Artificial sequence
<220>
   <223> Synthetic oligonucleotide
<400> 190
   tcaagcagct gctcggcctc 20
<210> 191
   <211> 20
   <212> DNA
   <213> Artificial sequence
<220>
   <223> Synthetic oligonucleotide
<400> 191
   ctcaagcagc tgctcggcct 20
<210> 192
   <211> 20
   <212> DNA
   <213> Artificial sequence
<220>
   <223> Synthetic oligonucleotide
<400> 192
   gctcaagcag ctgctcggcc 20
<210> 193
   <211> 20
   <212> DNA
   <213> Artificial sequence
<220>
   <223> Synthetic oligonucleotide
<400> 193
   ggctcaagca gctgctcggc 20
<210> 194
   <211> 20
   <212> DNA
   <213> Artificial sequence
<220>
   <223> Synthetic oligonucleotide
<400> 194
   tggctcaagc agctgctcgg 20
<210> 195
   <211> 20
   <212> DNA
   <213> Artificial sequence
<220>
   <223> Synthetic oligonucleotide
<400> 195
   gtggctcaag cagctgctcg 20
<210> 196
   <211> 20
   <212> DNA
   <213> Artificial sequence
<220>
   <223> Synthetic oligonucleotide
<400> 196
   tgtggctcaa gcagctgctc 20
<210> 197
   <211> 20
   <212> DNA
   <213> Artificial sequence
<220>
   <223> Synthetic oligonucleotide
<400> 197
   gtgtggctca agcagctgct 20
<210> 198
   <211> 20
   <212> DNA
   <213> Artificial sequence
<220>
   <223> Synthetic oligonucleotide
<400> 198
   ccacttcagc tgtttcatcc 20
<210> 199
   <211> 20
   <212> DNA
   <213> Artificial sequence
<220>
   <223> Synthetic oligonucleotide
<400> 199
   tgccacttca gctgtttcat 20
<210> 200
   <211> 20
   <212> DNA
   <213> Artificial sequence
<220>
   <223> Synthetic oligonucleotide
<400> 200
   ctgccacttc agctgtttca 20
<210> 201
   <211> 20
   <212> DNA
   <213> Artificial sequence
<220>
   <223> Synthetic oligonucleotide
<400> 201
   actgccactt cagctgtttc 20
<210> 202
   <211> 20
   <212> DNA
   <213> Artificial sequence
<220>
   <223> Synthetic oligonucleotide
<400> 202
   aactgccact tcagctgttt 20
<210> 203
   <211> 20
   <212> DNA
   <213> Artificial sequence
<220>
   <223> Synthetic oligonucleotide
<400> 203
   gaactgccac ttcagctgtt 20
<210> 204
   <211> 20
   <212> DNA
   <213> Artificial sequence
<220>
   <223> Synthetic oligonucleotide
<400> 204
   ggaactgcca cttcagctgt 20
<210> 205
   <211> 20
   <212> DNA
   <213> Artificial sequence
<220>
   <223> Synthetic oligonucleotide
<400> 205
   tggaactgcc acttcagctg 20
<210> 206
   <211> 20
   <212> DNA
   <213> Artificial sequence
<220>
   <223> Synthetic oligonucleotide
<400> 206
   ctggaactgc cacttcagct 20
<210> 207
   <211> 20
   <212> DNA
   <213> Artificial sequence
<220>
   <223> Synthetic oligonucleotide
<400> 207
   gctggaactg ccacttcagc 20
<210> 208
   <211> 20
   <212> DNA
   <213> Artificial sequence
<220>
   <223> Synthetic oligonucleotide
<400> 208
   cgctggaact gccacttcag 20
<210> 209
   <211> 20
   <212> DNA
   <213> Artificial sequence
<220>
   <223> Synthetic oligonucleotide
<400> 209
   ccgctggaac tgccacttca 20
<210> 210
   <211> 20
   <212> DNA
   <213> Artificial sequence
<220>
   <223> Synthetic oligonucleotide
<400> 210
   gccgctggaa ctgccacttc 20
<210> 211
   <211> 20
   <212> DNA
   <213> Artificial sequence
<220>
   <223> Synthetic oligonucleotide
<400> 211
   agccgctgga actgccactt 20
<210> 212
   <211> 20
   <212> DNA
   <213> Artificial sequence
<220>
   <223> Synthetic oligonucleotide
<400> 212
   ctcagcctct gccgcaggga 20
<210> 213
   <211> 20
   <212> DNA
   <213> Artificial sequence
<220>
   <223> Synthetic oligonucleotide
<400> 213
   cctcagcctc tgccgcaggg 20
<210> 214
   <211> 20
   <212> DNA
   <213> Artificial sequence
<220>
   <223> Synthetic oligonucleotide
<400> 214
   ggcctcagcc tctgccgcag 20
<210> 215
   <211> 20
   <212> DNA
   <213> Artificial sequence
<220>
   <223> Synthetic oligonucleotide
<400> 215
   cggcctcagc ctctgccgca 20
<210> 216
   <211> 20
   <212> DNA
   <213> Artificial sequence
<220>
   <223> Synthetic oligonucleotide
<400> 216
   tcggcctcag cctctgccgc 20
<210> 217
   <211> 20
   <212> DNA
   <213> Artificial sequence
<220>
   <223> Synthetic oligonucleotide
<400> 217
   ctcggcctca gcctctgccg 20
<210> 218
   <211> 20
   <212> DNA
   <213> Artificial sequence
<220>
   <223> Synthetic oligonucleotide
<400> 218
   cctcggcctc agcctctgcc 20
<210> 219
   <211> 20
   <212> DNA
   <213> Artificial sequence
<220>
   <223> Synthetic oligonucleotide
<400> 219
   acctcggcct cagcctctgc 20
<210> 220
   <211> 20
   <212> DNA
   <213> Artificial sequence
<220>
   <223> Synthetic oligonucleotide
<400> 220
   cacctcggcc tcagcctctg 20
<210> 221
   <211> 20
   <212> DNA
   <213> Artificial sequence
<220>
   <223> Synthetic oligonucleotide
<400> 221
   tcacctcggc ctcagcctct 20
<210> 222
   <211> 20
   <212> DNA
   <213> Artificial sequence
<220>
   <223> Synthetic oligonucleotide
<400> 222
   gtcacctcgg cctcagcctc 20
<210> 223
   <211> 20
   <212> DNA
   <213> Artificial sequence
<220>
   <223> Synthetic oligonucleotide
<400> 223
   cgtcacctcg gcctcagcct 20
<210> 224
   <211> 20
   <212> DNA
   <213> Artificial sequence
<220>
   <223> Synthetic oligonucleotide
<400> 224
   agcacctcct cctccagggc 20
<210> 225
   <211> 20
   <212> DNA
   <213> Artificial sequence
<220>
   <223> Synthetic oligonucleotide
<400> 225
   gagcacctcc tcctccaggg 20
<210> 226
   <211> 20
   <212> DNA
   <213> Artificial sequence
<220>
   <223> Synthetic oligonucleotide
<400> 226
   tgagcacctc ctcctccagg 20
<210> 227
   <211> 20
   <212> DNA
   <213> Artificial sequence
<220>
   <223> Synthetic oligonucleotide
<400> 227
   gtgagcacct cctcctccag 20
<210> 228
   <211> 20
   <212> DNA
   <213> Artificial sequence
<220>
   <223> Synthetic oligonucleotide
<400> 228
   ggtgagcacc tcctcctcca 20
<210> 229
   <211> 20
   <212> DNA
   <213> Artificial sequence
<220>
   <223> Synthetic oligonucleotide
<400> 229
   gggtgagcac ctcctcctcc 20
<210> 230
   <211> 20
   <212> DNA
   <213> Artificial sequence
<220>
   <223> Synthetic oligonucleotide
<400> 230
   cgggtgagca cctcctcctc 20
<210> 231
   <211> 20
   <212> DNA
   <213> Artificial sequence
<220>
   <223> Synthetic oligonucleotide
<400> 231
   ccgggtgagc acctcctcct 20
<210> 232
   <211> 20
   <212> DNA
   <213> Artificial sequence
<220>
   <223> Synthetic oligonucleotide
<400> 232
   gccgggtgag cacctcctcc 20
<210> 233
   <211> 20
   <212> DNA
   <213> Artificial sequence
<220>
   <223> Synthetic oligonucleotide
<400> 233
   tgccgggtga gcacctcctc 20
<210> 234
   <211> 20
   <212> DNA
   <213> Artificial sequence
<220>
   <223> Synthetic oligonucleotide
<400> 234
   ctgccgggtg agcacctcct 20
<210> 235
   <211> 20
   <212> DNA
   <213> Artificial sequence
<220>
   <223> Synthetic oligonucleotide
<400> 235
   tctgccgggt gagcacctcc 20
<210> 236
   <211> 20
   <212> DNA
   <213> Artificial sequence
<220>
   <223> Synthetic oligonucleotide
<400> 236
   gctctgccgg gtgagcacct 20
<210> 237
   <211> 20
   <212> DNA
   <213> Artificial sequence
<220>
   <223> Synthetic oligonucleotide
<400> 237
   ggctctgccg ggtgagcacc 20
<210> 238
   <211> 20
   <212> DNA
   <213> Artificial sequence
<220>
   <223> Synthetic oligonucleotide
<400> 238
   aggctctgcc gggtgagcac 20
<210> 239
   <211> 20
   <212> DNA
   <213> Artificial sequence
<220>
   <223> Synthetic oligonucleotide
<400> 239
   caggctctgc cgggtgagca 20
<210> 240
   <211> 20
   <212> DNA
   <213> Artificial sequence
<220>
   <223> Synthetic oligonucleotide
<400> 240
   tcaggctctg ccgggtgagc 20
<210> 241
   <211> 20
   <212> DNA
   <213> Artificial sequence
<220>
   <223> Synthetic oligonucleotide
<400> 241
   gctcaggctc tgccgggtga 20
<210> 242
   <211> 20
   <212> DNA
   <213> Artificial sequence
<220>
   <223> Synthetic oligonucleotide
<400> 242
   cggctcaggc tctgccgggt 20
<210> 243
   <211> 20
   <212> DNA
   <213> Artificial sequence
<220>
   <223> Synthetic oligonucleotide
<400> 243
   ccggctcagg ctctgccggg 20
<210> 244
   <211> 20
   <212> DNA
   <213> Artificial sequence
<220>
   <223> Synthetic oligonucleotide
<400> 244
   cccggctcag gctctgccgg 20
<210> 245
   <211> 20
   <212> DNA
   <213> Artificial sequence
<220>
   <223> Synthetic oligonucleotide
<400> 245
   tcccggctca ggctctgccg 20
<210> 246
   <211> 20
   <212> DNA
   <213> Artificial sequence
<220>
   <223> Synthetic oligonucleotide
<400> 246
   ctcccggctc aggctctgcc 20
<210> 247
   <211> 20
   <212> DNA
   <213> Artificial sequence
<220>
   <223> Synthetic oligonucleotide
<400> 247
   tctcccggct caggctctgc 20
<210> 248
   <211> 20
   <212> DNA
   <213> Artificial sequence
<220>
   <223> Synthetic oligonucleotide
<400> 248
   atctcccggc tcaggctctg 20
<210> 249
   <211> 20
   <212> DNA
   <213> Artificial sequence
<220>
   <223> Synthetic oligonucleotide
<400> 249
   catctcccgg ctcaggctct 20
<210> 250
   <211> 20
   <212> DNA
   <213> Artificial sequence
<220>
   <223> Synthetic oligonucleotide
<400> 250
   ccatctcccg gctcaggctc 20
<210> 251
   <211> 20
   <212> DNA
   <213> Artificial sequence
<220>
   <223> Synthetic oligonucleotide
<400> 251
   tccatctccc ggctcaggct 20
<210> 252
   <211> 20
   <212> DNA
   <213> Artificial sequence
<220>
   <223> Synthetic oligonucleotide
<400> 252
   ctccatctcc cggctcaggc 20
<210> 253
   <211> 20
   <212> DNA
   <213> Artificial sequence
<220>
   <223> Synthetic oligonucleotide
<400> 253
   cctccatctc ccggctcagg 20
<210> 254
   <211> 20
   <212> DNA
   <213> Artificial sequence
<220>
   <223> Synthetic oligonucleotide
<400> 254
   gcctccatct cccggctcag 20
<210> 255
   <211> 20
   <212> DNA
   <213> Artificial sequence
<220>
   <223> Synthetic oligonucleotide
<400> 255
   ggcctccatc tcccggctca 20
<210> 256
   <211> 20
   <212> DNA
   <213> Artificial sequence
<220>
   <223> Synthetic oligonucleotide
<400> 256
   tggcctccat ctcccggctc 20
<210> 257
   <211> 20
   <212> DNA
   <213> Artificial sequence
<220>
   <223> Synthetic oligonucleotide
<400> 257
   atggcctcca tctcccggct 20
<210> 258
   <211> 20
   <212> DNA
   <213> Artificial sequence
<220>
   <223> Synthetic oligonucleotide
<400> 258
   gatggcctcc atctcccggc 20
<210> 259
   <211> 20
   <212> DNA
   <213> Artificial sequence
<220>
   <223> Synthetic oligonucleotide
<400> 259
   ggatggcctc catctcccgg 20
<210> 260
   <211> 20
   <212> DNA
   <213> Artificial sequence
<220>
   <223> Synthetic oligonucleotide
<400> 260
   cggatggcct ccatctcccg 20
<210> 261
   <211> 20
   <212> DNA
   <213> Artificial sequence
<220>
   <223> Synthetic oligonucleotide
<400> 261
   gcggatggcc tccatctccc 20
<210> 262
   <211> 20
   <212> DNA
   <213> Artificial sequence
<220>
   <223> Synthetic oligonucleotide
<400> 262
   tgcggatggc ctccatctcc 20
<210> 263
   <211> 20
   <212> DNA
   <213> Artificial sequence
<220>
   <223> Synthetic oligonucleotide
<400> 263
   gttccgagcc tctgcctcgc 20
<210> 264
   <211> 20
   <212> DNA
   <213> Artificial sequence
<220>
   <223> Synthetic oligonucleotide
<400> 264
   gtcggaggac gaggtcaata 20
<210> 265
   <211> 20
   <212> DNA
   <213> Artificial sequence
<220>
   <223> Synthetic oligonucleotide
<400> 265
   agggcctcag cctggccgaa 20
<210> 266
   <211> 20
   <212> DNA
   <213> Artificial sequence
<220>
   <223> Synthetic oligonucleotide
<400> 266
   cagggcctca gcctggccga 20
<210> 267
   <211> 20
   <212> DNA
   <213> Artificial sequence
<220>
   <223> Synthetic oligonucleotide
<400> 267
   gtcagggcct cagcctggcc 20
<210> 268
   <211> 20
   <212> DNA
   <213> Artificial sequence
<220>
   <223> Synthetic oligonucleotide
<400> 268
   cgtcagggcc tcagcctggc 20
<210> 269
   <211> 20
   <212> DNA
   <213> Artificial sequence
<220>
   <223> Synthetic oligonucleotide
<400> 269
   agcaaatttc ccgagtaagc 20
<210> 270
   <211> 20
   <212> DNA
   <213> Artificial sequence
<220>
   <223> Synthetic oligonucleotide
<400> 270
   aagcaaattt cccgagtaag 20
<210> 271
   <211> 20
   <212> DNA
   <213> Artificial sequence
<220>
   <223> Synthetic oligonucleotide
<400> 271
   aaaagcaaat ttcccgagta 20
<210> 272
   <211> 20
   <212> DNA
   <213> Artificial sequence
<220>
   <223> Synthetic oligonucleotide
<400> 272
   caaaagcaaa tttcccgagt 20
<210> 273
   <211> 20
   <212> DNA
   <213> Artificial sequence
<220>
   <223> Synthetic oligonucleotide
<400> 273
   gcaaaagcaa atttcccgag 20
<210> 274
   <211> 20
   <212> DNA
   <213> Artificial sequence
<220>
   <223> Synthetic oligonucleotide
<400> 274
   ggcaaaagca aatttcccga 20
<210> 275
   <211> 20
   <212> DNA
   <213> Artificial sequence
<220>
   <223> Synthetic oligonucleotide
<400> 275
   tggcaaaagc aaatttcccg 20
<210> 276
   <211> 20
   <212> DNA
   <213> Artificial sequence
<220>
   <223> Synthetic oligonucleotide
<400> 276
   tttggcaaaa gcaaatttcc 20
<210> 277
   <211> 20
   <212> DNA
   <213> Artificial sequence
<220>
   <223> Synthetic oligonucleotide
<400> 277
   gtttggcaaa agcaaatttc 20
<210> 278
   <211> 20
   <212> DNA
   <213> Artificial sequence
<220>
   <223> Synthetic oligonucleotide
<400> 278
   gggtttggca aaagcaaatt 20
<210> 279
   <211> 20
   <212> DNA
   <213> Artificial sequence
<220>
   <223> Synthetic oligonucleotide
<400> 279
   cgggtttggc aaaagcaaat 20
<210> 280
   <211> 20
   <212> DNA
   <213> Artificial sequence
<220>
   <223> Synthetic oligonucleotide
<400> 280
   aagcgggttt ggcaaaagca 20
<210> 281
   <211> 20
   <212> DNA
   <213> Artificial sequence
<220>
   <223> Synthetic oligonucleotide
<400> 281
   aatatccaaa ccgccgaagc 20
<210> 282
   <211> 20
   <212> DNA
   <213> Artificial sequence
<220>
   <223> Synthetic oligonucleotide
<400> 282
   aaatatccaa accgccgaag 20
<210> 283
   <211> 20
   <212> DNA
   <213> Artificial sequence
<220>
   <223> Synthetic oligonucleotide
<400> 283
   ataaatatcc aaaccgccga 20
<210> 284
   <211> 20
   <212> DNA
   <213> Artificial sequence
<220>
   <223> Synthetic oligonucleotide
<400> 284
   aataaatatc caaaccgccg 20
<210> 285
   <211> 20
   <212> DNA
   <213> Artificial sequence
<220>
   <223> Synthetic oligonucleotide
<400> 285
   tcaataaata tccaaaccgc 20
<210> 286
   <211> 20
   <212> DNA
   <213> Artificial sequence
<220>
   <223> Synthetic oligonucleotide
<400> 286
   gtcaataaat atccaaaccg 20
<210> 287
   <211> 20
   <212> DNA
   <213> Artificial sequence
<220>
   <223> Synthetic oligonucleotide
<400> 287
   ggtcaataaa tatccaaacc 20
<210> 288
   <211> 20
   <212> DNA
   <213> Artificial sequence
<220>
   <223> Synthetic oligonucleotide
<400> 288
   aggtcaataa atatccaaac 20
<210> 289
   <211> 20
   <212> DNA
   <213> Artificial sequence
<220>
   <223> Synthetic oligonucleotide
<400> 289
   gaggtcaata aatatccaaa 20
<210> 290
   <211> 20
   <212> DNA
   <213> Artificial sequence
<220>
   <223> Synthetic oligonucleotide
<400> 290
   acgaggtcaa taaatatcca 20
<210> 291
   <211> 20
   <212> DNA
   <213> Artificial sequence
<220>
   <223> Synthetic oligonucleotide
<400> 291
   gacgaggtca ataaatatcc 20
<210> 292
   <211> 20
   <212> DNA
   <213> Artificial sequence
<220>
   <223> Synthetic oligonucleotide
<400> 292
   aggacgaggt caataaatat 20
<210> 293
   <211> 20
   <212> DNA
   <213> Artificial sequence
<220>
   <223> Synthetic oligonucleotide
<400> 293
   gaggacgagg tcaataaata 20
<210> 294
   <211> 20
   <212> DNA
   <213> Artificial sequence
<220>
   <223> Synthetic oligonucleotide
<400> 294
   cggaggacga ggtcaataaa 20
<210> 295
   <211> 20
   <212> DNA
   <213> Artificial sequence
<220>
   <223> Synthetic oligonucleotide
<400> 295
   tcggaggacg aggtcaataa 20
<210> 296
   <211> 20
   <212> DNA
   <213> Artificial sequence
<220>
   <223> Synthetic oligonucleotide
<400> 296
   agtcggagga cgaggtcaat 20
<210> 297
   <211> 20
   <212> DNA
   <213> Artificial sequence
<220>
   <223> Synthetic oligonucleotide
<400> 297
   gagtcggagg acgaggtcaa 20
<210> 298
   <211> 20
   <212> DNA
   <213> Artificial sequence
<220>
   <223> Synthetic oligonucleotide
<400> 298
   gcgagtcgga ggacgaggtc 20
<210> 299
   <211> 20
   <212> DNA
   <213> Artificial sequence
<220>
   <223> Synthetic oligonucleotide
<400> 299
   agcgagtcgg aggacgaggt 20
<210> 300
   <211> 20
   <212> DNA
   <213> Artificial sequence
<220>
   <223> Synthetic oligonucleotide
<400> 300
   cagcgagtcg gaggacgagg 20
<210> 301
   <211> 20
   <212> DNA
   <213> Artificial sequence
<220>
   <223> Synthetic oligonucleotide
<400> 301
   tcagcgagtc ggaggacgag 20
<210> 302
   <211> 20
   <212> DNA
   <213> Artificial sequence
<220>
   <223> Synthetic oligonucleotide
<400> 302
   gtcagcgagt cggaggacga 20
<210> 303
   <211> 20
   <212> DNA
   <213> Artificial sequence
<220>
   <223> Synthetic oligonucleotide
<400> 303
   ctgtcagcga gtcggaggac 20
<210> 304
   <211> 20
   <212> DNA
   <213> Artificial sequence
<220>
   <223> Synthetic oligonucleotide
<400> 304
   cctgtcagcg agtcggagga 20
<210> 305
   <211> 20
   <212> DNA
   <213> Artificial sequence
<220>
   <223> Synthetic oligonucleotide
<400> 305
   agcctgtcag cgagtcggag 20
<210> 306
   <211> 20
   <212> DNA
   <213> Artificial sequence
<220>
   <223> Synthetic oligonucleotide
<400> 306
   gtctcagtgc atccaaaacg 20
<210> 307
   <211> 20
   <212> DNA
   <213> Artificial sequence
<220>
   <223> Synthetic oligonucleotide
<400> 307
   ggtctcagtg catccaaaac 20
<210> 308
   <211> 20
   <212> DNA
   <213> Artificial sequence
<220>
   <223> Synthetic oligonucleotide
<400> 308
   gggtctcagt gcatccaaaa 20
<210> 309
   <211> 20
   <212> DNA
   <213> Artificial sequence
<220>
   <223> Synthetic oligonucleotide
<400> 309
   ggagggcctt ttattcgcga 20
<210> 310
   <211> 20
   <212> DNA
   <213> Artificial sequence
<220>
   <223> Synthetic oligonucleotide
<400> 310
   tggagggcct tttattcgcg 20
<210> 311
   <211> 20
   <212> DNA
   <213> Artificial sequence
<220>
   <223> Synthetic oligonucleotide
<400> 311
   atggagggcc ttttattcgc 20
<210> 312
   <211> 20
   <212> DNA
   <213> Artificial sequence
<220>
   <223> Synthetic oligonucleotide
<400> 312
   gatggagggc cttttattcg 20
<210> 313
   <211> 20
   <212> DNA
   <213> Artificial sequence
<220>
   <223> Synthetic oligonucleotide
<400> 313
   agatggaggg ccttttattc 20
<210> 314
   <211> 20
   <212> DNA
   <213> Artificial sequence
<220>
   <223> Synthetic oligonucleotide
<400> 314
   cagatggagg gccttttatt 20
<210> 315
   <211> 20
   <212> DNA
   <213> Artificial sequence
<220>
   <223> Synthetic oligonucleotide
<400> 315
   gcagatggag ggccttttat 20
<210> 316
   <211> 20
   <212> DNA
   <213> Artificial sequence
<220>
   <223> Synthetic oligonucleotide
<400> 316
   ccctcaggct ctctgcttta 20
<210> 317
   <211> 20
   <212> DNA
   <213> Artificial sequence
<220>
   <223> Synthetic oligonucleotide
<400> 317
   gccctcaggc tctctgcttt 20
<210> 318
   <211> 20
   <212> DNA
   <213> Artificial sequence
<220>
   <223> Synthetic oligonucleotide
<400> 318
   agccctcagg ctctctgctt 20
<210> 319
   <211> 20
   <212> DNA
   <213> Artificial sequence
<220>
   <223> Synthetic oligonucleotide
<400> 319
   tagccctcag gctctctgct 20
<210> 320
   <211> 20
   <212> DNA
   <213> Artificial sequence
<220>
   <223> Synthetic oligonucleotide
<400> 320
   ttagccctca ggctctctgc 20
<210> 321
   <211> 20
   <212> DNA
   <213> Artificial sequence
<220>
   <223> Synthetic oligonucleotide
<400> 321
   tttagccctc aggctctctg 20
<210> 322
   <211> 20
   <212> DNA
   <213> Artificial sequence
<220>
   <223> Synthetic oligonucleotide
<400> 322
   atttagccct caggctctct 20
<210> 323
   <211> 20
   <212> DNA
   <213> Artificial sequence
<220>
   <223> Synthetic oligonucleotide
<400> 323
   aatttagccc tcaggctctc 20
<210> 324
   <211> 20
   <212> DNA
   <213> Artificial sequence
<220>
   <223> Synthetic oligonucleotide
<400> 324
   aaatttagcc ctcaggctct 20
<210> 325
   <211> 20
   <212> DNA
   <213> Artificial sequence
<220>
   <223> Synthetic oligonucleotide
<400> 325
   taaatttagc cctcaggctc 20
<210> 326
   <211> 20
   <212> DNA
   <213> Artificial sequence
<220>
   <223> Synthetic oligonucleotide
<400> 326
   ttaaatttag ccctcaggct 20
<210> 327
   <211> 20
   <212> DNA
   <213> Artificial sequence
<220>
   <223> Synthetic oligonucleotide
<400> 327
   gttaaattta gccctcaggc 20
<210> 328
   <211> 20
   <212> DNA
   <213> Artificial sequence
<220>
   <223> Synthetic oligonucleotide
<400> 328
   agttaaattt agccctcagg 20
<210> 329
   <211> 20
   <212> DNA
   <213> Artificial sequence
<220>
   <223> Synthetic oligonucleotide
<400> 329
   cagttaaatt tagccctcag 20
<210> 330
   <211> 20
   <212> DNA
   <213> Artificial sequence
<220>
   <223> Synthetic oligonucleotide
<400> 330
   acagttaaat ttagccctca 20
<210> 331
   <211> 20
   <212> DNA
   <213> Artificial sequence
<220>
   <223> Synthetic oligonucleotide
<400> 331
   gacagttaaa tttagccctc 20
<210> 332
   <211> 20
   <212> DNA
   <213> Artificial sequence
<220>
   <223> Synthetic oligonucleotide
<400> 332
   ggacagttaa atttagccct 20
<210> 333
   <211> 20
   <212> DNA
   <213> Artificial sequence
<220>
   <223> Synthetic oligonucleotide
<400> 333
   cggacagtta aatttagccc 20
<210> 334
   <211> 20
   <212> DNA
   <213> Artificial sequence
<220>
   <223> Synthetic oligonucleotide
<400> 334
   tcggacagtt aaatttagcc 20
<210> 335
   <211> 20
   <212> DNA
   <213> Artificial sequence
<220>
   <223> Synthetic oligonucleotide
<400> 335
   ctcggacagt taaatttagc 20
<210> 336
   <211> 20
   <212> DNA
   <213> Artificial sequence
<220>
   <223> Synthetic oligonucleotide
<400> 336
   actcggacag ttaaatttag 20
<210> 337
   <211> 20
   <212> DNA
   <213> Artificial sequence
<220>
   <223> Synthetic oligonucleotide
<400> 337
   gactcggaca gttaaattta 20
<210> 338
   <211> 20
   <212> DNA
   <213> Artificial sequence
<220>
   <223> Synthetic oligonucleotide
<400> 338
   cgactcggac agttaaattt 20
<210> 339
   <211> 20
   <212> DNA
   <213> Artificial sequence
<220>
   <223> Synthetic oligonucleotide
<400> 339
   ccgactcgga cagttaaatt 20
<210> 340
   <211> 20
   <212> DNA
   <213> Artificial sequence
<220>
   <223> Synthetic oligonucleotide
<400> 340
   tccgactcgg acagttaaat 20
<210> 341
   <211> 16
   <212> DNA
   <213> Artificial sequence
<220>
   <223> Synthetic oligonucleotide
<400> 341
   tctcctcacg gaagca 16
<210> 342
   <211> 16
   <212> DNA
   <213> Artificial sequence
<220>
   <223> Synthetic oligonucleotide
<400> 342
   ctctcctcac ggaagc 16
<210> 343
   <211> 16
   <212> DNA
   <213> Artificial sequence
<220>
   <223> Synthetic oligonucleotide
<400> 343
   cctctcctca cggaag 16
<210> 344
   <211> 16
   <212> DNA
   <213> Artificial sequence
<220>
   <223> Synthetic oligonucleotide
<400> 344
   aaacttgctc agcagt 16
<210> 345
   <211> 16
   <212> DNA
   <213> Artificial sequence
<220>
   <223> Synthetic oligonucleotide
<400> 345
   caaacttgct cagcag 16
<210> 346
   <211> 16
   <212> DNA
   <213> Artificial sequence
<220>
   <223> Synthetic oligonucleotide
<400> 346
   ccaaacttgc tcagca 16
<210> 347
   <211> 16
   <212> DNA
   <213> Artificial sequence
<220>
   <223> Synthetic oligonucleotide
<400> 347
   cccaaacttg ctcagc 16
<210> 348
   <211> 16
   <212> DNA
   <213> Artificial sequence
<220>
   <223> Synthetic oligonucleotide
<400> 348
   ccccaaactt gctcag 16
<210> 349
   <211> 16
   <212> DNA
   <213> Artificial sequence
<220>
   <223> Synthetic oligonucleotide
<400> 349
   tccccaaact tgctca 16
<210> 350
   <211> 16
   <212> DNA
   <213> Artificial sequence
<220>
   <223> Synthetic oligonucleotide
<400> 350
   gtttgatgtc cctgtg 16
<210> 351
   <211> 16
   <212> DNA
   <213> Artificial sequence
<220>
   <223> Synthetic oligonucleotide
<400> 351
   ggtttgatgt ccctgt 16
<210> 352
   <211> 16
   <212> DNA
   <213> Artificial sequence
<220>
   <223> Synthetic oligonucleotide
<400> 352
   gggtttgatg tccctg 16
<210> 353
   <211> 16
   <212> DNA
   <213> Artificial sequence
<220>
   <223> Synthetic oligonucleotide
<400> 353
   acagcctgca ggatct 16
<210> 354
   <211> 16
   <212> DNA
   <213> Artificial sequence
<220>
   <223> Synthetic oligonucleotide
<400> 354
   cacagcctgc aggatc 16
<210> 355
   <211> 16
   <212> DNA
   <213> Artificial sequence
<220>
   <223> Synthetic oligonucleotide
<400> 355
   ccacagcctg caggat 16
<210> 356
   <211> 16
   <212> DNA
   <213> Artificial sequence
<220>
   <223> Synthetic oligonucleotide
<400> 356
   cccacagcct gcagga 16
<210> 357
   <211> 16
   <212> DNA
   <213> Artificial sequence
<220>
   <223> Synthetic oligonucleotide
<400> 357
   gcccacagcc tgcagg 16
<210> 358
   <211> 16
   <212> DNA
   <213> Artificial sequence
<220>
   <223> Synthetic oligonucleotide
<400> 358
   cgcccacagc ctgcag 16
<210> 359
   <211> 16
   <212> DNA
   <213> Artificial sequence
<220>
   <223> Synthetic oligonucleotide
<400> 359
   ccgcccacag cctgca 16
<210> 360
   <211> 16
   <212> DNA
   <213> Artificial sequence
<220>
   <223> Synthetic oligonucleotide
<400> 360
   accgcccaca gcctgc 16
<210> 361
   <211> 16
   <212> DNA
   <213> Artificial sequence
<220>
   <223> Synthetic oligonucleotide
<400> 361
   caccgcccac agcctg 16
<210> 362
   <211> 16
   <212> DNA
   <213> Artificial sequence
<220>
   <223> Synthetic oligonucleotide
<400> 362
   ccaccgccca cagcct 16
<210> 363
   <211> 16
   <212> DNA
   <213> Artificial sequence
<220>
   <223> Synthetic oligonucleotide
<400> 363
   cccaccgccc acagcc 16
<210> 364
   <211> 16
   <212> DNA
   <213> Artificial sequence
<220>
   <223> Synthetic oligonucleotide
<400> 364
   gcccaccgcc cacagc 16
<210> 365
   <211> 16
   <212> DNA
   <213> Artificial sequence
<220>
   <223> Synthetic oligonucleotide
<400> 365
   ccaggcccac cgccca 16
<210> 366
   <211> 16
   <212> DNA
   <213> Artificial sequence
<220>
   <223> Synthetic oligonucleotide
<400> 366
   cccaggccca ccgccc 16
<210> 367
   <211> 16
   <212> DNA
   <213> Artificial sequence
<220>
   <223> Synthetic oligonucleotide
<400> 367
   tcccaggccc accgcc 16
<210> 368
   <211> 16
   <212> DNA
   <213> Artificial sequence
<220>
   <223> Synthetic oligonucleotide
<400> 368
   tgcctgtccc aggccc 16
<210> 369
   <211> 16
   <212> DNA
   <213> Artificial sequence
<220>
   <223> Synthetic oligonucleotide
<400> 369
   ctgcctgtcc caggcc 16
<210> 370
   <211> 16
   <212> DNA
   <213> Artificial sequence
<220>
   <223> Synthetic oligonucleotide
<400> 370
   gctgcctgtc ccaggc 16
<210> 371
   <211> 16
   <212> DNA
   <213> Artificial sequence
<220>
   <223> Synthetic oligonucleotide
<400> 371
   ggtggcacct tcgaaa 16
<210> 372
   <211> 16
   <212> DNA
   <213> Artificial sequence
<220>
   <223> Synthetic oligonucleotide
<400> 372
   cggtggcacc ttcgaa 16
<210> 373
   <211> 16
   <212> DNA
   <213> Artificial sequence
<220>
   <223> Synthetic oligonucleotide
<400> 373
   tcggtggcac cttcga 16
<210> 374
   <211> 16
   <212> DNA
   <213> Artificial sequence
<220>
   <223> Synthetic oligonucleotide
<400> 374
   agtgagcccg tcctcc 16
<210> 375
   <211> 16
   <212> DNA
   <213> Artificial sequence
<220>
   <223> Synthetic oligonucleotide
<400> 375
   cagtgagccc gtcctc 16
<210> 376
   <211> 16
   <212> DNA
   <213> Artificial sequence
<220>
   <223> Synthetic oligonucleotide
<400> 376
   gcagtgagcc cgtcct 16
<210> 377
   <211> 16
   <212> DNA
   <213> Artificial sequence
<220>
   <223> Synthetic oligonucleotide
<400> 377
   tcccgaatgt ccgaca 16
<210> 378
   <211> 16
   <212> DNA
   <213> Artificial sequence
<220>
   <223> Synthetic oligonucleotide
<400> 378
   ttcccgaatg tccgac 16
<210> 379
   <211> 16
   <212> DNA
   <213> Artificial sequence
<220>
   <223> Synthetic oligonucleotide
<400> 379
   cttcccgaat gtccga 16
<210> 380
   <211> 16
   <212> DNA
   <213> Artificial sequence
<220>
   <223> Synthetic oligonucleotide
<400> 380
   ccttcccgaa tgtccg 16
<210> 381
   <211> 16
   <212> DNA
   <213> Artificial sequence
<220>
   <223> Synthetic oligonucleotide
<400> 381
   accttcccga atgtcc 16
<210> 382
   <211> 16
   <212> DNA
   <213> Artificial sequence
<220>
   <223> Synthetic oligonucleotide
<400> 382
   caccttcccg aatgtc 16
<210> 383
   <211> 16
   <212> DNA
   <213> Artificial sequence
<220>
   <223> Synthetic oligonucleotide
<400> 383
   gcaccttccc gaatgt 16
<210> 384
   <211> 16
   <212> DNA
   <213> Artificial sequence
<220>
   <223> Synthetic oligonucleotide
<400> 384
   cgcaccttcc cgaatg 16
<210> 385
   <211> 16
   <212> DNA
   <213> Artificial sequence
<220>
   <223> Synthetic oligonucleotide
<400> 385
   atccgctcct gcaact 16
<210> 386
   <211> 16
   <212> DNA
   <213> Artificial sequence
<220>
   <223> Synthetic oligonucleotide
<400> 386
   catccgctcc tgcaac 16
<210> 387
   <211> 16
   <212> DNA
   <213> Artificial sequence
<220>
   <223> Synthetic oligonucleotide
<400> 387
   ccatccgctc ctgcaa 16
<210> 388
   <211> 16
   <212> DNA
   <213> Artificial sequence
<220>
   <223> Synthetic oligonucleotide
<400> 388
   gctccctctg cctgca 16
<210> 389
   <211> 16
   <212> DNA
   <213> Artificial sequence
<220>
   <223> Synthetic oligonucleotide
<400> 389
   aggtggatcc gtggcc 16
<210> 390
   <211> 16
   <212> DNA
   <213> Artificial sequence
<220>
   <223> Synthetic oligonucleotide
<400> 390
   gggaaggtgg atccgt 16
<210> 391
   <211> 16
   <212> DNA
   <213> Artificial sequence
<220>
   <223> Synthetic oligonucleotide
<400> 391
   acaggagcag ggaaag 16
<210> 392
   <211> 16
   <212> DNA
   <213> Artificial sequence
<220>
   <223> Synthetic oligonucleotide
<400> 392
   cagactgcgg tgagtt 16
<210> 393
   <211> 16
   <212> DNA
   <213> Artificial sequence
<220>
   <223> Synthetic oligonucleotide
<400> 393
   ggctcctggg cggcgc 16
<210> 394
   <211> 16
   <212> DNA
   <213> Artificial sequence
<220>
   <223> Synthetic oligonucleotide
<400> 394
   ggcggctcct gggcgg 16
<210> 395
   <211> 16
   <212> DNA
   <213> Artificial sequence
<220>
   <223> Synthetic oligonucleotide
<400> 395
   cgcgggcggc tcctgg 16
<210> 396
   <211> 16
   <212> DNA
   <213> Artificial sequence
<220>
   <223> Synthetic oligonucleotide
<400> 396
   gagcgcgggc ggctcc 16
<210> 397
   <211> 16
   <212> DNA
   <213> Artificial sequence
<220>
   <223> Synthetic oligonucleotide
<400> 397
   ggttcaggga gcgcgg 16
<210> 398
   <211> 16
   <212> DNA
   <213> Artificial sequence
<220>
   <223> Synthetic oligonucleotide
<400> 398
   agttctaggg ttcagg 16
<210> 399
   <211> 16
   <212> DNA
   <213> Artificial sequence
<220>
   <223> Synthetic oligonucleotide
<400> 399
   cagttctagg gttcag 16
<210> 400
   <211> 16
   <212> DNA
   <213> Artificial sequence
<220>
   <223> Synthetic oligonucleotide
<400> 400
   acagttctag ggttca 16
<210> 401
   <211> 16
   <212> DNA
   <213> Artificial sequence
<220>
   <223> Synthetic oligonucleotide
<400> 401
   gacagttcta gggttc 16
<210> 402
   <211> 16
   <212> DNA
   <213> Artificial sequence
<220>
   <223> Synthetic oligonucleotide
<400> 402
   agacagttct agggtt 16
<210> 403
   <211> 16
   <212> DNA
   <213> Artificial sequence
<220>
   <223> Synthetic oligonucleotide
<400> 403
   aagacagttc tagggt 16
<210> 404
   <211> 16
   <212> DNA
   <213> Artificial sequence
<220>
   <223> Synthetic oligonucleotide
<400> 404
   gaagacagtt ctaggg 16
<210> 405
   <211> 16
   <212> DNA
   <213> Artificial sequence
<220>
   <223> Synthetic oligonucleotide
<400> 405
   cgaagacagt tctagg 16
<210> 406
   <211> 16
   <212> DNA
   <213> Artificial sequence
<220>
   <223> Synthetic oligonucleotide
<400> 406
   tcgaagacag ttctag 16
<210> 407
   <211> 16
   <212> DNA
   <213> Artificial sequence
<220>
   <223> Synthetic oligonucleotide
<400> 407
   gtcgaagaca gttcta 16
<210> 408
   <211> 16
   <212> DNA
   <213> Artificial sequence
<220>
   <223> Synthetic oligonucleotide
<400> 408
   agtcgaagac agttct 16
<210> 409
   <211> 16
   <212> DNA
   <213> Artificial sequence
<220>
   <223> Synthetic oligonucleotide
<400> 409
   gagtcgaaga cagttc 16
<210> 410
   <211> 16
   <212> DNA
   <213> Artificial sequence
<220>
   <223> Synthetic oligonucleotide
<400> 410
   ggagtcgaag acagtt 16
<210> 411
   <211> 16
   <212> DNA
   <213> Artificial sequence
<220>
   <223> Synthetic oligonucleotide
<400> 411
   cggagtcgaa gacagt 16
<210> 412
   <211> 16
   <212> DNA
   <213> Artificial sequence
<220>
   <223> Synthetic oligonucleotide
<400> 412
   ccggagtcga agacag 16
<210> 413
   <211> 16
   <212> DNA
   <213> Artificial sequence
<220>
   <223> Synthetic oligonucleotide
<400> 413
   cccggagtcg aagaca 16
<210> 414
   <211> 16
   <212> DNA
   <213> Artificial sequence
<220>
   <223> Synthetic oligonucleotide
<400> 414
   ccccggagtc gaagac 16
<210> 415
   <211> 16
   <212> DNA
   <213> Artificial sequence
<220>
   <223> Synthetic oligonucleotide
<400> 415
   gccccggagt cgaaga 16
<210> 416
   <211> 16
   <212> DNA
   <213> Artificial sequence
<220>
   <223> Synthetic oligonucleotide
<400> 416
   ggccccggag tcgaag 16
<210> 417
   <211> 16
   <212> DNA
   <213> Artificial sequence
<220>
   <223> Synthetic oligonucleotide
<400> 417
   gggccccgga gtcgaa 16
<210> 418
   <211> 16
   <212> DNA
   <213> Artificial sequence
<220>
   <223> Synthetic oligonucleotide
<400> 418
   cggttgtgaa ctggca 16
<210> 419
   <211> 16
   <212> DNA
   <213> Artificial sequence
<220>
   <223> Synthetic oligonucleotide
<400> 419
   agcggttgtg aactgg 16
<210> 420
   <211> 16
   <212> DNA
   <213> Artificial sequence
<220>
   <223> Synthetic oligonucleotide
<400> 420
   gcactttgcg aaccaa 16
<210> 421
   <211> 16
   <212> DNA
   <213> Artificial sequence
<220>
   <223> Synthetic oligonucleotide
<400> 421
   tgcactttgc gaacca 16
<210> 422
   <211> 16
   <212> DNA
   <213> Artificial sequence
<220>
   <223> Synthetic oligonucleotide
<400> 422
   actttgcgaa ccaacg 16
<210> 423
   <211> 16
   <212> DNA
   <213> Artificial sequence
<220>
   <223> Synthetic oligonucleotide
<400> 423
   aaagctttgc actttg 16
<210> 424
   <211> 16
   <212> DNA
   <213> Artificial sequence
<220>
   <223> Synthetic oligonucleotide
<400> 424
   tcccgagtaa gcaggc 16
<210> 425
   <211> 16
   <212> DNA
   <213> Artificial sequence
<220>
   <223> Synthetic oligonucleotide
<400> 425
   gcagcgcaag tgagga 16
<210> 426
   <211> 16
   <212> DNA
   <213> Artificial sequence
<220>
   <223> Synthetic oligonucleotide
<400> 426
   gtcagcgagt cggagg 16
<210> 427
   <211> 16
   <212> DNA
   <213> Artificial sequence
<220>
   <223> Synthetic oligonucleotide
<400> 427
   cctgtcagcg agtcgg 16
<210> 428
   <211> 16
   <212> DNA
   <213> Artificial sequence
<220>
   <223> Synthetic oligonucleotide
<400> 428
   gcctgtcagc gagtcg 16
<210> 429
   <211> 16
   <212> DNA
   <213> Artificial sequence
<220>
   <223> Synthetic oligonucleotide
<400> 429
   agcctgtcag cgagtc 16
<210> 430
   <211> 16
   <212> DNA
   <213> Artificial sequence
<220>
   <223> Synthetic oligonucleotide
<400> 430
   gggtctcagt gcatcc 16
<210> 431
   <211> 16
   <212> DNA
   <213> Artificial sequence
<220>
   <223> Synthetic oligonucleotide
<400> 431
   aggtttttcc agaggc 16
<210> 432
   <211> 16
   <212> DNA
   <213> Artificial sequence
<220>
   <223> Synthetic oligonucleotide
<400> 432
   aaggtttttc cagagg 16
<210> 433
   <211> 16
   <212> DNA
   <213> Artificial sequence
<220>
   <223> Synthetic oligonucleotide
<400> 433
   ggtcactgct gggtcc 16
<210> 434
   <211> 16
   <212> DNA
   <213> Artificial sequence
<220>
   <223> Synthetic oligonucleotide
<400> 434
   gtggtttctg tctgct 16
<210> 435
   <211> 16
   <212> DNA
   <213> Artificial sequence
<220>
   <223> Synthetic oligonucleotide
<400> 435
   cgtggtttct gtctgc 16
<210> 436
   <211> 16
   <212> DNA
   <213> Artificial sequence
<220>
   <223> Synthetic oligonucleotide
<400> 436
   gaactggcag gcggtg 16
<210> 437
   <211> 16
   <212> DNA
   <213> Artificial sequence
<220>
   <223> Synthetic oligonucleotide
<400> 437
   tgtgaactgg caggcg 16
<210> 438
   <211> 16
   <212> DNA
   <213> Artificial sequence
<220>
   <223> Synthetic oligonucleotide
<400> 438
   ggttgtgaac tggcag 16
<210> 439
   <211> 16
   <212> DNA
   <213> Artificial sequence
<220>
   <223> Synthetic oligonucleotide
<400> 439
   gagcggttgt gaactg 16
<210> 440
   <211> 16
   <212> DNA
   <213> Artificial sequence
<220>
   <223> Synthetic oligonucleotide
<400> 440
   actggagctg ggcgga 16
<210> 441
   <211> 16
   <212> DNA
   <213> Artificial sequence
<220>
   <223> Synthetic oligonucleotide
<400> 441
   aggactggag ctgggc 16
<210> 442
   <211> 16
   <212> DNA
   <213> Artificial sequence
<220>
   <223> Synthetic oligonucleotide
<400> 442
   tcacaggact ggagct 16
<210> 443
   <211> 16
   <212> DNA
   <213> Artificial sequence
<220>
   <223> Synthetic oligonucleotide
<400> 443
   atcacaggac tggagc 16
<210> 444
   <211> 16
   <212> DNA
   <213> Artificial sequence
<220>
   <223> Synthetic oligonucleotide
<400> 444
   ggatcacagg actgga 16
<210> 445
   <211> 16
   <212> DNA
   <213> Artificial sequence
<220>
   <223> Synthetic oligonucleotide
<400> 445
   cagcctggcc gaaaga 16
<210> 446
   <211> 16
   <212> DNA
   <213> Artificial sequence
<220>
   <223> Synthetic oligonucleotide
<400> 446
   ctcagcctgg ccgaaa 16
<210> 447
   <211> 16
   <212> DNA
   <213> Artificial sequence
<220>
   <223> Synthetic oligonucleotide
<400> 447
   gtcagggcct cagcct 16
<210> 448
   <211> 16
   <212> DNA
   <213> Artificial sequence
<220>
   <223> Synthetic oligonucleotide
<400> 448
   cgtcagggcc tcagcc 16
<210> 449
   <211> 16
   <212> DNA
   <213> Artificial sequence
<220>
   <223> Synthetic oligonucleotide
<400> 449
   tttgcacttt gcgaac 16
<210> 450
   <211> 16
   <212> DNA
   <213> Artificial sequence
<220>
   <223> Synthetic oligonucleotide
<400> 450
   gaaagctttg cacttt 16
<210> 451
   <211> 16
   <212> DNA
   <213> Artificial sequence
<220>
   <223> Synthetic oligonucleotide
<400> 451
   aatttcccga gtaagc 16
<210> 452
   <211> 16
   <212> DNA
   <213> Artificial sequence
<220>
   <223> Synthetic oligonucleotide
<400> 452
   gcaaatttcc cgagta 16
<210> 453
   <211> 16
   <212> DNA
   <213> Artificial sequence
<220>
   <223> Synthetic oligonucleotide
<400> 453
   agcaaatttc ccgagt 16
<210> 454
   <211> 16
   <212> DNA
   <213> Artificial sequence
<220>
   <223> Synthetic oligonucleotide
<400> 454
   aagcaaattt cccgag 16
<210> 455
   <211> 16
   <212> DNA
   <213> Artificial sequence
<220>
   <223> Synthetic oligonucleotide
<400> 455
   aaagcaaatt tcccga 16
<210> 456
   <211> 16
   <212> DNA
   <213> Artificial sequence
<220>
   <223> Synthetic oligonucleotide
<400> 456
   aaaagcaaat ttcccg 16
<210> 457
   <211> 16
   <212> DNA
   <213> Artificial sequence
<220>
   <223> Synthetic oligonucleotide
<400> 457
   gcaaaagcaa atttcc 16
<210> 458
   <211> 16
   <212> DNA
   <213> Artificial sequence
<220>
   <223> Synthetic oligonucleotide
<400> 458
   ggcaaaagca aatttc 16
<210> 459
   <211> 16
   <212> DNA
   <213> Artificial sequence
<220>
   <223> Synthetic oligonucleotide
<400> 459
   ttggcaaaag caaatt 16
<210> 460
   <211> 16
   <212> DNA
   <213> Artificial sequence
<220>
   <223> Synthetic oligonucleotide
<400> 460
   gtttggcaaa agcaaa 16
<210> 461
   <211> 16
   <212> DNA
   <213> Artificial sequence
<220>
   <223> Synthetic oligonucleotide
<400> 461
   ggtttggcaa aagcaa 16
<210> 462
   <211> 16
   <212> DNA
   <213> Artificial sequence
<220>
   <223> Synthetic oligonucleotide
<400> 462
   gggtttggca aaagca 16
<210> 463
   <211> 16
   <212> DNA
   <213> Artificial sequence
<220>
   <223> Synthetic oligonucleotide
<400> 463
   gcgggtttgg caaaag 16
<210> 464
   <211> 16
   <212> DNA
   <213> Artificial sequence
<220>
   <223> Synthetic oligonucleotide
<400> 464
   agcgggtttg gcaaaa 16
<210> 465
   <211> 16
   <212> DNA
   <213> Artificial sequence
<220>
   <223> Synthetic oligonucleotide
<400> 465
   aagcgggttt ggcaaa 16
<210> 466
   <211> 16
   <212> DNA
   <213> Artificial sequence
<220>
   <223> Synthetic oligonucleotide
<400> 466
   ctccgagagc agcgca 16
<210> 467
   <211> 16
   <212> DNA
   <213> Artificial sequence
<220>
   <223> Synthetic oligonucleotide
<400> 467
   gctccgagag cagcgc 16
<210> 468
   <211> 16
   <212> DNA
   <213> Artificial sequence
<220>
   <223> Synthetic oligonucleotide
<400> 468
   ggctccgaga gcagcg 16
<210> 469
   <211> 16
   <212> DNA
   <213> Artificial sequence
<220>
   <223> Synthetic oligonucleotide
<400> 469
   taaatatcca aaccgc 16
<210> 470
   <211> 16
   <212> DNA
   <213> Artificial sequence
<220>
   <223> Synthetic oligonucleotide
<400> 470
   gtcaataaat atccaa 16
<210> 471
   <211> 16
   <212> DNA
   <213> Artificial sequence
<220>
   <223> Synthetic oligonucleotide
<400> 471
   aggtcaataa atatcc 16
<210> 472
   <211> 16
   <212> DNA
   <213> Artificial sequence
<220>
   <223> Synthetic oligonucleotide
<400> 472
   cgaggtcaat aaatat 16
<210> 473
   <211> 16
   <212> DNA
   <213> Artificial sequence
<220>
   <223> Synthetic oligonucleotide
<400> 473
   acgaggtcaa taaata 16
<210> 474
   <211> 16
   <212> DNA
   <213> Artificial sequence
<220>
   <223> Synthetic oligonucleotide
<400> 474
   gacgaggtca ataaat 16
<210> 475
   <211> 16
   <212> DNA
   <213> Artificial sequence
<220>
   <223> Synthetic oligonucleotide
<400> 475
   ggacgaggtc aataaa 16
<210> 476
   <211> 16
   <212> DNA
   <213> Artificial sequence
<220>
   <223> Synthetic oligonucleotide
<400> 476
   aggacgaggt caataa 16
<210> 477
   <211> 16
   <212> DNA
   <213> Artificial sequence
<220>
   <223> Synthetic oligonucleotide
<400> 477
   gaggacgagg tcaata 16
<210> 478
   <211> 16
   <212> DNA
   <213> Artificial sequence
<220>
   <223> Synthetic oligonucleotide
<400> 478
   ggaggacgag gtcaat 16
<210> 479
   <211> 16
   <212> DNA
   <213> Artificial sequence
<220>
   <223> Synthetic oligonucleotide
<400> 479
   cggaggacga ggtcaa 16
<210> 480
   <211> 16
   <212> DNA
   <213> Artificial sequence
<220>
   <223> Synthetic oligonucleotide
<400> 480
   tcggaggacg aggtca 16
<210> 481
   <211> 16
   <212> DNA
   <213> Artificial sequence
<220>
   <223> Synthetic oligonucleotide
<400> 481
   gtcggaggac gaggtc 16
<210> 482
   <211> 16
   <212> DNA
   <213> Artificial sequence
<220>
   <223> Synthetic oligonucleotide
<400> 482
   agtcggagga cgaggt 16
<210> 483
   <211> 16
   <212> DNA
   <213> Artificial sequence
<220>
   <223> Synthetic oligonucleotide
<400> 483
   gagtcggagg acgagg 16
<210> 484
   <211> 16
   <212> DNA
   <213> Artificial sequence
<220>
   <223> Synthetic oligonucleotide
<400> 484
   cgagtcggag gacgag 16
<210> 485
   <211> 16
   <212> DNA
   <213> Artificial sequence
<220>
   <223> Synthetic oligonucleotide
<400> 485
   agcgagtcgg aggacg 16
<210> 486
   <211> 16
   <212> DNA
   <213> Artificial sequence
<220>
   <223> Synthetic oligonucleotide
<400> 486
   cagcgagtcg gaggac 16
<210> 487
   <211> 16
   <212> DNA
   <213> Artificial sequence
<220>
   <223> Synthetic oligonucleotide
<400> 487
   tcagcgagtc ggagga 16
<210> 488
   <211> 16
   <212> DNA
   <213> Artificial sequence
<220>
   <223> Synthetic oligonucleotide
<400> 488
   tgtcagcgag tcggag 16
<210> 489
   <211> 16
   <212> DNA
   <213> Artificial sequence
<220>
   <223> Synthetic oligonucleotide
<400> 489
   ctgtcagcga gtcgga 16
<210> 490
   <211> 16
   <212> DNA
   <213> Artificial sequence
<220>
   <223> Synthetic oligonucleotide
<400> 490
   cagtgcatcc aaaacg 16
<210> 491
   <211> 16
   <212> DNA
   <213> Artificial sequence
<220>
   <223> Synthetic oligonucleotide
<400> 491
   tcagtgcatc caaaac 16
<210> 492
   <211> 16
   <212> DNA
   <213> Artificial sequence
<220>
   <223> Synthetic oligonucleotide
<400> 492
   ctcagtgcat ccaaaa 16
<210> 493
   <211> 16
   <212> DNA
   <213> Artificial sequence
<220>
   <223> Synthetic oligonucleotide
<400> 493
   tctcagtgca tccaaa 16
<210> 494
   <211> 16
   <212> DNA
   <213> Artificial sequence
<220>
   <223> Synthetic oligonucleotide
<400> 494
   gtctcagtgc atccaa 16
<210> 495
   <211> 16
   <212> DNA
   <213> Artificial sequence
<220>
   <223> Synthetic oligonucleotide
<400> 495
   caataaatac cgagga 16
<210> 496
   <211> 16
   <212> DNA
   <213> Artificial sequence
<220>
   <223> Synthetic oligonucleotide
<400> 496
   agacaataaa taccga 16
<210> 497
   <211> 16
   <212> DNA
   <213> Artificial sequence
<220>
   <223> Synthetic oligonucleotide
<400> 497
   cagacaataa ataccg 16
<210> 498
   <211> 16
   <212> DNA
   <213> Artificial sequence
<220>
   <223> Synthetic oligonucleotide
<400> 498
   cacggaagca cgacac 16
<210> 499
   <211> 16
   <212> DNA
   <213> Artificial sequence
<220>
   <223> Synthetic oligonucleotide
<400> 499
   tcacggaagc acgaca 16
<210> 500
   <211> 16
   <212> DNA
   <213> Artificial sequence
<220>
   <223> Synthetic oligonucleotide
<400> 500
   ctcacggaag cacgac 16
<210> 501
   <211> 16
   <212> DNA
   <213> Artificial sequence
<220>
   <223> Synthetic oligonucleotide
<400> 501
   gtccctctcc tcacgg 16
<210> 502
   <211> 16
   <212> DNA
   <213> Artificial sequence
<220>
   <223> Synthetic oligonucleotide
<400> 502
   cgtccctctc ctcacg 16
<210> 503
   <211> 16
   <212> DNA
   <213> Artificial sequence
<220>
   <223> Synthetic oligonucleotide
<400> 503
   cccattcacc aacacg 16
<210> 504
   <211> 16
   <212> DNA
   <213> Artificial sequence
<220>
   <223> Synthetic oligonucleotide
<400> 504
   ccccattcac caacac 16
<210> 505
   <211> 16
   <212> DNA
   <213> Artificial sequence
<220>
   <223> Synthetic oligonucleotide
<400> 505
   tccccattca ccaaca 16
<210> 506
   <211> 16
   <212> DNA
   <213> Artificial sequence
<220>
   <223> Synthetic oligonucleotide
<400> 506
   gtccccattc accaac 16
<210> 507
   <211> 16
   <212> DNA
   <213> Artificial sequence
<220>
   <223> Synthetic oligonucleotide
<400> 507
   ggtccccatt caccaa 16
<210> 508
   <211> 16
   <212> DNA
   <213> Artificial sequence
<220>
   <223> Synthetic oligonucleotide
<400> 508
   cggtccccat tcacca 16
<210> 509
   <211> 16
   <212> DNA
   <213> Artificial sequence
<220>
   <223> Synthetic oligonucleotide
<400> 509
   ccggtcccca ttcacc 16
<210> 510
   <211> 16
   <212> DNA
   <213> Artificial sequence
<220>
   <223> Synthetic oligonucleotide
<400> 510
   gccggtcccc attcac 16
<210> 511
   <211> 16
   <212> DNA
   <213> Artificial sequence
<220>
   <223> Synthetic oligonucleotide
<400> 511
   cgccggtccc cattca 16
<210> 512
   <211> 16
   <212> DNA
   <213> Artificial sequence
<220>
   <223> Synthetic oligonucleotide
<400> 512
   ccgccggtcc ccattc 16
<210> 513
   <211> 16
   <212> DNA
   <213> Artificial sequence
<220>
   <223> Synthetic oligonucleotide
<400> 513
   accgccggtc cccatt 16
<210> 514
   <211> 16
   <212> DNA
   <213> Artificial sequence
<220>
   <223> Synthetic oligonucleotide
<400> 514
   caccgccggt ccccat 16
<210> 515
   <211> 16
   <212> DNA
   <213> Artificial sequence
<220>
   <223> Synthetic oligonucleotide
<400> 515
   ccaccgccgg tcccca 16
<210> 516
   <211> 16
   <212> DNA
   <213> Artificial sequence
<220>
   <223> Synthetic oligonucleotide
<400> 516
   tccaccgccg gtcccc 16
<210> 517
   <211> 16
   <212> DNA
   <213> Artificial sequence
<220>
   <223> Synthetic oligonucleotide
<400> 517
   atccaccgcc ggtccc 16
<210> 518
   <211> 16
   <212> DNA
   <213> Artificial sequence
<220>
   <223> Synthetic oligonucleotide
<400> 518
   gatccaccgc cggtcc 16
<210> 519
   <211> 16
   <212> DNA
   <213> Artificial sequence
<220>
   <223> Synthetic oligonucleotide
<400> 519
   tgatccaccg ccggtc 16
<210> 520
   <211> 16
   <212> DNA
   <213> Artificial sequence
<220>
   <223> Synthetic oligonucleotide
<400> 520
   gtgatccacc gccggt 16
<210> 521
   <211> 16
   <212> DNA
   <213> Artificial sequence
<220>
   <223> Synthetic oligonucleotide
<400> 521
   cgtgatccac cgccgg 16
<210> 522
   <211> 16
   <212> DNA
   <213> Artificial sequence
<220>
   <223> Synthetic oligonucleotide
<400> 522
   catcctggaa ggcgaa 16
<210> 523
   <211> 16
   <212> DNA
   <213> Artificial sequence
<220>
   <223> Synthetic oligonucleotide
<400> 523
   tcatcctgga aggcga 16
<210> 524
   <211> 16
   <212> DNA
   <213> Artificial sequence
<220>
   <223> Synthetic oligonucleotide
<400> 524
   agttctcatc ctggaa 16
<210> 525
   <211> 16
   <212> DNA
   <213> Artificial sequence
<220>
   <223> Synthetic oligonucleotide
<400> 525
   tagttctcat cctgga 16
<210> 526
   <211> 16
   <212> DNA
   <213> Artificial sequence
<220>
   <223> Synthetic oligonucleotide
<400> 526
   gtagttctca tcctgg 16
<210> 527
   <211> 16
   <212> DNA
   <213> Artificial sequence
<220>
   <223> Synthetic oligonucleotide
<400> 527
   caggtacagg tagttc 16
<210> 528
   <211> 16
   <212> DNA
   <213> Artificial sequence
<220>
   <223> Synthetic oligonucleotide
<400> 528
   ccaggtacag gtagtt 16
<210> 529
   <211> 16
   <212> DNA
   <213> Artificial sequence
<220>
   <223> Synthetic oligonucleotide
<400> 529
   gaccaggtac aggtag 16
<210> 530
   <211> 16
   <212> DNA
   <213> Artificial sequence
<220>
   <223> Synthetic oligonucleotide
<400> 530
   ctccatgacc aggtac 16
<210> 531
   <211> 16
   <212> DNA
   <213> Artificial sequence
<220>
   <223> Synthetic oligonucleotide
<400> 531
   actccatgac caggta 16
<210> 532
   <211> 16
   <212> DNA
   <213> Artificial sequence
<220>
   <223> Synthetic oligonucleotide
<400> 532
   tactccatga ccaggt 16
<210> 533
   <211> 16
   <212> DNA
   <213> Artificial sequence
<220>
   <223> Synthetic oligonucleotide
<400> 533
   atactccatg accagg 16
<210> 534
   <211> 16
   <212> DNA
   <213> Artificial sequence
<220>
   <223> Synthetic oligonucleotide
<400> 534
   aatactccat gaccag 16
<210> 535
   <211> 16
   <212> DNA
   <213> Artificial sequence
<220>
   <223> Synthetic oligonucleotide
<400> 535
   taatactcca tgacca 16
<210> 536
   <211> 16
   <212> DNA
   <213> Artificial sequence
<220>
   <223> Synthetic oligonucleotide
<400> 536
   gtaatactcc atgacc 16
<210> 537
   <211> 16
   <212> DNA
   <213> Artificial sequence
<220>
   <223> Synthetic oligonucleotide
<400> 537
   cgtaatactc catgac 16
<210> 538
   <211> 16
   <212> DNA
   <213> Artificial sequence
<220>
   <223> Synthetic oligonucleotide
<400> 538
   cttgctcagc agtgtc 16
<210> 539
   <211> 16
   <212> DNA
   <213> Artificial sequence
<220>
   <223> Synthetic oligonucleotide
<400> 539
   acttgctcag cagtgt 16
<210> 540
   <211> 16
   <212> DNA
   <213> Artificial sequence
<220>
   <223> Synthetic oligonucleotide
<400> 540
   aacttgctca gcagtg 16
<210> 541
   <211> 16
   <212> DNA
   <213> Artificial sequence
<220>
   <223> Synthetic oligonucleotide
<400> 541
   gctccccaaa cttgct 16
<210> 542
   <211> 16
   <212> DNA
   <213> Artificial sequence
<220>
   <223> Synthetic oligonucleotide
<400> 542
   cgctccccaa acttgc 16
<210> 543
   <211> 16
   <212> DNA
   <213> Artificial sequence
<220>
   <223> Synthetic oligonucleotide
<400> 543
   ccgctcccca aacttg 16
<210> 544
   <211> 16
   <212> DNA
   <213> Artificial sequence
<220>
   <223> Synthetic oligonucleotide
<400> 544
   tccgctcccc aaactt 16
<210> 545
   <211> 16
   <212> DNA
   <213> Artificial sequence
<220>
   <223> Synthetic oligonucleotide
<400> 545
   atccgctccc caaact 16
<210> 546
   <211> 16
   <212> DNA
   <213> Artificial sequence
<220>
   <223> Synthetic oligonucleotide
<400> 546
   aatccgctcc ccaaac 16
<210> 547
   <211> 16
   <212> DNA
   <213> Artificial sequence
<220>
   <223> Synthetic oligonucleotide
<400> 547
   gaatccgctc cccaaa 16
<210> 548
   <211> 16
   <212> DNA
   <213> Artificial sequence
<220>
   <223> Synthetic oligonucleotide
<400> 548
   ggaatccgct ccccaa 16
<210> 549
   <211> 16
   <212> DNA
   <213> Artificial sequence
<220>
   <223> Synthetic oligonucleotide
<400> 549
   cggaatccgc tcccca 16
<210> 550
   <211> 16
   <212> DNA
   <213> Artificial sequence
<220>
   <223> Synthetic oligonucleotide
<400> 550
   ccggaatccg ctcccc 16
<210> 551
   <211> 16
   <212> DNA
   <213> Artificial sequence
<220>
   <223> Synthetic oligonucleotide
<400> 551
   gccggaatcc gctccc 16
<210> 552
   <211> 16
   <212> DNA
   <213> Artificial sequence
<220>
   <223> Synthetic oligonucleotide
<400> 552
   agaagcgcgc catctc 16
<210> 553
   <211> 16
   <212> DNA
   <213> Artificial sequence
<220>
   <223> Synthetic oligonucleotide
<400> 553
   tagaagcgcg ccatct 16
<210> 554
   <211> 16
   <212> DNA
   <213> Artificial sequence
<220>
   <223> Synthetic oligonucleotide
<400> 554
   gtagaagcgc gccatc 16
<210> 555
   <211> 16
   <212> DNA
   <213> Artificial sequence
<220>
   <223> Synthetic oligonucleotide
<400> 555
   ggtagaagcg cgccat 16
<210> 556
   <211> 16
   <212> DNA
   <213> Artificial sequence
<220>
   <223> Synthetic oligonucleotide
<400> 556
   aggtagaagc gcgcca 16
<210> 557
   <211> 16
   <212> DNA
   <213> Artificial sequence
<220>
   <223> Synthetic oligonucleotide
<400> 557
   caggtagaag cgcgcc 16
<210> 558
   <211> 16
   <212> DNA
   <213> Artificial sequence
<220>
   <223> Synthetic oligonucleotide
<400> 558
   ccaggtagaa gcgcgc 16
<210> 559
   <211> 16
   <212> DNA
   <213> Artificial sequence
<220>
   <223> Synthetic oligonucleotide
<400> 559
   gccaggtaga agcgcg 16
<210> 560
   <211> 16
   <212> DNA
   <213> Artificial sequence
<220>
   <223> Synthetic oligonucleotide
<400> 560
   cgccaggtag aagcgc 16
<210> 561
   <211> 16
   <212> DNA
   <213> Artificial sequence
<220>
   <223> Synthetic oligonucleotide
<400> 561
   ccgccaggta gaagcg 16
<210> 562
   <211> 16
   <212> DNA
   <213> Artificial sequence
<220>
   <223> Synthetic oligonucleotide
<400> 562
   tccgccaggt agaagc 16
<210> 563
   <211> 16
   <212> DNA
   <213> Artificial sequence
<220>
   <223> Synthetic oligonucleotide
<400> 563
   tgacaatctc cgccag 16
<210> 564
   <211> 16
   <212> DNA
   <213> Artificial sequence
<220>
   <223> Synthetic oligonucleotide
<400> 564
   atgacaatct ccgcca 16
<210> 565
   <211> 16
   <212> DNA
   <213> Artificial sequence
<220>
   <223> Synthetic oligonucleotide
<400> 565
   catgacaatc tccgcc 16
<210> 566
   <211> 16
   <212> DNA
   <213> Artificial sequence
<220>
   <223> Synthetic oligonucleotide
<400> 566
   ccatgacaat ctccgc 16
<210> 567
   <211> 16
   <212> DNA
   <213> Artificial sequence
<220>
   <223> Synthetic oligonucleotide
<400> 567
   gccatgacaa tctccg 16
<210> 568
   <211> 16
   <212> DNA
   <213> Artificial sequence
<220>
   <223> Synthetic oligonucleotide
<400> 568
   ggccatgaca atctcc 16
<210> 569
   <211> 16
   <212> DNA
   <213> Artificial sequence
<220>
   <223> Synthetic oligonucleotide
<400> 569
   ccaactgttc tcttag 16
<210> 570
   <211> 16
   <212> DNA
   <213> Artificial sequence
<220>
   <223> Synthetic oligonucleotide
<400> 570
   aaccaactgt tctctt 16
<210> 571
   <211> 16
   <212> DNA
   <213> Artificial sequence
<220>
   <223> Synthetic oligonucleotide
<400> 571
   ccagtaataa aagctg 16
<210> 572
   <211> 16
   <212> DNA
   <213> Artificial sequence
<220>
   <223> Synthetic oligonucleotide
<400> 572
   gtccagtaat aaaagc 16
<210> 573
   <211> 16
   <212> DNA
   <213> Artificial sequence
<220>
   <223> Synthetic oligonucleotide
<400> 573
   gttgtccagt aataaa 16
<210> 574
   <211> 16
   <212> DNA
   <213> Artificial sequence
<220>
   <223> Synthetic oligonucleotide
<400> 574
   atgttgtcca gtaata 16
<210> 575
   <211> 16
   <212> DNA
   <213> Artificial sequence
<220>
   <223> Synthetic oligonucleotide
<400> 575
   taatgttgtc cagtaa 16
<210> 576
   <211> 16
   <212> DNA
   <213> Artificial sequence
<220>
   <223> Synthetic oligonucleotide
<400> 576
   ttcaatcctg acccac 16
<210> 577
   <211> 16
   <212> DNA
   <213> Artificial sequence
<220>
   <223> Synthetic oligonucleotide
<400> 577
   ggttcaatcc tgaccc 16
<210> 578
   <211> 16
   <212> DNA
   <213> Artificial sequence
<220>
   <223> Synthetic oligonucleotide
<400> 578
   tgggttcaat cctgac 16
<210> 579
   <211> 16
   <212> DNA
   <213> Artificial sequence
<220>
   <223> Synthetic oligonucleotide
<400> 579
   gatgggttca atcctg 16
<210> 580
   <211> 16
   <212> DNA
   <213> Artificial sequence
<220>
   <223> Synthetic oligonucleotide
<400> 580
   aggatgggtt caatcc 16
<210> 581
   <211> 16
   <212> DNA
   <213> Artificial sequence
<220>
   <223> Synthetic oligonucleotide
<400> 581
   agaggatggg ttcaat 16
<210> 582
   <211> 16
   <212> DNA
   <213> Artificial sequence
<220>
   <223> Synthetic oligonucleotide
<400> 582
   atagaggatg ggttca 16
<210> 583
   <211> 16
   <212> DNA
   <213> Artificial sequence
<220>
   <223> Synthetic oligonucleotide
<400> 583
   ccctcctgtg ggaaca 16
<210> 584
   <211> 16
   <212> DNA
   <213> Artificial sequence
<220>
   <223> Synthetic oligonucleotide
<400> 584
   gtccctcctg tgggaa 16
<210> 585
   <211> 16
   <212> DNA
   <213> Artificial sequence
<220>
   <223> Synthetic oligonucleotide
<400> 585
   cagtccctcc tgtggg 16
<210> 586
   <211> 16
   <212> DNA
   <213> Artificial sequence
<220>
   <223> Synthetic oligonucleotide
<400> 586
   agcagtccct cctgtg 16
<210> 587
   <211> 16
   <212> DNA
   <213> Artificial sequence
<220>
   <223> Synthetic oligonucleotide
<400> 587
   actcagctgt gggaag 16
<210> 588
   <211> 16
   <212> DNA
   <213> Artificial sequence
<220>
   <223> Synthetic oligonucleotide
<400> 588
   cccactcagc tgtggg 16
<210> 589
   <211> 16
   <212> DNA
   <213> Artificial sequence
<220>
   <223> Synthetic oligonucleotide
<400> 589
   accccactca gctgtg 16
<210> 590
   <211> 16
   <212> DNA
   <213> Artificial sequence
<220>
   <223> Synthetic oligonucleotide
<400> 590
   acaccccact cagctg 16
<210> 591
   <211> 16
   <212> DNA
   <213> Artificial sequence
<220>
   <223> Synthetic oligonucleotide
<400> 591
   gcacacccca ctcagc 16
<210> 592
   <211> 16
   <212> DNA
   <213> Artificial sequence
<220>
   <223> Synthetic oligonucleotide
<400> 592
   tcagcacacc ccactc 16
<210> 593
   <211> 16
   <212> DNA
   <213> Artificial sequence
<220>
   <223> Synthetic oligonucleotide
<400> 593
   gtggtcctaa gactgg 16
<210> 594
   <211> 16
   <212> DNA
   <213> Artificial sequence
<220>
   <223> Synthetic oligonucleotide
<400> 594
   gatgtggtcc taagac 16
<210> 595
   <211> 16
   <212> DNA
   <213> Artificial sequence
<220>
   <223> Synthetic oligonucleotide
<400> 595
   cagatgtggt cctaag 16
<210> 596
   <211> 16
   <212> DNA
   <213> Artificial sequence
<220>
   <223> Synthetic oligonucleotide
<400> 596
   cctccacaga tgtggt 16
<210> 597
   <211> 16
   <212> DNA
   <213> Artificial sequence
<220>
   <223> Synthetic oligonucleotide
<400> 597
   cacctccaca gatgtg 16
<210> 598
   <211> 16
   <212> DNA
   <213> Artificial sequence
<220>
   <223> Synthetic oligonucleotide
<400> 598
   ggccacctcc acagat 16
<210> 599
   <211> 16
   <212> DNA
   <213> Artificial sequence
<220>
   <223> Synthetic oligonucleotide
<400> 599
   tgcttggctc tggcca 16
<210> 600
   <211> 16
   <212> DNA
   <213> Artificial sequence
<220>
   <223> Synthetic oligonucleotide
<400> 600
   actgcttggc tctggc 16
<210> 601
   <211> 16
   <212> DNA
   <213> Artificial sequence
<220>
   <223> Synthetic oligonucleotide
<400> 601
   agactgcttg gctctg 16
<210> 602
   <211> 16
   <212> DNA
   <213> Artificial sequence
<220>
   <223> Synthetic oligonucleotide
<400> 602
   ggagactgct tggctc 16
<210> 603
   <211> 16
   <212> DNA
   <213> Artificial sequence
<220>
   <223> Synthetic oligonucleotide
<400> 603
   tgcagacccc tcttct 16
<210> 604
   <211> 16
   <212> DNA
   <213> Artificial sequence
<220>
   <223> Synthetic oligonucleotide
<400> 604
   ctcctccctt gacatg 16
<210> 605
   <211> 16
   <212> DNA
   <213> Artificial sequence
<220>
   <223> Synthetic oligonucleotide
<400> 605
   ccagaccccc atgttc 16
<210> 606
   <211> 16
   <212> DNA
   <213> Artificial sequence
<220>
   <223> Synthetic oligonucleotide
<400> 606
   gtccagaccc ccatgt 16
<210> 607
   <211> 16
   <212> DNA
   <213> Artificial sequence
<220>
   <223> Synthetic oligonucleotide
<400> 607
   gggtccagac ccccat 16
<210> 608
   <211> 16
   <212> DNA
   <213> Artificial sequence
<220>
   <223> Synthetic oligonucleotide
<400> 608
   accttctgca gggact 16
<210> 609
   <211> 16
   <212> DNA
   <213> Artificial sequence
<220>
   <223> Synthetic oligonucleotide
<400> 609
   taaaccttct gcaggg 16
<210> 610
   <211> 16
   <212> DNA
   <213> Artificial sequence
<220>
   <223> Synthetic oligonucleotide
<400> 610
   gaaaagccct gcccct 16
<210> 611
   <211> 16
   <212> DNA
   <213> Artificial sequence
<220>
   <223> Synthetic oligonucleotide
<400> 611
   taggaaaagc cctgcc 16
<210> 612
   <211> 16
   <212> DNA
   <213> Artificial sequence
<220>
   <223> Synthetic oligonucleotide
<400> 612
   cttaggaaaa gccctg 16
<210> 613
   <211> 16
   <212> DNA
   <213> Artificial sequence
<220>
   <223> Synthetic oligonucleotide
<400> 613
   tgcttaggaa aagccc 16
<210> 614
   <211> 16
   <212> DNA
   <213> Artificial sequence
<220>
   <223> Synthetic oligonucleotide
<400> 614
   tctgcttagg aaaagc 16
<210> 615
   <211> 16
   <212> DNA
   <213> Artificial sequence
<220>
   <223> Synthetic oligonucleotide
<400> 615
   ctcctctgct taggaa 16
<210> 616
   <211> 16
   <212> DNA
   <213> Artificial sequence
<220>
   <223> Synthetic oligonucleotide
<400> 616
   ccctcctctg cttagg 16
<210> 617
   <211> 16
   <212> DNA
   <213> Artificial sequence
<220>
   <223> Synthetic oligonucleotide
<400> 617
   ctgatttgag gaaggg 16
<210> 618
   <211> 16
   <212> DNA
   <213> Artificial sequence
<220>
   <223> Synthetic oligonucleotide
<400> 618
   tcctgatttg aggaag 16
<210> 619
   <211> 16
   <212> DNA
   <213> Artificial sequence
<220>
   <223> Synthetic oligonucleotide
<400> 619
   cctcctgatt tgagga 16
<210> 620
   <211> 16
   <212> DNA
   <213> Artificial sequence
<220>
   <223> Synthetic oligonucleotide
<400> 620
   gacctcctga tttgag 16
<210> 621
   <211> 16
   <212> DNA
   <213> Artificial sequence
<220>
   <223> Synthetic oligonucleotide
<400> 621
   aagacctcct gatttg 16
<210> 622
   <211> 16
   <212> DNA
   <213> Artificial sequence
<220>
   <223> Synthetic oligonucleotide
<400> 622
   ccaagacctc ctgatt 16
<210> 623
   <211> 16
   <212> DNA
   <213> Artificial sequence
<220>
   <223> Synthetic oligonucleotide
<400> 623
   ctgcttccaa gacctc 16
<210> 624
   <211> 16
   <212> DNA
   <213> Artificial sequence
<220>
   <223> Synthetic oligonucleotide
<400> 624
   agctgcttcc aagacc 16
<210> 625
   <211> 16
   <212> DNA
   <213> Artificial sequence
<220>
   <223> Synthetic oligonucleotide
<400> 625
   gcagctgctt ccaaga 16
<210> 626
   <211> 16
   <212> DNA
   <213> Artificial sequence
<220>
   <223> Synthetic oligonucleotide
<400> 626
   ctggtggaga accaga 16
<210> 627
   <211> 16
   <212> DNA
   <213> Artificial sequence
<220>
   <223> Synthetic oligonucleotide
<400> 627
   ctctggtgga gaacca 16
<210> 628
   <211> 16
   <212> DNA
   <213> Artificial sequence
<220>
   <223> Synthetic oligonucleotide
<400> 628
   ttctctggtg gagaac 16
<210> 629
   <211> 16
   <212> DNA
   <213> Artificial sequence
<220>
   <223> Synthetic oligonucleotide
<400> 629
   gattctctgg tggaga 16
<210> 630
   <211> 16
   <212> DNA
   <213> Artificial sequence
<220>
   <223> Synthetic oligonucleotide
<400> 630
   acttactgtt tcatcc 16
<210> 631
   <211> 16
   <212> DNA
   <213> Artificial sequence
<220>
   <223> Synthetic oligonucleotide
<400> 631
   cggaccccct cccctc 16
<210> 632
   <211> 16
   <212> DNA
   <213> Artificial sequence
<220>
   <223> Synthetic oligonucleotide
<400> 632
   gacggacccc ctcccc 16
<210> 633
   <211> 16
   <212> DNA
   <213> Artificial sequence
<220>
   <223> Synthetic oligonucleotide
<400> 633
   ctgacggacc ccctcc 16
<210> 634
   <211> 16
   <212> DNA
   <213> Artificial sequence
<220>
   <223> Synthetic oligonucleotide
<400> 634
   ccctgacgga ccccct 16
<210> 635
   <211> 16
   <212> DNA
   <213> Artificial sequence
<220>
   <223> Synthetic oligonucleotide
<400> 635
   aagccctcac cttttc 16
<210> 636
   <211> 16
   <212> DNA
   <213> Artificial sequence
<220>
   <223> Synthetic oligonucleotide
<400> 636
   ggaagccctc accttt 16
<210> 637
   <211> 16
   <212> DNA
   <213> Artificial sequence
<220>
   <223> Synthetic oligonucleotide
<400> 637
   cgggaagccc tcacct 16
<210> 638
   <211> 16
   <212> DNA
   <213> Artificial sequence
<220>
   <223> Synthetic oligonucleotide
<400> 638
   cccgggaagc cctcac 16
<210> 639
   <211> 16
   <212> DNA
   <213> Artificial sequence
<220>
   <223> Synthetic oligonucleotide
<400> 639
   cacccgggaa gccctc 16
<210> 640
   <211> 16
   <212> DNA
   <213> Artificial sequence
<220>
   <223> Synthetic oligonucleotide
<400> 640
   gccacccggg aagccc 16
<210> 641
   <211> 16
   <212> DNA
   <213> Artificial sequence
<220>
   <223> Synthetic oligonucleotide
<400> 641
   acgccacccg ggaagc 16
<210> 642
   <211> 16
   <212> DNA
   <213> Artificial sequence
<220>
   <223> Synthetic oligonucleotide
<400> 642
   ctgttcagga agtccc 16
<210> 643
   <211> 16
   <212> DNA
   <213> Artificial sequence
<220>
   <223> Synthetic oligonucleotide
<400> 643
   ttctgttcag gaagtc 16
<210> 644
   <211> 16
   <212> DNA
   <213> Artificial sequence
<220>
   <223> Synthetic oligonucleotide
<400> 644
   gcttctgttc aggaag 16
<210> 645
   <211> 16
   <212> DNA
   <213> Artificial sequence
<220>
   <223> Synthetic oligonucleotide
<400> 645
   gcttgggccc acccct 16
<210> 646
   <211> 16
   <212> DNA
   <213> Artificial sequence
<220>
   <223> Synthetic oligonucleotide
<400> 646
   aggcttgggc ccaccc 16
<210> 647
   <211> 16
   <212> DNA
   <213> Artificial sequence
<220>
   <223> Synthetic oligonucleotide
<400> 647
   cgaggcttgg gcccac 16
<210> 648
   <211> 16
   <212> DNA
   <213> Artificial sequence
<220>
   <223> Synthetic oligonucleotide
<400> 648
   agcgaggctt gggccc 16
<210> 649
   <211> 16
   <212> DNA
   <213> Artificial sequence
<220>
   <223> Synthetic oligonucleotide
<400> 649
   agagcgaggc ttgggc 16
<210> 650
   <211> 16
   <212> DNA
   <213> Artificial sequence
<220>
   <223> Synthetic oligonucleotide
<400> 650
   gcagagcgag gcttgg 16
<210> 651
   <211> 16
   <212> DNA
   <213> Artificial sequence
<220>
   <223> Synthetic oligonucleotide
<400> 651
   gagcagagcg aggctt 16
<210> 652
   <211> 16
   <212> DNA
   <213> Artificial sequence
<220>
   <223> Synthetic oligonucleotide
<400> 652
   aaaggagcag agcgag 16
<210> 653
   <211> 16
   <212> DNA
   <213> Artificial sequence
<220>
   <223> Synthetic oligonucleotide
<400> 653
   caaaaggagc agagcg 16
<210> 654
   <211> 16
   <212> DNA
   <213> Artificial sequence
<220>
   <223> Synthetic oligonucleotide
<400> 654
   tggaccaaaa ggagca 16
<210> 655
   <211> 16
   <212> DNA
   <213> Artificial sequence
<220>
   <223> Synthetic oligonucleotide
<400> 655
   cctggaccaa aaggag 16
<210> 656
   <211> 16
   <212> DNA
   <213> Artificial sequence
<220>
   <223> Synthetic oligonucleotide
<400> 656
   cacctggacc aaaagg 16
<210> 657
   <211> 16
   <212> DNA
   <213> Artificial sequence
<220>
   <223> Synthetic oligonucleotide
<400> 657
   cgcacctgga ccaaaa 16
<210> 658
   <211> 16
   <212> DNA
   <213> Artificial sequence
<220>
   <223> Synthetic oligonucleotide
<400> 658
   gaccgcacct ggacca 16
<210> 659
   <211> 16
   <212> DNA
   <213> Artificial sequence
<220>
   <223> Synthetic oligonucleotide
<400> 659
   accttgtagt ggacga 16
<210> 660
   <211> 16
   <212> DNA
   <213> Artificial sequence
<220>
   <223> Synthetic oligonucleotide
<400> 660
   tcaccttgta gtggac 16
<210> 661
   <211> 16
   <212> DNA
   <213> Artificial sequence
<220>
   <223> Synthetic oligonucleotide
<400> 661
   gctcaccttg tagtgg 16
<210> 662
   <211> 16
   <212> DNA
   <213> Artificial sequence
<220>
   <223> Synthetic oligonucleotide
<400> 662
   ggagaggagg cgatag 16
<210> 663
   <211> 16
   <212> DNA
   <213> Artificial sequence
<220>
   <223> Synthetic oligonucleotide
<400> 663
   agggagagga ggcgat 16
<210> 664
   <211> 16
   <212> DNA
   <213> Artificial sequence
<220>
   <223> Synthetic oligonucleotide
<400> 664
   ctcctgctca gaggga 16
<210> 665
   <211> 16
   <212> DNA
   <213> Artificial sequence
<220>
   <223> Synthetic oligonucleotide
<400> 665
   gtgctcctgc tcagag 16
<210> 666
   <211> 16
   <212> DNA
   <213> Artificial sequence
<220>
   <223> Synthetic oligonucleotide
<400> 666
   aggtgctcct gctcag 16
<210> 667
   <211> 16
   <212> DNA
   <213> Artificial sequence
<220>
   <223> Synthetic oligonucleotide
<400> 667
   agaggtgctc ctgctc 16
<210> 668
   <211> 16
   <212> DNA
   <213> Artificial sequence
<220>
   <223> Synthetic oligonucleotide
<400> 668
   agagaggtgc tcctgc 16
<210> 669
   <211> 16
   <212> DNA
   <213> Artificial sequence
<220>
   <223> Synthetic oligonucleotide
<400> 669
   accccgcccc cgctca 16
<210> 670
   <211> 16
   <212> DNA
   <213> Artificial sequence
<220>
   <223> Synthetic oligonucleotide
<400> 670
   ctaccccgcc cccgct 16
<210> 671
   <211> 16
   <212> DNA
   <213> Artificial sequence
<220>
   <223> Synthetic oligonucleotide
<400> 671
   acctaccccg cccccg 16
<210> 672
   <211> 16
   <212> DNA
   <213> Artificial sequence
<220>
   <223> Synthetic oligonucleotide
<400> 672
   gtacctaccc cgcccc 16
<210> 673
   <211> 16
   <212> DNA
   <213> Artificial sequence
<220>
   <223> Synthetic oligonucleotide
<400> 673
   aggtacctac cccgcc 16
<210> 674
   <211> 16
   <212> DNA
   <213> Artificial sequence
<220>
   <223> Synthetic oligonucleotide
<400> 674
   gggaggttcc cgcagc 16
<210> 675
   <211> 16
   <212> DNA
   <213> Artificial sequence
<220>
   <223> Synthetic oligonucleotide
<400> 675
   gtccttactc caactt 16
<210> 676
   <211> 16
   <212> DNA
   <213> Artificial sequence
<220>
   <223> Synthetic oligonucleotide
<400> 676
   ctgtccttac tccaac 16
<210> 677
   <211> 16
   <212> DNA
   <213> Artificial sequence
<220>
   <223> Synthetic oligonucleotide
<400> 677
   cactgtcctt actcca 16
<210> 678
   <211> 16
   <212> DNA
   <213> Artificial sequence
<220>
   <223> Synthetic oligonucleotide
<400> 678
   ggcactgtcc ttactc 16
<210> 679
   <211> 16
   <212> DNA
   <213> Artificial sequence
<220>
   <223> Synthetic oligonucleotide
<400> 679
   taggcactgt ccttac 16
<210> 680
   <211> 16
   <212> DNA
   <213> Artificial sequence
<220>
   <223> Synthetic oligonucleotide
<400> 680
   ggtaggcact gtcctt 16
<210> 681
   <211> 16
   <212> DNA
   <213> Artificial sequence
<220>
   <223> Synthetic oligonucleotide
<400> 681
   gtcactgctg ggtcct 16
<210> 682
   <211> 16
   <212> DNA
   <213> Artificial sequence
<220>
   <223> Synthetic oligonucleotide
<400> 682
   aggtcactgc tgggtc 16
<210> 683
   <211> 16
   <212> DNA
   <213> Artificial sequence
<220>
   <223> Synthetic oligonucleotide
<400> 683
   ctaggtcact gctggg 16
<210> 684
   <211> 16
   <212> DNA
   <213> Artificial sequence
<220>
   <223> Synthetic oligonucleotide
<400> 684
   gtctaggtca ctgctg 16
<210> 685
   <211> 16
   <212> DNA
   <213> Artificial sequence
<220>
   <223> Synthetic oligonucleotide
<400> 685
   aagtctaggt cactgc 16
<210> 686
   <211> 16
   <212> DNA
   <213> Artificial sequence
<220>
   <223> Synthetic oligonucleotide
<400> 686
   gcactccatt gtctca 16
<210> 687
   <211> 16
   <212> DNA
   <213> Artificial sequence
<220>
   <223> Synthetic oligonucleotide
<400> 687
   ctgcactcca ttgtct 16
<210> 688
   <211> 16
   <212> DNA
   <213> Artificial sequence
<220>
   <223> Synthetic oligonucleotide
<400> 688
   ccctgcactc cattgt 16
<210> 689
   <211> 16
   <212> DNA
   <213> Artificial sequence
<220>
   <223> Synthetic oligonucleotide
<400> 689
   ccccctgcac tccatt 16
<210> 690
   <211> 16
   <212> DNA
   <213> Artificial sequence
<220>
   <223> Synthetic oligonucleotide
<400> 690
   cttgctgagt caggag 16
<210> 691
   <211> 16
   <212> DNA
   <213> Artificial sequence
<220>
   <223> Synthetic oligonucleotide
<400> 691
   tccttgctga gtcagg 16
<210> 692
   <211> 16
   <212> DNA
   <213> Artificial sequence
<220>
   <223> Synthetic oligonucleotide
<400> 692
   cttccttgct gagtca 16
<210> 693
   <211> 16
   <212> DNA
   <213> Artificial sequence
<220>
   <223> Synthetic oligonucleotide
<400> 693
   accttccttg ctgagt 16
<210> 694
   <211> 16
   <212> DNA
   <213> Artificial sequence
<220>
   <223> Synthetic oligonucleotide
<400> 694
   ggaccttcct tgctga 16
<210> 695
   <211> 16
   <212> DNA
   <213> Artificial sequence
<220>
   <223> Synthetic oligonucleotide
<400> 695
   caggaccttc cttgct 16
<210> 696
   <211> 16
   <212> DNA
   <213> Artificial sequence
<220>
   <223> Synthetic oligonucleotide
<400> 696
   agccctccag gacctt 16
<210> 697
   <211> 16
   <212> DNA
   <213> Artificial sequence
<220>
   <223> Synthetic oligonucleotide
<400> 697
   tagctcccca ctccag 16
<210> 698
   <211> 16
   <212> DNA
   <213> Artificial sequence
<220>
   <223> Synthetic oligonucleotide
<400> 698
   gatagctccc cactcc 16
<210> 699
   <211> 16
   <212> DNA
   <213> Artificial sequence
<220>
   <223> Synthetic oligonucleotide
<400> 699
   cagatagctc cccact 16
<210> 700
   <211> 16
   <212> DNA
   <213> Artificial sequence
<220>
   <223> Synthetic oligonucleotide
<400> 700
   ctcagatagc tcccca 16
<210> 701
   <211> 16
   <212> DNA
   <213> Artificial sequence
<220>
   <223> Synthetic oligonucleotide
<400> 701
   agctcagata gctccc 16
<210> 702
   <211> 16
   <212> DNA
   <213> Artificial sequence
<220>
   <223> Synthetic oligonucleotide
<400> 702
   tcagctcaga tagctc 16
<210> 703
   <211> 16
   <212> DNA
   <213> Artificial sequence
<220>
   <223> Synthetic oligonucleotide
<400> 703
   tctcagctca gatagc 16
<210> 704
   <211> 16
   <212> DNA
   <213> Artificial sequence
<220>
   <223> Synthetic oligonucleotide
<400> 704
   gagtcctctc ctgctt 16
<210> 705
   <211> 16
   <212> DNA
   <213> Artificial sequence
<220>
   <223> Synthetic oligonucleotide
<400> 705
   ggaggagtcc tctcct 16
<210> 706
   <211> 16
   <212> DNA
   <213> Artificial sequence
<220>
   <223> Synthetic oligonucleotide
<400> 706
   gaggaggagt cctctc 16
<210> 707
   <211> 16
   <212> DNA
   <213> Artificial sequence
<220>
   <223> Synthetic oligonucleotide
<400> 707
   caaaagggca cccaga 16
<210> 708
   <211> 16
   <212> DNA
   <213> Artificial sequence
<220>
   <223> Synthetic oligonucleotide
<400> 708
   agcaaaaggg caccca 16
<210> 709
   <211> 16
   <212> DNA
   <213> Artificial sequence
<220>
   <223> Synthetic oligonucleotide
<400> 709
   ggatccccag tattgt 16
<210> 710
   <211> 16
   <212> DNA
   <213> Artificial sequence
<220>
   <223> Synthetic oligonucleotide
<400> 710
   ctggatcccc agtatt 16
<210> 711
   <211> 16
   <212> DNA
   <213> Artificial sequence
<220>
   <223> Synthetic oligonucleotide
<400> 711
   tgctggatcc ccagta 16
<210> 712
   <211> 16
   <212> DNA
   <213> Artificial sequence
<220>
   <223> Synthetic oligonucleotide
<400> 712
   attctctaga ctgcaa 16
<210> 713
   <211> 16
   <212> DNA
   <213> Artificial sequence
<220>
   <223> Synthetic oligonucleotide
<400> 713
   taattctcta gactgc 16
<210> 714
   <211> 16
   <212> DNA
   <213> Artificial sequence
<220>
   <223> Synthetic oligonucleotide
<400> 714
   tctaattctc tagact 16
<210> 715
   <211> 16
   <212> DNA
   <213> Artificial sequence
<220>
   <223> Synthetic oligonucleotide
<400> 715
   tctctaattc tctaga 16
<210> 716
   <211> 16
   <212> DNA
   <213> Artificial sequence
<220>
   <223> Synthetic oligonucleotide
<400> 716
   ctccataatt ctctaa 16
<210> 717
   <211> 16
   <212> DNA
   <213> Artificial sequence
<220>
   <223> Synthetic oligonucleotide
<400> 717
   actctccata attctc 16
<210> 718
   <211> 16
   <212> DNA
   <213> Artificial sequence
<220>
   <223> Synthetic oligonucleotide
<400> 718
   acactctcca taattc 16
<210> 719
   <211> 16
   <212> DNA
   <213> Artificial sequence
<220>
   <223> Synthetic oligonucleotide
<400> 719
   ccacactctc cataat 16
<210> 720
   <211> 16
   <212> DNA
   <213> Artificial sequence
<220>
   <223> Synthetic oligonucleotide
<400> 720
   tgccacactc tccata 16
<210> 721
   <211> 16
   <212> DNA
   <213> Artificial sequence
<220>
   <223> Synthetic oligonucleotide
<400> 721
   cccatgccca tcctgc 16
<210> 722
   <211> 16
   <212> DNA
   <213> Artificial sequence
<220>
   <223> Synthetic oligonucleotide
<400> 722
   ggacagagaa atgttg 16
<210> 723
   <211> 16
   <212> DNA
   <213> Artificial sequence
<220>
   <223> Synthetic oligonucleotide
<400> 723
   ggcataggac agagaa 16
<210> 724
   <211> 16
   <212> DNA
   <213> Artificial sequence
<220>
   <223> Synthetic oligonucleotide
<400> 724
   gtggcatagg acagag 16
<210> 725
   <211> 16
   <212> DNA
   <213> Artificial sequence
<220>
   <223> Synthetic oligonucleotide
<400> 725
   tggtggcata ggacag 16
<210> 726
   <211> 16
   <212> DNA
   <213> Artificial sequence
<220>
   <223> Synthetic oligonucleotide
<400> 726
   cttactctgc ccctcc 16
<210> 727
   <211> 16
   <212> DNA
   <213> Artificial sequence
<220>
   <223> Synthetic oligonucleotide
<400> 727
   accttactct gcccct 16
<210> 728
   <211> 16
   <212> DNA
   <213> Artificial sequence
<220>
   <223> Synthetic oligonucleotide
<400> 728
   tgaccttact ctgccc 16
<210> 729
   <211> 16
   <212> DNA
   <213> Artificial sequence
<220>
   <223> Synthetic oligonucleotide
<400> 729
   gctgacctta ctctgc 16
<210> 730
   <211> 16
   <212> DNA
   <213> Artificial sequence
<220>
   <223> Synthetic oligonucleotide
<400> 730
   ctgctgacct tactct 16
<210> 731
   <211> 16
   <212> DNA
   <213> Artificial sequence
<220>
   <223> Synthetic oligonucleotide
<400> 731
   ctctgctgac cttact 16
<210> 732
   <211> 16
   <212> DNA
   <213> Artificial sequence
<220>
   <223> Synthetic oligonucleotide
<400> 732
   gcctctgctg acctta 16
<210> 733
   <211> 16
   <212> DNA
   <213> Artificial sequence
<220>
   <223> Synthetic oligonucleotide
<400> 733
   ccatggctct gagtca 16
<210> 734
   <211> 16
   <212> DNA
   <213> Artificial sequence
<220>
   <223> Synthetic oligonucleotide
<400> 734
   agccatggct ctgagt 16
<210> 735
   <211> 16
   <212> DNA
   <213> Artificial sequence
<220>
   <223> Synthetic oligonucleotide
<400> 735
   taagccatgg ctctga 16
<210> 736
   <211> 16
   <212> DNA
   <213> Artificial sequence
<220>
   <223> Synthetic oligonucleotide
<400> 736
   tagcctgctg tgactc 16
<210> 737
   <211> 16
   <212> DNA
   <213> Artificial sequence
<220>
   <223> Synthetic oligonucleotide
<400> 737
   atgggaggct gttggc 16
<210> 738
   <211> 16
   <212> DNA
   <213> Artificial sequence
<220>
   <223> Synthetic oligonucleotide
<400> 738
   ccatgggagg ctgttg 16
<210> 739
   <211> 16
   <212> DNA
   <213> Artificial sequence
<220>
   <223> Synthetic oligonucleotide
<400> 739
   ggccatggga ggctgt 16
<210> 740
   <211> 16
   <212> DNA
   <213> Artificial sequence
<220>
   <223> Synthetic oligonucleotide
<400> 740
   gtgcagagag gccatg 16
<210> 741
   <211> 16
   <212> DNA
   <213> Artificial sequence
<220>
   <223> Synthetic oligonucleotide
<400> 741
   gagctcccag catgac 16
<210> 742
   <211> 16
   <212> DNA
   <213> Artificial sequence
<220>
   <223> Synthetic oligonucleotide
<400> 742
   agggagctcc cagcat 16
<210> 743
   <211> 16
   <212> DNA
   <213> Artificial sequence
<220>
   <223> Synthetic oligonucleotide
<400> 743
   gccatagagc ccactt 16
<210> 744
   <211> 16
   <212> DNA
   <213> Artificial sequence
<220>
   <223> Synthetic oligonucleotide
<400> 744
   gggccataga gcccac 16
<210> 745
   <211> 16
   <212> DNA
   <213> Artificial sequence
<220>
   <223> Synthetic oligonucleotide
<400> 745
   atgctggccc tcctgg 16
<210> 746
   <211> 16
   <212> DNA
   <213> Artificial sequence
<220>
   <223> Synthetic oligonucleotide
<400> 746
   agctgcccca tgctgg 16
<210> 747
   <211> 16
   <212> DNA
   <213> Artificial sequence
<220>
   <223> Synthetic oligonucleotide
<400> 747
   cgcccctggc agctgc 16
<210> 748
   <211> 16
   <212> DNA
   <213> Artificial sequence
<220>
   <223> Synthetic oligonucleotide
<400> 748
   tgcgcccctg gcagct 16
<210> 749
   <211> 16
   <212> DNA
   <213> Artificial sequence
<220>
   <223> Synthetic oligonucleotide
<400> 749
   gctgcgcccc tggcag 16
<210> 750
   <211> 16
   <212> DNA
   <213> Artificial sequence
<220>
   <223> Synthetic oligonucleotide
<400> 750
   cggctgcgcc cctggc 16
<210> 751
   <211> 16
   <212> DNA
   <213> Artificial sequence
<220>
   <223> Synthetic oligonucleotide
<400> 751
   gtcggctgcg cccctg 16
<210> 752
   <211> 16
   <212> DNA
   <213> Artificial sequence
<220>
   <223> Synthetic oligonucleotide
<400> 752
   ctgtcggctg cgcccc 16
<210> 753
   <211> 16
   <212> DNA
   <213> Artificial sequence
<220>
   <223> Synthetic oligonucleotide
<400> 753
   gcctgtcggc tgcgcc 16
<210> 754
   <211> 16
   <212> DNA
   <213> Artificial sequence
<220>
   <223> Synthetic oligonucleotide
<400> 754
   ctgcctgtcg gctgcg 16
<210> 755
   <211> 16
   <212> DNA
   <213> Artificial sequence
<220>
   <223> Synthetic oligonucleotide
<400> 755
   acctgcctgt cggctg 16
<210> 756
   <211> 16
   <212> DNA
   <213> Artificial sequence
<220>
   <223> Synthetic oligonucleotide
<400> 756
   acacctgcct gtcggc 16
<210> 757
   <211> 16
   <212> DNA
   <213> Artificial sequence
<220>
   <223> Synthetic oligonucleotide
<400> 757
   gaacacctgc ctgtcg 16
<210> 758
   <211> 16
   <212> DNA
   <213> Artificial sequence
<220>
   <223> Synthetic oligonucleotide
<400> 758
   ccgaacacct gcctgt 16
<210> 759
   <211> 16
   <212> **DNA**
   <213> Artificial sequence
<220>
   <223> Synthetic oligonucleotide
<400> 759
   cgccgaacac ctgcct 16
<210> 760
   <211> 16
   <212> DNA
   <213> Artificial sequence
<220>
   <223> Synthetic oligonucleotide
<400> 760
   cctgggcacc tgttgg 16
<210> 761
   <211> 16
   <212> DNA
   <213> Artificial sequence
<220>
   <223> Synthetic oligonucleotide
<400> 761
   gtgcctgggc acctgt 16
<210> 762
   <211> 16
   <212> DNA
   <213> Artificial sequence
<220>
   <223> Synthetic oligonucleotide
<400> 762
   cgccctccca gtgcct 16
<210> 763
   <211> 16
   <212> DNA
   <213> Artificial sequence
<220>
   <223> Synthetic oligonucleotide
<400> 763
   accgccctcc cagtgc 16
<210> 764
   <211> 16
   <212> DNA
   <213> Artificial sequence
<220>
   <223> Synthetic oligonucleotide
<400> 764
   tcaccgccct cccagt 16
<210> 765
   <211> 16
   <212> DNA
   <213> Artificial sequence
<220>
   <223> Synthetic oligonucleotide
<400> 765
   agtcaccgcc ctccca 16
<210> 766
   <211> 16
   <212> DNA
   <213> Artificial sequence
<220>
   <223> Synthetic oligonucleotide
<400> 766
   tgagtcaccg ccctcc 16
<210> 767
   <211> 16
   <212> DNA
   <213> Artificial sequence
<220>
   <223> Synthetic oligonucleotide
<400> 767
   cgtgagtcac cgccct 16
<210> 768
   <211> 16
   <212> DNA
   <213> Artificial sequence
<220>
   <223> Synthetic oligonucleotide
<400> 768
   caaagctggt tctccc 16
<210> 769
   <211> 16
   <212> DNA
   <213> Artificial sequence
<220>
   <223> Synthetic oligonucleotide
<400> 769
   tgcaaagctg gttctc 16
<210> 770
   <211> 16
   <212> DNA
   <213> Artificial sequence
<220>
   <223> Synthetic oligonucleotide
<400> 770
   tctgcaaagc tggttc 16
<210> 771
   <211> 16
   <212> DNA
   <213> Artificial sequence
<220>
   <223> Synthetic oligonucleotide
<400> 771
   tgtctgcaaa gctggt 16
<210> 772
   <211> 16
   <212> DNA
   <213> Artificial sequence
<220>
   <223> Synthetic oligonucleotide
<400> 772
   cctgtctgca aagctg 16
<210> 773
   <211> 16
   <212> DNA
   <213> Artificial sequence
<220>
   <223> Synthetic oligonucleotide
<400> 773
   cgcctgtctg caaagc 16
<210> 774
   <211> 16
   <212> DNA
   <213> Artificial sequence
<220>
   <223> Synthetic oligonucleotide
<400> 774
   ttgtccctcc tggatc 16
<210> 775
   <211> 16
   <212> DNA
   <213> Artificial sequence
<220>
   <223> Synthetic oligonucleotide
<400> 775
   agttgtccct cctgga 16
<210> 776
   <211> 16
   <212> DNA
   <213> Artificial sequence
<220>
   <223> Synthetic oligonucleotide
<400> 776
   aaagttgtcc ctcctg 16
<210> 777
   <211> 16
   <212> DNA
   <213> Artificial sequence
<220>
   <223> Synthetic oligonucleotide
<400> 777
   ccaaagttgt ccctcc 16
<210> 778
   <211> 16
   <212> DNA
   <213> Artificial sequence
<220>
   <223> Synthetic oligonucleotide
<400> 778
   acccaaagtt gtccct 16
<210> 779
   <211> 16
   <212> DNA
   <213> Artificial sequence
<220>
   <223> Synthetic oligonucleotide
<400> 779
   gaacccaaag ttgtcc 16
<210> 780
   <211> 16
   <212> DNA
   <213> Artificial sequence
<220>
   <223> Synthetic oligonucleotide
<400> 780
   gaagaaccca aagttg 16
<210> 781
   <211> 16
   <212> DNA
   <213> Artificial sequence
<220>
   <223> Synthetic oligonucleotide
<400> 781
   ccagaagaac ccaaag 16
<210> 782
   <211> 16
   <212> DNA
   <213> Artificial sequence
<220>
   <223> Synthetic oligonucleotide
<400> 782
   cacccagaag aaccca 16
<210> 783
   <211> 16
   <212> DNA
   <213> Artificial sequence
<220>
   <223> Synthetic oligonucleotide
<400> 783
   gcagaaccta caaaag 16
<210> 784
   <211> 16
   <212> DNA
   <213> Artificial sequence
<220>
   <223> Synthetic oligonucleotide
<400> 784
   gtgcagaacc tacaaa 16
<210> 785
   <211> 16
   <212> DNA
   <213> Artificial sequence
<220>
   <223> Synthetic oligonucleotide
<400> 785
   gggtgcagaa cctaca 16
<210> 786
   <211> 16
   <212> DNA
   <213> Artificial sequence
<220>
   <223> Synthetic oligonucleotide
<400> 786
   gtgggtgcag aaccta 16
<210> 787
   <211> 16
   <212> DNA
   <213> Artificial sequence
<220>
   <223> Synthetic oligonucleotide
<400> 787
   ccacacggct catagg 16
<210> 788
   <211> 16
   <212> DNA
   <213> Artificial sequence
<220>
   <223> Synthetic oligonucleotide
<400> 788
   acccacacgg ctcata 16
<210> 789
   <211> 16
   <212> DNA
   <213> Artificial sequence
<220>
   <223> Synthetic oligonucleotide
<400> 789
   tgacccacac ggctca 16
<210> 790
   <211> 16
   <212> DNA
   <213> Artificial sequence
<220>
   <223> Synthetic oligonucleotide
<400> 790
   gctgacccac acggct 16
<210> 791
   <211> 16
   <212> DNA
   <213> Artificial sequence
<220>
   <223> Synthetic oligonucleotide
<400> 791
   tggctgaccc acacgg 16
<210> 792
   <211> 16
   <212> DNA
   <213> Artificial sequence
<220>
   <223> Synthetic oligonucleotide
<400> 792
   ggtggctgac ccacac 16
<210> 793
   <211> 16
   <212> DNA
   <213> Artificial sequence
<220>
   <223> Synthetic oligonucleotide
<400> 793
   atggtggctg acccac 16
<210> 794
   <211> 16
   <212> DNA
   <213> Artificial sequence
<220>
   <223> Synthetic oligonucleotide
<400> 794
   gaatggtggc tgaccc 16
<210> 795
   <211> 16
   <212> DNA
   <213> Artificial sequence
<220>
   <223> Synthetic oligonucleotide
<400> 795
   ctaaaggacg caggga 16
<210> 796
   <211> 16
   <212> DNA
   <213> Artificial sequence
<220>
   <223> Synthetic oligonucleotide
<400> 796
   aactaaagga cgcagg 16
<210> 797
   <211> 16
   <212> DNA
   <213> Artificial sequence
<220>
   <223> Synthetic oligonucleotide
<400> 797
   gagaactaaa ggacgc 16
<210> 798
   <211> 16
   <212> DNA
   <213> Artificial sequence
<220>
   <223> Synthetic oligonucleotide
<400> 798
   attggtccca agcccc 16
<210> 799
   <211> 16
   <212> DNA
   <213> Artificial sequence
<220>
   <223> Synthetic oligonucleotide
<400> 799
   ccattggtcc caagcc 16
<210> 800
   <211> 16
   <212> DNA
   <213> Artificial sequence
<220>
   <223> Synthetic oligonucleotide
<400> 800
   gcccattggt cccaag 16
<210> 801
   <211> 16
   <212> DNA
   <213> Artificial sequence
<220>
   <223> Synthetic oligonucleotide
<400> 801
   acgcccattg gtccca 16
<210> 802
   <211> 16
   <212> DNA
   <213> Artificial sequence
<220>
   <223> Synthetic oligonucleotide
<400> 802
   ccacgcccat tggtcc 16
<210> 803
   <211> 16
   <212> DNA
   <213> Artificial sequence
<220>
   <223> Synthetic oligonucleotide
<400> 803
   caccacgccc attggt 16
<210> 804
   <211> 16
   <212> DNA
   <213> Artificial sequence
<220>
   <223> Synthetic oligonucleotide
<400> 804
   agacccaact ccaccc 16
<210> 805
   <211> 16
   <212> DNA
   <213> Artificial sequence
<220>
   <223> Synthetic oligonucleotide
<400> 805
   tcacctcgcc cctctt 16
<210> 806
   <211> 16
   <212> DNA
   <213> Artificial sequence
<220>
   <223> Synthetic oligonucleotide
<400> 806
   cctcacctcg cccctc 16
<210> 807
   <211> 16
   <212> DNA
   <213> Artificial sequence
<220>
   <223> Synthetic oligonucleotide
<400> 807
   agcccctcac ctcgcc 16
<210> 808
   <211> 16
   <212> DNA
   <213> Artificial sequence
<220>
   <223> Synthetic oligonucleotide
<400> 808
   ctcaaagccc cccacg 16
<210> 809
   <211> 16
   <212> DNA
   <213> Artificial sequence
<220>
   <223> Synthetic oligonucleotide
<400> 809
   atcctcaaag cccccc 16
<210> 810
   <211> 16
   <212> DNA
   <213> Artificial sequence
<220>
   <223> Synthetic oligonucleotide
<400> 810
   ggatcctcaa agcccc 16
<210> 811
   <211> 16
   <212> DNA
   <213> Artificial sequence
<220>
   <223> Synthetic oligonucleotide
<400> 811
   gcggatcctc aaagcc 16
<210> 812
   <211> 16
   <212> DNA
   <213> Artificial sequence
<220>
   <223> Synthetic oligonucleotide
<400> 812
   gcgcggatcc tcaaag 16
<210> 813
   <211> 16
   <212> DNA
   <213> Artificial sequence
<220>
   <223> Synthetic oligonucleotide
<400> 813
   gggcgcggat cctcaa 16
<210> 814
   <211> 16
   <212> DNA
   <213> Artificial sequence
<220>
   <223> Synthetic oligonucleotide
<400> 814
   gagctgcagc cggaga 16
<210> 815
   <211> 16
   <212> DNA
   <213> Artificial sequence
<220>
   <223> Synthetic oligonucleotide
<400> 815
   aggagctgca gccgga 16
<210> 816
   <211> 16
   <212> DNA
   <213> Artificial sequence
<220>
   <223> Synthetic oligonucleotide
<400> 816
   cggaggagct gcagcc 16
<210> 817
   <211> 16
   <212> DNA
   <213> Artificial sequence
<220>
   <223> Synthetic oligonucleotide
<400> 817
   acccggagga gctgca 16
<210> 818
   <211> 16
   <212> DNA
   <213> Artificial sequence
<220>
   <223> Synthetic oligonucleotide
<400> 818
   gcacccggag gagctg 16
<210> 819
   <211> 16
   <212> DNA
   <213> Artificial sequence
<220>
   <223> Synthetic oligonucleotide
<400> 819
   gggcacccgg aggagc 16
<210> 820
   <211> 16
   <212> DNA
   <213> Artificial sequence
<220>
   <223> Synthetic oligonucleotide
<400> 820
   cagggcaccc ggagga 16
<210> 821
   <211> 16
   <212> DNA
   <213> Artificial sequence
<220>
   <223> Synthetic oligonucleotide
<400> 821
   tgcagggcac ccggag 16
<210> 822
   <211> 16
   <212> DNA
   <213> Artificial sequence
<220>
   <223> Synthetic oligonucleotide
<400> 822
   cctgcagggc acccgg 16
<210> 823
   <211> 16
   <212> DNA
   <213> Artificial sequence
<220>
   <223> Synthetic oligonucleotide
<400> 823
   cgacacctgc agggca 16
<210> 824
   <211> 16
   <212> DNA
   <213> Artificial sequence
<220>
   <223> Synthetic oligonucleotide
<400> 824
   cacgacacct gcaggg 16
<210> 825
   <211> 16
   <212> DNA
   <213> Artificial sequence
<220>
   <223> Synthetic oligonucleotide
<400> 825
   agcacgacac ctgcag 16
<210> 826
   <211> 16
   <212> DNA
   <213> Artificial sequence
<220>
   <223> Synthetic oligonucleotide
<400> 826
   gaagcacgac acctgc 16
<210> 827
   <211> 16
   <212> DNA
   <213> Artificial sequence
<220>
   <223> Synthetic oligonucleotide
<400> 827
   ccaggtagtt ctcatc 16
<210> 828
   <211> 16
   <212> DNA
   <213> Artificial sequence
<220>
   <223> Synthetic oligonucleotide
<400> 828
   caccaggtag ttctca 16
<210> 829
   <211> 16
   <212> DNA
   <213> Artificial sequence
<220>
   <223> Synthetic oligonucleotide
<400> 829
   ctcaccaggt agttct 16
<210> 830
   <211> 16
   <212> DNA
   <213> Artificial sequence
<220>
   <223> Synthetic oligonucleotide
<400> 830
   agctcaccag gtagtt 16
<210> 831
   <211> 16
   <212> DNA
   <213> Artificial sequence
<220>
   <223> Synthetic oligonucleotide
<400> 831
   ggagctcacc aggtag 16
<210> 832
   <211> 16
   <212> DNA
   <213> Artificial sequence
<220>
   <223> Synthetic oligonucleotide
<400> 832
   ccggagctca ccaggt 16
<210> 833
   <211> 16
   <212> DNA
   <213> Artificial sequence
<220>
   <223> Synthetic oligonucleotide
<400> 833
   gcccggagct caccag 16
<210> 834
   <211> 16
   <212> DNA
   <213> Artificial sequence
<220>
   <223> Synthetic oligonucleotide
<400> 834
   tagagcttcc tctccc 16
<210> 835
   <211> 16
   <212> DNA
   <213> Artificial sequence
<220>
   <223> Synthetic oligonucleotide
<400> 835
   cctagagctt cctctc 16
<210> 836
   <211> 16
   <212> DNA
   <213> Artificial sequence
<220>
   <223> Synthetic oligonucleotide
<400> 836
   atcctagagc ttcctc 16
<210> 837
   <211> 16
   <212> DNA
   <213> Artificial sequence
<220>
   <223> Synthetic oligonucleotide
<400> 837
   caatcctaga gcttcc 16
<210> 838
   <211> 16
   <212> DNA
   <213> Artificial sequence
<220>
   <223> Synthetic oligonucleotide
<400> 838
   cccaatccta gagctt 16
<210> 839
   <211> 16
   <212> DNA
   <213> Artificial sequence
<220>
   <223> Synthetic oligonucleotide
<400> 839
   cccccaatcc tagagc 16
<210> 840
   <211> 16
   <212> DNA
   <213> Artificial sequence
<220>
   <223> Synthetic oligonucleotide
<400> 840
   cacccccaat cctaga 16
<210> 841
   <211> 16
   <212> DNA
   <213> Artificial sequence
<220>
   <223> Synthetic oligonucleotide
<400> 841
   agcaccccca atccta 16
<210> 842
   <211> 16
   <212> DNA
   <213> Artificial sequence
<220>
   <223> Synthetic oligonucleotide
<400> 842
   gcagcacccc caatcc 16
<210> 843
   <211> 16
   <212> DNA
   <213> Artificial sequence
<220>
   <223> Synthetic oligonucleotide
<400> 843
   gggcagcacc cccaat 16
<210> 844
   <211> 16
   <212> DNA
   <213> Artificial sequence
<220>
   <223> Synthetic oligonucleotide
<400> 844
   tgacacaccc tcttac 16
<210> 845
   <211> 16
   <212> DNA
   <213> Artificial sequence
<220>
   <223> Synthetic oligonucleotide
<400> 845
   cctgacacac cctctt 16
<210> 846
   <211> 16
   <212> DNA
   <213> Artificial sequence
<220>
   <223> Synthetic oligonucleotide
<400> 846
   cacctgacac accctc 16
<210> 847
   <211> 16
   <212> DNA
   <213> Artificial sequence
<220>
   <223> Synthetic oligonucleotide
<400> 847
   tccacctgac acaccc 16
<210> 848
   <211> 16
   <212> DNA
   <213> Artificial sequence
<220>
   <223> Synthetic oligonucleotide
<400> 848
   catccacctg acacac 16
<210> 849
   <211> 16
   <212> DNA
   <213> Artificial sequence
<220>
   <223> Synthetic oligonucleotide
<400> 849
   ctcatccacc tgacac 16
<210> 850
   <211> 16
   <212> DNA
   <213> Artificial sequence
<220>
   <223> Synthetic oligonucleotide
<400> 850
   ccctcatcca cctgac 16
<210> 851
   <211> 16
   <212> DNA
   <213> Artificial sequence
<220>
   <223> Synthetic oligonucleotide
<400> 851
   gcccctcatc cacctg 16
<210> 852
   <211> 16
   <212> DNA
   <213> Artificial sequence
<220>
   <223> Synthetic oligonucleotide
<400> 852
   aggcccctca tccacc 16
<210> 853
   <211> 16
   <212> DNA
   <213> Artificial sequence
<220>
   <223> Synthetic oligonucleotide
<400> 853
   gaaggcccct catcca 16
<210> 854
   <211> 16
   <212> DNA
   <213> Artificial sequence
<220>
   <223> Synthetic oligonucleotide
<400> 854
   aggtaagaga cccccc 16
<210> 855
   <211> 16
   <212> DNA
   <213> Artificial sequence
<220>
   <223> Synthetic oligonucleotide
<400> 855
   ccaggtaaga gacccc 16
<210> 856
   <211> 16
   <212> DNA
   <213> Artificial sequence
<220>
   <223> Synthetic oligonucleotide
<400> 856
   ttccaggtaa gagacc 16
<210> 857
   <211> 16
   <212> DNA
   <213> Artificial sequence
<220>
   <223> Synthetic oligonucleotide
<400> 857
   ccattccagg taagag 16
<210> 858
   <211> 16
   <212> DNA
   <213> Artificial sequence
<220>
   <223> Synthetic oligonucleotide
<400> 858
   tcccattcca ggtaag 16
<210> 859
   <211> 16
   <212> DNA
   <213> Artificial sequence
<220>
   <223> Synthetic oligonucleotide
<400> 859
   tatcccattc caggta 16
<210> 860
   <211> 16
   <212> DNA
   <213> Artificial sequence
<220>
   <223> Synthetic oligonucleotide
<400> 860
   cctatcccat tccagg 16
<210> 861
   <211> 16
   <212> DNA
   <213> Artificial sequence
<220>
   <223> Synthetic oligonucleotide
<400> 861
   gacctatccc attcca 16
<210> 862
   <211> 16
   <212> DNA
   <213> Artificial sequence
<220>
   <223> Synthetic oligonucleotide
<400> 862
   aagacctatc ccattc 16
<210> 863
   <211> 16
   <212> DNA
   <213> Artificial sequence
<220>
   <223> Synthetic oligonucleotide
<400> 863
   tgaagaccta tcccat 16
<210> 864
   <211> 16
   <212> DNA
   <213> Artificial sequence
<220>
   <223> Synthetic oligonucleotide
<400> 864
   tggccccgtt agaatt 16
<210> 865
   <211> 16
   <212> DNA
   <213> Artificial sequence
<220>
   <223> Synthetic oligonucleotide
<400> 865
   agtggccccg ttagaa 16
<210> 866
   <211> 16
   <212> DNA
   <213> Artificial sequence
<220>
   <223> Synthetic oligonucleotide
<400> 866
   gcagtggccc cgttag 16
<210> 867
   <211> 16
   <212> DNA
   <213> Artificial sequence
<220>
   <223> Synthetic oligonucleotide
<400> 867
   aggcagtggc cccgtt 16
<210> 868
   <211> 16
   <212> DNA
   <213> Artificial sequence
<220>
   <223> Synthetic oligonucleotide
<400> 868
   ctaggcagtg gccccg 16
<210> 869
   <211> 16
   <212> DNA
   <213> Artificial sequence
<220>
   <223> Synthetic oligonucleotide
<400> 869
   ccctaggcag tggccc 16
<210> 870
   <211> 16
   <212> DNA
   <213> Artificial sequence
<220>
   <223> Synthetic oligonucleotide
<400> 870
   aggtcccaga cactcc 16
<210> 871
   <211> 16
   <212> DNA
   <213> Artificial sequence
<220>
   <223> Synthetic oligonucleotide
<400> 871
   ataggtccca gacact 16
<210> 872
   <211> 16
   <212> DNA
   <213> Artificial sequence
<220>
   <223> Synthetic oligonucleotide
<400> 872
   gaataggtcc cagaca 16
<210> 873
   <211> 16
   <212> DNA
   <213> Artificial sequence
<220>
   <223> Synthetic oligonucleotide
<400> 873
   gagaataggt cccaga 16
<210> 874
   <211> 16
   <212> DNA
   <213> Artificial sequence
<220>
   <223> Synthetic oligonucleotide
<400> 874
   cagagaatag gtccca 16